# EUROPEAN PATENT SPECIFICATION

(11) **EP 2 996 718 B1**
(45) Date of publication and mention of the grant of the patent: **15.01.2020**
(21) Application number: 14884476.4
(22) Date of filing: 15.05.2014
(51) Int. Cl.: A61K 39/29, A61P 31/14

(54) **E1E2 HCV VACCINES AND METHODS OF USE**
E1E2-HCV-IMPFSTOFFE UND VERFAHREN ZUR VERWENDUNG
VACCINS CONTRE LE VHC E1E2 ET PROCÉDÉS D'UTILISATION DESDITS VACCINS

(30) Priority: 15.05.2013 US 201361823712 P; 04.10.2013 US 201361887229 P
(43) Date of publication of application: 23.03.2016
(73) Proprietor: The Governors of the University of Alberta, Edmonton, Alberta T5J 4P6 (CA)
(72) Inventor: HOUGHTON, Michael, Edmonton, Alberta T6G 2E1 (CA); HOCKMAN, Darren, Edmonton, Alberta T6G 2E1 (CA); LAW, John, L., Edmonton, Alberta T6G 2E1 (CA); LOGAN, Michael, Edmonton, Alberta T6G 2E1 (CA); TYRRELL, Lorne D., Edmonton, Alberta T5R 3M7 (CA)
(74) Representative: Mewburn Ellis LLP
(86) International application number: PCT/IB2014/001972
(87) International publication number: WO 2015/132619

(56) References cited:
- WO-A1-2009/061739
- WO-A2-2007/041432
- WO-A2-2014/060851
- GROLLO LARA ET AL: "Exploiting information inherent in binding sites of virus-specific antibodies: design of an HCV vaccine candidate cross-reactive with multiple genotypes", ANTIVIRAL THERAPY- AN OFFICIAL PUBLICATION OF THE INTERNATIONAL SOCIETY FOR ANTIVIRAL RESEARCH, MTM PUBLICATIONS, LONDON, GB, vol. 11, no. 8, 1 January 2006 (2006-01-01), pages 1005-1014, XP009192451, ISSN: 1359-6535
- MARLOES A NAARDING ET AL: "Hepatitis C virus soluble E2 in combination with QuilA and CpG ODN induces neutralizing antibodies in mice", VACCINE, ELSEVIER, AMSTERDAM, NL, vol. 29, no. 16, 5 February 2011 (2011-02-05), pages 2910-2917, XP028160596, ISSN: 0264-410X, DOI: 10.1016/J.VACCINE.2011.02.009 [retrieved on 2011-02-10]
- JOHN LOK MAN LAW ET AL: "A Hepatitis C Virus (HCV) Vaccine Comprising Envelope Glycoproteins gpE1/gpE2 Derived from a Single Isolate Elicits Broad Cross-Genotype Neutralizing Antibodies in Humans", PLOS ONE, vol. 8, no. 3, 19 March 2013 (2013-03-19), page e59776, XP055293182, DOI: 10.1371/journal.pone.0059776
- TORRESI JOSEPH ET AL: "A self-adjuvanting multiepitope immunogen that induces a broadly cross-reactive antibody to hepatitis C virus", HEPATOLOGY, WILEY, vol. 45, no. 4, 1 April 2007 (2007-04-01), pages 911-920, XP009090063, ISSN: 0270-9139, DOI: 10.1002/HEP.21538
- F. HELLE ET AL: "The Neutralizing Activity of Anti-Hepatitis C Virus Antibodies Is Modulated by Specific Glycans on the E2 Envelope Protein", JOURNAL OF VIROLOGY., vol. 81, no. 15, 1 August 2007 (2007-08-01), pages 8101-8111, XP055365953, US ISSN: 0022-538X, DOI: 10.1128/JVI.00127-07
- MEUNIER J C ET AL: "Vaccine-induced cross-genotype reactive neutralizing antibodies against hepatitis C virus", THE JOURNAL OF INFECTIOUS DISEASES UNITED STATES 15 DEC 2010, INFECTIOUS DISEASES SOCIETY OF AMERICA, US, vol. 204, no. 8, 15 October 2011 (2011-10-15), pages 1186-1190, XP002690713, ISSN: 1537-6613, DOI: 10.1093/INFDIS/JIR511
- LAW, JL.: 'A hepatitis C virus (HCV) vaccine comprising envelope glycoproteins gpEl/gpE2 derived from a single isolate elicits broad cross-genotype neutralizing antibodies in humans.' PLOS ONE. vol. 8, no. ISSUE, 19 March 2013, page E59776, XP055293182
- TORRESI, J.: 'Progress in the development of preventive and therapeutic vaccines for hepatitis C virus.' J HEPATOL. vol. 54, no. ISSUE, June 2011, pages 1273 - 1285, XP028210471
- SIMMONDS, P.: 'The Origin of Hepatitis C Virus.' HEPATITIS C VIRUS: FROM MOLECULAR VIROLOGY TO ANTIVIRAL THERAPY CURRENT TOPICS IN MICROBIOLOGY AND IMMUNOLOGY vol. 369, 06 March 2013, pages 1 - 15, XP055293180
- JENS BUKH ET AL: "At least 12 genotypes of hepatitis C virus predicted by sequence analysis of the putative El gene of isolates collected worldwide", PNAS, vol. 90, no. 09, 1 September 1993 (1993-09-01), pages 8234-8238, XP055548369,

## Description

### INTRODUCTION

Hepatitis C virus (HCV) is a blood-borne pathogen that is estimated to infect 150-200 million people worldwide. Infection by HCV may be non-symptomatic, and can be cleared by patients, sometimes without the need for medical intervention. However, the majority of patients develop a chronic HCV infection, which may lead to liver inflammation, scarring and even to liver failure or liver cancer. In the United States alone, over 3 million people have a chronic infection.

The HCV virion contains a positive-sense single stranded RNA genome of about 9.5 kb. The genome encodes a single polyprotein of 3,010 to 3,030 amino acids. The structural proteins comprise a core protein forming the viral nucleocapsid and two envelope glycoproteins, E1 and E2.

There is a need in the art for immunogenic compositions that induce an immune response to HCV in an individual so as to provide induction of an immune response effective against multiple HCV genotypes found around the world, e.g. genotypes 1 and 3. WO 2007/041432 describes immunogenic compositions comprising HCV envelope polypeptides that cross-neutralize the infectivity of at least two HCV genotypes selected from HCV-1, HCV-2, HCV-3, HCV-4, HCV-5 and HCV-6.

### SUMMARY

The invention is defined by the appended claims. The present disclosure provides immunogenic compositions comprising HCV E1, E2 or E1/E2 polypeptides from two or more different HCV genotypes. The present disclosure provides immunogenic compositions comprising HCV E2 or E1/E2 polypeptides from two or more different HCV genotypes. The immunogenic compositions are useful in carrying out methods of inducing an immune response to HCV. The present disclosure further provides methods of stimulating an immune response to HCV in an individual.

In some cases, the present disclosure provides an immunogenic composition comprising: a) an hepatitis C virus (HCV) E1 polypeptide, E2 polypeptide or E1E2 polypeptide from a first HCV genotype; b) an HCV E1 polypeptide, E2 polypeptide, or E1E2 polypeptide from a second HCV genotype; and c) a pharmaceutically acceptable excipient, with the proviso that the composition comprises at least one E1 polypeptide and at least one E2 polypeptide. In some of these cases, the first HCV genotype is genotype 1; and the second HCV genotype is genotype 2. In other cases, the first HCV genotype is genotype 1; and the second HCV genotype is genotype 3. The following clauses i) through xiv) are non-limiting examples, where a subject immunogenic composition comprises:
i) an E1/E2 heterodimeric polypeptide complex of HCV genotype 1; and E1/E2 polypeptide of HCV genotype 3;
ii) an E1/E2 heterodimeric polypeptide complex of HCV genotype 1; and an E2 polypeptide of HCV genotype 3;
iii) an E2 polypeptide of HCV genotype 1; and an E1/E2 heterodimeric polypeptide complex of HCV genotype 3;
iv) an E1/E2 heterodimeric polypeptide complex of HCV genotype 1; and an E1/E2 heterodimeric polypeptide complex of HCV genotype 2;
v) an E1/E2 heterodimeric polypeptide complex of HCV genotype 1; and an E2 polypeptide of HCV genotype 2;
vi) an E2 polypeptide of HCV genotype 1; and an E1/E2 heterodimeric polypeptide complex of HCV genotype 2;
vii) an E1 polypeptide of HCV genotype 1; and an E2 polypeptide of HCV genotype 3;
viii) an E1 polypeptide of HCV genotype 1; and an E1/E2 heterodimeric polypeptide complex of HCV genotype 3;
ix) an E2 polypeptide of HCV genotype 1; and an E1 polypeptide of HCV genotype 3;
x) an E2 polypeptide of HCV genotype 1; and an E1/E2 heterodimeric polypeptide complex of HCV genotype 3;
xi) an E1 polypeptide of HCV genotype 1; and an E2 polypeptide of HCV genotype 2;
xii) an E1 polypeptide of HCV genotype 1; and an E1/E2 heterodimeric polypeptide complex of HCV genotype 2;
xiii) an E2 polypeptide of HCV genotype 1; and an E1 polypeptide of HCV genotype 2; or
xiv) an E2 polypeptide of HCV genotype 1; and an E1/E2 heterodimeric polypeptide complex of HCV genotype 2.

In any of the above-described cases, the composition can further include an HCV E1, E2, or E1/E2 polypeptide of a third HCV genotype.

In any of the above-described cases, the HCV E2 polypeptide from the first HCV genotype can be wild-type, and the HCV E2 polypeptide from the second HCV genotype can be wild-type. In any of the above-described cases, the HCV E2 polypeptide from the first HCV genotype can be wild-type, and the HCV E2 polypeptide from the second HCV genotype can comprise an amino acid substitution of asparagine in an E2N1 site and/or an E2N6 site. In any of the above-described cases, the HCV E2 polypeptide from the first HCV genotype can comprise an amino acid substitution of asparagine in an E2N1 site and/or an E2N6 site, and the HCV E2 polypeptide from the second HCV genotype can be wild-type.

In a subject immunogenic composition, the pharmaceutically acceptable excipient can comprise an adjuvant; in some cases, the adjuvant is MF59, alum, poly(DL-lactide co-glycolide), or a CpG oligonucleotide.

In other cases, the present disclosure provides an immmunogenic composition comprising: a) a hepatitis C virus (HCV) E1 polypeptide, E2 polypeptide, or E1/E2 polypeptide from a first HCV genotype; b) an HCV E1 polypeptide, E2 polypeptide, or E1/E2 polypeptide from a second HCV genotype; c) an HCV E1 polypeptide, E2 polypeptide, or E1/E2 polypeptide from a third HCV genotype; and d) a pharmaceutically acceptable excipient, with the proviso that the composition comprises at least one E1 polypeptide and at least one E2 polypeptide. In some cases, the first HCV genotype is genotype 1, the second HCV genotype is genotype 2, and the third HCV genotype is genotype 3. The following clauses i) through xvii) are non-limiting examples, where a subject immunogenic composition comprises:
i) an E1/E2 heterodimeric polypeptide complex of HCV genotype 1; an E1/E2 heterodimeric polypeptide complex of HCV genotype 2; and an E1/E2 heterodimeric polypeptide complex of HCV genotype 3;
ii) an E1/E2 heterodimeric polypeptide complex of HCV genotype 1; an E1/E2 heterodimeric polypeptide complex of HCV genotype 2; and an E2 polypeptide of HCV genotype 3;
iii) an E1/E2 heterodimeric polypeptide complex of HCV genotype 1; an E2 polypeptide of HCV genotype 2; and an E1/E2 heterodimeric polypeptide complex of HCV genotype 3;
iv) an E2 polypeptide of HCV genotype 1; an E1/E2 heterodimeric polypeptide complex of HCV genotype 2; and an E1/E2 heterodimeric polypeptide complex of HCV genotype 3;
v) an E1/E2 heterodimeric polypeptide complex of HCV genotype 1; an E2 polypeptide of HCV genotype 2; and an E2 polypeptide of HCV genotype 3;
vi) an E2 polypeptide of HCV genotype 1; an E1/E2 heterodimeric polypeptide complex of HCV genotype 2; and an E2 polypeptide of HCV genotype 3;
vii) an E2 polypeptide of HCV genotype 1; an E2 polypeptide of HCV genotype 2; and an E1/E2 heterodimeric polypeptide complex of HCV genotype 3;
viii) an E1 polypeptide of HCV genotype 1; an E1/E2 heterodimeric polypeptide complex of HCV genotype 2; and an E1/E2 heterodimeric polypeptide complex of HCV genotype 3;
ix) an E1/E2 heterodimeric polypeptide complex of HCV genotype 1; an E1 polypeptide of HCV genotype 2; and an E1/E2 heterodimeric polypeptide complex of HCV genotype 3;
x) an E1/E2 heterodimeric polypeptide complex of HCV genotype 1; an E1/E2 heterodimeric polypeptide complex of HCV genotype 2; and an E1 polypeptide of HCV genotype 3;
xi) an E1 polypeptide of HCV genotype 1; an E2 polypeptide of HCV genotype 2; and an E1/E2 heterodimeric polypeptide complex of HCV genotype 3;
xii) an E2 polypeptide of HCV genotype 1; an E1 polypeptide of HCV genotype 2; and an E1/E2 heterodimeric polypeptide complex of HCV genotype 3;
xiii) an E1/E2 heterodimeric polypeptide complex of HCV genotype 1; an E2 polypeptide of HCV genotype 2; and an E1 polypeptide of HCV genotype 3;
xiv) an E1 polypeptide of HCV genotype 1; an E2 polypeptide of HCV genotype 2; and an E2 polypeptide of HCV genotype 3;
xv) an E2 polypeptide of HCV genotype 1; an E1 polypeptide of HCV genotype 2; and an E2 polypeptide of HCV genotype 3;
xvi) an E2 polypeptide of HCV genotype 1; an E2 polypeptide of HCV genotype 2; and an E1 polypeptide of HCV genotype 3; or
xvii) an E1 polypeptide of HCV genotype 1; an E1/E2 heterodimeric polypeptide complex of HCV genotype 2; and an E2 polypeptide of HCV genotype 3. The combination of clauses (i)-(iii), (v), (ix), (x) and (xiii) are embodiments of the invention.

In any of the above-mentioned embodiments, the HCV E2 polypeptide from the first HCV genotype can be wild-type, the HCV E2 polypeptide from the second HCV genotype can be wild-type, and the HCV E2 polypeptide from the third HCV genotype can be wild-type. In other cases, the HCV E2 polypeptide from the first HCV genotype is wild-type, the HCV E2 polypeptide from the second HCV genotype is wild-type, and the HCV E2 polypeptide from the third HCV genotype comprises an amino acid substitution of asparagine in an E2N1 site and/or an E2N6 site. In other cases, the HCV E2 polypeptide from the first HCV genotype is wild-type, the HCV E2 polypeptide from the second HCV genotype comprises an amino acid substitution of asparagine in an E2N1 site and/or an E2N6 site, and the HCV E2 polypeptide from the third HCV genotype is wild-type. In other cases, the HCV E2 polypeptide from the first HCV genotype comprises an amino acid substitution of asparagine in an E2N1 site and/or an E2N6 site, the HCV E2 polypeptide from the second HCV genotype is wild-type, and the HCV E2 polypeptide from the third HCV genotype is wild-type.

In any of the above-mentioned embodiments, the pharmaceutically acceptable excipient can include an adjuvant. In any of the above-mentioned embodiments, the adjuvant can be MF59, alum, poly(DL-lactide co-glycolide), or a CpG oligonucleotide.

The present disclosure provides a method of inducing an immune response in an individual, the method comprising administering to the individual an effective amount of an immunogenic composition according to any of the embodiments described herein.

### BRIEF DESCRIPTION OF THE DRAWINGS

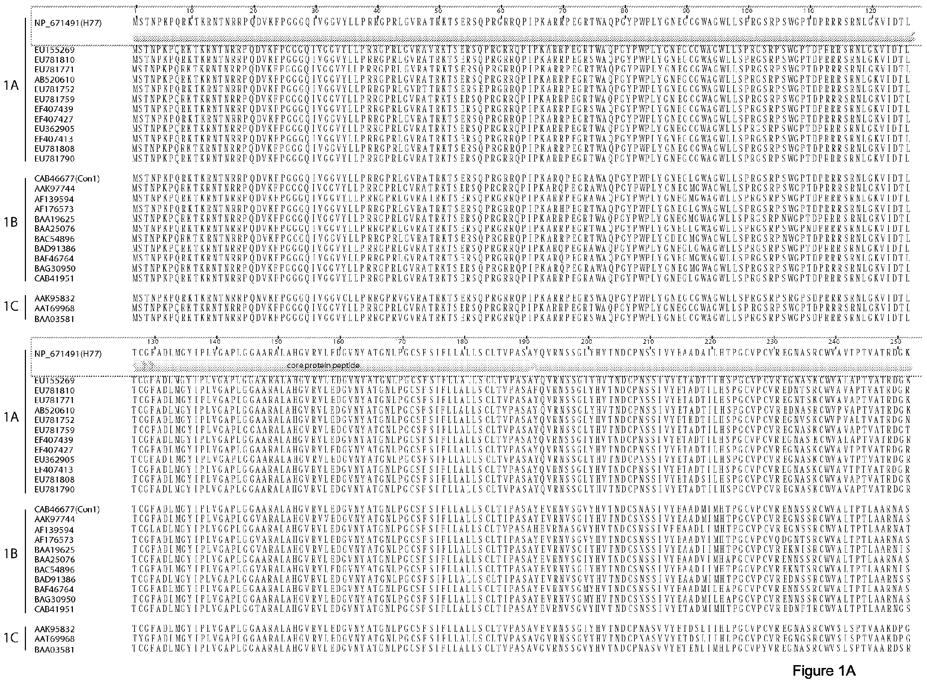

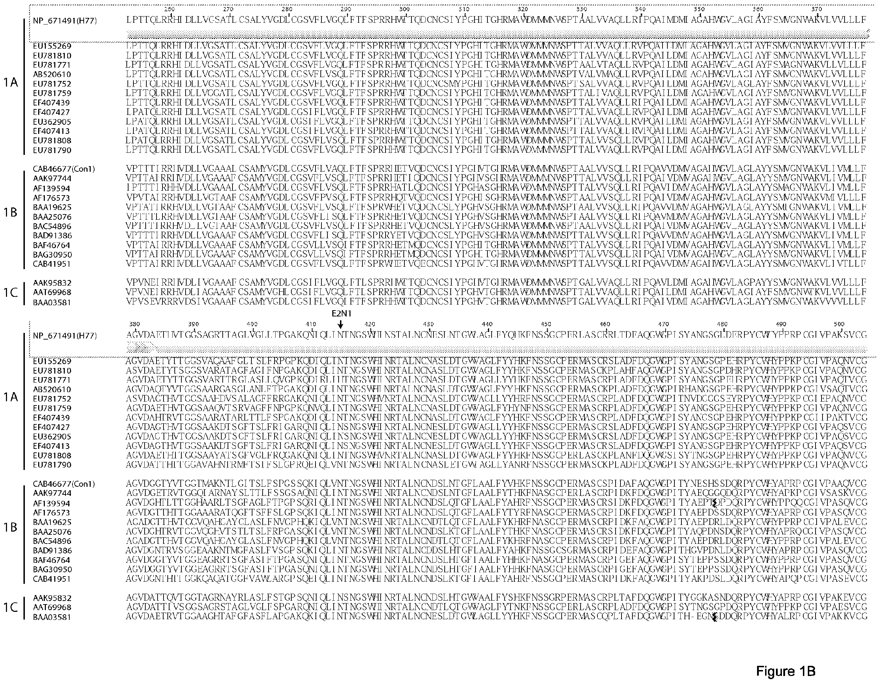

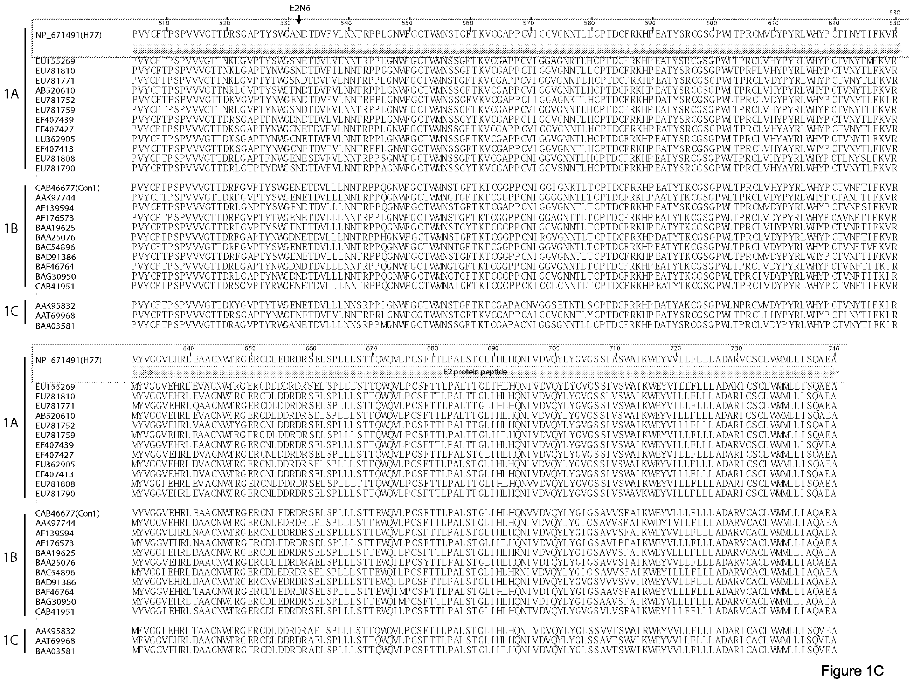

Figures 1A-C provide a protein alignment of examples of the core-E1-E2 coding regions of a HCV genotype 1 virus, specifically representative HCV 1A, 1B and 1C genotypes. Genbank database sequences for the coding region core-E1-E2 were aligned using Geneious software v5.6.4. Glycosylation sites N417 (E2N1) and N532 (E2N6) are highlighted (arrows). Numbering of amino acids is according to strain NP_671941 (H77). To maintain amino acid numbering, strains AF139594 and BAA03581 have an amino acid insertion between amino acid 478 and 479 (lysine (K) and alanine (A), respectively) that is not shown (hashed line). From top to bottom SEQ ID NOs:1-27.

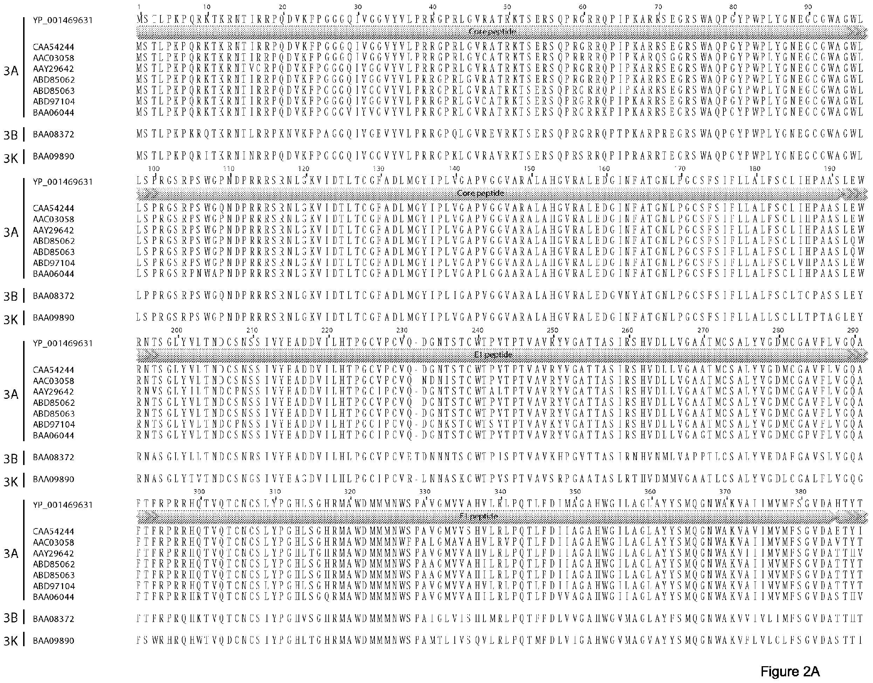

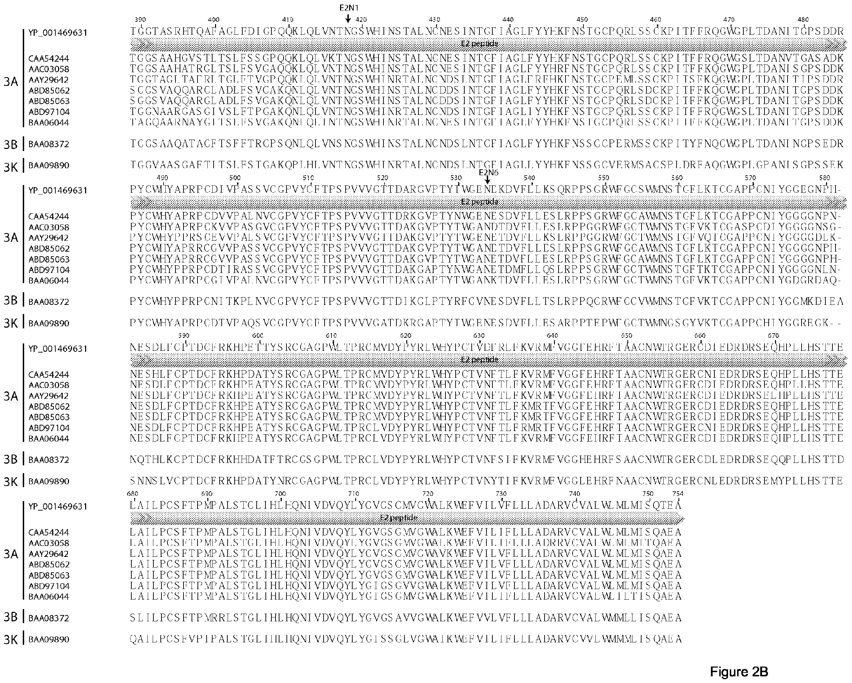

Figures 2A and 2B provide a protein alignment of the core-E1-E2 coding region for representative HCV 3A, 3B and 3K genotypes. Genbank database sequences for the coding region core-E1-E2 were aligned using Geneious software v5.6.4. Glycosylation sites N417 (E2N1) and N532 (E2N6) are highlighted (arrows). From top to bottom SEQ ID NOs:28-37.

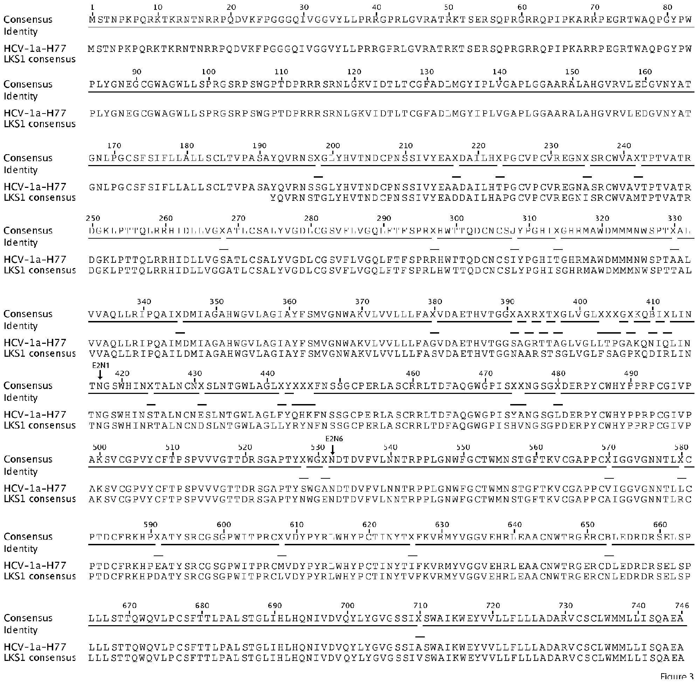

Figure 3 provides a consensus E1E2 protein sequence for Alberta HCV genotype 1A isolate (LKS1). A consensus sequence for the E1E2 coding region was obtained from a patient with HCV genotype 1A (LKS1) (Li Ka Shing Institute of Virology, University of Alberta, Edmonton, AB, Canada). LKS1 E1E2 is shown in comparison to H77 genotype 1A (core-E1-E2). LKS1 has 515/555 (92.8%) amino acid identities with H77 in the E1E2 coding region. Glycosylation sites N417 (E2N1) and N532 (E2N6) are highlighted (arrows). From top to bottom SEQ ID NO:38, SEQ ID NO:1, and SEQ ID NO:39.

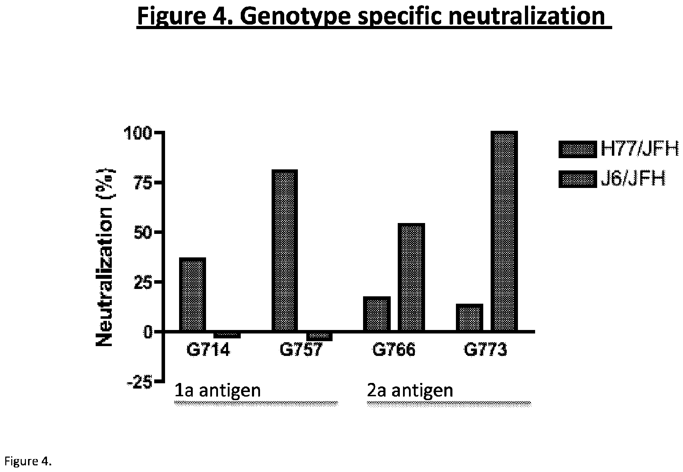

Figure 4 depicts genotype-specific neutralization by sera of goats immunized with either HCV1 (genotype la)-derived E1E2, or with J6 (genotype 2)-derived E2.

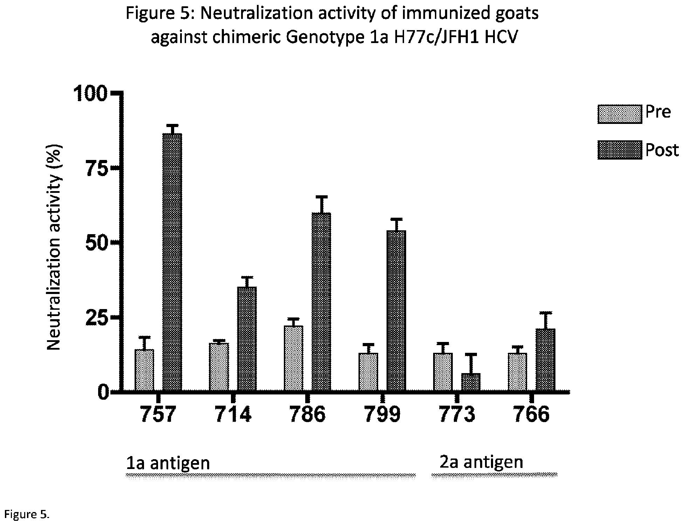

Figure 5 depicts neutralization activity of sera from goats immunized against Genotype 1a chimeric H77c/JFH1 HCV.

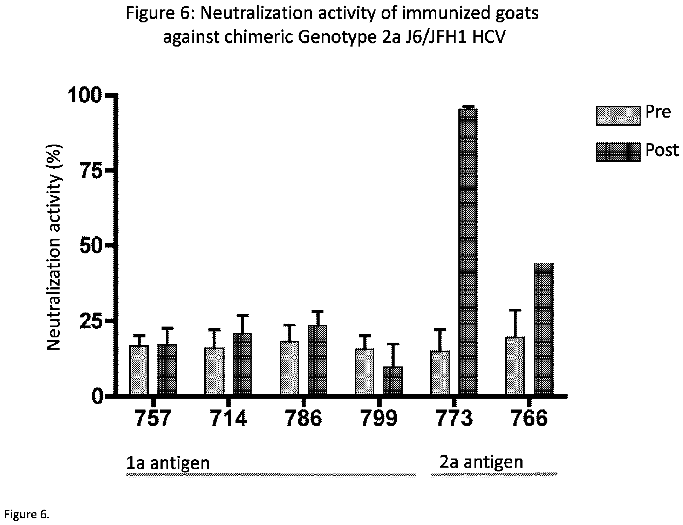

Figure 6 depicts neutralization activity of sera from goats immunized against Genotype 2a chimeric J6/JFH1 HCV.

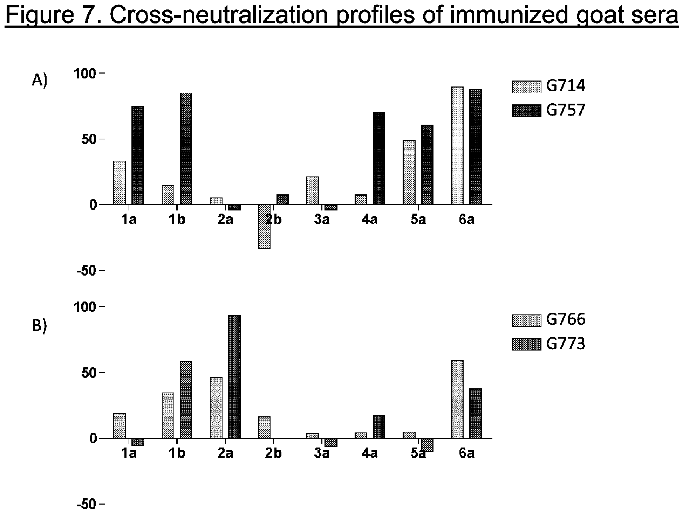

Figures 7A and 7B depict genotype-specific neutralization and cross-neutralization of HCV by sera of goats immunized with HCV antigens. A) Goats 757 and 714 were immunized with recombinant E1E2 derived from genotype 1a HCV1 sequence. B) Goats 766 and 773 were immunized with E2 derived from genotype 2a J6 sequence. Neutralization activity against chimeric HCVcc of genotype 1a-6a was measured. Results were normalized using neutralization activity of sera prior to immunization as control.

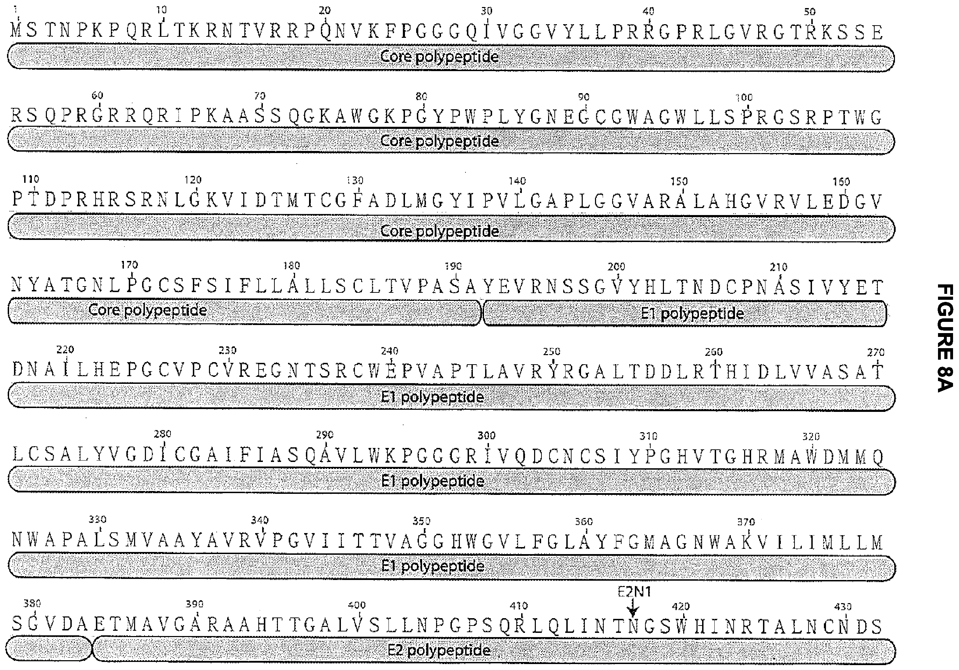

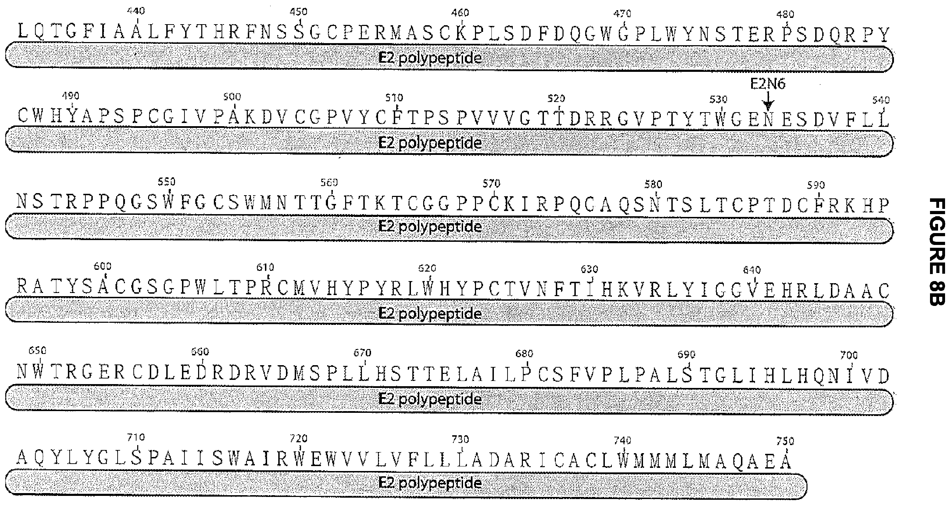

Figures 8A and 8B provide an amino acid sequence of the core-E1-E2 coding region for HCV genotype 7a. Amino acid sequence for the coding region core-E1-E2 of genotype 7a (isolate QC69; Genbank: ABN05226.1) is shown according to the numbering scheme of the reference strain, NP_671941 (H77). Glycosylation sites N417 (E2N1) and N533 (E2N6) are highlighted (arrows) (SEQ ID NO:40).

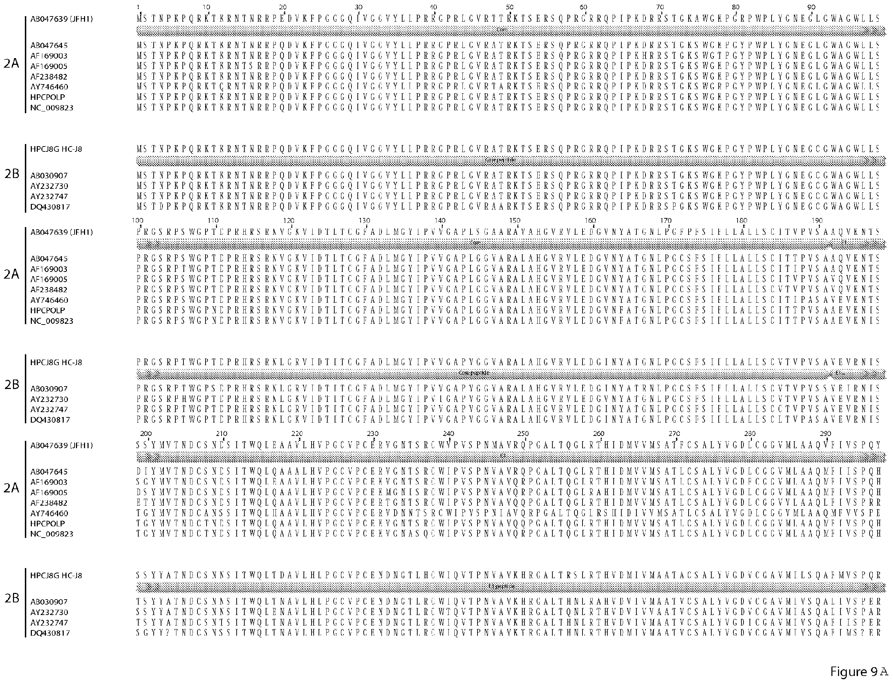

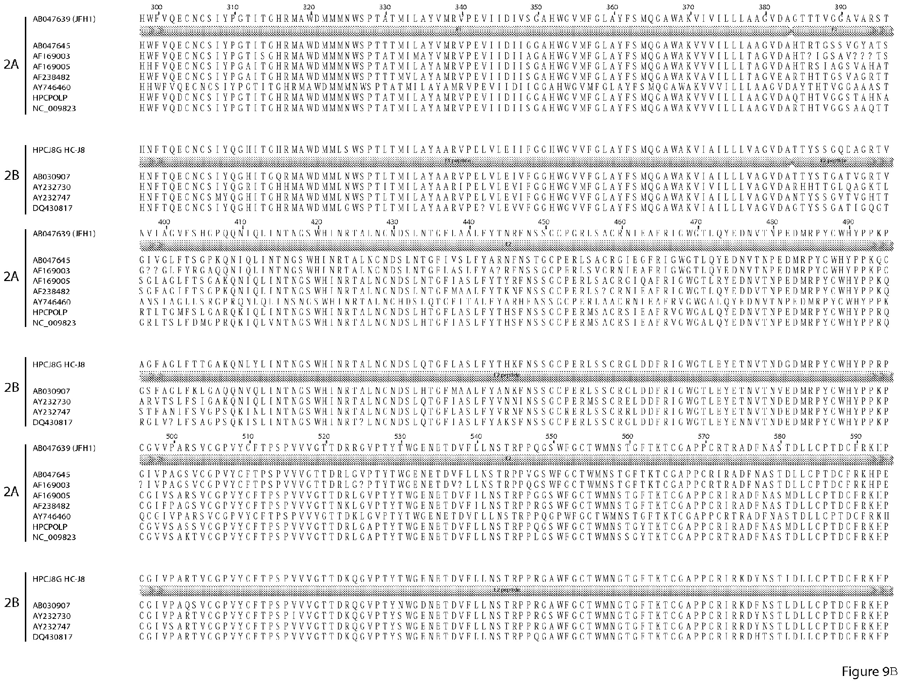

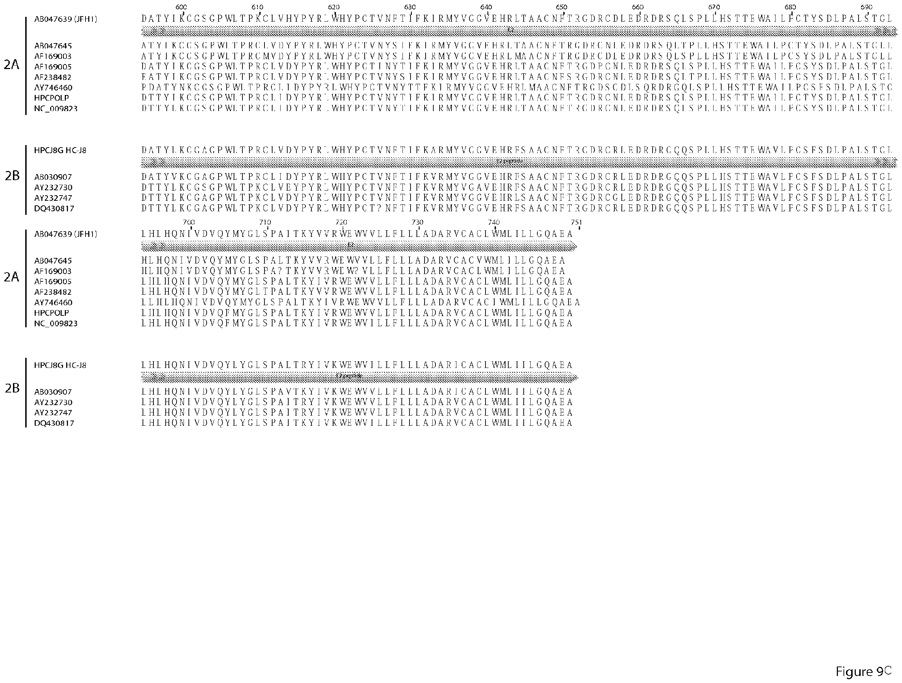

Figures 9A-C provide an alignment of amino acid sequences of the core-E1-E2 coding region of representative HCV 2A and HCV2B subtypes. Genbank database sequences for the coding region core-E1-E2 were aligned using Geneious software v5.6.4. The amino acid numbering depicted is in accordance to the common HCV strains: ABO47639 (JFH1) and HPCJ8G-J8 (J8) for HCV2A and HCV2B, respectively. From top to bottom SEQ ID NOs:41-53.

### DEFINITIONS

The term "hepatitis C virus" ("HCV"), as used herein, refers to any one of a number of different genotypes and isolates of hepatitis C virus. Thus, "HCV" encompasses any of a number of genotypes, subtypes, or quasispecies, of HCV, including, e.g., genotype 1, 2, 3, 4, 6, 7, etc. and subtypes (e.g., 1a, 1b, 2a, 2b, 3a, 4a, 4c, etc.), and quasispecies. Representative HCV genotypes and isolates include: the "Chiron" isolate HCV-1, H77, J6, Con1, isolate 1, BK, EC1, EC10, HC-J2, HC-J5; HC-J6, HC-J7, HC-J8, HC-JT, HCT18, HCT27, HCV-476, HCV-KF, "Hunan", "Japanese", "Taiwan", TH, type 1, type 1a, H77 type 1b, type 1c, type 1d, type 1e, type 1f, type 10, type 2, type 2a, type 2b, type 2c, type 2d, type 2f, type 3, type 3a, type 3b, type 3g, type 4, type 4a, type 4c, type 4d, type 4f, type 4h, type 4k, type 5, type 5a, type 6 and type 6a.

The terms "individual," "host," "subject," and "patient" are used interchangeably herein, and refer to a mammal, including, but not limited to, non-human primates (e.g., simians), and humans.

As used herein, the term "isolated," in reference to a polypeptide, refers to a polypeptide that is in an environment different from that in which the polypeptide naturally occurs. An isolated polypeptide can be purified. By "purified" is meant a compound of interest (e.g., a polypeptide) has been separated from components that accompany it in nature. "Purified" can also be used to refer to a polypeptide separated from components that can accompany it during production of the polypeptide (e.g., during synthesis in vitro, etc.). In some embodiments, a polypeptide (or a mixture of polypeptides) is substantially pure when the polypeptide (or mixture of polypeptides) is at least 60% or at least 75% by weight free from organic molecules with which it is naturally associated or with which it is associated during production. In some embodiments, the polypeptide is from 30% to 60% pure. In some embodiments, the polypeptide (or mixture of polypeptides) is at least 60%, at least 75%, at least 80%, at least 85%, at least 90%, at least 95%, or at least 99%, by weight, pure. For example, in some embodiments, an E1 or an E2 polypeptide (or a mixture of E1 and E2 polypeptides) is substantially pure when the E1 or E2 polypeptide (or mixture of E1 and E2 polypeptides) is at least 60% or at least 75% by weight free from organic molecules with which the polypeptide(s) is naturally associated or with which it is associated during production. In some embodiments, the E1 or E2 polypeptide (or mixture of E1 and E2 polypeptides) is at least 60%, at least 75%, at least 80%, at least 85%, at least 90%, at least 95%, or at least 99%, by weight, pure. In some embodiments, where a composition comprises an E2 polypeptide, the E2 polypeptide is at least 60%, at least 75%, at least 80%, at least 85%, at least 90%, at least 95%, or at least 99%, by weight, pure. In some embodiments, where a composition comprises an E1/E2 heterodimeric complex polypeptide, the E1/E2 heterodimeric complex polypeptide is at least 60%, at least 75%, at least 80%, at least 85%, at least 90%, at least 95%, or at least 99%, by weight, pure. In some embodiments, where a composition comprises an E2 polypeptide and an E1/E2 heterodimeric complex polypeptide, the E2 polypeptide and the E1/E2 heterodimeric complex polypeptide are at least 60%, at least 75%, at least 80%, at least 85%, at least 90%, at least 95%, or at least 99%, by weight, pure.

Before the present invention is further described, it is to be understood that this invention is not limited to particular embodiments described, as such may, of course, vary. It is also to be understood that the terminology used herein is for the purpose of describing particular embodiments only, and is not intended to be limiting, since the scope of the present invention will be limited only by the appended claims.

Where a range of values is provided, it is understood that each intervening value, to the tenth of the unit of the lower limit unless the context clearly dictates otherwise, between the upper and lower limit of that range and any other stated or intervening value in that stated range, is encompassed within the invention. The upper and lower limits of these smaller ranges may independently be included in the smaller ranges, and are also encompassed within the invention, subject to any specifically excluded limit in the stated range. Where the stated range includes one or both of the limits, ranges excluding either or both of those included limits are also included in the invention.

Unless defined otherwise, all technical and scientific terms used herein have the same meaning as commonly understood by one of ordinary skill in the art to which this invention belongs. Although any methods and materials similar or equivalent to those described herein can also be used in the practice or testing of the present invention, the preferred methods and materials are now described. All publications mentioned herein are to disclose and describe the methods and/or materials in connection with which the publications are cited.

It must be noted that as used herein and in the appended claims, the singular forms "a," "an," and "the" include plural referents unless the context clearly dictates otherwise. Thus, for example, reference to "an HCV E1 polypeptide" includes a plurality of such polypeptide and reference to "the HCV genotype" includes reference to one or more genotypes and equivalents thereof known to those skilled in the art, and so forth. It is further noted that the claims may be drafted to exclude any optional element. As such, this statement is intended to serve as antecedent basis for use of such exclusive terminology as "solely," "only" and the like in connection with the recitation of claim elements, or use of a "negative" limitation.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable sub-combination. All combinations of the embodiments pertaining to the invention are specifically embraced by the present invention and are disclosed herein just as if each and every combination was individually and explicitly disclosed. In addition, all sub-combinations of the various embodiments and elements thereof are also specifically embraced by the present invention and are disclosed herein just as if each and every such sub-combination was individually and explicitly disclosed herein.

The publications discussed herein are provided solely for their disclosure prior to the filing date of the present application. Nothing herein is to be construed as an admission that the present invention is not entitled to antedate such publication by virtue of prior invention. Further, the dates of publication provided may be different from the actual publication dates which may need to be independently confirmed.

### DETAILED DESCRIPTION

The present disclosure provides immunogenic compositions comprising HCV E1, E2, or E1/E2 polypeptides from two or more different HCV genotypes. The present disclosure provides immunogenic compositions comprising HCV E2 or E1/E2 polypeptides from two or more different HCV genotypes. The immunogenic compositions are useful in carrying out methods of inducing an immune response to HCV. The present disclosure further provides methods of stimulating an immune response to HCV in an individual.

### IMMUNOGENIC COMPOSITIONS

The present disclosure provides immunogenic compositions comprising HCV structural polypeptides, e.g., E1, E2, and/or E1/E2, where the composition comprises HCV structural polypeptides from at least two different HCV genotypes. The present disclosure provides immunogenic compositions comprising HCV E2 or E1/E2 polypeptides from two or more different HCV genotypes.

A subject immunogenic composition includes HCV polypeptides E1, E2, and/or E1/E2. E1 and E2 polypeptides present in a subject immunogenic composition may be present in the composition as a covalently or non-covalently linked heterodimer. The E1 and E2 polypeptides can be present in the composition as a single polypeptide chain, or can be present as two separate polypeptide chains (which may or may not be covalently linked via a disulfide bond).

The E1 and E2 polypeptides are isolated, and can be purified. In some cases, a subject immunogenic composition comprises E1 and E2 polypeptides, where the polypeptides (or mixtures of E1 and E2 polypeptides) are from 30% to 60% pure, or from 60% to about 80% pure. In some cases, a subject immunogenic composition comprises E1 and E2 polypeptides, where the polypeptides (or mixtures of E1 and E2 polypeptides) are at least about 80% pure, at least about 85% pure, at least about 90% pure, at least about 95% pure, at least about 98% pure, at least about 99% pure, or greater than 99% pure. In some embodiments, a subject immunogenic composition does not include any other polypeptides (e.g. no other HCV polypeptides) other than HCV E1 and HCV E2 polypeptides.

The terms "E1 polypeptide" and "E2 polypeptide" encompass proteins which include additional modifications to the native sequence, such as deletions (e.g., C-terminal truncations, e.g., C-terminally truncated E2 lacking of the naturally-occurring C terminus as described herein), additions and substitutions (e.g., conservative amino acid substitutions). These modifications may be deliberate, as through site-directed mutagenesis, or may occur as a result of mutational events during naturally-occurring or recombinant expression of E1 or E2 (e.g., *in vivo* in the course of HCV infection, during HCV virus production in cell culture, during recombinant expression or E1 or E2 in in vitro cell culture.

### E1

An HCV E1 polypeptide suitable for use in an immunogenic composition of the present disclosure can have a length of from about 150 amino acids (aa) to about 175 aa, from about 175 aa to about 195 aa, from about 131 aa to about 175 aa, or from about 175 aa to about 193 aa.

In Figures 1A-C, the amino acid sequence of E1 is amino acid 192 to amino acid 383. In Figures 2A-2B, the amino acid sequence of E1 is amino acid 192 to amino acid 384. In Figure 3, the amino acid sequence of E1 is amino acid 192 to amino acid 385. Amino acids at around 170 through approximately 191 serve as a signal sequence for E1. As used herein, "E1 polypeptide" includes a precursor E1 protein, including the signal sequence; includes a mature E1 polypeptide which lacks this sequence; and includes an E1 polypeptide with a heterologous signal sequence. An E1 polypeptide can include a C-terminal membrane anchor sequence which occurs at approximately amino acid positions 360-383 (see, e.g., WO 96/04301). In some cases, a suitable E1 polypeptide lacks a C-terminal portion that includes a transmembrane region. For example, in some cases, a suitable E1 polypeptide lacks the C-terminal portion from amino acid 330 to amino acid 384, or from amino acid 360 to amino acid 384. E1 polypeptides can be an E1 polypeptide of any genotype, subtype or isolate of HCV. E1 polypeptides of genotype 1 and E1 polypeptides of genotype 3 are of particular interest.

An E1 polypeptide can comprise an amino acid sequence having at least about 70%, at least about 75%, at least about 80%, at least about 85%, at least about 90%, at least about 95%, at least about 98%, at least about 99%, or 100%, amino acid sequence identity to an amino acid sequence of an E1 polypeptide depicted in Figures 1A-C, Figures 2A-2B, or Figure 3.

An E1 polypeptide can comprise an amino acid sequence having at least about 70%, at least about 75%, at least about 80%, at least about 85%, at least about 90%, at least about 95%, at least about 98%, at least about 99%, or 100%, amino acid sequence identity to the consensus E1 polypeptide amino acid sequence depicted in Figure 3.

An E1 polypeptide can comprise an amino acid sequence having at least about 70%, at least about 75%, at least about 80%, at least about 85%, at least about 90%, at least about 95%, at least about 98%, at least about 99%, or 100%, amino acid sequence identity to an amino acid sequence of an E1 polypeptide depicted in Figures 8A and 8B. For example, an E1 polypeptide of genotype 7A can comprise an amino acid sequence having at least about 70%, at least about 75%, at least about 80%, at least about 85%, at least about 90%, at least about 95%, at least about 98%, at least about 99%, or 100%, amino acid sequence identity to amino acids 192-383 of the amino acid sequence depicted in Figures 8A and 8B.

An E1 polypeptide can comprise an amino acid sequence having at least about 70%, at least about 75%, at least about 80%, at least about 85%, at least about 90%, at least about 95%, at least about 98%, at least about 99%, or 100%, amino acid sequence identity to an amino acid sequence of an E1 polypeptide depicted in Figures 9A-C. For example, an E1 polypeptide of genotype 2A can comprise an amino acid sequence having at least about 70%, at least about 75%, at least about 80%, at least about 85%, at least about 90%, at least about 95%, at least about 98%, at least about 99%, or 100%, amino acid sequence identity to amino acids 192-383 of an amino acid sequence identified as 2A and depicted in Figures 9A-C. For example, an E1 polypeptide of genotype 2B can comprise an amino acid sequence having at least about 70%, at least about 75%, at least about 80%, at least about 85%, at least about 90%, at least about 95%, at least about 98%, at least about 99%, or 100%, amino acid sequence identity to amino acids 384-751 of an amino acid sequence identified as 2B and depicted in Figures 9A-C.

### E2

An E2 polypeptide suitable for use in a subject immunogenic composition can have a length of from about 200 amino acids (aa) to about 250 aa, from about 250 aa to about 275 aa, from about 275 aa to about 300 aa, from about 300 aa to about 325 aa, from about 325 aa to about 350 aa, or from about 350 aa to about 365 aa.

In Figures 1A-C, the amino acid sequence of E2 is amino acid 384 to amino acid 746. In Figures 2A-2B, the amino acid sequence of E2 is amino acid 385 to amino acid 754. In Figure 3, the amino acid sequence of E2 is amino acid 386 to amino acid 746. As used herein, an "E2 polypeptide" includes a precursor E2 protein, including the signal sequence; includes a mature E2 polypeptide which lacks this sequence; and includes an E2 polypeptide with a heterologous signal sequence. An E2 polypeptide can include a C-terminal membrane anchor sequence which occurs at approximately amino acid positions 715-730 and may extend as far as approximately amino acid residue 746 (see, Lin et al., J. Virol. (1994) 68:5063-5073).

In some cases, a E2 polypeptide suitable for use in an immunogenic composition of the present disclosure lacks a portion of its C-terminal region, e.g., from about amino acid 715 to the C-terminus; from about amino acid 625 to the C-terminus; from about amino acid 661 to the C-terminus; from about amino acid 655 to the C-terminus; from about amino acid 500 to the C-terminus, where the amino acid numbering is with reference to the numbering in Figures 1A-C. See, e.g., U.S. Patent No. 6,521,423.

An E2 polypeptide suitable for use in an immunogenic composition of the present disclosure can comprise an amino acid sequence having at least about 70%, at least about 75%, at least about 80%,at least about 85%, at least about 90%, at least about 95%, at least about 98%, at least about 99%, or 100%, amino acid sequence identity to the amino acid sequence of an E2 polypeptide depicted in Figures 1A-C, Figures 2A-2B, or Figure 3.

An E2 polypeptide suitable for use in an immunogenic composition of the present disclosure can comprise an amino acid sequence having at least about 70%, at least about 75%, at least about 80%, at least about 85%, at least about 90%, at least about 95%, at least about 98%, at least about 99%, or 100%, amino acid sequence identity to the amino acid sequence of an E2 polypeptide depicted in Figures 8A and 8B. For example, an E2 polypeptide of genotype 7A can comprise an amino acid sequence having at least about 70%, at least about 75%, at least about 80%, at least about 85%, at least about 90%, at least about 95%, at least about 98%, at least about 99%, or 100%, amino acid sequence identity to amino acids 384-750 of the amino acid sequence depicted in Figures 8A and 8B.

An E2 polypeptide suitable for use in an immunogenic composition of the present disclosure can comprise an amino acid sequence having at least about 70%, at least about 75%, at least about 80%, at least about 85%, at least about 90%, at least about 95%, at least about 98%, at least about 99%, or 100%, amino acid sequence identity to the amino acid sequence of an E2 polypeptide depicted in Figures 9A-C. For example, an E2 polypeptide of genotype 2A can comprise an amino acid sequence having at least about 70%, at least about 75%, at least about 80%, at least about 85%, at least about 90%, at least about 95%, at least about 98%, at least about 99%, or 100%, amino acid sequence identity to amino acids 384-751 of an amino acid sequence identified as 2A and depicted in Figures 9A-C. For example, an E2 polypeptide of genotype 2B can comprise an amino acid sequence having at least about 70%, at least about 75%, at least about 80%, at least about 85%, at least about 90%, at least about 95%, at least about 98%, at least about 99%, or 100%, amino acid sequence identity to amino acids 384-751 of an amino acid sequence identified as 2B and depicted in Figures 9A-C.

### Modified E2 polypeptide

In some cases, the E2 polypeptide present in a subject immunogenic composition is modified such that the E2 polypeptide has reduced glycosylation compared to wild-type E2 polypeptide. Wild-type HCV E2 is glycosylated at E2N1 and/or E2N6, which in the reference strain H77 includes residues Asn-417 and/or Asn-532, respectively. "E2N1" and "E2N6" refer to amino acid sequence motifs at which naturally-occurring HCV E2 polypeptides can be N-linked glycosylated, which motifs are positioned in the E2 polypeptide as shown in the alignments of Figures 1A-1C, 2A-2E and 5. For example, as shown in Figures 1A-C, in the reference genotype 1 strain H77, the E2N1 site includes Asn-417 and the E2N6 site includes Asn-532. As another example, as shown in Figures 2A and 2B, the E2N1 site includes Asn-418, and the E2N6 site includes Asn-534. As another example, as shown in Figures 8A and 8B, the E2N1 site includes Asn-417, and the E2N6 site includes Asn-533.

A modified E2 polypeptide suitable for inclusion in a subject immunogenic composition has a substitution of the asparagine at E2N1 and/or E2N6 (e.g., by modification (e.g., substitution) of Asn-417 and/or Asn-532), such that N-linked glycosylation does not occur at these positions. E2 polypeptides for production of modified E2 polypeptides can be an E2 polypeptide of any genotype, subtype, or isolate of HCV. E2 polypeptides of genotype 1 and E2 polypeptides of genotype 3 are of particular interest.

A modified E2 polypeptide can comprise a modification, e.g., a substitution, of an asparagine in the sites E2N1 and/or E2N6 (e.g., Asn-417 and/or Asn-532), such that N-linked glycosylation does not occur at these positions. The asparagine of E2N1 and/or E2N6 (e.g., Asn-417 and/or the Asn-532) can be substituted with any amino acid that is not subject to glycosylation (including N-glycosylation and O-glycosylation). Examples of substitutions include, but are not limited to, N417T (substitution of Asn-417 with a threonine), N417S (substitution of Asn-417 with a serine), N417Q (substitution of Asn-417 with a glutamine), N417Y (substitution of Asn-417 with a tyrosine), N417C (substitution of Asn-417 with a cysteine), N532T, N532S, N532Q, N532Y, N532C, and the like. The position of the substituted asparagine(s) is based on the numbering depicted in Figures 1A-C (HCV genotype 1). The positions of the substituted asparagine(s) in genotype 3 are shown by arrows in Figures 2A-2B and Figure 3. Positions of substituted asparagine(s) are also depicted in Figure 5.

A modified E2 polypeptide can have a length of from about 200 amino acids (aa) to about 250 aa, from about 250 aa to about 275 aa, from about 275 aa to about 300 aa, from about 300 aa to about 325 aa, from about 325 aa to about 350 aa, or from about 350 aa to about 365 aa.

In Figures 1A-C, the amino acid sequence of E2 is amino acid 384 to amino acid 746. In Figures 2A-2B, the amino acid sequence of E2 is amino acid 385 to amino acid 754. In Figure 3, the amino acid sequence of E2 is amino acid 386 to amino acid 746. As used herein, an "E2 polypeptide" includes a precursor E2 protein, including the signal sequence; includes a mature E2 polypeptide which lacks this sequence; and includes an E2 polypeptide with a heterologous signal sequence. An E2 polypeptide can include a C-terminal membrane anchor sequence which occurs at approximately amino acid positions 715-730 and may extend as far as approximately amino acid residue 746 (see, Lin et al., J. Virol. (1994) 68:5063-5073).

In some cases, a modified E2 polypeptide lacks a portion of its C-terminal region, e.g., from about amino acid 715 to the C-terminus; from about amino acid 625 to the C-terminus; from about amino acid 650 to the C-terminus; from about amino acid 661 to the C-terminus; from about amino acid 655 to the C-terminus; from about amino acid 500 to the C-terminus, where the amino acid numbering is with reference to the numbering in Figures 1A-C. See, e.g., U.S. Patent No. 6,521,423.

A modified E2 polypeptide can comprise an amino acid sequence having at least about 70%, at least about 75%, at least about 80%, at least about 85%, at least about 90%, at least about 95%, at least about 98%, at least about 99%, or 100%, amino acid sequence identity to the amino acid sequence of an E2 polypeptide depicted in Figures 1A-C, Figures 2A-2B, or Figure 3, where the modified E2 polypeptide has an amino acid substitution of asparagine at E2N1 and/or E2N6 (e.g., Asn-417 and/or Asn-532).

A modified E2 polypeptide can comprise an amino acid sequence having at least about 70%, at least about 75%, at least about 80%, at least about 85%, at least about 90%, at least about 95%, at least about 98%, at least about 99%, or 100%, amino acid sequence identity to the amino acid sequence of an E2 polypeptide depicted in Figures 1A-C, Figures 2A-F, or Figure 3, where the modified E2 polypeptide has an amino acid substitution of asparagine at E2N2 (e.g., Asn-417), where the amino acid sequence comprises an asparagine at E2N6 (e.g., Asn-532).

A modified E2 polypeptide can comprise an amino acid sequence having at least about 70%, at least about 75%, at least about 80%, at least about 85%, at least about 90%, at least about 95%, at least about 98%, at least about 99%, or 100%, amino acid sequence identity to the amino acid sequence of an E2 polypeptide depicted in Figures 1A-C, Figures 2A-F, or Figure 3, where the modified E2 polypeptide has an amino acid substitution of asparagine at E2N6 (e.g., Asn-532), where the amino acid sequence comprises asparagine at E2N1 (e.g., Asn-417).

A modified E2 polypeptide can comprise an amino acid sequence having at least about 70%, at least about 75%, at least about 80%, at least about 85%, at least about 90%, at least about 95%, at least about 98%, at least about 99%, or 100%, amino acid sequence identity to the amino acid sequence of an E2 polypeptide depicted in Figures 1A-C, Figures 2A-F, or Figure 3, where the modified E2 polypeptide has an amino acid substitution of asparagine at E2N1 (e.g., Asn-417) and an amino acid substitution of asparagine at E2N6 (e.g., Asn-532).

In some cases, in addition to the above-described modifications, a modified E2 polypeptide includes an amino acid substitution of an asparagine at one or more of: 1) the E2N2 site (e.g., residue 423); 2) the E2N4 site (e.g., residue 448); 3) the E2N11 site (e.g., residue 645).

### Compositions comprising HCV E1 of HCV genotype 1

The present disclosure provides an immunogenic composition comprising E1/E2 of HCV genotype 1. In some cases, the immunogenic composition comprises HCV genotype 1 E1/E2 alone, e.g., the immunogenic composition does not include an E1 polypeptide, an E2 polypeptide, or an E1/E2 heterodimer of any other HCV genotype.

### Compositions comprising E1, E2, and/or E1/E2 of two different HCV genotypes

Compositions of the present disclosure comprise: a) an HCV E1 polypeptide, an E2 polypeptide, or an E1/E2 heterodimeric polypeptide complex from a first HCV genotype; b) an HCV E1 polypeptide, an E2 polypeptide, or an E1/E2 heterodimeric polypeptide complex from a second HCV genotype; and c) a pharmaceutically acceptable excipient, with the proviso that the composition comprises at least one E1 polypeptide and at least one E2 polypeptide. The E2 polypeptide can be present in the composition in a heterodimeric complex with an E1 polypeptide, or can be present in the composition uncomplexed with an E1 polypeptide.

In the compositions described below, "E2" refers to an E2 polypeptide uncomplexed with an E1 polypeptide, while "E1/E2" refers to E1 and E2 present together in a heterodimeric polypeptide complex. Similarly, the E1 polypeptide can be present in the composition in a heterodimeric complex with an E2 polypeptide, or can be present in the composition uncomplexed with an E2 polypeptide. In the compositions described below, "E1" refers to an E1 polypeptide uncomplexed with an E2 polypeptide, while "E1/E2" refers to E1 and E2 present together in a heterodimeric polypeptide complex.

Composition of the present disclosure can comprise:
a) an E1/E2 heterodimeric polypeptide complex of a first HCV genotype; and an E1/E2 heterodimeric polypeptide complex of a second HCV genotype;
b) an E1/E2 heterodimeric polypeptide complex of a first HCV genotype; and an E2 polypeptide of a second HCV genotype;
c) an E2 polypeptide of a first HCV genotype; and an E1/E2 heterodimeric polypeptide complex of a second HCV genotype;
d) an E1 polypeptide of a first HCV genotype; and an E2 polypeptide of a second HCV genotype;
e) an E1 polypeptide of a first HCV genotype; and an E1/E2 heterodimeric polypeptide complex of a second HCV genotype;
f) an E2 polypeptide of a first HCV genotype; and an E1 polypeptide of a second HCV genotype; or
g) an E2 polypeptide of a first HCV genotype; and an E1/E2 heterodimeric polypeptide complex of a second HCV genotype.

Exemplary compositions of the present disclosure include:
1) an E1/E2 heterodimeric polypeptide complex of HCV genotype 1; and an E1/E2 heterodimeric polypeptide complex of HCV genotype 3, e.g., where all of the polypeptides are wild-type;
2) an E1/E2 heterodimeric polypeptide complex of HCV genotype 1; and an E2 polypeptide of HCV genotype 3, e.g., where all of the polypeptides are wild-type;
3) an E2 polypeptide of HCV genotype 1; and an E1/E2 heterodimeric polypeptide complex of HCV genotype 3, e.g., where all of the polypeptides are wild-type;
4) an E1/E2 heterodimeric polypeptide complex of HCV genotype 1; and an E1/E2 heterodimeric polypeptide complex of HCV genotype 2, e.g., where all of the polypeptides are wild-type;
5) an E1/E2 heterodimeric polypeptide complex of HCV genotype 1; and an E2 polypeptide of HCV genotype 2, e.g., where all of the polypeptides are wild-type;
6) an E2 polypeptide of HCV genotype 1; and an E1/E2 heterodimeric polypeptide complex of HCV genotype 2, e.g., where all of the polypeptides are wild-type;
7) an E1 polypeptide of HCV genotype 1; and an E2 polypeptide of HCV genotype 3, e.g., where all of the polypeptides are wild-type;
8) an E1 polypeptide of HCV genotype 1; and an E1/E2 heterodimeric polypeptide complex of HCV genotype 3, e.g., where all of the polypeptides are wild-type;
9) an E2 polypeptide of HCV genotype 1; and an E1 polypeptide of HCV genotype 3, e.g., where all of the polypeptides are wild-type;
10) an E2 polypeptide of HCV genotype 1; and an E1/E2 heterodimeric polypeptide complex of HCV genotype 3, e.g., where all of the polypeptides are wild-type;
11) an E1 polypeptide of HCV genotype 1; and an E2 polypeptide of HCV genotype 2, e.g., where all of the polypeptides are wild-type;
12) an E1 polypeptide of HCV genotype 1; and an E1/E2 heterodimeric polypeptide complex of HCV genotype 2, e.g., where all of the polypeptides are wild-type;
13) an E2 polypeptide of HCV genotype 1; and an E1 polypeptide of HCV genotype 2, e.g., where all of the polypeptides are wild-type;
14) an E2 polypeptide of HCV genotype 1; and an E1/E2 heterodimeric polypeptide complex of HCV genotype 2, e.g., where all of the polypeptides are wild-type.
   In some embodiments, a subject composition does not include an E1 polypeptide, and E2 polypeptide, and/or an E1/E2 polypeptide of any HCV genotype other than the above-mentioned genotype 1 and 2 combinations or genotype 1 and 3 combinations.
   As noted above, the E2 polypeptide can have a wild-type HCV E2 amino acid sequence, e.g., where the E2 polypeptide does not have an amino acid substitution of asparagine in an E2N1 site and/or an E2N6 site. A "wild-type" HCV E2 polypeptide comprises an amino acid sequence of an HCV E2 polypeptide found in nature.
   As noted above, in some embodiments, the E2 polypeptide can comprise an amino acid substitution of asparagine in an E2N1 site and/or an E2N6 site. The following are exemplary compositions:
15) an E1/E2 heterodimeric polypeptide complex of HCV genotype 1, where the E2 polypeptide comprises a modification (e.g., a substitution) of an asparagine at the E2N1 and/or E2N6 site such as described above; and an E1/E2 heterodimeric polypeptide complex of HCV genotype 3, where the E2 polypeptide is wild-type;
16) an E1/E2 heterodimeric polypeptide complex of HCV genotype 1, where the E2 polypeptide is wild-type; and an E1/E2 heterodimeric polypeptide complex of HCV genotype 3, where the E2 polypeptide comprises a modification (e.g., a substitution) of an asparagine at the E2N1 and/or E2N6 site such as described above;
17) an E1/E2 heterodimeric polypeptide complex of HCV genotype 1, where the E2 polypeptide comprises a modification (e.g., a substitution) of an asparagine at the E2N1 and/or E2N6 site such as described above; and an E2 polypeptide of HCV genotype 3, where the E2 polypeptide is wild-type;
18) an E1/E2 heterodimeric polypeptide complex of HCV genotype 1, where the E2 polypeptide is wild-type; and an E2 polypeptide of HCV genotype 3, where the E2 polypeptide comprises a modification (e.g., a substitution) of an asparagine at the E2N1 and/or E2N6 site such as described above;
19) an E2 polypeptide of HCV genotype 1, where the E2 polypeptide comprises a modification (e.g., a substitution) of an asparagine at the E2N1 and/or E2N6 site such as described above; and an E1/E2 heterodimeric polypeptide complex of HCV genotype 3, where the E2 polypeptide is wild-type;
20) an E2 polypeptide of HCV genotype 1, where the E2 polypeptide is wild-type; and an E1/E2 heterodimeric polypeptide complex of HCV genotype 3, where the E2 polypeptide comprises a modification (e.g., a substitution) of an asparagine at the E2N1 and/or E2N6 site such as described above;
21) an E1/E2 heterodimeric polypeptide complex of HCV genotype 1, where the E2 polypeptide comprises a modification (e.g., a substitution) of an asparagine at the E2N1 and/or E2N6 site such as described above; and an E1/E2 heterodimeric polypeptide complex of HCV genotype 2, where the E2 polypeptide is wild-type;
22) an E1/E2 heterodimeric polypeptide complex of HCV genotype 1, where the E2 polypeptide is wild-type; and an E1/E2 heterodimeric polypeptide complex of HCV genotype 2, where the E2 polypeptide comprises a modification (e.g., a substitution) of an asparagine at the E2N1 and/or E2N6 site such as described above;
23) an E1/E2 heterodimeric polypeptide complex of HCV genotype 1, where the E2 polypeptide comprises a modification (e.g., a substitution) of an asparagine at the E2N1 and/or E2N6 site such as described above; and an E2 polypeptide of HCV genotype 2, where the E2 polypeptide is wild-type;
24) an E1/E2 heterodimeric polypeptide complex of HCV genotype 1, where the E2 polypeptide is wild-type; and an E2 polypeptide of HCV genotype 2, where the E2 polypeptide comprises a modification (e.g., a substitution) of an asparagine at the E2N1 and/or E2N6 site such as described above;
25) an E2 polypeptide of HCV genotype 1, where the E2 polypeptide comprises a modification (e.g., a substitution) of an asparagine at the E2N1 and/or E2N6 site such as described above; and an E1/E2 heterodimeric polypeptide complex of HCV genotype 2, where the E2 polypeptide is wild-type;
26) an E2 polypeptide of HCV genotype 1, where the E2 polypeptide is wild-type; and an E1/E2 heterodimeric polypeptide complex of HCV genotype 2, where the E2 polypeptide comprises a modification (e.g., a substitution) of an asparagine at the E2N1 and/or E2N6 site such as described above.

### Compositions comprising E2 or E1/E2 of two different HCV genotypes

Compositions of the present disclosure comprise: a) an HCV E2 polypeptide, or an E1/E2 heterodimeric polypeptide complex from a first HCV genotype; b) an HCV E2 polypeptide, or an E1/E2 heterodimeric polypeptide complex, from a second HCV genotype; and c) a pharmaceutically acceptable excipient. The E2 polypeptide can be present in the composition in a heterodimeric complex with an E1 polypeptide, or can be present in the composition uncomplexed with an E1 polypeptide. The E2 polypeptide can be soluble.

A composition of the present disclosure can comprise one of a) through k), as described below, where the composition comprises a pharmaceutically acceptable excipient:
a) an E2 polypeptide of HCV genotype 1a; and an E1/E2 heterodimeric polypeptide complex of HCV genotype 3a, where all of the polypeptides are wild-type. In some cases, the composition is administered in an amount that is effective to neutralize HCV genotypes most prevalent in North America and among i.v. drug users. In some cases, the composition is administered in an amount that is effective to neutralize HCV genotypes 1a and 3a. In some cases, the composition is administered in an amount that is effective to induce neutralizing antibody to HCV genotypes 1a and 3a. In some cases, the composition is administered in an amount that is effective to induce a CTL response to HCV genotypes 1a and 3a. In some cases, the composition is administered in an amount that is effective to achieve 50% or greater than 50% neutralization of HCV genotypes 1a and 3a. In some cases, the composition does not include an E1 polypeptide, an E2 polypeptide, and/or an E1/E2 polypeptide of any HCV genotype other than the above-mentioned genotypes 1a and 3a. In some cases, a subject composition also includes an E1 polypeptide, and E2 polypeptide, and/or an E1/E2 polypeptide of at least one additional HCV genotype.
b) a full-length E2 polypeptide of HCV genotype 1a; and an E1/E2 heterodimeric polypeptide complex of HCV genotype 3a, where all of the polypeptides are wild-type. In some cases, the composition is administered in an amount that is effective to neutralize HCV genotypes most prevalent in North America and among i.v. drug users. In some cases, the composition is administered in an amount that is effective to neutralize HCV genotypes 1a and 3a. In some cases, the composition is administered in an amount that is effective to induce neutralizing antibody to HCV genotypes 1a and 3a. In some cases, the composition is administered in an amount that is effective to induce a CTL response to HCV genotypes 1a and 3a. In some cases, the composition is administered in an amount that is effective to achieve 50% or greater than 50% neutralization of HCV genotypes 1a and 3a. In some cases, the composition does not include an E1 polypeptide, an E2 polypeptide, and/or an E1/E2 polypeptide of any HCV genotype other than the above-mentioned genotypes 1a and 3a. In some cases, a subject composition also includes an E1 polypeptide, and E2 polypeptide, and/or an E1/E2 polypeptide of at least one additional HCV genotype.
c) a soluble E2 polypeptide of HCV genotype 1a; and an E1/E2 heterodimeric polypeptide complex of HCV genotype 3a, where all of the polypeptides are wild-type. In some cases, the composition is administered in an amount that is effective to neutralize HCV genotypes most prevalent in North America and among i.v. drug users. In some cases, the composition is administered in an amount that is effective to neutralize HCV genotypes 1a and 3a. In some cases, the composition is administered in an amount that is effective to induce neutralizing antibody to HCV genotypes 1a and 3a. In some cases, the composition is administered in an amount that is effective to induce a CTL response to HCV genotypes 1a and 3a. In some cases, the composition is administered in an amount that is effective to achieve 50% or greater than 50% neutralization of HCV genotypes 1a and 3a. In some cases, the composition does not include an E1 polypeptide, an E2 polypeptide, and/or an E1/E2 polypeptide of any HCV genotype other than the above-mentioned genotypes 1a and 3a. In some cases, a subject composition also includes an E1 polypeptide, and E2 polypeptide, and/or an E1/E2 polypeptide of at least one additional HCV genotype.
d) an E2 polypeptide of HCV genotype 1a, where the E2 polypeptide comprises a modification (e.g., a substitution) of an asparagine at the E2N1 and/or E2N6 site such as described above; and an E1/E2 heterodimeric polypeptide complex of HCV genotype 3a, where the E2 polypeptide of the E1/E2 heterodimer is wild-type. In some cases, the composition is administered in an amount that is effective to neutralize HCV genotypes most prevalent in North America and among i.v. drug users. In some cases, the composition is administered in an amount that is effective to neutralize HCV genotypes 1a and 3a. In some cases, the composition is administered in an amount that is effective to induce neutralizing antibody to HCV genotypes 1a and 3a. In some cases, the composition is administered in an amount that is effective to induce a CTL response to HCV genotypes 1a and 3a. In some cases, the composition is administered in an amount that is effective to achieve 50% or greater than 50% neutralization of HCV genotypes 1a and 3a. In some cases, the composition does not include an E1 polypeptide, an E2 polypeptide, and/or an E1/E2 polypeptide of any HCV genotype other than the above-mentioned genotypes 1a and 3a. In some cases, a subject composition also includes an E1 polypeptide, and E2 polypeptide, and/or an E1/E2 polypeptide of at least one additional HCV genotype.
e) an E2 polypeptide of HCV genotype 1a, where the E2 polypeptide is wild-type; and an E1/E2 heterodimeric polypeptide complex of HCV genotype 3a, where the E2 polypeptide of the E1/E2 heterodimer comprises a modification (e.g., a substitution) of an asparagine at the E2N1 and/or E2N6 site such as described above. In some cases, the composition is administered in an amount that is effective to neutralize HCV genotypes most prevalent in North America and among i.v. drug users. In some cases, the composition is administered in an amount that is effective to neutralize HCV genotypes 1a and 3a. In some cases, the composition is administered in an amount that is effective to induce neutralizing antibody to HCV genotypes 1a and 3a. In some cases, the composition is administered in an amount that is effective to induce a CTL response to HCV genotypes 1a and 3a. In some cases, the composition is administered in an amount that is effective to achieve 50% or greater than 50% neutralization of HCV genotypes 1a and 3a. In some cases, the composition does not include an E1 polypeptide, an E2 polypeptide, and/or an E1/E2 polypeptide of any HCV genotype other than the above-mentioned genotypes 1a and 3a. In some cases, a subject composition also includes an E1 polypeptide, and E2 polypeptide, and/or an E1/E2 polypeptide of at least one additional HCV genotype.
f) an E2 polypeptide of HCV genotype 3a; and an E1/E2 heterodimeric polypeptide complex of HCV genotype 1a, where all of the polypeptides are wild-type. In some cases, the composition is administered in an amount that is effective to neutralize HCV genotypes most prevalent in North America and among i.v. drug users. In some cases, the composition is administered in an amount that is effective to neutralize HCV genotypes 1a and 3a. In some cases, the composition is administered in an amount that is effective to induce neutralizing antibody to HCV genotypes 1a and 3a. In some cases, the composition is administered in an amount that is effective to induce a CTL response to HCV genotypes 1a and 3a. In some cases, the composition is administered in an amount that is effective to achieve 50% or greater than 50% neutralization of HCV genotypes 1a and 3a. In some cases, the composition does not include an E1 polypeptide, an E2 polypeptide, and/or an E1/E2 polypeptide of any HCV genotype other than the above-mentioned genotypes 1a and 3a. In some cases a subject composition also includes an E1 polypeptide, and E2 polypeptide, and/or an E1/E2 polypeptide of at least one additional HCV genotype.
g) a full-length E2 polypeptide of HCV genotype 3a; and an E1/E2 heterodimeric polypeptide complex of HCV genotype 1a, where all of the polypeptides are wild-type. In some cases, the composition is administered in an amount that is effective to neutralize HCV genotypes most prevalent in North America and among i.v. drug users. In some cases, the composition is administered in an amount that is effective to neutralize HCV genotypes 1a and 3a. In some cases, the composition is administered in an amount that is effective to induce neutralizing antibody to HCV genotypes 1a and 3a. In some cases, the composition is administered in an amount that is effective to induce a CTL response to HCV genotypes 1a and 3a. In some cases, the composition is administered in an amount that is effective to achieve 50% or greater than 50% neutralization of HCV genotypes 1a and 3a. In some cases, the composition does not include an E1 polypeptide, an E2 polypeptide, and/or an E1/E2 polypeptide of any HCV genotype other than the above-mentioned genotypes 1a and 3a. In some cases, a subject composition also includes an E1 polypeptide, and E2 polypeptide, and/or an E1/E2 polypeptide of at least one additional HCV genotype.
h) a soluble E2 polypeptide of HCV genotype 3a; and an E1/E2 heterodimeric polypeptide complex of HCV genotype 1a, where all of the polypeptides are wild-type. In some cases, the composition is administered in an amount that is effective to neutralize HCV genotypes most prevalent in North America and among i.v. drug users. In some cases, the composition is administered in an amount that is effective to neutralize HCV genotypes 1a and 3a. In some cases, the composition is administered in an amount that is effective to induce neutralizing antibody to HCV genotypes 1a and 3a. In some cases, the composition is administered in an amount that is effective to induce a CTL response to HCV genotypes 1a and 3a. In some cases, the composition is administered in an amount that is effective to achieve 50% or greater than 50% neutralization of HCV genotypes 1a and 3a. In some cases, the composition does not include an E1 polypeptide, an E2 polypeptide, and/or an E1/E2 polypeptide of any HCV genotype other than the above-mentioned genotypes 1a and 3a. In some cases, a subject composition also includes an E1 polypeptide, and E2 polypeptide, and/or an E1/E2 polypeptide of at least one additional HCV genotype.
i) an E2 polypeptide of HCV genotype 3a, where the E2 polypeptide comprises a modification (e.g., a substitution) of an asparagine at the E2N1 and/or E2N6 site such as described above; and an E1/E2 heterodimeric polypeptide complex of HCV genotype 1a, where the E2 polypeptide of the E1/E2 heterodimer is wild-type. In some cases, the composition is administered in an amount that is effective to neutralize HCV genotypes most prevalent in North America and among i.v. drug users. In some cases, the composition is administered in an amount that is effective to neutralize HCV genotypes 1a and 3a. In some cases, the composition is administered in an amount that is effective to induce neutralizing antibody to HCV genotypes 1a and 3a. In some cases, the composition is administered in an amount that is effective to induce a CTL response to HCV genotypes 1a and 3a. In some cases, the composition is administered in an amount that is effective to achieve 50% or greater than 50% neutralization of HCV genotypes 1a and 3a. In some cases, the composition does not include an E1 polypeptide, an E2 polypeptide, and/or an E1/E2 polypeptide of any HCV genotype other than the above-mentioned genotypes 1a and 3a. In some cases, a subject composition also includes an E1 polypeptide, and E2 polypeptide, and/or an E1/E2 polypeptide of at least one additional HCV genotype.
j) an E2 polypeptide of HCV genotype 3a, where the E2 polypeptide is wild-type; and an E1/E2 heterodimeric polypeptide complex of HCV genotype 1a, where the E2 polypeptide of the E1/E2 heterodimer comprises a modification (e.g., a substitution) of an asparagine at the E2N1 and/or E2N6 site such as described above. In some cases, the composition is administered in an amount that is effective to neutralize HCV genotypes most prevalent in North America and among i.v. drug users. In some cases, the composition is administered in an amount that is effective to neutralize HCV genotypes 1a and 3a. In some cases, the composition is administered in an amount that is effective to induce neutralizing antibody to HCV genotypes 1a and 3a. In some cases, the composition is administered in an amount that is effective to induce a CTL response to HCV genotypes 1a and 3a. In some cases, the composition is administered in an amount that is effective to achieve 50% or greater than 50% neutralization of HCV genotypes 1a and 3a. In some cases, the composition does not include an E1 polypeptide, an E2 polypeptide, and/or an E1/E2 polypeptide of any HCV genotype other than the above-mentioned genotypes 1a and 3a. In some cases, a subject composition also includes an E1 polypeptide, and E2 polypeptide, and/or an E1/E2 polypeptide of at least one additional HCV genotype.
k) an E2 polypeptide or an E1/E2 heterodimeric polypeptide complex of HCV genotype 1a; and an E2 polypeptide or an E1/E2 heterodimeric polypeptide complex of HCV genotype 2a. In some cases, all of the polypeptides are wild-type. In some cases, the genotype 1a and the genotype 2a polypeptides are both E1/E2 heterodimeric polypeptide. In some cases, the genotype 1a polypeptide is an E2 polypeptide; and the genotype 2a polypeptide is an E1/E2 heterodimeric polypeptide complex. In some cases, the genotype 1a polypeptide is an E1/E2 heterodimeric polypeptide complex; and the genotype 2a polypeptide is an E2 polypeptide. In some cases, the genotype 1a and the genotype 2a polypeptides are both E2 polypeptides. In some cases, the genotype 1a and the genotype 2a polypeptides are both soluble E2 polypeptides. In some cases, the composition is administered in an amount that is effective to neutralize HCV genotypes such as genotypes 1a, 1b, 2a, 4, 5, and 6a. In some cases, the composition is administered in an amount that is effective to neutralize HCV genotypes 1a and 2a. In some cases, the composition is administered in an amount that is effective to induce neutralizing antibody to HCV genotypes 1a and 2a. In some cases, the composition is administered in an amount that is effective to induce a CTL response to HCV genotypes 1a and 2a. In some cases, the composition is administered in an amount that is effective to achieve 50% or greater than 50% neutralization of HCV genotypes 1a and 2a. In some cases, the composition is administered in an amount that is effective to achieve 50% or greater than 50% neutralization of HCV genotypes 1a, 1b, 2a, 4, 5, and 6a. In some cases the composition does not include an E1 polypeptide, an E2 polypeptide, and/or an E1/E2 polypeptide of any HCV genotype other than the above-mentioned genotypes 1a and 2a. In some cases, a subject composition also includes an E1 polypeptide, and E2 polypeptide, and/or an E1/E2 polypeptide of at least one additional HCV genotype.

In any of the above-described embodiments, E2 (either as uncomplexed E2 or as an E1/E2 heterodimeric complex) of HCV genotype 1a can be substituted with E2 (either as uncomplexed E2 or as an E1/E2 heterodimeric complex) of HCV genotype 1, 4, 5, or 6.

### Compositions comprising E1, E2, and/or E1/E2 of three different HCV genotypes

Compositions of the present disclosure comprise: a) an HCV E1 polypeptide, an E2 polypeptide, or an E1/E2 heterodimeric polypeptide complex from a first HCV genotype; b) an HCV E1 polypeptide, an E2 polypeptide, or an E1/E2 heterodimeric polypeptide complex from a second HCV genotype; c) an HCV E1 polypeptide, an E2 polypeptide, or an E1/E2 heterodimeric polypeptide complex from a third HCV genotype; and d) a pharmaceutically acceptable excipient, with the proviso that the composition comprises at least one E1 polypeptide and at least one E2 polypeptide. The E2 polypeptide can be present in the composition in a heterodimeric complex with an E1 polypeptide, or can be present in the composition uncomplexed with an E1 polypeptide. In the compositions described below, "E2" refers to an E2 polypeptide uncomplexed with an E1 polypeptide, while "E1/E2" refers to E1 and E2 present together in a heterodimeric polypeptide complex. Similarly, the E1 polypeptide can be present in the composition in a heterodimeric complex with an E2 polypeptide, or can be present in the composition uncomplexed with an E2 polypeptide. In the compositions described below, "E1" refers to an E1 polypeptide uncomplexed with an E2 polypeptide, while "E1/E2" refers to E1 and E2 present together in a heterodimeric polypeptide complex.

Composition of the present disclosure can comprise:
i) an E1/E2 heterodimeric polypeptide complex of HCV genotype 1; an E1/E2 heterodimeric polypeptide complex of HCV genotype 2; and an E1/E2 heterodimeric polypeptide complex of HCV genotype 3, e.g., where all of the polypeptides are wild-type;
ii) an E1/E2 heterodimeric polypeptide complex of HCV genotype 1; an E1/E2 heterodimeric polypeptide complex of HCV genotype 2; and an E2 polypeptide of HCV genotype 3, e.g., where all of the polypeptides are wild-type;
iii) an E1/E2 heterodimeric polypeptide complex of HCV genotype 1; an E2 polypeptide of HCV genotype 2; and an E1/E2 heterodimeric polypeptide complex of HCV genotype 3, e.g., where all of the polypeptides are wild-type;
iv) an E2 polypeptide of HCV genotype 1; an E1/E2 heterodimeric polypeptide complex of HCV genotype 2; and an E1/E2 heterodimeric polypeptide complex of HCV genotype 3, e.g., where all of the polypeptides are wild-type;
v) an E1/E2 heterodimeric polypeptide complex of HCV genotype 1; an E2 polypeptide of HCV genotype 2; and an E2 polypeptide of HCV genotype 3, e.g., where all of the polypeptides are wild-type;
vi) an E2 polypeptide of HCV genotype 1; an E1/E2 heterodimeric polypeptide complex of HCV genotype 2; and an E2 polypeptide of HCV genotype 3, e.g., where all of the polypeptides are wild-type;
vii) an E2 polypeptide of HCV genotype 1; an E2 polypeptide of HCV genotype 2; and an E1/E2 heterodimeric polypeptide complex of HCV genotype 3, e.g., where all of the polypeptides are wild-type.
viii) an E1 polypeptide of HCV genotype 1; an E1/E2 heterodimeric polypeptide complex of HCV genotype 2; and an E1/E2 heterodimeric polypeptide complex of HCV genotype 3;
ix) an E1/E2 heterodimeric polypeptide complex of HCV genotype 1; an E1 polypeptide of HCV genotype 2; and an E1/E2 heterodimeric polypeptide complex of HCV genotype 3;
x) an E1/E2 heterodimeric polypeptide complex of HCV genotype 1; an E1/E2 heterodimeric polypeptide complex of HCV genotype 2; and an E1 polypeptide of HCV genotype 3;
xi) an E1 polypeptide of HCV genotype 1; an E2 polypeptide of HCV genotype 2; and an E1/E2 heterodimeric polypeptide complex of HCV genotype 3;
xii) an E2 polypeptide of HCV genotype 1; an E1 polypeptide of HCV genotype 2; and an E1/E2 heterodimeric polypeptide complex of HCV genotype 3;
xiii) an E1/E2 heterodimeric polypeptide complex of HCV genotype 1; an E2 polypeptide of HCV genotype 2; and an E1 polypeptide of HCV genotype 3;
xiv) an E1 polypeptide of HCV genotype 1; an E2 polypeptide of HCV genotype 2; and an E2 polypeptide of HCV genotype 3;
xv) an E2 polypeptide of HCV genotype 1; an E1 polypeptide of HCV genotype 2; and an E2 polypeptide of HCV genotype 3;
xvi) an E2 polypeptide of HCV genotype 1; an E2 polypeptide of HCV genotype 2; and an E1 polypeptide of HCV genotype 3;
xvii) an E1 polypeptide of HCV genotype 1; an E1/E2 heterodimeric polypeptide complex of HCV genotype 2; and an E2 polypeptide of HCV genotype 3. The combinations of clauses (i)-(iii), (v), (ix), (x) and (xiii) are embodiments of the invention.

In the invention, a subject composition does not include an E1 polypeptide, and E2 polypeptide, and/or an E1/E2 polypeptide of any HCV genotype other than the above-mentioned genotypes 1, 2, and 3. In some embodiments, a subject composition also includes an E1 polypeptide, and E2 polypeptide, and/or an E1/E2 polypeptide of HCV genotype 7.

In any of the above-noted compositions, the E2 polypeptide can have a wild-type HCV E2 amino acid sequence, e.g., where the E2 polypeptide does not have an amino acid substitution of asparagine in an E2N1 site and/or an E2N6 site. A "wild-type" HCV E2 polypeptide comprises an amino acid sequence of an HCV E2 polypeptide found in nature. In some embodiments, an E2 polypeptide can comprise an amino acid substitution of asparagine in an E2N1 site and/or an E2N6 site.

### Compositions comprising E2, and/or E1/E2 of three different HCV genotypes

Compositions of the present disclosure comprise: a) an HCV E2 polypeptide, or an E1/E2 heterodimeric polypeptide complex from a first HCV genotype; b) an HCV E2 polypeptide, or an E1/E2 heterodimeric polypeptide complex from a second HCV genotype; c) an HCV E2 polypeptide, or an E1/E2 heterodimeric polypeptide complex from a third HCV genotype; and d) a pharmaceutically acceptable excipient. The E2 polypeptide can be present in the composition in a heterodimeric complex with an E1 polypeptide, or can be present in the composition uncomplexed with an E1 polypeptide. The E2 polypeptide can be soluble.

A composition of the present disclosure can comprise one of a) through f), as described below, where the composition comprises a pharmaceutically acceptable excipient:
a) an E2 polypeptide of HCV genotype 1a; an E1/E2 heterodimeric polypeptide complex of HCV genotype 2a; and an E2 polypeptide of HCV genotype 3a. In some cases, all of the polypeptides are wild-type. In other cases, the E2 polypeptide of the E1/E2 heterodimer comprises a modification (e.g., a substitution) of an asparagine at the E2N1 and/or E2N6 site such as described above. In other cases, the E2 polypeptide that is not in a heterodimeric complex with E1 comprises a modification (e.g., a substitution) of an asparagine at the E2N1 and/or E2N6 site such as described above. In some cases, at least one of the E2 polypeptides is full length. In some cases, at least one of the E2 polypeptides is a soluble E2 polypeptide. In some cases, the composition is administered in an amount that is effective to neutralize HCV genotypes prevalent globally. In some cases, the composition is administered in an amount that is effective to neutralize HCV genotypes 1a, 1b, 2a, 2b, 3a, 4a, 5a, 6a, and 7a. In some cases, the composition is administered in an amount that is effective to induce neutralizing antibody to HCV genotypes 1a, 1b, 2a, 2b, 3a, 4a, 5a, 6a, and 7a. In some cases, the composition is administered in an amount that is effective to induce a CTL response to HCV genotypes 1a, 1b, 2a, 2b, 3a, 4a, 5a, 6a, and 7a. In some cases, the composition is administered in an amount that is effective to achieve 50% or greater than 50% neutralization of HCV genotypes 1a, 1b, 2a, 2b, 3a, 4a, 5a, 6a, and 7a. In some cases the composition does not include an E1 polypeptide, an E2 polypeptide, and/or an E1/E2 polypeptide of any HCV genotype other than the above-mentioned genotypes 1a, 2a, and 3a. In some cases the composition also includes an E1 polypeptide, and E2 polypeptide, and/or an E1/E2 polypeptide of at least one additional HCV genotype. In some cases, the composition also includes an E1 polypeptide, and E2 polypeptide, and/or an E1/E2 polypeptide of HCV genotype 2b. In some cases, the composition also includes an E1 polypeptide, and E2 polypeptide, and/or an E1/E2 polypeptide of HCV genotype 7a.
b) an E2 polypeptide of HCV genotype 1a; an E2 polypeptide of HCV genotype 2a; and an E1/E2 heterodimeric polypeptide complex of HCV genotype 3a. In some cases, all of the polypeptides are wild-type. In other cases, the E2 polypeptide of the E1/E2 heterodimer comprises a modification (e.g., a substitution) of an asparagine at the E2N1 and/or E2N6 site such as described above. In other cases, the E2 polypeptide that is not in a heterodimeric complex with E1 comprises a modification (e.g., a substitution) of an asparagine at the E2N1 and/or E2N6 site such as described above. In some cases, at least one of the E2 polypeptides is full length. In some cases, at least one of the E2 polypeptides is a soluble E2 polypeptide. In some cases, the composition is administered in an amount that is effective to neutralize HCV genotypes prevalent globally. In some cases, the composition is administered in an amount that is effective to neutralize HCV genotypes 1a, 1b, 2a, 2b, 3a, 4a, 5a, 6a, and 7a. In some cases, the composition is administered in an amount that is effective to induce neutralizing antibody to HCV genotypes 1a, 1b, 2a, 2b, 3a, 4a, 5a, 6a, and 7a. In some cases, the composition is administered in an amount that is effective to induce a CTL response to HCV genotypes 1a, 1b, 2a, 2b, 3a, 4a, 5a, 6a, and 7a. In some cases, the composition is administered in an amount that is effective to achieve 50% or greater than 50% neutralization of HCV genotypes 1a, 1b, 2a, 2b, 3a, 4a, 5a, 6a, and 7a. In some cases the composition does not include an E1 polypeptide, an E2 polypeptide, and/or an E1/E2 polypeptide of any HCV genotype other than the above-mentioned genotypes 1a, 2a, and 3a. In some cases the composition also includes an E1 polypeptide, and E2 polypeptide, and/or an E1/E2 polypeptide of at least one additional HCV genotype. In some cases the composition also includes an E1 polypeptide, and E2 polypeptide, and/or an E1/E2 polypeptide of HCV genotype 2b. In some cases, the composition also includes an E1 polypeptide, and E2 polypeptide, and/or an E1/E2 polypeptide of HCV genotype 7a.
c) an E1/E2 heterodimeric polypeptide complex of HCV genotype 1a; an E2 polypeptide of HCV genotype 2a; and an E2 polypeptide of HCV genotype 3a. In some cases, all of the polypeptides are wild-type. In other cases, the E2 polypeptide of the E1/E2 heterodimer comprises a modification (e.g., a substitution) of an asparagine at the E2N1 and/or E2N6 site such as described above. In other cases, the E2 polypeptide that is not in a heterodimeric complex with E1 comprises a modification (e.g., a substitution) of an asparagine at the E2N1 and/or E2N6 site such as described above. In some cases, at least one of the E2 polypeptides is full length. In some cases, at least one of the E2 polypeptides is a soluble E2 polypeptide. In some cases, the composition is administered in an amount that is effective to neutralize HCV genotypes prevalent globally. In some cases, the composition is administered in an amount that is effective to neutralize HCV genotypes 1a, 1b, 2a, 2b, 3a, 4a, 5a, 6a, and 7a. In some cases, the composition is administered in an amount that is effective to induce neutralizing antibody to HCV genotypes 1a, 1b, 2a, 2b, 3a, 4a, 5a, 6a, and 7a. In some cases, the composition is administered in an amount that is effective to induce a CTL response to HCV genotypes 1a, 1b, 2a, 2b, 3a, 4a, 5a, 6a, and 7a. In some cases, the composition is administered in an amount that is effective to achieve 50% or greater than 50% neutralization of HCV genotypes 1a, 1b, 2a, 2b, 3a, 4a, 5a, 6a, and 7a. In some embodiments, the composition does not include an E1 polypeptide, an E2 polypeptide, and/or an E1/E2 polypeptide of any HCV genotype other than the above-mentioned genotypes 1a, 2a, and 3a. In some embodiments, the composition also includes an E1 polypeptide, and E2 polypeptide, and/or an E1/E2 polypeptide of at least one additional HCV genotype. In some embodiments, the composition also includes an E1 polypeptide, and E2 polypeptide, and/or an E1/E2 polypeptide of HCV genotype 2b. In some embodiments, the composition also includes an E1 polypeptide, and E2 polypeptide, and/or an E1/E2 polypeptide of HCV genotype 7a.
d) an E1/E2 heterodimeric polypeptide complex of HCV genotype 1a; an E1/E2 heterodimeric polypeptide complex of HCV genotype 2a; and an E2 polypeptide of HCV genotype 3a. In some cases, all of the polypeptides are wild-type. In other cases, the E2 polypeptide of the E1/E2 heterodimer comprises a modification (e.g., a substitution) of an asparagine at the E2N1 and/or E2N6 site such as described above. In other cases, the E2 polypeptide that is not in a heterodimeric complex with E1 comprises a modification (e.g., a substitution) of an asparagine at the E2N1 and/or E2N6 site such as described above. In some cases, at least one of the E2 polypeptides is full length. In some cases, at least one of the E2 polypeptides is a soluble E2 polypeptide. In some cases, the composition is administered in an amount that is effective to neutralize HCV genotypes prevalent globally. In some cases, the composition is administered in an amount that is effective to neutralize HCV genotypes 1a, 1b, 2a, 2b, 3a, 4a, 5a, 6a, and 7a. In some cases, the composition is administered in an amount that is effective to induce neutralizing antibody to HCV genotypes 1a, 1b, 2a, 2b, 3a, 4a, 5a, 6a, and 7a. In some cases, the composition is administered in an amount that is effective to induce a CTL response to HCV genotypes 1a, 1b, 2a, 2b, 3a, 4a, 5a, 6a, and 7a. In some cases, the composition is administered in an amount that is effective to achieve 50% or greater than 50% neutralization of HCV genotypes 1a, 1b, 2a, 2b, 3a, 4a, 5a, 6a, and 7a. In some embodiments, the composition does not include an E1 polypeptide, an E2 polypeptide, and/or an E1/E2 polypeptide of any HCV genotype other than the above-mentioned genotypes 1a, 2a, and 3a. In some embodiments, the composition also includes an E1 polypeptide, and E2 polypeptide, and/or an E1/E2 polypeptide of at least one additional HCV genotype. In some embodiments, the composition also includes an E1 polypeptide, and E2 polypeptide, and/or an E1/E2 polypeptide of HCV genotype 2b. In some embodiments, the composition also includes an E1 polypeptide, and E2 polypeptide, and/or an E1/E2 polypeptide of HCV genotype 7a.
e) an E2 polypeptide of HCV genotype 1a; an E1/E2 heterodimeric polypeptide complex of HCV genotype 2a; and an E1/E2 heterodimeric polypeptide complex of HCV genotype 3a. In some cases, all of the polypeptides are wild-type. In other cases, the E2 polypeptide of the E1/E2 heterodimer comprises a modification (e.g., a substitution) of an asparagine at the E2N1 and/or E2N6 site such as described above. In other cases, the E2 polypeptide that is not in a heterodimeric complex with E1 comprises a modification (e.g., a substitution) of an asparagine at the E2N1 and/or E2N6 site such as described above. In some cases, at least one of the E2 polypeptides is full length. In some cases, at least one of the E2 polypeptides is a soluble E2 polypeptide. In some cases, the composition is administered in an amount that is effective to neutralize HCV genotypes prevalent globally. In some cases, the composition is administered in an amount that is effective to neutralize HCV genotypes 1a, 1b, 2a, 2b, 3a, 4a, 5a, 6a, and 7a. In some cases, the composition is administered in an amount that is effective to induce neutralizing antibody to HCV genotypes 1a, 1b, 2a, 2b, 3a, 4a, 5a, 6a, and 7a. In some cases, the composition is administered in an amount that is effective to induce a CTL response to HCV genotypes 1a, 1b, 2a, 2b, 3a, 4a, 5a, 6a, and 7a. In some cases, the composition is administered in an amount that is effective to achieve 50% or greater than 50% neutralization of HCV genotypes 1a, 1b, 2a, 2b, 3a, 4a, 5a, 6a, and 7a. In some cases, the composition does not include an E1 polypeptide, an E2 polypeptide, and/or an E1/E2 polypeptide of any HCV genotype other than the above-mentioned genotypes 1a, 2a, and 3a. In some cases, the composition also includes an E1 polypeptide, and E2 polypeptide, and/or an E1/E2 polypeptide of at least one additional HCV genotype. In some cases, the composition also includes an E1 polypeptide, and E2 polypeptide, and/or an E1/E2 polypeptide of HCV genotype 2b. In some cases, the composition also includes an E1 polypeptide, and E2 polypeptide, and/or an E1/E2 polypeptide of HCV genotype 7a.
f) an E1/E2 heterodimeric polypeptide complex of HCV genotype 1a; an E2 polypeptide of HCV genotype 2a; and an E1/E2 heterodimeric polypeptide complex of HCV genotype 3a. In some cases, all of the polypeptides are wild-type. In other cases, the E2 polypeptide of the E1/E2 heterodimer comprises a modification (e.g., a substitution) of an asparagine at the E2N1 and/or E2N6 site such as described above. In other cases, the E2 polypeptide that is not in a heterodimeric complex with E1 comprises a modification (e.g., a substitution) of an asparagine at the E2N1 and/or E2N6 site such as described above. In some cases, at least one of the E2 polypeptides is full length. In some cases, at least one of the E2 polypeptides is a soluble E2 polypeptide. In some cases, the composition is administered in an amount that is effective to neutralize HCV genotypes prevalent globally. In some cases, the composition is administered in an amount that is effective to neutralize HCV genotypes 1a, 1b, 2a, 2b, 3a, 4a, 5a, 6a, and 7a. In some cases, the composition is administered in an amount that is effective to induce neutralizing antibody to HCV genotypes 1a, 1b, 2a, 2b, 3a, 4a, 5a, 6a, and 7a. In some cases, the composition is administered in an amount that is effective to induce a CTL response to HCV genotypes 1a, 1b, 2a, 2b, 3a, 4a, 5a, 6a, and 7a. In some cases, the composition is administered in an amount that is effective to achieve 50% or greater than 50% neutralization of HCV genotypes 1a, 1b, 2a, 2b, 3a, 4a, 5a, 6a, and 7a. In some embodiments, the composition does not include an E1 polypeptide, an E2 polypeptide, and/or an E1/E2 polypeptide of any HCV genotype other than the above-mentioned genotypes 1a, 2a, and 3a. In some embodiments, the composition also includes an E1 polypeptide, and E2 polypeptide, and/or an E1/E2 polypeptide of at least one additional HCV genotype. In some embodiments, the composition also includes an E1 polypeptide, and E2 polypeptide, and/or an E1/E2 polypeptide of HCV genotype 2b. In some embodiments, the composition also includes an E1 polypeptide, and E2 polypeptide, and/or an E1/E2 polypeptide of HCV genotype 7a.

### Methods of making E1 and E2 polypeptides

E1 and E2 polypeptides can be produced using any suitable method, including recombinant and non-recombinant methods (e.g., chemical synthesis).

Where a polypeptide is chemically synthesized, the synthesis may proceed via liquid phase or solid-phase. Solid-phase peptide synthesis (SPPS) allows the incorporation of unnatural amino acids and/or peptide/protein backbone modification. Various forms of SPPS, such as Fmoc and Boc, are available for synthesizing polypeptides. Details of the chemical synthesis are known in the art (e.g., Ganesan A. 2006 Mini Rev. Med Chem. 6:3-10 and Camarero JA et al. 2005 Protein Pept Lett. 12:723-8).

Where a polypeptide is produced using recombinant techniques, the polypeptide may be produced as an intracellular protein or as an secreted protein, using any suitable construct and any suitable host cell, which can be a prokaryotic or eukaryotic cell, such as a bacterial (e.g., *Escherichia coli*) cell or a yeast host cell, respectively. Other examples of eukaryotic cells that may be used as host cells include insect cells, mammalian cells, filamentous fungi, and plant cells. Suitable yeast cells include, e.g., Saccharomyces cerevisiae and Pichia (e.g., *Pichia pastoris*)*.*

Suitable mammalian cell lines include human cell lines, non-human primate cell lines, rodent (e.g., mouse, rat) cell lines, and the like. Suitable mammalian cell lines include, but are not limited to, HeLa cells (e.g., American Type Culture Collection (ATCC) No. CCL-2), CHO cells (e.g., ATCC Nos. CRL9618, CCL61, CRL9096), 293 cells (e.g., ATCC No. CRL-1573), Vero cells, NIH 3T3 cells (e.g., ATCC No. CRL-1658), Huh-7 cells, BHK cells (e.g., ATCC No. CCL10), PC12 cells (ATCC No. CRL1721), COS cells, COS-7 cells (ATCC No. CRL1651), RAT1 cells, mouse L cells (ATCC No. CCLI.3), human embryonic kidney (HEK) cells (ATCC No. CRL1573), HLHepG2 cells, MRC5 cells (ATCC No. CCL-171), and the like. Where mammalian host cells are used, such host cells may include human cells (e.g., HeLa, 293, H9 and Jurkat cells); mouse cells (e.g., NIH3T3, L cells, and C127 cells); primate cells (e.g., Cos 1, Cos 7 and CV1) and hamster cells (e.g., Chinese hamster ovary (CHO) cells).

A variety of host-vector systems suitable for the expression of a polypeptide may be employed according to standard procedures known in the art. See, e.g., Sambrook et al., 1989 Current Protocols in Molecular Biology Cold Spring Harbor Press, New York; Ausubel et al. 1995 Current Protocols in Molecular Biology, Eds. Wiley and Sons; "Protein Expression: A Practical Approach" (1999) S.J. Higgins and B.D. James, eds., Oxford University Press; "Protein Expression in Mammalian Cells: Methods and Protocols (Methods in Molecular Biology)" (2012) James L. Hartley, ed., Humana Press; and "Production of Recombinant Proteins" (2005) Gerd Gellisen, ed., Wiley-VCH. Methods for introduction of nucleic acids into host cells include, for example, transformation, electroporation, conjugation, transfection, calcium phosphate methods, and the like. The method for transfer can be selected so as to provide for stable expression of the introduced polypeptide-encoding nucleic acid. The polypeptide-encoding nucleic acid can be provided as an inheritable episomal element (e.g., a plasmid) or can be genomically-integrated. A variety of appropriate vectors for use in production of a peptide of interest are available commercially.

Suitable expression vectors include, but are not limited to, baculovirus vectors, bacteriophage vectors, plasmids, phagemids, cosmids, fosmids, bacterial artificial chromosomes, viral vectors (e.g. viral vectors based on vaccinia virus, poliovirus, adenovirus, adeno-associated virus, SV40, herpes simplex virus, HIV-based lentivirus vectors, murine leukemia virus (MVL)-based gamma retrovirus vectors, and the like), P1-based artificial chromosomes, yeast plasmids, yeast artificial chromosomes, and any other vectors specific for specific hosts of interest (such as E. coli, mammalian cells, insect cells, or yeast cells).

E1 and E2 polypeptides can be produced by introducing a recombinant expression vector comprising a nucleotide sequence encoding the E1 and/or the E2 polypeptide into an appropriate host cell, where the host cell produces the encoded E1 and/or E1 polypeptide. In the expression vector, a polynucleotide comprising a nucleotide sequence(s) encoding E1 and/or E2 is linked to a regulatory sequence as appropriate to obtain the desired expression properties. These regulatory sequences can include promoters, enhancers, terminators, operators, repressors, and inducers. The promoters can be regulated or constitutive. Expression vectors generally have convenient restriction sites located near the promoter sequence to provide for the insertion of nucleic acid sequences encoding a protein of interest. A selectable marker operative in the expression host cell may be present.

In some cases, the E1 and E2 polypeptides are encoded in a recombinant expression vector suitable for expression in a eukaryotic host cell (e.g., an insect cell; a yeast cell; a mammalian host cell, such as CHO cells, HeLa cells, 293 cells, MRC5 cells, etc.). In some cases, a recombinant expression vector comprises a nucleotide sequence encoding E1 and E2 as a single polypeptide chain; the recombinant expression vector is introduced into a eukaryotic host cell to generate a genetically modified host cell. The E1 and E2 polypeptides are initially produced as a single polypeptide chain, which is cleaved in the endoplasmic reticulum (ER) of the genetically modified host cell to produce separate E1 and E2 polypeptides. The separate E1 and E2 polypeptides can form a heterodimer (e.g., a non-covalently linked heterodimer) in the ER. The E1/E2 heterodimer can be isolated from the genetically modified host cell by, e.g., lysis using a non-ionic detergent. See, e.g., Frey et al. (2010) Vaccine 28:6367.

Alternatively, the E1 and E2 polypeptides can be encoded on separate recombinant expression vectors; and produced in a cell (e.g., the same host cell or separate host cells) as separate polypeptides.

If full-length E1 and E2 polypeptides are expressed in a eukaryotic host cell, the E1 and E2 polypeptides remain in the lumen of the endoplasmic reticulum (ER) as asialoglycoproteins. If the E1 and E2 polypeptides have C-terminal truncations, such that the C-terminal transmembrane regions are removed, the truncated polypeptides are secreted as complex glycoproteins (where the glycosylations include sialic acid). An E1 polypeptide that has a C-terminal truncation, such that the C-terminal transmembrane removed, and such that the E1 polypeptide is secreted from a eukaryotic cell producing the E1 polypeptide, is referred to as a "soluble E1 polypeptide." Similarly, an E2 polypeptide that has a C-terminal truncation, such that the C-terminal transmembrane removed, and such that the E2 polypeptide is secreted from a eukaryotic cell producing the E2 polypeptide, is referred to as a "soluble E2 polypeptide." Removal of approximately amino acids 662-746 of E2, or amino acids 715-746 of D2, and removal of approximately amino acids 330-384 of E1, results in secretion of E2 and E1 from a eukaryotic host cell. If E1 and E2 are co-expressed in the same eukaryotic host cell as full-length polypeptides, they remain in the lumen of the ER as a heterodimer.

After production in a host cell, the E1 and E2 polypeptides (e.g., separately or as a heterodimer) can be purified from the host cell. Methods of purification of recombinantly produced polypeptides from a host cell are known in the art and include, e.g., detergent lysis (e.g., with a non-ionic detergent), followed by one or more of size exclusion column chromatography, high performance liquid chromatography, affinity chromatography, and the like.

### Formulations

HCV E1, E2, and E1/E2 polypeptides can be formulated with a pharmaceutically acceptable excipient(s) to generate a subject immunogenic composition. A wide variety of pharmaceutically acceptable excipients is known in the art and need not be discussed in detail herein. Pharmaceutically acceptable excipients have been amply described in a variety of publications, including, for example, A. Gennaro (2000) "Remington: The Science and Practice of Pharmacy", 20th edition, Lippincott, Williams, & Wilkins; Pharmaceutical Dosage Forms and Drug Delivery Systems (1999) H. C. Ansel et al., eds 7th ed., Lippincott, Williams, & Wilkins; and Handbook of Pharmaceutical Excipients (2000) A. H. Kibbe et al., eds., 3rd ed. Amer. Pharmaceutical Assoc.

In some embodiments, the E1 and E2 polypeptides are formulated in an aqueous buffer. Suitable aqueous buffers include, but are not limited to, acetate, succinate, citrate, and phosphate buffers varying in strengths from about 5 mM to about 100 mM. In some embodiments, the aqueous buffer includes reagents that provide for an isotonic solution. Such reagents include, but are not limited to, sodium chloride; and sugars e.g., mannitol, dextrose, sucrose, and the like. In some embodiments, the aqueous buffer further includes a non-ionic surfactant such as polysorbate 20 (TWEEN®20) or polysorbate 80 (TWEEN®80). For example, a formulation of E1 and E2 polypeptides in an aqueous buffer can include, e.g., from about 0.01% to about 0.05% polysorbate-20 (TWEEN®20) non-ionic detergent. Optionally the formulations may further include a preservative. Suitable preservatives include, but are not limited to, a benzyl alcohol, phenol, chlorobutanol, benzalkonium chloride, and the like. In many cases, the formulation is stored at about 4°C. Formulations may also be lyophilized, in which case they generally include cryoprotectants such as sucrose, trehalose, lactose, maltose, mannitol, and the like. Lyophilized formulations can be stored over extended periods of time, even at ambient temperatures.

E1 and E2 polypeptides can be formulated into preparations for injection by dissolving, suspending or emulsifying them in an aqueous or nonaqueous solvent, such as vegetable or other similar oils, synthetic aliphatic acid glycerides, esters of higher aliphatic acids or propylene glycol; and if desired, with conventional additives such as solubilizers, isotonic agents, suspending agents, emulsifying agents, stabilizers and preservatives.

An immunogenic composition of the present disclosure can include, e.g., pharmaceutical grades of mannitol, lactose, starch, magnesium stearate, sodium saccharin, talcum, cellulose, glucose, sucrose, magnesium, carbonate, and the like. The compositions may contain pharmaceutically acceptable auxiliary substances as required to approximate physiological conditions such as pH adjusting and buffering agents, toxicity adjusting agents and the like, for example, sodium acetate, sodium chloride, potassium chloride, calcium chloride, sodium lactate and the like.

The concentration of HCV E1, E2, and E1/E2 polypeptides in a formulation can vary widely (e.g., from less than about 0.1% to at least about 2%, to as much as 20% to 50% or more by weight) and can be selected primarily based on fluid volumes, viscosities, and patient-based factors in accordance with the particular mode of administration selected and the patient's needs.

The HCV polypeptide-containing formulations of the present disclosure can be provided in the form of a solution, suspension, tablet, pill, capsule, powder, gel, cream, lotion, ointment, aerosol or the like. It is recognized that oral administration can require protection of the compositions from digestion. This is typically accomplished either by association of the composition with an agent that renders it resistant to acidic and enzymatic hydrolysis or by packaging the composition in an appropriately resistant carrier. Means of protecting from digestion are well known in the art.

The HCV polypeptide-containing formulations of the present disclosure can also be provided so as to enhance serum half-life of the heterodimer following administration. For example, where isolated HCV E1, E2, or E1/E2 polypeptides are formulated for injection, the HCV polypeptide may be provided in a liposome formulation, prepared as a colloid, or other conventional techniques for extending serum half-life. A variety of methods are available for preparing liposomes, as described in, e.g., Szoka et al., Ann. Rev. Biophys. Bioeng. 9:467 (1980), U.S. Pat. Nos. 4,235,871, 4,501,728 and 4,837,028. The preparations may also be provided in controlled release or slow-release forms.

### Adjuvant

An immunogenic composition of the present disclosure can include an adjuvant. Examples of known suitable adjuvants that can be used in humans include, but are not necessarily limited to, alum, aluminum phosphate, aluminum hydroxide, MF59 (4.3% w/v squalene, 0.5% w/v Tween 80™, 0.5% w/v Span 85), CpG-containing nucleic acid (where the cytosine is unmethylated), QS21, MPL, 3DMPL, extracts from Aquilla, ISCOMS, LT/CT mutants, poly(D,L-lactide-co-glycolide) (PLG) microparticles, Quil A, interleukins, and the like. For experimental animals, one can use Freund's, N-acetyl-muramyl-L-threonyl-D-isoglutamine (thr-MDP), N-acetyl-nor-muramyl-L-alanyl-D-isoglutamine (CGP 11637, referred to as nor-MDP), N-acetylmuramyl-L-alanyl-D-isoglutaminyl-L-alanine-2-(1'-2'-dip- almitoyl-sn-glycero-3-hydroxyphosphoryloxy)-ethylamine (CGP 19835A, referred to as MTP-PE), and RIBI, which contains three components extracted from bacteria, monophosphoryl lipid A, trehalose dimycolate and cell wall skeleton (MPL+TDM+CWS) in a 2% squalene/Tween 80 emulsion. The effectiveness of an adjuvant may be determined by one or more of measuring the amount of antibodies directed against the immunogenic antigen or antigenic epitope thereof, measuring a cytotoxic T lymphocyte response to the antigen, and measuring a helper T cell response to the antigen.

Further exemplary adjuvants to enhance effectiveness of the composition include, but are not limited to: (1) oil-in-water emulsion formulations (with or without other specific immunostimulating agents such as muramyl peptides (see below) or bacterial cell wall components), such as for example (a) MF59TM (see, e.g., WO 90/14837), containing 5% Squalene, 0.5% Tween 80, and 0.5% Span 85 (optionally containing MTP-PE) formulated into submicron particles using a microfluidizer, (b) SAF, containing 10% Squalane, 0.4% Tween 80,5% pluronic-blocked polymer L121, and thr-MDP either microfluidized into a submicron emulsion or vortexed to generate a larger particle size emulsion, and (c) RIBI TM adjuvant system (RAS), (Ribi Immunochem, Hamilton, Mont.) containing 2% Squalene, 0.2% Tween 80, and one or more bacterial cell wall components such as monophosphorylipid A (MPL), trehalose dimycolate (TDM), and cell wall skeleton (CWS), e.g., MPL+CWS (Detox TM); (2) saponin adjuvants, such as QS21 or StimulonTM (Cambridge Bioscience, Worcester, Mass.) may be used or particles generated therefrom such as ISCOMs (immunostimulating complexes), which ISCOMS may be devoid of additional detergent e.g. WO 00/07621; (3) Complete Freund's Adjuvant (CFA) and Incomplete Freund's Adjuvant (IFA); (4) cytokines, such as interleukins (e.g. IL-1, IL-2, IL-4, IL-5, IL-6, IL-7, IL-12 (WO99/44636), etc.), interferons (e.g. gamma interferon), macrophage colony stimulating factor (M-CSF), tumor necrosis factor (TNF), etc.; (5) monophosphoryl lipid A (MPL) or 3-O-deacylated MPL (3dMPL) e.g. GB-2220221, EP-A-0689454, optionally in the substantial absence of alum when used with pneumococcal saccharides e.g. WO 00/56358; (6) combinations of 3dMPL with, for example, QS21 and/or oil-in-water emulsions (see, e.g. EP-A-0835318, EP-A-0735898, EP-A-0761231); (7) oligonucleotides comprising a CpG motif containing at least one CG dinucleotide, where the cytosine is unmethylated (see, e.g., WO 96/02555, WO 98/16247, WO 98/18810, WO 98/40100, WO 98/55495, WO 98/37919 and WO 98/52581); (8) a polyoxyethylene ether or a polyoxyethylene ester (see, e.g. WO 99/52549); (9) a polyoxyethylene sorbitan ester surfactant in combination with an octoxynol (WO 01/21207) or a polyoxyethylene alkyl ether or ester surfactant in combination with at least one additional non-ionic surfactant such as an octoxynol (WO 01/21152); (10) a saponin and an immunostimulatory oligonucleotide (e.g. a CpG oligonucleotide) (WO 00/62800); (11) an immunostimulant and a particle of metal salt (see, e.g. WO 00/23105); (12) a saponin and an oil-in-water emulsion (see e.g. WO 99/11241); (13) a saponin (e.g. QS21)+3dMPL+IM2 (optionally including a sterol) (see, e.g. WO 98/57659); (14) other substances that act as immunostimulating agents to enhance the efficacy of the composition. Muramyl peptides include N-acetyl-muramyl-L-threonyl-D-isoglutamine (thr-MDP), N-25 acetyl-normuramyl-L-alanyl-D-isoglutamine (nor-MDP), N-acetylmuramyl-L-alanyl-D-isoglutarninyl-L-alanine-2-(1'-2'-dipalmitoyl-sn-glycero-3-hydroxyphosphoryloxy)-ethylamine MTP-PE), etc. Also suitable for use is Matrix-M™; Matrix-M™ is an adjuvant that comprises 40 nm nanoparticles comprising *Quillaja* saponins, cholesterol, and phospholipid. Adjuvants suitable for administration to a human are of particular interest. In some cases, the adjuvant is one that enhances a CD4+ T helper response to the immunogen.

In some instances, the adjuvant is MF59, with or without a CpG-containing oligonucleotide. In other instances, the adjuvant is alum, with or without a CpG-containing oligonucleotide. In other instances, the adjuvant is poly(D,L-lactide-co-glycolide), with or without a CpG-containing oligonucleotide. In other instances, the adjuvant is MPL, with or without a CpG-containing oligonucleotide. In other cases, the adjuvant is Matrix-M™, with or without a CpG-containing oligonucleotide.

### METHODS OF INDUCING AN IMMUNE RESPONSE TO HCV

The present disclosure provides a method of inducing an immune response to at least one HCV genotype in a mammalian subject. The methods generally involve administering to an individual in need thereof an effective amount of a subject immunogenic composition.

An HCV immunogenic composition of the present disclosure is generally administered to a human subject who has an HCV infection or who is at risk of acquiring an HCV infection so as to prevent or at least partially arrest the development of disease and its complications. An amount adequate to accomplish this is defined as a "therapeutically effective dose" or a "therapeutically effective amount." "Prophylactic" use of a subject immunogenic composition generally refers to administration to an individual who has not been infected with HCV. "Therapeutic" use of a subject immunogenic composition can refer to "prophylactic" use (administration to an individual who has not been infected with HCV) and/or to administration to an individual who has an HCV infection. A "therapeutically effective amount" of an immunogenic composition of the present disclosure, can be an amount that, when administered in one or more doses to an individual who is not infected with HCV, is effective to induce an immune response in the individual to HCV. A "therapeutically effective amount" of an immunogenic composition of the present disclosure, can be an amount that, when administered in one or more doses to an individual who is infected with HCV, is effective to enhance an immune response in the individual to HCV.

Amounts effective for therapeutic use will depend on, e.g., the immunogenic composition, the manner of administration, the weight and general state of health of the patient, and the judgment of the prescribing physician. Single or multiple doses of a subject immunogenic composition can be administered depending on the dosage and frequency required and tolerated by the patient, and route of administration.

In some cases, an effective amount (e.g., a therapeutically effective amount) of an HCV immunogenic composition of the present disclosure is an amount that, when administered to an individual in one or more doses, is effective to induce an antibody response to HCV in the individual. For example, antibody to HCV (e.g., extracellular HCV), and/or to an HCV-infected cell, can be induced.

An effective amount of an immunogenic composition of the present disclosure can be an amount that, when administered to an individual in one or more doses, is effective to induce a neutralizing antibody response to HCV of a variety of genotypes (e.g., genotype 1; genotype 3; genotype 2; genotype 7; genotype 5; genotype 6; etc.). A neutralizing antibody response reduces binding of HCV to CD81 and/or other cellular receptors, and inhibits entry of HCV into a cell. In some cases, an effective amount of an immunogenic composition of the present disclosure is an amount that is effective to induce a neutralizing antibody response to HCV of genotype 1 and genotype 3. In some cases, an effective amount of an immunogenic composition of the present disclosure is an amount that is effective to induce a neutralizing antibody response to HCV of genotype 1, genotype 2, and genotype 3. In some cases, an effective amount of an immunogenic composition of the present disclosure is an amount that is effective to induce a neutralizing antibody response to HCV of genotype 1, genotype 2, genotype 3, and genotype 7. In some cases, an effective amount of an immunogenic composition of the present disclosure is an amount that is effective to induce a neutralizing antibody response to HCV of genotype 1, genotype 2, genotype 3, genotype 4, genotype 5, genotype 6, and genotype 7. For example, in some cases, an effective amount of an immunogenic composition of the present disclosure is an amount that is effective to induce a neutralizing antibody response to HCV of genotype 1a/b and genotype 3a. As another example, in some cases, an effective amount of an immunogenic composition of the present disclosure is an amount that is effective to induce a neutralizing antibody response to HCV of genotype 1a/b, genotype 2a, and genotype 3a. As another example, in some cases, an effective amount of an immunogenic composition of the present disclosure is an amount that is effective to induce a neutralizing antibody response to HCV of genotype 1a/b, genotype 2b, and genotype 3a. As another example, in some cases, an effective amount of an immunogenic composition of the present disclosure is an amount that is effective to induce a neutralizing antibody response to HCV of genotype 1a/b, genotype 2a, genotype 3a, genotype 4a, genotype 5a, genotype 6a, and genotype 7a.

In some cases, an effective amount (e.g., a therapeutically effective amount) of an immunogenic composition of the present disclosure is an amount that, when administered to an individual in one or more doses, is effective to induce a cytotoxic T lymphocyte (CTL) response to HCV. For example, a CTL response to an HCV-infected cell can be induced.

In some cases, an effective amount of an immunogenic composition of the present disclosure is an amount that is effective to induce a CTL response to HCV of genotype 1 and genotype 3. In some cases, an effective amount of an immunogenic composition of the present disclosure is an amount that is effective to induce a CTL response to HCV of genotype 1, genotype 2, and genotype 3. In some cases, an effective amount of an immunogenic composition of the present disclosure is an amount that is effective to induce a CTL response to HCV of genotype 1, genotype 2, genotype 3, and genotype 7. In some cases, an effective amount of an immunogenic composition of the present disclosure is an amount that is effective to induce a CTL response to HCV of genotype 1, genotype 2, genotype 3, genotype 4, genotype 5, genotype 6, and genotype 7. For example, in some cases, an effective amount of an immunogenic composition of the present disclosure is an amount that is effective to induce a CTL response to HCV of genotype 1a/b and genotype 3a. As another example, in some cases, an effective amount of an immunogenic composition of the present disclosure is an amount that is effective to induce a CTL response to HCV of genotype 1a/b, genotype 2a, and genotype 3a. As another example, in some cases, an effective amount of an immunogenic composition of the present disclosure is an amount that is effective to induce a CTL response to HCV of genotype 1a/b, genotype 2b, and genotype 3a. As another example, in some cases, an effective amount of an immunogenic composition of the present disclosure is an amount that is effective to induce a CTL response to HCV of genotype 1a/b, genotype 2a, genotype 3a, genotype 4a, genotype 5a, genotype 6a, and genotype 7a.

In some cases, an effective amount (e.g., a therapeutically effective amount) of an immunogenic composition of the present disclosure is an amount that, when administered to an individual in one or more doses, is effective to induce a helper T lymphocyte (e.g., CD4+ T cell) to HCV in an individual.

In some cases, an effective amount (e.g., a therapeutically effective amount) of an immunogenic composition of the present disclosure is an amount that, when administered to an individual in one or more doses, is effective to induce an antibody response (e.g., a neutralizing antibody response) and/or a CTL response and/or a helper T cell response to HCV genotype 1. In some cases, an effective amount (e.g., a therapeutically effective amount) of an immunogenic composition of the present disclosure is an amount that, when administered to an individual in one or more doses, is effective to induce an antibody response (e.g., a neutralizing antibody response) and/or a CTL response and/or a helper T cell response to HCV genotype 3. In some cases, an effective amount (e.g., a therapeutically effective amount) of an immunogenic composition of the present disclosure is an amount that, when administered to an individual in one or more doses, is effective to induce an antibody response (e.g., a neutralizing antibody response) and/or a CTL response and/or a helper T cell response to HCV genotype 1 and HCV genotype 3. In some cases, an effective amount (e.g., a therapeutically effective amount) of an immunogenic composition of the present disclosure is an amount that, when administered to an individual in one or more doses, is effective to induce an antibody response (e.g., a neutralizing antibody response) and/or a CTL response and/or a helper T cell response to HCV of any genotype.

An immunogenic composition of the present disclosure is generally administered in an amount effective to elicit an immune response, e.g., a humoral immune response (e.g., an antibody response) and/or a CTL response, in the mammalian subject. Effective amounts for immunization will vary, and can generally range from about 1 µg to 100 µg per 70 kg patient, e.g., from about 5 µg/70 kg to about 50 µg/70 kg. Substantially higher dosages (e.g. 10 mg to 100 mg or more) may be suitable in oral, nasal, or topical administration routes. The initial administration can be followed by booster immunization of the same HCV immunogenic composition or a different HCV immunogenic composition. In some instances, a subject method of inducing an immune response involves an initial administration of an HCV immunogenic composition of the present disclosure, followed by at least one booster, and in some instances involves two or more (e.g., three, four, or five) boosters. The interval between an initial administration and a booster, or between a give booster and a subsequent booster, can be from about 1 week to about 12 weeks, e.g., from about 1 week to about 2 weeks, from about 2 weeks to about 4 weeks, from about 4 weeks to about 6 weeks, from about 6 weeks to about 8 weeks, from about 8 weeks to about 10 weeks, or from about 10 weeks to about 12 weeks.

In general, immunization can be accomplished by administration of an HCV immunogenic composition of the present disclosure by any suitable route, including administration of the composition orally, nasally, nasopharyngeally, parenterally, enterically, gastrically, topically, transdermally, subcutaneously, intramuscularly, in tablet, solid, powdered, liquid, aerosol form, locally or systemically, with or without added excipients. Actual methods for preparing parenterally administrable compositions will be known or apparent to those skilled in the art and are described in more detail in such publications as Remington's Pharmaceutical Science, 15th ed., Mack Publishing Company, Easton, Pa. (1980). In some instances, immunization is accomplished by intramuscular injection of an HCV immunogenic composition of the present disclosure.

In some cases, subject method comprises administering to an individual in need thereof an effective amount of a composition of the present disclosure that comprises an E2 polypeptide and/or an E1/E2 heterodimeric complex from two different HCV genotypes, as described hereinabove and below, where the composition comprises a pharmaceutically acceptable excipient.

In some cases, subject method comprises administering to an individual in need thereof an effective amount of a composition comprising: an E2 polypeptide of HCV genotype 1a; and an E1/E2 heterodimeric polypeptide complex of HCV genotype 3a, where all of the polypeptides are wild-type. In some cases, the composition is administered in an amount that is effective to neutralize HCV genotypes most prevalent in North America and among i.v. drug users. In some cases, the composition is administered in an amount that is effective to neutralize HCV genotypes 1a and 3a. In some cases, the composition is administered in an amount that is effective to induce neutralizing antibody to HCV genotypes 1a and 3a. In some cases, the composition is administered in an amount that is effective to induce a CTL response to HCV genotypes 1a and 3a. In some cases, the composition is administered in an amount that is effective to achieve 50% or greater than 50% neutralization of HCV genotypes 1a and 3a. In some cases, the composition does not include an E1 polypeptide, an E2 polypeptide, and/or an E1/E2 polypeptide of any HCV genotype other than the above-mentioned genotypes 1a and 3a. In some cases, a subject composition also includes an E1 polypeptide, and E2 polypeptide, and/or an E1/E2 polypeptide of at least one additional HCV genotype.

In some cases, subject method comprises administering to an individual in need thereof an effective amount of a composition comprising: a full-length E2 polypeptide of HCV genotype 1a; and an E1/E2 heterodimeric polypeptide complex of HCV genotype 3a, where all of the polypeptides are wild-type. In some cases, the composition is administered in an amount that is effective to neutralize HCV genotypes most prevalent in North America and among i.v. drug users. In some cases, the composition is administered in an amount that is effective to neutralize HCV genotypes 1a and 3a. In some cases, the composition is administered in an amount that is effective to induce neutralizing antibody to HCV genotypes 1a and 3a. In some cases, the composition is administered in an amount that is effective to induce a CTL response to HCV genotypes 1a and 3a. In some cases, the composition is administered in an amount that is effective to achieve 50% or greater than 50% neutralization of HCV genotypes 1a and 3a. In some cases, the composition does not include an E1 polypeptide, an E2 polypeptide, and/or an E1/E2 polypeptide of any HCV genotype other than the above-mentioned genotypes 1a and 3a. In some cases a subject composition also includes an E1 polypeptide, and E2 polypeptide, and/or an E1/E2 polypeptide of at least one additional HCV genotype.

In some cases, subject method comprises administering to an individual in need thereof an effective amount of a composition comprising: a soluble E2 polypeptide of HCV genotype 1a; and an E1/E2 heterodimeric polypeptide complex of HCV genotype 3a, where all of the polypeptides are wild-type. In some cases, the composition is administered in an amount that is effective to neutralize HCV genotypes most prevalent in North America and among i.v. drug users. In some cases, the composition is administered in an amount that is effective to neutralize HCV genotypes 1a and 3a. In some cases, the composition is administered in an amount that is effective to induce neutralizing antibody to HCV genotypes 1a and 3a. In some cases, the composition is administered in an amount that is effective to induce a CTL response to HCV genotypes 1a and 3a. In some cases, the composition is administered in an amount that is effective to achieve 50% or greater than 50% neutralization of HCV genotypes 1a and 3a. In some cases, the composition does not include an E1 polypeptide, an E2 polypeptide, and/or an E1/E2 polypeptide of any HCV genotype other than the above-mentioned genotypes 1a and 3a. In some cases, a subject composition also includes an E1 polypeptide, and E2 polypeptide, and/or an E1/E2 polypeptide of at least one additional HCV genotype.

In some cases, subject method comprises administering to an individual in need thereof an effective amount of a composition comprising: an E2 polypeptide of HCV genotype 1a, where the E2 polypeptide comprises a modification (e.g., a substitution) of an asparagine at the E2N1 and/or E2N6 site such as described above; and an E1/E2 heterodimeric polypeptide complex of HCV genotype 3a, where the E2 polypeptide of the E1/E2 heterodimer is wild-type. In some cases, the composition is administered in an amount that is effective to neutralize HCV genotypes most prevalent in North America and among i.v. drug users. In some cases, the composition is administered in an amount that is effective to neutralize HCV genotypes 1a and 3a. In some cases, the composition is administered in an amount that is effective to induce neutralizing antibody to HCV genotypes 1a and 3a. In some cases, the composition is administered in an amount that is effective to induce a CTL response to HCV genotypes 1a and 3a. In some cases, the composition is administered in an amount that is effective to achieve 50% or greater than 50% neutralization of HCV genotypes 1a and 3a. In some cases, the composition does not include an E1 polypeptide, an E2 polypeptide, and/or an E1/E2 polypeptide of any HCV genotype other than the above-mentioned genotypes 1a and 3a. In some cases embodiments, a subject composition also includes an E1 polypeptide, and E2 polypeptide, and/or an E1/E2 polypeptide of at least one additional HCV genotype.

In some cases, subject method comprises administering to an individual in need thereof an effective amount of a composition comprising: an E2 polypeptide of HCV genotype 1a, where the E2 polypeptide is wild-type; and an E1/E2 heterodimeric polypeptide complex of HCV genotype 3a, where the E2 polypeptide of the E1/E2 heterodimer comprises a modification (e.g., a substitution) of an asparagine at the E2N1 and/or E2N6 site such as described above. In some cases, the composition is administered in an amount that is effective to neutralize HCV genotypes most prevalent in North America and among i.v. drug users. In some cases, the composition is administered in an amount that is effective to neutralize HCV genotypes 1a and 3a. In some cases, the composition is administered in an amount that is effective to induce neutralizing antibody to HCV genotypes 1a and 3a. In some cases, the composition is administered in an amount that is effective to induce a CTL response to HCV genotypes 1a and 3a. In some cases, the composition is administered in an amount that is effective to achieve 50% or greater than 50% neutralization of HCV genotypes 1a and 3a. In some cases,the composition does not include an E1 polypeptide, an E2 polypeptide, and/or an E1/E2 polypeptide of any HCV genotype other than the above-mentioned genotypes 1a and 3a. In some cases, a subject composition also includes an E1 polypeptide, and E2 polypeptide, and/or an E1/E2 polypeptide of at least one additional HCV genotype.

In some cases, subject method comprises administering to an individual in need thereof an effective amount of a composition comprising: an E2 polypeptide of HCV genotype 3a; and an E1/E2 heterodimeric polypeptide complex of HCV genotype 1a, where all of the polypeptides are wild-type. In some cases, the composition is administered in an amount that is effective to neutralize HCV genotypes most prevalent in North America and among i.v. drug users. In some cases, the composition is administered in an amount that is effective to neutralize HCV genotypes 1a and 3a. In some cases, the composition is administered in an amount that is effective to induce neutralizing antibody to HCV genotypes 1a and 3a. In some cases, the composition is administered in an amount that is effective to induce a CTL response to HCV genotypes 1a and 3a. In some cases, the composition is administered in an amount that is effective to achieve 50% or greater than 50% neutralization of HCV genotypes 1a and 3a. In some cases, the composition does not include an E1 polypeptide, an E2 polypeptide, and/or an E1/E2 polypeptide of any HCV genotype other than the above-mentioned genotypes 1a and 3a. In some cases a subject composition also includes an E1 polypeptide, and E2 polypeptide, and/or an E1/E2 polypeptide of at least one additional HCV genotype.

In some cases, subject method comprises administering to an individual in need thereof an effective amount of a composition comprising: a full-length E2 polypeptide of HCV genotype 3a; and an E1/E2 heterodimeric polypeptide complex of HCV genotype 1a, where all of the polypeptides are wild-type. In some cases, the composition is administered in an amount that is effective to neutralize HCV genotypes most prevalent in North America and among i.v. drug users. In some cases, the composition is administered in an amount that is effective to neutralize HCV genotypes 1a and 3a. In some cases, the composition is administered in an amount that is effective to induce neutralizing antibody to HCV genotypes 1a and 3a. In some cases, the composition is administered in an amount that is effective to induce a CTL response to HCV genotypes 1a and 3a. In some cases, the composition is administered in an amount that is effective to achieve 50% or greater than 50% neutralization of HCV genotypes 1a and 3a. In some cases, the composition does not include an E1 polypeptide, an E2 polypeptide, and/or an E1/E2 polypeptide of any HCV genotype other than the above-mentioned genotypes 1a and 3a. In some cases, a subject composition also includes an E1 polypeptide, and E2 polypeptide, and/or an E1/E2 polypeptide of at least one additional HCV genotype.

In some cases, subject method comprises administering to an individual in need thereof an effective amount of a composition comprising: a soluble E2 polypeptide of HCV genotype 3a; and an E1/E2 heterodimeric polypeptide complex of HCV genotype 1a, where all of the polypeptides are wild-type. In some cases, the composition is administered in an amount that is effective to neutralize HCV genotypes most prevalent in North America and among i.v. drug users. In some cases, the composition is administered in an amount that is effective to neutralize HCV genotypes 1a and 3a. In some cases, the composition is administered in an amount that is effective to induce neutralizing antibody to HCV genotypes 1a and 3a. In some cases, the composition is administered in an amount that is effective to induce a CTL response to HCV genotypes 1a and 3a. In some cases, the composition is administered in an amount that is effective to achieve 50% or greater than 50% neutralization of HCV genotypes 1a and 3a. In some cases, the composition does not include an E1 polypeptide, an E2 polypeptide, and/or an E1/E2 polypeptide of any HCV genotype other than the above-mentioned genotypes 1a and 3a. In some cases a subject composition also includes an E1 polypeptide, and E2 polypeptide, and/or an E1/E2 polypeptide of at least one additional HCV genotype.

In some cases, subject method comprises administering to an individual in need thereof an effective amount of a composition comprising: an E2 polypeptide of HCV genotype 3a, where the E2 polypeptide comprises a modification (e.g., a substitution) of an asparagine at the E2N1 and/or E2N6 site such as described above; and an E1/E2 heterodimeric polypeptide complex of HCV genotype 1a, where the E2 polypeptide of the E1/E2 heterodimer is wild-type. In some cases, the composition is administered in an amount that is effective to neutralize HCV genotypes most prevalent in North America and among i.v. drug users. In some cases, the composition is administered in an amount that is effective to neutralize HCV genotypes 1a and 3a. In some cases, the composition is administered in an amount that is effective to induce neutralizing antibody to HCV genotypes 1a and 3a. In some cases, the composition is administered in an amount that is effective to induce a CTL response to HCV genotypes 1a and 3a. In some cases, the composition is administered in an amount that is effective to achieve 50% or greater than 50% neutralization of HCV genotypes 1a and 3a. In some cases, the composition does not include an E1 polypeptide, an E2 polypeptide, and/or an E1/E2 polypeptide of any HCV genotype other than the above-mentioned genotypes 1a and 3a. In some cases, a subject composition also includes an E1 polypeptide, and E2 polypeptide, and/or an E1/E2 polypeptide of at least one additional HCV genotype.

In some cases, subject method comprises administering to an individual in need thereof an effective amount of a composition comprising: an E2 polypeptide of HCV genotype 3a, where the E2 polypeptide is wild-type; and an E1/E2 heterodimeric polypeptide complex of HCV genotype 1a, where the E2 polypeptide of the E1/E2 heterodimer comprises a modification (e.g., a substitution) of an asparagine at the E2N1 and/or E2N6 site such as described above. In some cases, the composition is administered in an amount that is effective to neutralize HCV genotypes most prevalent in North America and among i.v. drug users. In some cases, the composition is administered in an amount that is effective to neutralize HCV genotypes 1a and 3a. In some cases, the composition is administered in an amount that is effective to induce neutralizing antibody to HCV genotypes 1a and 3a. In some cases, the composition is administered in an amount that is effective to induce a CTL response to HCV genotypes 1a and 3a. In some cases, the composition is administered in an amount that is effective to achieve 50% or greater than 50% neutralization of HCV genotypes 1a and 3a. In some cases, the composition does not include an E1 polypeptide, an E2 polypeptide, and/or an E1/E2 polypeptide of any HCV genotype other than the above-mentioned genotypes 1a and 3a. In some cases a subject composition also includes an E1 polypeptide, and E2 polypeptide, and/or an E1/E2 polypeptide of at least one additional HCV genotype.

In some cases, subject method comprises administering to an individual in need thereof an effective amount of a composition comprising: an E2 polypeptide or an E1/E2 heterodimeric polypeptide complex of HCV genotype 1a; and an E2 polypeptide or an E1/E2 heterodimeric polypeptide complex of HCV genotype 2a. In some cases, all of the polypeptides are wild-type. In some cases, the genotype 1a and the genotype 2a polypeptides are both E1/E2 heterodimeric polypeptide. In some cases, the genotype 1a polypeptide is an E2 polypeptide; and the genotype 2a polypeptide is an E1/E2 heterodimeric polypeptide complex. In some cases, the genotype 1a polypeptide is an E1/E2 heterodimeric polypeptide complex; and the genotype 2a polypeptide is an E2 polypeptide. In some cases, the genotype 1a and the genotype 2a polypeptides are both E2 polypeptides. In some cases, the genotype 1a and the genotype 2a polypeptides are both soluble E2 polypeptides. In some cases, the composition is administered in an amount that is effective to neutralize HCV genotypes such as genotypes 1a, 1b, 2a, 4, 5, and 6a. In some cases, the composition is administered in an amount that is effective to neutralize HCV genotypes 1a and 2a. In some cases, the composition is administered in an amount that is effective to induce neutralizing antibody to HCV genotypes 1a and 2a. In some cases, the composition is administered in an amount that is effective to induce a CTL response to HCV genotypes 1a and 2a. In some cases, the composition is administered in an amount that is effective to achieve 50% or greater than 50% neutralization of HCV genotypes 1a and 2a. In some cases, the composition is administered in an amount that is effective to achieve 50% or greater than 50% neutralization of HCV genotypes 1a, 1b, 2a, 4, 5, and 6a. In some cases the composition does not include an E1 polypeptide, an E2 polypeptide, and/or an E1/E2 polypeptide of any HCV genotype other than the above-mentioned genotypes 1a and 2a. In some cases a subject composition also includes an E1 polypeptide, and E2 polypeptide, and/or an E1/E2 polypeptide of at least one additional HCV genotype.

In some cases, subject method comprises administering to an individual in need thereof an effective amount of a composition of the present disclosure that comprises an E2 polypeptide and/or an E1/E2 heterodimeric complex from three different HCV genotypes, as described hereinabove and below, where the composition comprises a pharmaceutically acceptable excipient.

In some cases, subject method comprises administering to an individual in need thereof an effective amount of a composition comprising: an E2 polypeptide of HCV genotype 1a; an E1/E2 heterodimeric polypeptide complex of HCV genotype 2a; and an E2 polypeptide of HCV genotype 3a. In some cases, all of the polypeptides are wild-type. In other cases, the E2 polypeptide of the E1/E2 heterodimer comprises a modification (e.g., a substitution) of an asparagine at the E2N1 and/or E2N6 site such as described above. In other cases, the E2 polypeptide that is not in a heterodimeric complex with E1 comprises a modification (e.g., a substitution) of an asparagine at the E2N1 and/or E2N6 site such as described above. In some cases, at least one of the E2 polypeptides is full length. In some cases, at least one of the E2 polypeptides is a soluble E2 polypeptide. In some cases, the composition is administered in an amount that is effective to neutralize HCV genotypes prevalent globally. In some cases, the composition is administered in an amount that is effective to neutralize HCV genotypes 1a, 1b, 2a, 2b, 3a, 4a, 5a, 6a, and 7a. In some cases, the composition is administered in an amount that is effective to induce neutralizing antibody to HCV genotypes 1a, 1b, 2a, 2b, 3a, 4a, 5a, 6a, and 7a. In some cases, the composition is administered in an amount that is effective to induce a CTL response to HCV genotypes 1a, 1b, 2a, 2b, 3a, 4a, 5a, 6a, and 7a. In some cases, the composition is administered in an amount that is effective to achieve 50% or greater than 50% neutralization of HCV genotypes 1a, 1b, 2a, 2b, 3a, 4a, 5a, 6a, and 7a. In some cases, the composition does not include an E1 polypeptide, an E2 polypeptide, and/or an E1/E2 polypeptide of any HCV genotype other than the above-mentioned genotypes 1a, 2a, and 3a. In some cases, the composition also includes an E1 polypeptide, and E2 polypeptide, and/or an E1/E2 polypeptide of at least one additional HCV genotype. In some cases, the composition also includes an E1 polypeptide, and E2 polypeptide, and/or an E1/E2 polypeptide of HCV genotype 2b. In some cases, the composition also includes an E1 polypeptide, and E2 polypeptide, and/or an E1/E2 polypeptide of HCV genotype 7a.

In some cases, subject method comprises administering to an individual in need thereof an effective amount of a composition comprising: an E2 polypeptide of HCV genotype 1a; an E2 polypeptide of HCV genotype 2a; and an E1/E2 heterodimeric polypeptide complex of HCV genotype 3a. In some cases, all of the polypeptides are wild-type. In other cases, the E2 polypeptide of the E1/E2 heterodimer comprises a modification (e.g., a substitution) of an asparagine at the E2N1 and/or E2N6 site such as described above. In other cases, the E2 polypeptide that is not in a heterodimeric complex with E1 comprises a modification (e.g., a substitution) of an asparagine at the E2N1 and/or E2N6 site such as described above. In some cases, at least one of the E2 polypeptides is full length. In some cases, at least one of the E2 polypeptides is a soluble E2 polypeptide. In some cases, the composition is administered in an amount that is effective to neutralize HCV genotypes prevalent globally. In some cases, the composition is administered in an amount that is effective to neutralize HCV genotypes 1a, 1b, 2a, 2b, 3a, 4a, 5a, 6a, and 7a. In some cases, the composition is administered in an amount that is effective to induce neutralizing antibody to HCV genotypes 1a, 1b, 2a, 2b, 3a, 4a, 5a, 6a, and 7a. In some cases, the composition is administered in an amount that is effective to induce a CTL response to HCV genotypes 1a, 1b, 2a, 2b, 3a, 4a, 5a, 6a, and 7a. In some cases, the composition is administered in an amount that is effective to achieve 50% or greater than 50% neutralization of HCV genotypes 1a, 1b, 2a, 2b, 3a, 4a, 5a, 6a, and 7a. In some cases, the composition does not include an E1 polypeptide, an E2 polypeptide, and/or an E1/E2 polypeptide of any HCV genotype other than the above-mentioned genotypes 1a, 2a, and 3a. In some cases, the composition also includes an E1 polypeptide, and E2 polypeptide, and/or an E1/E2 polypeptide of at least one additional HCV genotype. In some cases, the composition also includes an E1 polypeptide, and E2 polypeptide, and/or an E1/E2 polypeptide of HCV genotype 2b. In some cases, the composition also includes an E1 polypeptide, and E2 polypeptide, and/or an E1/E2 polypeptide of HCV genotype 7a.

In some cases, subject method comprises administering to an individual in need thereof an effective amount of a composition comprising: an E1/E2 heterodimeric polypeptide complex of HCV genotype 1a; an E2 polypeptide of HCV genotype 2a; and an E2 polypeptide of HCV genotype 3a. In some cases, all of the polypeptides are wild-type. In other cases, the E2 polypeptide of the E1/E2 heterodimer comprises a modification (e.g., a substitution) of an asparagine at the E2N1 and/or E2N6 site such as described above. In other cases, the E2 polypeptide that is not in a heterodimeric complex with E1 comprises a modification (e.g., a substitution) of an asparagine at the E2N1 and/or E2N6 site such as described above. In some cases, at least one of the E2 polypeptides is full length. In some cases, at least one of the E2 polypeptides is a soluble E2 polypeptide. In some cases, the composition is administered in an amount that is effective to neutralize HCV genotypes prevalent globally. In some cases, the composition is administered in an amount that is effective to neutralize HCV genotypes 1a, 1b, 2a, 2b, 3a, 4a, 5a, 6a, and 7a. In some cases, the composition is administered in an amount that is effective to induce neutralizing antibody to HCV genotypes 1a, 1b, 2a, 2b, 3a, 4a, 5a, 6a, and 7a. In some cases, the composition is administered in an amount that is effective to induce a CTL response to HCV genotypes 1a, 1b, 2a, 2b, 3a, 4a, 5a, 6a, and 7a. In some cases, the composition is administered in an amount that is effective to achieve 50% or greater than 50% neutralization of HCV genotypes 1a, 1b, 2a, 2b, 3a, 4a, 5a, 6a, and 7a. In some embodiments, the composition does not include an E1 polypeptide, an E2 polypeptide, and/or an E1/E2 polypeptide of any HCV genotype other than the above-mentioned genotypes 1a, 2a, and 3a. In some embodiments, the composition also includes an E1 polypeptide, and E2 polypeptide, and/or an E1/E2 polypeptide of at least one additional HCV genotype. In some embodiments, the composition also includes an E1 polypeptide, and E2 polypeptide, and/or an E1/E2 polypeptide of HCV genotype 2b. In some embodiments, the composition also includes an E1 polypeptide, and E2 polypeptide, and/or an E1/E2 polypeptide of HCV genotype 7a.

In some cases, subject method comprises administering to an individual in need thereof an effective amount of a composition comprising: an E1/E2 heterodimeric polypeptide complex of HCV genotype 1a; an E1/E2 heterodimeric polypeptide complex of HCV genotype 2a; and an E2 polypeptide of HCV genotype 3a. In some cases, all of the polypeptides are wild-type. In other cases, the E2 polypeptide of the E1/E2 heterodimer comprises a modification (e.g., a substitution) of an asparagine at the E2N1 and/or E2N6 site such as described above. In other cases, the E2 polypeptide that is not in a heterodimeric complex with E1 comprises a modification (e.g., a substitution) of an asparagine at the E2N1 and/or E2N6 site such as described above. In some cases, at least one of the E2 polypeptides is full length. In some cases, at least one of the E2 polypeptides is a soluble E2 polypeptide. In some cases, the composition is administered in an amount that is effective to neutralize HCV genotypes prevalent globally. In some cases, the composition is administered in an amount that is effective to neutralize HCV genotypes 1a, 1b, 2a, 2b, 3a, 4a, 5a, 6a, and 7a. In some cases, the composition is administered in an amount that is effective to induce neutralizing antibody to HCV genotypes 1a, 1b, 2a, 2b, 3a, 4a, 5a, 6a, and 7a. In some cases, the composition is administered in an amount that is effective to induce a CTL response to HCV genotypes 1a, 1b, 2a, 2b, 3a, 4a, 5a, 6a, and 7a. In some cases, the composition is administered in an amount that is effective to achieve 50% or greater than 50% neutralization of HCV genotypes 1a, 1b, 2a, 2b, 3a, 4a, 5a, 6a, and 7a. In some embodiments, the composition does not include an E1 polypeptide, an E2 polypeptide, and/or an E1/E2 polypeptide of any HCV genotype other than the above-mentioned genotypes 1a, 2a, and 3a. In some embodiments, the composition also includes an E1 polypeptide, and E2 polypeptide, and/or an E1/E2 polypeptide of at least one additional HCV genotype. In some embodiments, the composition also includes an E1 polypeptide, and E2 polypeptide, and/or an E1/E2 polypeptide of HCV genotype 2b. In some embodiments, the composition also includes an E1 polypeptide, and E2 polypeptide, and/or an E1/E2 polypeptide of HCV genotype 7a.

In some cases, subject method comprises administering to an individual in need thereof an effective amount of a composition comprising: an E2 polypeptide of HCV genotype 1a; an E1/E2 heterodimeric polypeptide complex of HCV genotype 2a; and an E1/E2 heterodimeric polypeptide complex of HCV genotype 3a. In some cases, all of the polypeptides are wild-type. In other cases, the E2 polypeptide of the E1/E2 heterodimer comprises a modification (e.g., a substitution) of an asparagine at the E2N1 and/or E2N6 site such as described above. In other cases, the E2 polypeptide that is not in a heterodimeric complex with E1 comprises a modification (e.g., a substitution) of an asparagine at the E2N1 and/or E2N6 site such as described above. In some cases, at least one of the E2 polypeptides is full length. In some cases, at least one of the E2 polypeptides is a soluble E2 polypeptide. In some cases, the composition is administered in an amount that is effective to neutralize HCV genotypes prevalent globally. In some cases, the composition is administered in an amount that is effective to neutralize HCV genotypes 1a, 1b, 2a, 2b, 3a, 4a, 5a, 6a, and 7a. In some cases, the composition is administered in an amount that is effective to induce neutralizing antibody to HCV genotypes 1a, 1b, 2a, 2b, 3a, 4a, 5a, 6a, and 7a. In some cases, the composition is administered in an amount that is effective to induce a CTL response to HCV genotypes 1a, 1b, 2a, 2b, 3a, 4a, 5a, 6a, and 7a. In some cases, the composition is administered in an amount that is effective to achieve 50% or greater than 50% neutralization of HCV genotypes 1a, 1b, 2a, 2b, 3a, 4a, 5a, 6a, and 7a. In some cases, the composition does not include an E1 polypeptide, an E2 polypeptide, and/or an E1/E2 polypeptide of any HCV genotype other than the above-mentioned genotypes 1a, 2a, and 3a. In some cases, the composition also includes an E1 polypeptide, and E2 polypeptide, and/or an E1/E2 polypeptide of at least one additional HCV genotype. In some cases, the composition also includes an E1 polypeptide, and E2 polypeptide, and/or an E1/E2 polypeptide of HCV genotype 2b. In some cases, the composition also includes an E1 polypeptide, and E2 polypeptide, and/or an E1/E2 polypeptide of HCV genotype 7a.

In some cases, subject method comprises administering to an individual in need thereof an effective amount of a composition comprising: an E1/E2 heterodimeric polypeptide complex of HCV genotype 1a; an E2 polypeptide of HCV genotype 2a; and an E1/E2 heterodimeric polypeptide complex of HCV genotype 3a. In some cases, all of the polypeptides are wild-type. In other cases, the E2 polypeptide of the E1/E2 heterodimer comprises a modification (e.g., a substitution) of an asparagine at the E2N1 and/or E2N6 site such as described above. In other cases, the E2 polypeptide that is not in a heterodimeric complex with E1 comprises a modification (e.g., a substitution) of an asparagine at the E2N1 and/or E2N6 site such as described above. In some cases, at least one of the E2 polypeptides is full length. In some cases, at least one of the E2 polypeptides is a soluble E2 polypeptide. In some cases, the composition is administered in an amount that is effective to neutralize HCV genotypes prevalent globally. In some cases, the composition is administered in an amount that is effective to neutralize HCV genotypes 1a, 1b, 2a, 2b, 3a, 4a, 5a, 6a, and 7a. In some cases, the composition is administered in an amount that is effective to induce neutralizing antibody to HCV genotypes 1a, 1b, 2a, 2b, 3a, 4a, 5a, 6a, and 7a. In some cases, the composition is administered in an amount that is effective to induce a CTL response to HCV genotypes 1a, 1b, 2a, 2b, 3a, 4a, 5a, 6a, and 7a. In some cases, the composition is administered in an amount that is effective to achieve 50% or greater than 50% neutralization of HCV genotypes 1a, 1b, 2a, 2b, 3a, 4a, 5a, 6a, and 7a. In some cases, the composition does not include an E1 polypeptide, an E2 polypeptide, and/or an E1/E2 polypeptide of any HCV genotype other than the above-mentioned genotypes 1a, 2a, and 3a. In some cases, the composition also includes an E1 polypeptide, and E2 polypeptide, and/or an E1/E2 polypeptide of at least one additional HCV genotype. In some cases, the composition also includes an E1 polypeptide, and E2 polypeptide, and/or an E1/E2 polypeptide of HCV genotype 2b. In some cases, the composition also includes an E1 polypeptide, and E2 polypeptide, and/or an E1/E2 polypeptide of HCV genotype 7a.

### INDIVIDUALS SUITABLE FOR ADMINISTRATION

Individuals who are suitable for administration with an HCV composition of the present disclosure include immunologically naive individuals (e.g., individuals who have not been infected with HCV and/or who have not been administered with an HCV vaccine).

Individuals who are suitable for administration with an HCV composition of the present disclosure include individuals who are at greater risk than the general population of becoming infected with HCV, where such individuals include, e.g., intravenous drug users; individuals who are the recipients, or the prospective recipients, of blood or blood products from another (donor) individual(s); individuals who are the recipients, or the prospective recipients, of non-autologous cells, tissues, or organs from another (donor) individual; health care workers; emergency medical and non-medical personnel (e.g., first responders; fire fighters; emergency medical team personnel; etc.) and the like.

Individuals who are suitable for administration with an HCV composition of the present disclosure include individuals who recently became exposed to HCV or who recently became infected with HCV. For example, a subject immunogenic composition can be administered to an individual within from about 24 hours to about 48 hours, from about 48 hours to about 1 week, or from about 1 week to about 4 weeks, following possible or suspected exposure to HCV or following infection with HCV.

Individuals who are suitable for administration with an HCV composition of the present disclosure include individuals who have been diagnosed as having an HCV infection, and include chronically infected individuals. In some cases, an individual who has been diagnosed as having an HCV infection is treated with an anti-viral agent and a subject HCV immunogenic composition. Suitable anti-viral agents for treating HCV infection include, e.g., ribavirin (1-β-D-ribofuranosyl-1H-1,2,4-triazole-3-carboxamide); interferon-alpha (IFN-α) (where "IFN-α" includes IFN-α2a; IFN-α2b; IFN-α that is conjugated with poly(ethylene glycol) ("pegylated IFN-α), where the pegylated IFN-α can be pegylated IFN-α2a or pegylated IFN-α 2b); an HCV NS3 protease inhibitor (e.g., boceprevir; telaprevir); and an HCV NS5 protease inhibitor. In some cases, an individual who has been diagnosed as having an HCV infection is treated with, e.g.: 1) IFN-α + ribavirin; and a subject HCV immunogenic composition; or 2) IFN-α + ribavirin + an HCV protease inhibitor (e.g., boceprevir or telaprevir); and a subject HCV immunogenic composition. As one non-limiting example, and a subject HCV immunogenic composition is administered to an individual who has been diagnosed as having an HCV infection once a month for 6 months.

### EXAMPLES

The following examples are put forth so as to provide those of ordinary skill in the art with a complete disclosure and description of how to make and use the present invention, and are not intended to limit the scope of what the inventors regard as their invention nor are they intended to represent that the experiments below are all or the only experiments performed. Efforts have been made to ensure accuracy with respect to numbers used (e.g. amounts, temperature, etc.) but some experimental errors and deviations should be accounted for. Unless indicated otherwise, parts are parts by weight, molecular weight is weight average molecular weight, temperature is in degrees Celsius, and pressure is at or near atmospheric. Standard abbreviations may be used, e.g., bp, base pair(s); kb, kilobase(s); pl, picoliter(s); s or sec, second(s); min, minute(s); h or hr, hour(s); aa, amino acid(s); kb, kilobase(s); bp, base pair(s); nt, nucleotide(s); i.m., intramuscular(ly); i.p., intraperitoneal(ly); s.c., subcutaneous(ly); and the like.

### Example 1: Genotype-specific neutralization

### MATERIALS AND METHODS

### Goat Immunization

Goats (G714 and G757) and Goats (G766 and G773) were immunized with HCV1 (Genotype la)-derived E1E2 and with J6 (genotype 2a)-derived E2, respectively. Recombinant E1E2 and soluble E2 proteins were derived from the sequence of an HCV genotype 1a strain and HCV genotype 2a strain respectively. The recombinant E1E2 proteins were purified from Chinese hamster ovary cell line constitutively expressing the glycoproteins; and the E2 protein was purified from culture medium of 293T cells expressing the E2 glycoprotein. 0.5ml experimental vaccine, which was composed of 10 µg of purified protein antigen (E2E1 or E2) along with either squalene-based Addavax or Freund's adjuvant were administered to goat. Post-vaccinated serum used in this data was collected after four vaccinations at 129 days post-immunization. Serum collected prior to vaccination was used as a negative control.

### In vitro neutralization assay

Post-vaccinated goat sera were tested for neutralization activity against chimeric genotype 1a H77c/JFH1 or genotype 2a J6/JFH1 HCV produced in cell culture (HCVcc) *in vitro.*

1x 10⁴ Huh-7.5 cells per well were seeded in a multi-well plate one day prior to infection. For infection, 300 TCID50 (median tissue culture infectious dose) HCVcc were premixed with heat-inactivated goat sera diluted at 1 in 50 (by volume), for 1 hour at 37 °C. The pre-mixed HCVcc/goat sera were then added to cells. 12 hour post-infection, the antibody-virus inoculum was replaced with fresh culture media. Cells were then fixed 48 hours post-infection with 2% paraformaldehyde. The amount of infection was quantitated by flow cytometry, counting the number of NS5A-positive cells detected using mouse monoclonal NS5A antibody (9E10). The percentage of neutralization was reported by comparison with pre-vaccination serum.

### RESULTS

The neutralization activity of post-vaccinated goat serum was tested against tissue culture-derived chimeric genotype 1a (H77c/JFH1) or 2a (J6/JFH1) using an *in vitro* neutralization assay. Goats (G714 and G757) were immunized with recombinant E1E2 derived from sequence of genotype 1a (G1a), whereas goats (G766 and G773) were immunized with E2 derived from sequence of genotype 2a (G2a). The G1a antigen induced strong neutralization activity against G1a chimeric virus H77/JFH, but showed a reduced efficiency against G2a virus J6/JFH (Figure 4). On the other hand, the E2 antigen derived from G2a induced more effective neutralizing antibodies against G2a virus than G1a virus. Together, the data indicate the need for an antigen cocktail to confer cross-genotype protection in a global vaccine.

### Example 2: Genotype-specific neutralization

### MATERIALS AND METHODS

### Goat Immunization

Goats (G757, G714) were immunized with HCV1 (Genotype la)-derived E1E2; Goats (G004, G752) were immunized with HCV1 (Genotype 1a)-derived ectodomain of E1; Goats (G786, G799) were immunized with HCV1 (Genotype 1a)-derived ectodomain of E2; Goats (G766, G733) were immunized with J6 (Genotype 2a)-derived ectodomain of E2, respectively. E1E2 proteins, ectodomain of E1 and E2 were derived from the sequence of an HCV genotype 1a (H77) strain and HCV genotype 2a (J6) strain, respectively. The recombinant antigens were purified from Chinese hamster ovary cell line constitutively expressing the glycoproteins. 0.5ml experimental vaccine, which was composed of 10 µg of purified protein antigen (E1E2) or the molar equivalent of E1 or E2 along with either squalene-based AddaVax or Incomplete Freund's Adjuvant (IFA) were administered to goat. Post-vaccinated serum used in this data was collected after four vaccinations at 129 days post-immunization. Serum collected prior to vaccination was used as a negative control. Ectodomain of E1 and ectodomain of E2 are soluble E1 and E2 polypeptides with the C-terminal transmembrane regions removed.

### In vitro neutralization assay

Post-vaccinated goat sera were tested for neutralization activity against chimeric genotype 1a H77c/JFH1 or genotype 2a J6/JFH1 HCV produced in cell culture (HCVcc) *in vitro.*

1x 10⁴ Huh-7.5 cells per well were seeded in a multi-well plate one day prior to infection. For infection, 300 TCID50 (median tissue culture infectious dose) HCVcc were premixed with heat-inactivated goat sera diluted at 1 in 50 (by volume), for 1 hour at 37 °C. The pre-mixed HCVcc/goat sera were then added to cells. 12 hour post-infection, the antibody-virus inoculum was replaced with fresh culture media. Cells were then fixed 48 hours post-infection with 2% paraformaldehyde. The amount of infection was quantitated by flow cytometry, counting the number of NS5A-positive cells detected using mouse monoclonal NS5A antibody (9E10). The percentage of neutralization was reported by comparison with pre-vaccination serum.

### RESULTS

Cross neutralization of vaccine-induced antibody was tested. Sera diluted 1:50 were pre-incubated with either tissue culture-derived HCV of H77/JFH1 (G1a) or J6/JFH (G2a), then used to infect Huh-7.5 cells. The level of infection was monitored by NS5a staining using flow cytometry. The level of neutralization activity was normalized with one set of pre-vaccinated control to 0%. A second set of pre-vaccination sera was used to show the variation of the assay.

The results are shown in Figures 5 and 6. In Figure 5, the ability of goat sera to neutralize HCV genotype 1a (HCV of H77/JFH1 as the "challenge virus") is shown. In Figure 6, the ability of goat sera to neutralize HCV genotype 2a (HCV of J6/JFH as the "challenge virus") is shown.

**Figure 5****.** The sera of goats 757, 714, 786, and 799 (immunized with HCV1 (G1a) derived antigen) showed induction of neutralizing antibodies to block G1a infection; however, the sera of goats 766 and 773 (immunized with J6 (G2a derived antigen) had limited effectiveness in blocking infection of G1a (genotype 1a) virus.

**Figure 6****.** The sera of goats 766 and 733 (immunized with antigen derived from genotype 2a) exhibited greater blocking of infection of G2a (genotype 2a) virus than of G1a virus. Sera from Goats (757, 714, 004, 752,786, 799), immunized with antigen derived from genotype 1a, have limited neutralization activity against G2a virus. Example 3: **Cross-neutralization**

Goats were immunized with E1/E2 heterodimeric complex from HCV strain HCV1 (genotype 1a) or with soluble E2 (sE2) from HCV strain J6 (genotype 2a). Post-vaccinated goat sera were tested for neutralization activity against HCV genotype 1a, 1b, 2a, 2b, 3a, 4a, 5a, and 6a. The data are shown in Figure 7A and 7B. The data presented in Figure 7a show that immunization with E1/E2 from HCV genotype 1a elicited neutralizing antibodies against HCV of all genotypes tested except 2a, 2b, and 3a; and Figure 7b show that immunization with sE2 from HCV genotype 2a elicited neutralizing antibodies against HCV of genotypes 1b, 2a, 4a, and 6a.

## Claims

1. An immunogenic composition for use in a method of inducing an immune response in an individual, the composition comprising:
a) a hepatitis C virus (HCV) E1/E2 heterodimeric polypeptide of a first HCV genotype, wherein the first HCV genotype is genotype 1, wherein the E1 polypeptide comprises an amino acid sequence having at least 75% amino acid sequence identity to any one of the E1 amino acid sequences depicted in SEQ ID NO: 1-27 of FIG. 1A-1C, and wherein the E2 polypeptide comprises an amino acid sequence having at least 80% amino acid sequence identity to any one of the E2 amino acid sequences depicted in SEQ ID NO: 1-27 of FIG. 1A-1C;
b) an HCV E1 polypeptide, E2 polypeptide or E1/E2 heterodimeric polypeptide of a second HCV genotype, wherein the second HCV genotype is genotype 2, wherein the E1 polypeptide comprises an amino acid sequence having at least 70% amino acid sequence identity to any one of the E1 amino acid sequences depicted in SEQ ID NO: 41-53 of FIG. 9A-9C, and wherein the E2 polypeptide comprises an amino acid sequence having at least 75% amino acid sequence identity to any one of the E2 amino acid sequences depicted in SEQ ID NO: 41-53 of FIG. 9A-9C;
c) an HCV E1 polypeptide, E2 polypeptide or E1/E2 heterodimeric polypeptide of a third HCV genotype, wherein the third HCV genotype is genotype 3, wherein the E1 polypeptide comprises an amino acid sequence having at least 75% amino acid sequence identity to any one of the E1 amino acid sequences depicted in SEQ ID NO:28-31 of FIG. 2A-2B, and wherein the E2 polypeptide comprises an amino acid sequence having at least 75% amino acid sequence identity to any one of the E2 amino acid sequences depicted in SEQ ID NO: 28-37 of FIG. 2A-2B; and
d) a pharmaceutically acceptable excipient,
wherein the composition does not include an HCV E1/E2 polypeptide, and E1 polypeptide, or an E2 polypeptide of any other genotype other than HCV genotype 1, HCV genotype 2 and HCV genotype 3; and
wherein the E1 polypeptide has a length of from about 150 amino acids to about 193 amino acids, and wherein the E2 polypeptide has a length of from about 200 amino acids to about 365 amino acids;
wherein the immune response:
i) induces neutralizing antibody to HCV genotypes 1, 2, 3, 4, 5 and 6; or
ii) induces a cytotoxic T lymphocyte response to HCV genotypes 1, 2, 3, 4, 5 and 6.

2. The immunogenic composition for use according to claim 1, wherein the composition comprises:
i) an E1/E2 heterodimeric polypeptide complex of HCV genotype 1, an E1/E2 heterodimeric polypeptide complex of HCV genotype 2 and an E1/E2 heterodimeric polypeptide complex of HCV genotype 3;
ii) an E1/E2 heterodimeric polypeptide complex of HCV genotype 1, an E1/E2 heterodimeric polypeptide complex of HCV genotype 2 and an E2 polypeptide of HCV genotype 3;
iii) an E1/E2 heterodimeric polypeptide complex of HCV genotype 1, an E2 polypeptide of HCV genotype 2, and an E1/E2 heterodimeric polypeptide complex of HCV genotype 3;
iv) an E1/E2 heterodimeric polypeptide complex of HCV genotype 1, an E2 polypeptide of HCV genotype 2 and an E2 polypeptide of HCV genotype 3;
v) an E1/E2 heterodimeric polypeptide complex of HCV genotype 1, an E1 polypeptide of HCV genotype 2 and an E1/E2 heterodimeric polypeptide complex of HCV genotype 3;
vi) an E1/E2 heterodimeric polypeptide complex of HCV genotype 1, an E1/E2 heterodimeric polypeptide complex of HCV genotype 2 and an E1 polypeptide of HCV genotype 3; or
vii) an E1/E2 heterodimeric polypeptide complex of HCV genotype 1, an E2 polypeptide of HCV genotype 2 and an E1 polypeptide of HCV genotype 3.

3. The immunogenic composition for use according to claim 1, wherein the composition comprises an E1/E2 heterodimeric polypeptide complex of HCV genotype 1, an E1/E2 heterodimeric polypeptide complex of HCV genotype 2 and an E1/E2 heterodimeric polypeptide complex of HCV genotype 3.

4. The immunogenic composition for use according to claim 2, wherein the composition comprises an E1/E2 heterodimeric polypeptide of HCV genotype 1, a soluble E2 polypeptide of HCV genotype 2 and an E2 polypeptide of HCV genotype 3.

5. The composition for use according to any one of claims 1 to 4 wherein an E2 polypeptide has reduced N-linked glycosylation compared to naturally-occurring glycosylated E2 polypeptide.

6. The immunogenic composition for use according to any one of claims 1 to 5, wherein the E2 polypeptide is a soluble E2 polypeptide.

7. The immunogenic composition for use according to any one of claims 1 to 6, wherein the pharmaceutically acceptable excipient comprises an adjuvant, optionally wherein the adjuvant is MF59, alum, poly(DL-lactide co-glycolide), monophosphoryl lipid A or a CpG oligonucleotide.

8. The immunogenic composition for use according to any one of claims 1 to 7, wherein:
a) the genotype 1 E1 polypeptide comprises an amino acid sequence having at least 80% amino acid sequence identity to any one of the E1 amino acid sequences depicted in SEQ ID NO: 1-27 of FIG. 1A-1C, and wherein the genotype 1 E2 polypeptide comprises an amino acid sequence having at least 80% amino acid sequence identity to any one of the E2 amino acid sequences depicted in SEQ ID NO: 1-27 of FIG. 1A-1C;
b) the genotype 2 E1 polypeptide comprises an amino acid sequence having at least 80% amino acid sequence identity to any one of the E1 amino acid sequences depicted in SEQ ID NO: 1-27 of FIG. 9A-9C, and wherein the genotype 2 E2 polypeptide comprises an amino acid sequence having at least 80% amino acid sequence identity to any one of the E2 amino acid sequences depicted in SEQ ID NO: 1-27 of FIG. 9A-9C; and
c) the genotype 3 E1 polypeptide comprises an amino acid sequence having at least 80% amino acid sequence identity to any one of the E1 amino acid sequences depicted in SEQ ID NO:28-31 of FIG. 2A-2B, and wherein the genotype 3 E2 polypeptide comprises an amino acid sequence having at least 80% amino acid sequence identity to any one of the E2 amino acid sequences depicted in SEQ ID NO:28-31 of FIG. 2A-2B.

9. The immunogenic composition for use according to any one of claims 1 to 7, wherein:
a) the genotype 1 E1 polypeptide comprises an amino acid sequence having at least 90% amino acid sequence identity to any one of the E1 amino acid sequences depicted in SEQ ID NO: 1-27 of FIG. 1A-1C, and wherein the genotype 1 E2 polypeptide comprises an amino acid sequence having at least 90% amino acid sequence identity to any one of the E2 amino acid sequences depicted in SEQ ID NO: 1-27 of FIG. 1A-1C;
b) the genotype 2 E1 polypeptide comprises an amino acid sequence having at least 90% amino acid sequence identity to any one of the E1 amino acid sequences depicted in SEQ ID NO: 1-27 of FIG. 9A-9C, and wherein the genotype 2 E2 polypeptide comprises an amino acid sequence having at least 90% amino acid sequence identity to any one of the E2 amino acid sequences depicted in SEQ ID NO: 1-27 of FIG. 9A-9C; and
c) the genotype 3 E1 polypeptide comprises an amino acid sequence having at least 90% amino acid sequence identity to any one of the E1 amino acid sequences depicted in SEQ ID NO:28-31 of FIG. 2A-2B, and wherein the genotype 3 E2 polypeptide comprises an amino acid sequence having at least 90% amino acid sequence identity to any one of the E2 amino acid sequences depicted in SEQ ID NO:28-31 of FIG. 2A-2B.

## Patentansprüche

1. Immunogene Zusammensetzung zur Verwendung in einem Verfahren zum Hervorrufen einer Immunantwort in einem Individuum, wobei die Zusammensetzung Folgendes umfasst:
a) ein heterodimeres Hepatitis-C-Virus- (HCV-) E1/E2-Polypeptid eines ersten HCV-Genotyps, wobei der erste HCV-Genotyp Genotyp 1 ist, wobei das E1-Polypeptid eine Aminosäuresequenz mit zumindest 75 % Aminosäuresequenzidentität mit einer beliebigen der E1-Aminosäuresequenzen, die in SEQ ID NO: 1-27 der FIG. 1A-1C dargestellt sind, umfasst und wobei das E2-Polypeptid eine Aminosäuresequenz mit zumindest 80 % Aminosäuresequenzidentität mit einer beliebigen der E2-Aminosäuresequenzen, die in SEQ ID NO: 1-27 der FIG. 1A-1C dargestellt sind, umfasst;
b) ein HCV-E1-Polypeptid, -E2-Polypeptid oder heterodimeres -E1/E2-Polypeptid eines zweiten HCV-Genotyps, wobei der zweite HCV-Genotyp Genotyp 2 ist, wobei das E1-Polypeptid eine Aminosäuresequenz mit zumindest 70 % Aminosäuresequenzidentität mit einer beliebigen der E1-Aminosäuresequenzen, die in SEQ ID NO: 41-53 der FIG. 9A-9C dargestellt sind, umfasst und wobei das E2-Polypeptid eine Aminosäuresequenz mit zumindest 75 % Aminosäuresequenzidentität mit einer beliebigen der E2-Aminosäuresequenzen, die in SEQ ID NO: 41-53 der FIG. 9A-9C dargestellt sind, umfasst;
c) ein HCV-E1-Polypeptid, -E2-Polypeptid oder heterodimeres -E1/E2-Polypeptid eines dritten HCV-Genotyps, wobei der dritte HCV-Genotyp Genotyp 3 ist, wobei das E1-Polypeptid eine Aminosäuresequenz mit zumindest 75 % Aminosäuresequenzidentität mit einer beliebigen der E1-Aminosäuresequenzen, die in SEQ ID NO: 28-31 der FIG. 2A-2B dargestellt sind, umfasst und wobei das E2-Polypeptid eine Aminosäuresequenz mit zumindest 75 % Aminosäuresequenzidentität mit einer beliebigen der E2-Aminosäuresequenzen, die in SEQ ID NO: 28-37 der FIG. 2A-2B dargestellt sind, umfasst; und
d) einen pharmazeutisch annehmbaren Exzipienten,
wobei die Zusammensetzung kein HCV-E1/E2-Polypeptid und -E1-Polypeptid oder-E2-Polypeptid umfasst, die einen anderen Genotyp als HCV-Genotyp 1, HCV-Genotyp 2 und HVC-Genotyp 3 aufweisen; und
wobei das E1-Polypeptid eine Länge von etwa 150 Aminosäuren bis etwa 193 Aminosäuren aufweist und wobei das E2-Polypeptid eine Länge von etwa 200 Aminosäuren bis etwa 365 Aminosäuren aufweist;
wobei die Immunantwort:
i) neutralisierende Antikörper gegen die HCV-Genotypen 1, 2, 3, 4, 5 und 6 hervorruft; oder
ii) eine zytotoxische T-Lymphozyten-Antwort gegen die HCV-Genotypen 1, 2, 3, 4, 5 und 6 hervorruft.

2. Immunogene Zusammensetzung zur Verwendung nach Anspruch 1, wobei die Zusammensetzung Folgendes umfasst:
i) einen heterodimeren E1/E2-Polypeptidkomplex des HCV-Genotyps 1, einen heterodimeren E1/E2-Polypeptidkomplex des HCV-Genotyps 2 und einen heterodimeren E1/E2-Polypeptidkomplex des HCV-Genotyps 3;
ii) einen heterodimeren E1/E2-Polypeptidkomplex des HCV-Genotyps 1, einen heterodimeren E1/E2-Polypeptidkomplex des HCV-Genotyps 2 und ein E2-Polypeptid des HCV-Genotyps 3;
iii) einen heterodimeren E1/E2-Polypeptidkomplex des HCV-Genotyps 1, ein E2-Polypeptid des HCV-Genotyps 2 und einen heterodimeren E1/E2-Polypeptidkomplex des HCV-Genotyps 3;
iv) einen heterodimeren E1/E2-Polypeptidkomplex des HCV-Genotyps 1, ein E2-Polypeptid des HCV-Genotyps 2 und ein E2-Polypeptid des HCV-Genotyps 3;
v) einen heterodimeren E1/E2-Polypeptidkomplex des HCV-Genotyps 1, ein E1-Polypeptid des HCV-Genotyps 2 und einen heterodimeren E1/E2-Polypeptidkomplex des HCV-Genotyps 3;
vi) einen heterodimeren E1/E2-Polypeptidkomplex des HCV-Genotyps 1, einen heterodimeren E1/E2-Polypeptidkomplex des HCV-Genotyps 2 und ein E1-Polypeptid des HCV-Genotyps 3; oder
vii) einen heterodimeren E1/E2-Polypeptidkomplex des HCV-Genotyps 1, ein E2-Polypeptid des HCV-Genotyps 2 und ein E1-Polypeptid des HCV-Genotyps 3.

3. Immunogene Zusammensetzung zur Verwendung nach Anspruch 1, wobei die Zusammensetzung einen heterodimeren E1/E2-Polypeptidkomplex des HCV-Genotyps 1, einen heterodimeren E1/E2-Polypeptidkomplex des HCV-Genotyps 2 und einen heterodimeren E1/E2-Polypeptidkomplex des HCV-Genotyps 3 umfasst.

4. Immunogene Zusammensetzung zur Verwendung nach Anspruch 2, wobei die Zusammensetzung ein heterodimeres E1/E2-Polypeptid des HCV-Genotyps 1, ein lösliches E2-Polypeptid des HCV-Genotyps 2 und ein E2-Polypeptid des HCV-Genotyps 3 umfasst.

5. Zusammensetzung zur Verwendung nach einem der Ansprüche 1 bis 4, wobei ein E2-Polypeptid im Vergleich mit einem natürlich vorkommenden glykosylierten E2-Polypeptid eine verringerte N-gebundene Glykosylierung aufweist.

6. Immunogene Zusammensetzung zur Verwendung nach einem der Ansprüche 1 bis 5, wobei das E2-Polypeptid ein lösliches E2-Polypeptid ist.

7. Immunogene Zusammensetzung zur Verwendung nach einem der Ansprüche 1 bis 6, wobei der pharmazeutisch annehmbare Exzipient ein Adjuvans umfasst, gegebenenfalls wobei das Adjuvans MF59, Alaun, Poly(DL-Lactid-co-glykolid), Monophosphoryl-Lipid-A oder ein CpG-Oligonucleotid ist.

8. Immunogene Zusammensetzung zur Verwendung nach einem der Ansprüche 1 bis 7, wobei:
a) das E1-Polypeptid mit Genotyp 1 eine Aminosäuresequenz mit zumindest 80 % Aminosäuresequenzidentität mit einer beliebigen der in SEQ ID NO: 1-27 der FIG. 1A-1C dargestellten E1-Aminosäuresequenzen umfasst und wobei das E2-Polypeptid mit Genotyp 1 eine Aminosäuresequenz mit zumindest 80 % Aminosäuresequenzidentität mit einer beliebigen der in SEQ ID NO: 1-27 der FIG. 1A-1C dargestellten E2-Aminosäuresequenzen umfasst;
b) das E1-Polypeptid mit Genotyp 2 eine Aminosäuresequenz mit zumindest 80 % Aminosäuresequenzidentität mit einer beliebigen der in SEQ ID NO: 1-27 der FIG. 9A-9C dargestellten E1-Aminosäuresequenzen umfasst und wobei das E2-Polypeptid mit Genotyp 2 eine Aminosäuresequenz mit zumindest 80 % Aminosäuresequenzidentität mit einer beliebigen der in SEQ ID NO: 1-27 der FIG. 9A-9C dargestellten E2-Aminosäuresequenzen umfasst; und
c) das E1-Polypeptid mit Genotyp 3 eine Aminosäuresequenz mit zumindest 80 % Aminosäuresequenzidentität mit einer beliebigen der in SEQ ID NO: 28-31 der FIG. 2A-2B dargestellten E1-Aminosäuresequenzen umfasst und wobei das E2-Polypeptid mit Genotyp 3 eine Aminosäuresequenz mit zumindest 80 % Aminosäuresequenzidentität mit einer beliebigen der in SEQ ID NO: 28-31 der FIG. 2A-2B dargestellten E2-Aminosäuresequenzen umfasst.

9. Immunogene Zusammensetzung zur Verwendung nach einem der Ansprüche 1 bis 7, wobei:
a) das E1-Polypeptid mit Genotyp 1 eine Aminosäuresequenz mit zumindest 90 % Aminosäuresequenzidentität mit einer beliebigen der in SEQ ID NO: 1-27 der FIG. 1A-1C dargestellten E1-Aminosäuresequenzen umfasst und wobei das E2-Polypeptid mit Genotyp 1 eine Aminosäuresequenz mit zumindest 90 % Aminosäuresequenzidentität mit einer beliebigen der in SEQ ID NO: 1-27 der FIG. 1A-1C dargestellten E2-Aminosäuresequenzen umfasst;
b) das E1-Polypeptid mit Genotyp 2 eine Aminosäuresequenz mit zumindest 90 % Aminosäuresequenzidentität mit einer beliebigen der in SEQ ID NO: 1-27 der FIG. 9A-9C dargestellten E1-Aminosäuresequenzen umfasst und wobei das E2-Polypeptid mit Genotyp 2 eine Aminosäuresequenz mit zumindest 90 % Aminosäuresequenzidentität mit einer beliebigen der in SEQ ID NO: 1-27 der FIG. 9A-9C dargestellten E2-Aminosäuresequenzen umfasst; und
c) das E1-Polypeptid mit Genotyp 3 eine Aminosäuresequenz mit zumindest 90 % Aminosäuresequenzidentität mit einer beliebigen der in SEQ ID NO: 28-31 der FIG. 2A-2B dargestellten E1-Aminosäuresequenzen umfasst und wobei das E2-Polypeptid mit Genotyp 3 eine Aminosäuresequenz mit zumindest 90 % Aminosäuresequenzidentität mit einer beliebigen der in SEQ ID NO: 28-31 der FIG. 2A-2B dargestellten E2-Aminosäuresequenzen umfasst.

## Revendications

1. Composition immunogène destinée à être utilisée dans un procédé d'induction d'une réponse immunitaire chez un individu, la composition comprenant :
a) un polypeptide hétérodimérique E1/E2 du virus de l'hépatite C (VHC) d'un premier génotype du VHC, où le premier génotype du VHC est le génotype 1, où le polypeptide E1 comprend une séquence d'acides aminés présentant au moins 75 % d'identité de séquence d'acides aminés avec l'une quelconque des séquences d'acides aminés de E1 représentées dans SEQ ID NO: 1-27 des FIG. 1A-1C, et où le polypeptide E2 comprend une séquence d'acides aminés présentant au moins 80 % d'identité de séquence d'acides aminés avec l'une quelconque des séquences d'acides aminés de E2 représentées dans SEQ ID NO: 1-27 des FIG. 1A-1C ;
b) un polypeptide El, un polypeptide E2 ou un polypeptide hétérodimérique E1/E2 du VHC d'un deuxième génotype du VHC, où le deuxième génotype du VHC est le génotype 2, où le polypeptide E1 comprend une séquence d'acides aminés présentant au moins 70 % d'identité de séquence d'acides aminés avec l'une quelconque des séquences d'acides aminés de E1 représentées dans SEQ ID NO: 41-53 des FIG. 9A-9C, et où le polypeptide E2 comprend une séquence d'acides aminés présentant au moins 75 % d'identité de séquence d'acides aminés avec l'une quelconque des séquences d'acides aminés de E2 représentées dans SEQ ID NO: 41-53 des FIG. 9A-9C ;
c) un polypeptide El, un polypeptide E2 ou un polypeptide hétérodimérique E1/E2 du VHC d'un troisième génotype du VHC, où le troisième génotype du VHC est le génotype 3, où le polypeptide E1 comprend une séquence d'acides aminés présentant au moins 75 % d'identité de séquence d'acides aminés avec l'une quelconque des séquences d'acides aminés de E1 représentées dans SEQ ID NO: 28-31 des FIG. 2A-2B, et où le polypeptide E2 comprend une séquence d'acides aminés présentant au moins 75 % d'identité de séquence d'acides aminés avec l'une quelconque des séquences d'acides aminés de E2 représentées dans SEQ ID NO: 28-37 des FIG. 2A-2B ; et
d) un excipient pharmaceutiquement acceptable,
où la composition n'inclut pas de polypeptide E1/E2 du VHC, et de polypeptide El, ou de polypeptide E2 d'un quelconque autre génotype autre que le génotype 1 du VHC, le génotype 2 du VHC et le génotype 3 du VHC ; et
où le polypeptide E1 possède une longueur comprise entre environ 150 acides aminés et environ 193 acides aminés, et où le polypeptide E2 possède une longueur comprise entre environ 200 acides aminés et environ 365 acides aminés ;
où la réponse immunitaire :
i) induit un anticorps neutralisant contre les génotypes 1, 2, 3, 4, 5 et 6 du VHC ; ou
ii) induit une réponse de lymphocytes T cytotoxiques contre les génotypes 1, 2, 3, 4, 5 et 6 du VHC.

2. Composition immunogène destinée à être utilisée selon la revendication 1, où la composition comprend :
i) un complexe de polypeptide hétérodimérique E1/E2 du génotype 1 du VHC, un complexe de polypeptide hétérodimérique E1/E2 du génotype 2 du VHC et un complexe de polypeptide hétérodimérique E1/E2 du génotype 3 du VHC ;
ii) un complexe de polypeptide hétérodimérique E1/E2 du génotype 1 du VHC, un complexe de polypeptide hétérodimérique E1/E2 du génotype 2 du VHC et un polypeptide E2 du génotype 3 du VHC ;
iii) un complexe de polypeptide hétérodimérique E1/E2 du génotype 1 du VHC, un polypeptide E2 du génotype 2 du VHC, et un complexe de polypeptide hétérodimérique E1/E2 du génotype 3 du VHC ;
iv) un complexe de polypeptide hétérodimérique E1/E2 du génotype 1 du VHC, un polypeptide E2 du génotype 2 du VHC et un polypeptide E2 du génotype 3 du VHC ;
v) un complexe de polypeptide hétérodimérique E1/E2 du génotype 1 du VHC, un polypeptide E1 du génotype 2 du VHC et un complexe de polypeptide hétérodimérique E1/E2 du génotype 3 du VHC ;
vi) un complexe de polypeptide hétérodimérique E1/E2 du génotype 1 du VHC, un complexe de polypeptide hétérodimérique E1/E2 du génotype 2 du VHC et un polypeptide E1 du génotype 3 du VHC ; ou
vii) un complexe de polypeptide hétérodimérique E1/E2 du génotype 1 du VHC, un polypeptide E2 du génotype 2 du VHC et un polypeptide E1 du génotype 3 du VHC.

3. Composition immunogène destinée à être utilisée selon la revendication 1, où la composition comprend un complexe de polypeptide hétérodimérique E1/E2 du génotype 1 du VHC, un complexe de polypeptide hétérodimérique E1/E2 du génotype 2 du VHC et un complexe de polypeptide hétérodimérique E1/E2 du génotype 3 du VHC.

4. Composition immunogène destinée à être utilisée selon la revendication 2, où la composition comprend un polypeptide hétérodimérique E1/E2 du génotype 1 du VHC, un polypeptide E2 soluble du génotype 2 du VHC et un polypeptide E2 du génotype 3 du VHC.

5. Composition destinée à être utilisée selon l'une quelconque des revendications 1 à 4, dans laquelle un polypeptide E2 possède une N-glycosylation réduite par comparaison à un polypeptide E2 glycosylé survenant à l'état naturel.

6. Composition immunogène destinée à être utilisée selon l'une quelconque des revendications 1 à 5, dans laquelle le polypeptide E2 est un polypeptide E2 soluble.

7. Composition immunogène destinée à être utilisée selon l'une quelconque des revendications 1 à 6, dans laquelle l'excipient pharmaceutiquement acceptable comprend un adjuvant, facultativement dans laquelle l'adjuvant est le MF59, l'alun, le poly(DL-lactide-co-glycolide), le monophosphoryle lipide A ou un oligonucléotide CpG.

8. Composition immunogène destinée à être utilisée selon l'une quelconque des revendications 1 à 7, dans laquelle :
a) le polypeptide E1 du génotype 1 comprend une séquence d'acides aminés présentant au moins 80 % d'identité de séquence d'acides aminés avec l'une quelconque des séquences d'acides aminés de E1 représentées dans SEQ ID NO: 1-27 des FIG. 1A-1C, et dans laquelle le polypeptide E2 du génotype 1 comprend une séquence d'acides aminés présentant au moins 80 % d'identité de séquence d'acides aminés avec l'une quelconque des séquences d'acides aminés de E2 représentées dans SEQ ID NO: 1-27 des FIG. 1A-1C ;
b) le polypeptide E1 du génotype 2 comprend une séquence d'acides aminés présentant au moins 80 % d'identité de séquence d'acides aminés avec l'une quelconque des séquences d'acides aminés de E1 représentées dans SEQ ID NO: 1-27 des FIG. 9A-9C, et dans laquelle le polypeptide E2 du génotype 2 comprend une séquence d'acides aminés présentant au moins 80 % d'identité de séquence d'acides aminés avec l'une quelconque des séquences d'acides aminés de E2 représentées dans SEQ ID NO: 1-27 des FIG. 9A-9C ; et
c) le polypeptide E1 du génotype 3 comprend une séquence d'acides aminés présentant au moins 80 % d'identité de séquence d'acides aminés avec l'une quelconque des séquences d'acides aminés de E1 représentées dans SEQ ID NO: 28-31 des FIG. 2A-2B, et dans laquelle le polypeptide E2 du génotype 3 comprend une séquence d'acides aminés présentant au moins 80 % d'identité de séquence d'acides aminés avec l'une quelconque des séquences d'acides aminés de E2 représentées dans SEQ ID NO: 28-31 des FIG. 2A-2B.

9. Composition immunogène destinée à être utilisée selon l'une quelconque des revendications 1 à 7, dans laquelle :
a) le polypeptide E1 du génotype 1 comprend une séquence d'acides aminés présentant au moins 90 % d'identité de séquence d'acides aminés avec l'une quelconque des séquences d'acides aminés de E1 représentées dans SEQ ID NO: 1-27 des FIG. 1A-1C, et dans laquelle le polypeptide E2 du génotype 1 comprend une séquence d'acides aminés présentant au moins 90 % d'identité de séquence d'acides aminés avec l'une quelconque des séquences d'acides aminés de E2 représentées dans SEQ ID NO: 1-27 des FIG. 1A-1C ;
b) le polypeptide E1 du génotype 2 comprend une séquence d'acides aminés présentant au moins 90 % d'identité de séquence d'acides aminés avec l'une quelconque des séquences d'acides aminés de E1 représentées dans SEQ ID NO: 1-27 des FIG. 9A-9C, et dans laquelle le polypeptide E2 du génotype 2 comprend une séquence d'acides aminés présentant au moins 90 % d'identité de séquence d'acides aminés avec l'une quelconque des séquences d'acides aminés de E2 représentées dans SEQ ID NO: 1-27 des FIG. 9A-9C ; et
c) le polypeptide E1 du génotype 3 comprend une séquence d'acides aminés présentant au moins 90 % d'identité de séquence d'acides aminés avec l'une quelconque des séquences d'acides aminés de E1 représentées dans SEQ ID NO: 28-31 des FIG. 2A-2B, et dans laquelle le polypeptide E2 du génotype 3 comprend une séquence d'acides aminés présentant au moins 90 % d'identité de séquence d'acides aminés avec l'une quelconque des séquences d'acides aminés de E2 représentées dans SEQ ID NO: 28-31 des FIG. 2A-2B.


### SEQUENCE LISTING

<110> Houghton, Michael Hockman, Darren Law, John
<120> E1E2 HCV VACCINES AND METHODS OF USE
<130> UALB-017WO
<150> US 61/823,712
   <151> 2013-05-15
<150> US 61/887,229
   <151> 2013-10-04
<160> 53
<170> PatentIn version 3.5
<210> 1
   <211> 746
   <212> PRT
   <213> Hepatitis C virus
<400> 1
   Met Ser Thr Asn Pro Lys Pro Gln Arg Lys Thr Lys Arg Asn Thr Asn
   1               5                   10                  15
   Arg Arg Pro Gln Asp Val Lys Phe Pro Gly Gly Gly Gln Ile Val Gly
               20                  25                  30
   Gly Val Tyr Leu Leu Pro Arg Arg Gly Pro Arg Leu Gly Val Arg Ala
           35                  40                  45
   Thr Arg Lys Thr Ser Glu Arg Ser Gln Pro Arg Gly Arg Arg Gln Pro
       50                  55                  60
   Ile Pro Lys Ala Arg Arg Pro Glu Gly Arg Thr Trp Ala Gln Pro Gly
   65                  70                  75                  80
   Tyr Pro Trp Pro Leu Tyr Gly Asn Glu Gly Cys Gly Trp Ala Gly Trp
                   85                  90                  95
   Leu Leu Ser Pro Arg Gly Ser Arg Pro Ser Trp Gly Pro Thr Asp Pro
               100                 105                 110
   Arg Arg Arg Ser Arg Asn Leu Gly Lys Val Ile Asp Thr Leu Thr Cys
           115                 120                 125
   Gly Phe Ala Asp Leu Met Gly Tyr Ile Pro Leu Val Gly Ala Pro Leu
       130                 135                 140
   Gly Gly Ala Ala Arg Ala Leu Ala His Gly Val Arg Val Leu Glu Asp
   145                 150                 155                 160
   Gly Val Asn Tyr Ala Thr Gly Asn Leu Pro Gly Cys Ser Phe Ser Ile
                   165                 170                 175
   Phe Leu Leu Ala Leu Leu Ser Cys Leu Thr Val Pro Ala Ser Ala Tyr
               180                 185                 190
   Gln Val Arg Asn Ser Ser Gly Leu Tyr His Val Thr Asn Asp Cys Pro
           195                 200                 205
   Asn Ser Ser Ile Val Tyr Glu Ala Ala Asp Ala Ile Leu His Thr Pro
       210                 215                 220
   Gly Cys Val Pro Cys Val Arg Glu Gly Asn Ala Ser Arg Cys Trp Val
   225                 230                 235                 240
   Ala Val Thr Pro Thr Val Ala Thr Arg Asp Gly Lys Leu Pro Thr Thr
                   245                 250                 255
   Gln Leu Arg Arg His Ile Asp Leu Leu Val Gly Ser Ala Thr Leu Cys
               260                 265                 270
   Ser Ala Leu Tyr Val Gly Asp Leu Cys Gly Ser Val Phe Leu Val Gly
           275                 280                 285
   Gln Leu Phe Thr Phe Ser Pro Arg Arg His Trp Thr Thr Gln Asp Cys
       290                 295                 300
   Asn Cys Ser Ile Tyr Pro Gly His Ile Thr Gly His Arg Met Ala Trp
   305                 310                 315                 320
   Asp Met Met Met Asn Trp Ser Pro Thr Ala Ala Leu Val Val Ala Gln
                   325                 330                 335
   Leu Leu Arg Ile Pro Gln Ala Ile Met Asp Met Ile Ala Gly Ala His
               340                 345                 350
   Trp Gly Val Leu Ala Gly Ile Ala Tyr Phe Ser Met Val Gly Asn Trp
           355                 360                 365
   Ala Lys Val Leu Val Val Leu Leu Leu Phe Ala Gly Val Asp Ala Glu
       370                 375                 380
   Thr His Val Thr Gly Gly Ser Ala Gly Arg Thr Thr Ala Gly Leu Val
   385                 390                 395                 400
   Gly Leu Leu Thr Pro Gly Ala Lys Gln Asn Ile Gln Leu Ile Asn Thr
                   405                 410                 415
   Asn Gly Ser Trp His Ile Asn Ser Thr Ala Leu Asn Cys Asn Glu Ser
               420                 425                 430
   Leu Asn Thr Gly Trp Leu Ala Gly Leu Phe Tyr Gln His Lys Phe Asn
           435                 440                 445
   Ser Ser Gly Cys Pro Glu Arg Leu Ala Ser Cys Arg Arg Leu Thr Asp
       450                 455                 460
   Phe Ala Gln Gly Trp Gly Pro Ile Ser Tyr Ala Asn Gly Ser Gly Leu
   465                 470                 475                 480
   Asp Glu Arg Pro Tyr Cys Trp His Tyr Pro Pro Arg Pro Cys Gly Ile
                   485                 490                 495
   Val Pro Ala Lys Ser Val Cys Gly Pro Val Tyr Cys Phe Thr Pro Ser
               500                 505                 510
   Pro Val Val Val Gly Thr Thr Asp Arg Ser Gly Ala Pro Thr Tyr Ser
           515                 520                 525
   Trp Gly Ala Asn Asp Thr Asp Val Phe Val Leu Asn Asn Thr Arg Pro
       530                 535                 540
   Pro Leu Gly Asn Trp Phe Gly Cys Thr Trp Met Asn Ser Thr Gly Phe
   545                 550                 555                 560
   Thr Lys Val Cys Gly Ala Pro Pro Cys Val Ile Gly Gly Val Gly Asn
                   565                 570                 575
   Asn Thr Leu Leu Cys Pro Thr Asp Cys Phe Arg Lys His Pro Glu Ala
               580                 585                 590
   Thr Tyr Ser Arg Cys Gly Ser Gly Pro Trp Ile Thr Pro Arg Cys Met
           595                 600                 605
   Val Asp Tyr Pro Tyr Arg Leu Trp His Tyr Pro Cys Thr Ile Asn Tyr
       610                 615                 620
   Thr Ile Phe Lys Val Arg Met Tyr Val Gly Gly Val Glu His Arg Leu
   625                 630                 635                 640
   Glu Ala Ala Cys Asn Trp Thr Arg Gly Glu Arg Cys Asp Leu Glu Asp
                   645                 650                 655
   Arg Asp Arg Ser Glu Leu Ser Pro Leu Leu Leu Ser Thr Thr Gln Trp
               660                 665                 670
   Gln Val Leu Pro Cys Ser Phe Thr Thr Leu Pro Ala Leu Ser Thr Gly
           675                 680                 685
   Leu Ile His Leu His Gln Asn Ile Val Asp Val Gln Tyr Leu Tyr Gly
       690                 695                 700
   Val Gly Ser Ser Ile Ala Ser Trp Ala Ile Lys Trp Glu Tyr Val Val
   705                 710                 715                 720
   Leu Leu Phe Leu Leu Leu Ala Asp Ala Arg Val Cys Ser Cys Leu Trp
                   725                 730                 735
   Met Met Leu Leu Ile Ser Gln Ala Glu Ala
               740                 745
<210> 2
   <211> 746
   <212> PRT
   <213> Hepatitis C virus
<400> 2
   Met Ser Thr Asn Pro Lys Pro Gln Arg Lys Thr Lys Arg Asn Thr Asn
   1               5                   10                  15
   Arg Arg Pro Gln Asp Val Lys Phe Pro Gly Gly Gly Gln Ile Val Gly
               20                  25                  30
   Gly Val Tyr Leu Leu Pro Arg Arg Gly Pro Arg Leu Gly Val Arg Ala
           35                  40                  45
   Val Arg Lys Thr Ser Glu Arg Ser Gln Pro Arg Gly Arg Arg Gln Pro
       50                  55                  60
   Ile Pro Lys Ala Arg Arg Pro Glu Gly Arg Thr Trp Ala Gln Pro Gly
   65                  70                  75                  80
   Tyr Pro Trp Pro Leu Tyr Gly Asn Glu Gly Cys Gly Trp Ala Gly Trp
                   85                  90                  95
   Leu Leu Ser Pro Arg Gly Ser Arg Pro Ser Trp Gly Pro Thr Asp Pro
               100                 105                 110
   Arg Arg Arg Ser Arg Asn Leu Gly Lys Val Ile Asp Thr Leu Thr Cys
           115                 120                 125
   Gly Phe Ala Asp Leu Met Gly Tyr Ile Pro Leu Val Gly Ala Pro Leu
       130                 135                 140
   Gly Gly Ala Ala Arg Ala Leu Ala His Gly Val Arg Val Leu Glu Asp
   145                 150                 155                 160
   Gly Val Asn Tyr Ala Thr Gly Asn Leu Pro Gly Cys Ser Phe Ser Ile
                   165                 170                 175
   Phe Leu Leu Ala Leu Leu Ser Cys Leu Thr Val Pro Ala Ser Ala Tyr
               180                 185                 190
   Gln Val Arg Asn Ser Ser Gly Ile Tyr His Val Thr Asn Asp Cys Pro
           195                 200                 205
   Asn Ser Ser Ile Val Tyr Glu Thr Ala Asp Thr Ile Leu His Ser Pro
       210                 215                 220
   Gly Cys Val Pro Cys Val Arg Glu Gly Asn Ala Ser Lys Cys Trp Val
   225                 230                 235                 240
   Ala Leu Ala Pro Thr Val Ala Thr Arg Asp Gly Lys Leu Pro Thr Thr
                   245                 250                 255
   Gln Leu Arg Arg His Ile Asp Leu Leu Val Gly Ser Ala Thr Leu Cys
               260                 265                 270
   Ser Ala Leu Tyr Val Gly Asp Leu Cys Gly Ser Val Phe Leu Val Gly
           275                 280                 285
   Gln Leu Phe Thr Phe Ser Pro Arg Arg His Trp Thr Thr Gln Asp Cys
       290                 295                 300
   Asn Cys Ser Ile Tyr Pro Gly His Ile Thr Gly His Arg Met Ala Trp
   305                 310                 315                 320
   Asp Met Met Met Asn Trp Ser Pro Thr Thr Ala Leu Val Val Ala Gln
                   325                 330                 335
   Leu Leu Arg Val Pro Gln Ala Ile Leu Asp Met Ile Ala Gly Ala His
               340                 345                 350
   Trp Gly Val Leu Ala Gly Ile Ala Tyr Phe Ser Met Val Gly Asn Trp
           355                 360                 365
   Ala Lys Val Leu Val Val Leu Leu Leu Phe Ala Gly Val Asp Ala Glu
           370                 375                 380
   Thr Tyr Thr Thr Gly Gly Ser Val Ala Gln Ala Ala Phe Gly Leu Thr
   385                 390                 395                 400
   Ser Leu Phe Arg Pro Gly Pro Lys Gln Asp Ile Gln Leu Ile Asn Thr
                   405                 410                 415
   Asn Gly Ser Trp His Ile Asn Arg Thr Ala Leu Asn Cys Asn Ala Ser
               420                 425                 430
   Leu Asp Thr Gly Trp Val Ala Gly Leu Phe Tyr Tyr His Lys Phe Asn
           435                 440                 445
   Ser Ser Gly Cys Pro Glu Arg Met Ala Ser Cys Arg Ser Leu Ala Asp
       450                 455                 460
   Phe Asp Gln Gly Trp Gly Pro Ile Ser Tyr Ala Asn Gly Ser Gly Pro
   465                 470                 475                 480
   Glu His Arg Pro Tyr Cys Trp His Tyr Pro Pro Lys Pro Cys Gly Ile
                   485                 490                 495
   Val Pro Ala Gln Asn Val Cys Gly Pro Val Tyr Cys Phe Thr Pro Ser
               500                 505                 510
   Pro Val Val Val Gly Thr Thr Asn Lys Leu Gly Val Pro Thr Tyr Ser
           515                 520                 525
   Trp Gly Ser Asn Glu Thr Asp Val Leu Val Leu Asn Asn Thr Arg Pro
       530                 535                 540
   Pro Leu Gly Asn Trp Phe Gly Cys Thr Trp Met Asn Ser Ser Gly Phe
   545                 550                 555                 560
   Thr Lys Val Cys Gly Ala Pro Pro Cys Val Ile Gly Gly Ala Gly Asn
                   565                 570                 575
   Arg Thr Leu His Cys Pro Thr Asp Cys Phe Arg Lys His Pro Glu Ala
               580                 585                 590
   Thr Tyr Ser Arg Cys Gly Ser Gly Pro Trp Ile Thr Pro Arg Cys Leu
           595                 600                 605
   Val His Tyr Pro Tyr Arg Leu Trp His Tyr Pro Cys Thr Val Asn Tyr
       610                 615                 620
   Thr Met Phe Lys Val Arg Met Tyr Val Gly Gly Val Glu His Arg Leu
   625 630 635 640
   Glu Val Ala Cys Asn Trp Thr Arg Gly Glu Arg Cys Asp Leu Asp Asp
   645 650 655
   Arg Asp Arg Ser Glu Leu Ser Pro Leu Leu Leu Ser Thr Thr Gln Trp
   660 665 670
   Gln Val Leu Pro Cys Ser Phe Thr Thr Leu Pro Ala Leu Thr Thr Gly
   675 680 685
   Leu Ile His Leu His Gln Asn Ile Val Asp Val Gln Tyr Leu Tyr Gly
   690 695 700
   Val Gly Ser Ser Ile Val Ser Trp Ala Ile Lys Trp Glu Tyr Val Ile
   705 710 715 720
   Leu Leu Phe Leu Leu Leu Ala Asp Ala Arg Ile Cys Ser Cys Leu Trp
   725 730 735
   Met Met Leu Leu Ile Ser Gln Ala Glu Ala
   740 745
<210> 3
   <211> 746
   <212> PRT
   <213> Hepatitis C virus
<400> 3
   Met Ser Thr Asn Pro Lys Pro Gln Arg Lys Thr Lys Arg Asn Thr Asn
   1 5 10 15
   Arg Arg Pro Gln Asp Val Lys Phe Pro Gly Gly Gly Gln Ile Val Gly
   20 25 30
   Gly Val Tyr Leu Leu Pro Arg Arg Gly Pro Arg Leu Gly Val Arg Ala
   35 40 45
   Thr Arg Lys Thr Ser Glu Arg Ser Gln Pro Arg Gly Arg Arg Gln Pro
   50 55 60
   Ile Pro Lys Ala Arg Arg Pro Glu Gly Arg Ser Trp Ala Gln Pro Gly
   65 70 75 80
   Tyr Pro Trp Pro Leu Tyr Gly Asn Glu Gly Cys Gly Trp Ala Gly Trp
   85 90 95
   Leu Leu Ser Pro Arg Gly Ser Arg Pro Ser Trp Gly Pro Thr Asp Pro
               100                 105                 110
   Arg Arg Arg Ser Arg Asn Leu Gly Lys Val Ile Asp Thr Leu Thr Cys
           115                 120                 125
   Gly Phe Ala Asp Leu Met Gly Tyr Ile Pro Leu Val Gly Ala Pro Leu
       130                 135                 140
   Gly Gly Ala Ala Arg Ala Leu Ala His Gly Val Arg Val Leu Glu Asp
   145                 150                 155                 160
   Gly Val Asn Tyr Ala Thr Gly Asn Leu Pro Gly Cys Ser Phe Ser Ile
                   165                 170                 175
   Phe Leu Leu Ala Leu Leu Ser Cys Leu Thr Val Pro Ala Ser Ala Phe
               180                 185                 190
   Gln Val Arg Asn Ser Ser Gly Leu Tyr His Val Thr Asn Asp Cys Pro
           195                 200                 205
   Asn Ser Ser Ile Val Tyr Glu Thr Ala Asp Thr Ile Leu His Ser Pro
       210                 215                 220
   Gly Cys Val Pro Cys Val Arg Glu Asp Asn Thr Ser Arg Cys Trp Val
   225                 230                 235                 240
   Ala Val Ala Pro Thr Val Ala Thr Arg Asp Gly Arg Leu Pro Thr Thr
                   245                 250                 255
   Gln Leu Arg Arg His Ile Asp Leu Leu Val Gly Ser Ala Thr Leu Cys
               260                 265                 270
   Ser Ala Leu Tyr Val Gly Asp Leu Cys Gly Ser Val Phe Leu Val Gly
           275                 280                 285
   Gln Leu Phe Thr Phe Ser Pro Arg Arg His Trp Thr Thr Gln Asp Cys
       290                 295                 300
   Asn Cys Ser Ile Tyr Pro Gly His Ile Thr Gly His Arg Met Ala Trp
   305                 310                 315                 320
   Asp Met Met Met Asn Trp Ser Pro Thr Thr Ala Leu Val Val Ala Gln
                   325                 330                 335
   Leu Leu Arg Val Pro Gln Ala Ile Leu Asp Met Ile Ala Gly Ala His
               340                 345                 350
   Trp Gly Val Leu Ala Gly Ile Ala Tyr Phe Ser Met Val Gly Asn Trp
   355 360 365
   Ala Lys Val Leu Val Val Leu Leu Leu Phe Ala Ser Val Asp Ala Glu
   370 375 380
   Thr Tyr Thr Ser Gly Gly Ser Val Ala Arg Ala Thr Ala Gly Phe Ala
   385 390 395 400
   Gly Ile Phe Asn Pro Gly Ala Lys Gln Asp Ile Gln Leu Ile Asn Thr
   405 410 415
   Asn Gly Ser Trp His Ile Asn Arg Thr Ala Leu Asn Cys Asn Ala Ser
   420 425 430
   Leu Asp Thr Gly Trp Val Ala Gly Leu Phe Tyr Tyr His Lys Phe Asn
   435 440 445
   Ser Ser Gly Cys Pro Glu Arg Met Ala Ser Cys Lys Pro Leu Ala His
   450 455 460
   Phe Ala Gln Gly Trp Gly Pro Ile Ser Tyr Ala Asn Gly Ser Gly Pro
   465 470 475 480
   Asp His Arg Pro Tyr Cys Trp His Tyr Pro Pro Lys Pro Cys Gly Ile
   485 490 495
   Val Pro Ala Gln Asn Val Cys Gly Pro Val Tyr Cys Phe Thr Pro Ser
   500 505 510
   Pro Val Val Val Gly Thr Thr Asn Lys Leu Gly Ala Pro Thr Tyr Asn
   515 520 525
   Trp Gly Ser Asn Asp Thr Asp Val Phe Ile Leu Asn Asn Thr Arg Pro
   530 535 540
   Pro Gly Gly Asn Trp Phe Gly Cys Thr Trp Met Asn Ser Ser Gly Phe
   545 550 555 560
   Thr Lys Val Cys Gly Ala Pro Pro Cys Thr Ile Gly Gly Val Gly Asn
   565 570 575
   Asn Thr Leu Leu Cys Pro Thr Asp Cys Phe Arg Lys His Pro Glu Ala
   580 585 590
   Thr Tyr Ser Arg Cys Gly Ser Gly Pro Trp Val Thr Pro Arg Phe Leu
   595 600 605
   Val His Tyr Pro Tyr Arg Leu Trp His Tyr Pro Cys Thr Val Asn Tyr
   610 615 620
   Thr Leu Phe Lys Val Arg Met Tyr Val Gly Gly Val Glu His Arg Leu
   625 630 635 640
   Glu Val Ala Cys Asn Trp Thr Arg Gly Glu Arg Cys Asp Leu Asp Asp
   645 650 655
   Arg Asp Arg Ser Glu Leu Ser Pro Leu Leu Leu Ser Thr Thr Gln Trp
   660 665 670
   Gln Val Leu Pro Cys Ser Phe Thr Thr Leu Pro Ala Leu Thr Thr Gly
   675 680 685
   Leu Ile His Leu His Gln Asn Ile Val Asp Val Gln Tyr Leu Tyr Gly
   690 695 700
   Val Gly Ser Ser Leu Val Ser Trp Ala Ile Lys Trp Glu Tyr Val Ile
   705 710 715 720
   Leu Leu Phe Leu Leu Leu Ala Asp Ala Arg Ile Cys Ser Cys Leu Trp
   725 730 735
   Met Met Leu Leu Ile Ser Gln Ala Glu Ala
   740 745
<210> 4
   <211> 746
   <212> PRT
   <213> Hepatitis C virus
<400> 4
   Met Ser Thr Asn Pro Lys Pro Gln Arg Lys Thr Lys Arg Asn Thr Asn
   1 5 10 15
   Arg Arg Pro Gln Asp Val Lys Phe Pro Gly Gly Gly Gln Ile Val Gly
   20 25 30
   Gly Val Tyr Leu Leu Pro Arg Arg Gly Pro Arg Leu Gly Val Arg Ala
   35 40 45
   Thr Arg Lys Thr Ser Glu Arg Ser Gln Pro Arg Gly Arg Arg Gln Pro
   50 55 60
   Ile Pro Lys Ala Arg Arg Pro Glu Gly Arg Thr Trp Ala Gln Pro Gly
   65 70 75 80
   Tyr Pro Trp Pro Leu Tyr Gly Asn Glu Gly Cys Gly Trp Ala Gly Trp
                   85                  90                  95
   Leu Leu Ser Pro Arg Gly Ser Arg Pro Ser Trp Gly Pro Thr Asp Pro
               100                 105                 110
   Arg Arg Arg Ser Arg Asn Leu Gly Lys Val Ile Asp Thr Leu Thr Cys
           115                 120                 125
   Gly Phe Ala Asp Leu Met Gly Tyr Ile Pro Leu Val Gly Ala Pro Leu
       130                 135                 140
   Gly Gly Ala Ala Arg Ala Leu Ala His Gly Val Arg Val Leu Glu Asp
   145                 150                 155                 160
   Gly Val Asn Tyr Ala Thr Gly Asn Leu Pro Gly Cys Ser Phe Ser Ile
                   165                 170                 175
   Phe Leu Leu Ala Leu Leu Ser Cys Leu Thr Val Pro Ala Ser Ala Tyr
               180                 185                 190
   Gln Val Arg Asn Ser Ser Gly Leu Tyr His Val Thr Asn Asp Cys Pro
           195                 200                 205
   Asn Ser Ser Ile Val Tyr Glu Thr Ala Asp Thr Ile Leu His Ser Pro
       210                 215                 220
   Gly Cys Val Pro Cys Val Arg Glu Gly Asn Thr Ser Lys Cys Trp Val
   225                 230                 235                 240
   Ala Val Ala Pro Thr Val Ala Thr Arg Asp Gly Lys Leu Pro Thr Thr
                   245                 250                 255
   Gln Leu Arg Arg His Ile Asp Leu Leu Val Gly Ser Ala Thr Leu Cys
               260                 265                 270
   Ser Ala Leu Tyr Val Gly Asp Leu Cys Gly Ser Val Phe Leu Val Gly
           275                 280                 285
   Gln Leu Phe Thr Phe Ser Pro Arg Arg His Trp Thr Thr Gln Asp Cys
       290                 295                 300
   Asn Cys Ser Ile Tyr Pro Gly His Ile Thr Gly His Arg Met Ala Trp
   305                 310                 315                 320
   Asp Met Met Met Asn Trp Ser Pro Thr Ala Ala Leu Val Thr Ala Gln
   325 330 335
   Leu Leu Arg Val Pro Gln Ala Ile Leu Asp Met Ile Ala Gly Ala His
   340 345 350
   Trp Gly Val Leu Ala Gly Ile Ala Tyr Phe Ser Met Ala Gly Asn Trp
   355 360 365
   Ala Lys Val Leu Leu Val Leu Leu Leu Phe Ala Gly Val Asp Ala Glu
   370 375 380
   Thr Tyr Thr Thr Gly Gly Ser Val Ala Arg Thr Thr Arg Gly Leu Ala
   385 390 395 400
   Ser Leu Leu Gln Val Gly Pro Lys Gln Asp Ile Arg Leu Ile His Thr
   405 410 415
   Asn Gly Ser Trp His Ile Asn Arg Thr Ala Leu Asn Cys Asn Ala Ser
   420 425 430
   Leu Asp Thr Gly Trp Leu Ala Gly Leu Leu Tyr Tyr His Lys Phe Asn
   435 440 445
   Ser Ser Gly Cys Pro Glu Arg Met Ala Ser Cys Arg Pro Leu Ala Asp
   450 455 460
   Phe Asp Gln Gly Trp Gly Pro Ile Ser Tyr Ala Asn Gly Ser Gly Pro
   465 470 475 480
   Glu His Arg Pro Tyr Cys Trp His Tyr Pro Pro Lys Pro Cys Gly Ile
   485 490 495
   Val Pro Ala Gln Thr Val Cys Gly Pro Val Tyr Cys Phe Thr Pro Ser
   500 505 510
   Pro Val Val Val Gly Thr Thr Asn Lys Leu Gly Val Pro Thr Tyr Thr
   515 520 525
   Trp Gly Ser Asn Asp Thr Asp Val Phe Val Leu Asn Asn Thr Arg Pro
   530 535 540
   Pro Leu Gly Asn Trp Phe Gly Cys Thr Trp Met Asn Ser Thr Gly Phe
   545 550 555 560
   Thr Lys Val Cys Gly Ala Pro Pro Cys Val Ile Gly Gly Val Gly Asn
   565 570 575
   Asn Thr Leu His Cys Pro Thr Asp Cys Phe Arg Lys His Pro Glu Ala
   580 585 590
   Thr Tyr Ser Arg Cys Gly Ser Gly Pro Trp Ile Thr Pro Arg Cys Leu
   595 600 605
   Val Asp Tyr Pro Tyr Arg Leu Trp His Tyr Pro Cys Thr Val Asn Tyr
   610 615 620
   Thr Leu Phe Lys Val Arg Met Tyr Val Gly Gly Val Glu His Arg Leu
   625 630 635 640
   Gln Ala Ala Cys Asn Trp Thr Arg Gly Glu Arg Cys Asp Leu Asp Asp
   645 650 655
   Arg Asp Arg Ser Glu Leu Ser Pro Leu Leu Leu Ser Thr Thr Gln Trp
   660 665 670
   Gln Val Leu Pro Cys Ser Phe Thr Thr Leu Pro Ala Leu Thr Thr Gly
   675 680 685
   Leu Ile His Leu His Gln Asn Ile Val Asp Val Gln Tyr Leu Tyr Gly
   690 695 700
   Val Gly Ser Ser Ile Val Ser Trp Ala Ile Lys Trp Glu Tyr Val Ile
   705 710 715 720
   Leu Leu Phe Leu Leu Leu Ala Asp Ala Arg Ile Cys Ser Cys Leu Trp
   725 730 735
   Met Met Leu Leu Ile Ser Gln Ala Glu Ala
   740 745
<210> 5
   <211> 746
   <212> PRT
   <213> Hepatitis C virus
<400> 5
   Met Ser Thr Asn Pro Lys Pro Gln Arg Lys Thr Lys Arg Asn Thr Asn
   1 5 10 15
   Arg Arg Pro Gln Asp Val Lys Phe Pro Gly Gly Gly Gln Ile Val Gly
   20 25 30
   Gly Val Tyr Leu Leu Pro Arg Arg Gly Pro Arg Leu Gly Val Arg Ala
   35 40 45
   Thr Arg Lys Thr Ser Glu Arg Ser Gln Pro Arg Gly Arg Arg Gln Pro
       50                  55                  60
   Ile Pro Lys Ala Arg Arg Pro Glu Gly Arg Thr Trp Ala Gln Pro Gly
   65                  70                  75                  80
   Tyr Pro Trp Pro Leu Tyr Gly Asn Glu Gly Cys Gly Trp Ala Gly Trp
                   85                  90                  95
   Leu Leu Ser Pro Arg Gly Ser Arg Pro Ser Trp Gly Pro Thr Asp Pro
               100                 105                 110
   Arg Arg Arg Ser Arg Asn Leu Gly Lys Val Ile Asp Thr Leu Thr Cys
           115                 120                 125
   Gly Phe Ala Asp Leu Met Gly Tyr Ile Pro Leu Val Gly Ala Pro Leu
       130                 135                 140
   Gly Gly Ala Ala Arg Ala Leu Ala His Gly Val Arg Val Leu Glu Asp
   145                 150                 155                 160
   Gly Val Asn Tyr Ala Thr Gly Asn Leu Pro Gly Cys Ser Phe Ser Ile
                   165                 170                 175
   Phe Leu Leu Ala Leu Leu Ser Cys Leu Thr Val Pro Ala Ser Ala Tyr
               180                 185                 190
   Gln Val Arg Asn Ser Ser Gly Leu Tyr His Val Thr Asn Asp Cys Pro
           195                 200                 205
   Asn Ser Ser Ile Val Tyr Glu Thr Ala Asp Thr Ile Leu His Ser Pro
       210                 215                 220
   Gly Cys Val Pro Cys Val Arg Glu Asp Asn Ala Ser Arg Cys Trp Val
   225                 230                 235                 240
   Pro Val Ala Pro Thr Val Ala Thr Arg Asp Gly Lys Leu Pro Thr Thr
                   245                 250                 255
   Gln Leu Arg Arg His Ile Asp Leu Leu Val Gly Ser Ala Thr Leu Cys
               260                 265                 270
   Ser Ala Leu Tyr Val Gly Asp Leu Cys Gly Ser Val Phe Leu Val Ser
           275                 280                 285
   Gln Leu Phe Thr Phe Ser Pro Arg Arg His Trp Thr Thr Gln Asp Cys
       290                 295                 300
   Asn Cys Ser Ile Tyr Pro Gly His Ile Thr Gly His Arg Met Ala Trp
   305 310 315 320
   Asp Met Met Met Asn Trp Ser Pro Thr Val Ala Leu Val Met Ala Gln
   325 330 335
   Leu Leu Arg Val Pro Gln Ala Ile Leu Asp Met Ile Ala Gly Ala His
   340 345 350
   Trp Gly Val Leu Ala Gly Ile Ala Tyr Phe Ser Met Val Gly Asn Trp
   355 360 365
   Ala Lys Val Leu Val Val Leu Leu Leu Phe Ala Gly Val Asp Ala Gln
   370 375 380
   Thr Tyr Val Thr Gly Gly Ser Ala Ala Arg Gly Ala Ser Gly Leu Ala
   385 390 395 400
   Asn Leu Phe Thr Pro Gly Ala Lys Gln Asp Ile Gln Leu Ile Asn Thr
   405 410 415
   Asn Gly Ser Trp His Ile Asn Arg Thr Ala Leu Asn Cys Asn Ala Ser
   420 425 430
   Leu Asp Thr Gly Trp Val Ala Gly Leu Phe Tyr Tyr His Lys Phe Asn
   435 440 445
   Ser Ser Gly Cys Pro Glu Arg Met Ala Ser Cys Lys Pro Leu Ala Asp
   450 455 460
   Phe Asp Gln Gly Trp Gly Pro Ile Arg His Ala Asn Gly Ser Gly Pro
   465 470 475 480
   Glu His Arg Pro Tyr Cys Trp His Tyr Pro Pro Lys Pro Cys Gly Ile
   485 490 495
   Val Ser Ala Gln Thr Val Cys Gly Pro Val Tyr Cys Phe Thr Pro Ser
   500 505 510
   Pro Val Val Val Gly Thr Thr Asn Arg Leu Gly Val Pro Thr Tyr Ser
   515 520 525
   Trp Gly Thr Asn Asp Thr Asp Val Phe Val Leu Asn Asn Thr Arg Pro
   530 535 540
   Pro Leu Gly Asn Trp Phe Gly Cys Thr Trp Met Asn Ser Ser Gly Phe
   545 550 555 560
   Thr Lys Val Cys Gly Ala Pro Pro Cys Val Ile Gly Gly Val Gly Asn
   565 570 575
   Asn Thr Leu His Cys Pro Thr Asp Cys Phe Arg Lys His Pro Glu Ala
   580 585 590
   Thr Tyr Ser Arg Cys Gly Ser Gly Pro Trp Ile Thr Pro Arg Cys Leu
   595 600 605
   Val His Tyr Pro Tyr Arg Leu Trp His Tyr Pro Cys Thr Val Asn Tyr
   610 615 620
   Thr Leu Phe Lys Val Arg Met Tyr Val Gly Gly Val Glu His Arg Leu
   625 630 635 640
   Glu Val Ala Cys Asn Trp Thr Arg Gly Glu Arg Cys Asp Leu Asp Asp
   645 650 655
   Arg Asp Arg Ser Glu Leu Ser Pro Leu Leu Leu Ser Thr Thr Gln Trp
   660 665 670
   Gln Val Leu Pro Cys Ser Phe Thr Thr Leu Pro Ala Leu Thr Thr Gly
   675 680 685
   Leu Ile His Leu His Gln Asn Ile Val Asp Val Gln Tyr Leu Tyr Gly
   690 695 700
   Val Gly Ser Ser Ile Val Ser Trp Ala Ile Lys Trp Glu Tyr Val Ile
   705 710 715 720
   Leu Leu Phe Leu Leu Leu Ala Asp Ala Arg Ile Cys Ser Cys Leu Trp
   725 730 735
   Met Met Leu Leu Ile Ser Gln Ala Glu Ala
   740 745
<210> 6
   <211> 746
   <212> PRT
   <213> Hepatitis C virus
<400> 6
   Met Ser Thr Asn Pro Lys Pro Gln Arg Lys Thr Lys Arg Asn Thr Asn
   1 5 10 15
   Arg Arg Pro Gln Asp Val Lys Phe Pro Gly Gly Gly Gln Ile Val Gly
   20 25 30
   Gly Val Tyr Leu Leu Pro Arg Arg Gly Pro Arg Leu Gly Val Arg Thr
           35                  40                  45
   Thr Arg Lys Thr Ser Glu Arg Ser Glu Pro Arg Gly Arg Arg Gln Pro
       50                  55                  60
   Ile Pro Lys Ala Arg Arg Pro Glu Gly Arg Thr Trp Ala Gln Pro Gly
   65                  70                  75                  80
   Tyr Pro Trp Pro Leu Tyr Gly Asn Glu Gly Cys Gly Trp Ala Gly Trp
                   85                  90                  95
   Leu Leu Ser Pro Arg Gly Ser Arg Pro Ser Trp Gly Pro Thr Asp Pro
               100                 105                 110
   Arg Arg Arg Ser Arg Asn Leu Gly Lys Val Ile Asp Thr Leu Thr Cys
           115                 120                 125
   Gly Phe Ala Asp Leu Met Gly Tyr Ile Pro Leu Val Gly Ala Pro Leu
       130                 135                 140
   Gly Gly Ala Ala Arg Ala Leu Ala His Gly Val Arg Val Leu Glu Asp
   145                 150                 155                 160
   Gly Val Asn Tyr Ala Thr Gly Asn Leu Pro Gly Cys Ser Phe Ser Ile
                   165                 170                 175
   Phe Leu Leu Ala Leu Leu Ser Cys Leu Thr Val Pro Ala Ser Ala Tyr
               180                 185                 190
   Gln Val Arg Asn Ser Ser Gly Leu Tyr His Val Thr Asn Asp Cys Pro
           195                 200                 205
   Asn Ser Ser Ile Val Tyr Glu Thr Lys Asp Thr Ile Leu His Ser Pro
       210                 215                 220
   Gly Cys Val Pro Cys Val Arg Glu Gly Asn Val Ser Lys Cys Trp Val
   225                 230                 235                 240
   Pro Val Ala Leu Thr Val Ala Thr Arg Asp Gly Asn Leu Pro Thr Thr
                   245                 250                 255
   Gln Leu Arg Arg His Ile Asp Leu Leu Val Gly Ser Ala Thr Leu Cys
               260                 265                 270
   Ser Ala Leu Tyr Val Gly Asp Leu Cys Gly Ser Val Phe Leu Val Gly
           275                 280                 285
   Gln Leu Phe Thr Phe Ser Pro Arg Arg His Trp Thr Thr Gln Asp Cys
       290                 295                 300
   Asn Cys Ser Met Tyr Pro Gly His Ile Thr Gly His Arg Met Ala Trp
   305                 310                 315                 320
   Asp Met Met Met Asn Trp Ser Pro Thr Ser Ala Leu Val Val Ala Gln
                   325                 330                 335
   Leu Leu Arg Val Pro Gln Ala Ile Leu Asp Met Ile Ala Gly Ala His
               340                 345                 350
   Trp Gly Val Leu Ala Gly Leu Ala Tyr Phe Ser Met Val Gly Asn Trp
           355                 360                 365
   Ala Lys Val Leu Val Val Leu Leu Leu Phe Ala Ser Val Asp Ala Gly
       370                 375                 380
   Thr His Val Thr Gly Gly Ser Ala Ala His Asp Val Ser Ala Leu Ala
   385                 390                 395                 400
   Gly Phe Phe Arg Arg Gly Ala Lys Gln Asn Ile Gln Leu Ile Asn Thr
                   405                 410                 415
   Asn Gly Ser Trp His Val Asn Arg Thr Ala Leu Asn Cys Asn Ala Ser
               420                 425                 430
   Leu Asp Thr Gly Trp Val Ala Gly Leu Leu Tyr Tyr His Arg Phe Asn
           435                 440                 445
   Ser Ser Gly Cys Pro Glu Arg Met Ala Ser Cys Arg Pro Leu Ala Asp
       450                 455                 460
   Phe Asp Gln Gly Trp Gly Pro Ile Thr Asn Val Asp Gly Gly Gly Ser
   465                 470                 475                 480
   Glu Tyr Arg Pro Tyr Cys Trp His Tyr Pro Pro Lys Pro Cys Gly Ile
                   485                 490                 495
   Glu Pro Ala Gln Asn Val Cys Gly Pro Val Tyr Cys Phe Thr Pro Ser
               500                 505                 510
   Pro Val Val Val Gly Thr Thr Asp Lys Val Gly Val Pro Thr Tyr Asn
           515                 520                 525
   Trp Gly Glu Asn Asp Thr Asp Val Phe Val Leu Asn Asn Thr Arg Pro
       530                 535                 540
   Pro Leu Gly Asn Trp Phe Gly Cys Thr Trp Met Asn Ser Ser Gly Phe
   545                 550                 555                 560
   Val Lys Val Cys Gly Ala Pro Pro Cys Ile Ile Gly Gly Ala Gly Asn
                   565                 570                 575
   Lys Thr Leu His Cys Pro Thr Asp Cys Phe Arg Lys His Pro Asp Ala
               580                 585                 590
   Thr Tyr Ser Arg Cys Gly Ser Gly Pro Trp Leu Thr Pro Arg Cys Leu
           595                 600                 605
   Val Asp Tyr Pro Tyr Arg Leu Trp His Tyr Pro Cys Thr Val Asn Tyr
       610                 615                 620
   Thr Leu Phe Lys Ile Arg Met Tyr Val Gly Gly Val Glu His Arg Leu
   625                 630                 635                 640
   Val Ala Ala Cys Asn Trp Thr Tyr Gly Glu Arg Cys Asn Leu Asp Asp
                   645                 650                 655
   Arg Asp Arg Ser Glu Leu Ser Pro Leu Leu Leu Ser Thr Thr Gln Trp
               660                 665                 670
   Gln Val Leu Pro Cys Ser Phe Thr Thr Leu Pro Ala Leu Thr Thr Gly
           675                 680                 685
   Leu Ile His Leu His Gln Asn Ile Val Asp Val Gln Tyr Leu Tyr Gly
       690                 695                 700
   Val Gly Ser Ser Ile Val Ser Trp Ala Ile Lys Trp Glu Tyr Val Ile
   705                 710                 715                 720
   Leu Leu Phe Leu Leu Leu Ala Asp Ala Arg Ile Cys Ser Cys Leu Trp
                   725                 730                 735
   Met Met Leu Leu Ile Ser Gln Ala Glu Ala
               740                 745
<210> 7
   <211> 746
   <212> PRT
   <213> Hepatitis C virus
<400> 7
   Met Ser Thr Asn Pro Lys Pro Gln Arg Lys Thr Lys Arg Asn Thr Asn
   1               5                   10                  15
   Arg Arg Pro Gln Asp Val Lys Phe Pro Gly Gly Gly Gln Ile Val Gly
               20                  25                  30
   Gly Val Tyr Leu Leu Pro Arg Arg Gly Pro Arg Leu Gly Val Arg Ala
           35                  40                  45
   Thr Arg Lys Thr Ser Glu Arg Ser Gln Pro Arg Gly Arg Arg Gln Pro
       50                  55                  60
   Ile Pro Lys Ala Arg Arg Pro Glu Gly Arg Thr Trp Ala Gln Pro Gly
   65                  70                  75                  80
   Tyr Pro Trp Pro Leu Tyr Gly Asn Glu Gly Cys Gly Trp Ala Gly Trp
                   85                  90                  95
   Leu Leu Ser Pro Arg Gly Ser Arg Pro Ser Trp Gly Pro Thr Asp Pro
               100                 105                 110
   Arg Arg Arg Ser Arg Asn Leu Gly Lys Val Ile Asp Thr Leu Thr Cys
           115                 120                 125
   Gly Phe Ala Asp Leu Met Gly Tyr Ile Pro Leu Val Gly Ala Pro Leu
       130                 135                 140
   Gly Gly Ala Ala Arg Ala Leu Ala His Gly Val Arg Val Leu Glu Asp
   145                 150                 155                 160
   Gly Val Asn Tyr Ala Thr Gly Asn Leu Pro Gly Cys Ser Phe Ser Ile
                   165                 170                 175
   Phe Leu Leu Ala Leu Leu Ser Cys Leu Thr Val Pro Ala Ser Ala Tyr
               180                 185                 190
   Gln Val Arg Asn Ser Ser Gly Leu Tyr His Val Thr Asn Asp Cys Pro
           195                 200                 205
   Asn Ser Ser Ile Val Tyr Glu Thr Ala Asp Thr Ile Leu His Ser Pro
       210                 215                 220
   Gly Cys Val Pro Cys Val Arg Glu Gly Asn Ala Ser Lys Cys Trp Val
   225                 230                 235                 240
   Ala Val Ala Pro Thr Val Ala Thr Arg Asp Gly Thr Leu Pro Thr Thr
                   245                 250                 255
   Gln Leu Arg Arg His Ile Asp Leu Leu Val Gly Gly Ala Thr Leu Cys
   260 265 270
   Ser Ala Leu Tyr Val Gly Asp Leu Cys Gly Ser Val Phe Leu Val Gly
   275 280 285
   Gln Leu Phe Thr Phe Ser Pro Arg Arg His Trp Thr Thr Gln Asp Cys
   290 295 300
   Asn Cys Ser Ile Tyr Pro Gly His Ile Thr Gly His Arg Met Ala Trp
   305 310 315 320
   Asp Met Met Met Asn Trp Ser Pro Thr Thr Ala Leu Ile Val Ala Gln
   325 330 335
   Leu Leu Arg Val Pro Gln Ala Ile Leu Asp Met Ile Ala Gly Ala His
   340 345 350
   Trp Gly Val Leu Ala Gly Ile Ala Tyr Phe Ser Met Val Gly Asn Trp
   355 360 365
   Ala Lys Val Leu Val Val Leu Leu Leu Phe Ala Gly Val Asp Ala Glu
   370 375 380
   Thr His Val Thr Gly Gly Ser Ala Ala Gln Val Thr Ser Arg Val Ala
   385 390 395 400
   Gly Phe Phe Asn Pro Gly Pro Lys Gln Asn Val Gln Leu Ile Asn Thr
   405 410 415
   Asn Gly Ser Trp His Ile Asn Arg Thr Ala Leu Asn Cys Asn Ala Ser
   420 425 430
   Leu Asp Thr Gly Trp Val Ala Gly Leu Phe Tyr His Tyr Asn Phe Asn
   435 440 445
   Ser Ser Gly Cys Pro Glu Arg Met Ala Ser Cys Arg Pro Leu Ala Asp
   450 455 460
   Phe Asp Gln Gly Trp Gly Pro Ile Ser Tyr Ala Asn Gly Ser Gly Pro
   465 470 475 480
   Glu His Arg Pro Tyr Cys Trp His Tyr Pro Pro Lys Pro Cys Gly Ile
   485 490 495
   Val Pro Ala Gln Asn Val Cys Gly Pro Val Tyr Cys Phe Thr Pro Ser
   500 505 510
   Pro Val Val Val Gly Thr Thr Asn Arg Leu Gly Val Pro Thr Tyr Asn
   515 520 525
   Trp Gly Ser Asn Asp Thr Asp Val Phe Val Leu Asn Asn Thr Arg Pro
   530 535 540
   Pro Leu Gly Asn Trp Phe Gly Cys Thr Trp Met Asn Ser Ser Gly Phe
   545 550 555 560
   Thr Lys Val Cys Gly Ala Pro Pro Cys Val Ile Gly Gly Val Gly Asn
   565 570 575
   Asn Thr Leu His Cys Pro Thr Asp Cys Phe Arg Lys His Pro Glu Ala
   580 585 590
   Thr Tyr Ser Arg Cys Gly Ser Gly Pro Trp Ile Thr Pro Arg Cys Leu
   595 600 605
   Val His Tyr Pro Tyr Arg Leu Trp His Tyr Pro Cys Thr Val Asn Tyr
   610 615 620
   Thr Leu Phe Lys Val Arg Met Tyr Val Gly Gly Val Glu His Arg Leu
   625 630 635 640
   Glu Ala Ala Cys Asn Trp Thr Arg Gly Glu Arg Cys Asp Leu Asp Asp
   645 650 655
   Arg Asp Arg Ser Glu Leu Ser Pro Leu Leu Leu Ser Thr Thr Gln Trp
   660 665 670
   Gln Val Leu Pro Cys Ser Phe Thr Thr Leu Pro Ala Leu Thr Thr Gly
   675 680 685
   Leu Ile His Leu His Gln Asn Ile Val Asp Val Gln Tyr Leu Tyr Gly
   690 695 700
   Val Gly Ser Ser Ile Val Ser Trp Ala Ile Lys Trp Glu Tyr Val Ile
   705 710 715 720
   Leu Leu Phe Leu Leu Leu Ala Asp Ala Arg Ile Cys Ser Cys Leu Trp
   725 730 735
   Met Met Leu Leu Ile Ser Gln Ala Glu Ala
   740 745
<210> 8
   <211> 746
   <212> PRT
   <213> Hepatitis C virus
<400> 8
   Met Ser Thr Asn Pro Lys Pro Gln Arg Lys Thr Lys Arg Asn Thr Asn
   1               5                   10                  15
   Arg Arg Pro Gln Asp Val Lys Phe Pro Gly Gly Gly Gln Ile Val Gly
               20                  25                  30
   Gly Val Tyr Leu Leu Pro Arg Arg Gly Pro Arg Leu Gly Val Arg Ala
           35                  40                  45
   Thr Arg Lys Thr Ser Glu Arg Ser Gln Pro Arg Gly Arg Arg Gln Pro
       50                  55                  60
   Ile Pro Lys Ala Arg Arg Pro Glu Gly Arg Ser Trp Ala Gln Pro Gly
   65                  70                  75                  80
   Tyr Pro Trp Pro Leu Tyr Gly Asn Glu Gly Cys Gly Trp Ala Gly Trp
                   85                  90                  95
   Leu Leu Ser Pro Arg Gly Ser Arg Pro Ser Trp Gly Pro Thr Asp Pro
               100                 105                 110
   Arg Arg Arg Ser Arg Asn Leu Gly Lys Val Ile Asp Thr Leu Thr Cys
           115                 120                 125
   Gly Phe Ala Asp Leu Met Gly Tyr Ile Pro Leu Val Gly Ala Pro Leu
       130                 135                 140
   Gly Gly Ala Ala Arg Ala Leu Ala His Gly Val Arg Val Leu Glu Asp
   145                 150                 155                 160
   Gly Val Asn Tyr Ala Thr Gly Asn Leu Pro Gly Cys Ser Phe Ser Ile
                   165                 170                 175
   Phe Leu Leu Ala Leu Leu Ser Cys Leu Thr Val Pro Ala Ser Ala Tyr
               180                 185                 190
   Gln Val Arg Asn Ser Ser Gly Ile Tyr His Val Thr Asn Asp Cys Pro
           195                 200                 205
   Asn Ser Ser Ile Val Tyr Glu Thr Ala Asp Thr Ile Leu His Ser Pro
       210                 215                 220
   Gly Cys Val Pro Cys Val Arg Glu Gly Asn Ala Ser Lys Cys Trp Val
   225 230 235 240
   Ala Leu Ala Pro Thr Val Ala Thr Arg Asp Gly Arg Leu Pro Thr Thr
   245 250 255
   Gln Leu Arg Arg His Ile Asp Leu Leu Val Gly Ser Ala Thr Leu Cys
   260 265 270
   Ser Ala Leu Tyr Val Gly Asp Leu Cys Gly Ser Val Phe Leu Val Gly
   275 280 285
   Gln Leu Phe Thr Phe Ser Pro Arg Arg His Trp Thr Thr Gln Asp Cys
   290 295 300
   Asn Cys Ser Ile Tyr Pro Gly His Ile Thr Gly His Arg Met Ala Trp
   305 310 315 320
   Asp Met Met Met Asn Trp Ser Pro Thr Thr Ala Leu Val Val Ala Gln
   325 330 335
   Leu Leu Arg Ile Pro Gln Ala Ile Leu Asp Met Ile Ala Gly Ala His
   340 345 350
   Trp Gly Val Leu Ala Gly Ile Ala Tyr Phe Ser Met Val Gly Asn Trp
   355 360 365
   Ala Lys Val Leu Val Val Leu Leu Leu Phe Ala Gly Val Asp Ala His
   370 375 380
   Thr Arg Val Thr Gly Gly Ser Ala Ala Arg Ala Thr Ala Arg Leu Thr
   385 390 395 400
   Thr Leu Phe Ser Pro Gly Ala Lys Gln Asp Ile Gln Leu Ile Asn Thr
   405 410 415
   Asn Gly Ser Trp His Ile Asn Arg Thr Ala Leu Asn Cys Asn Ala Ser
   420 425 430
   Leu Asp Thr Gly Trp Val Ala Gly Leu Phe Tyr Tyr His Lys Phe Asn
   435 440 445
   Ser Ser Gly Cys Pro Glu Arg Met Ala Ser Cys Arg Pro Leu Ala Asp
   450 455 460
   Phe Asp Gln Gly Trp Gly Pro Ile Ser Tyr Ala Asn Gly Ser Gly Pro
   465 470 475 480
   Glu His Arg Pro Tyr Cys Trp His Tyr Pro Pro Lys Pro Cys Gly Ile
   485 490 495
   Ile Pro Ala Lys Thr Val Cys Gly Pro Val Tyr Cys Phe Thr Pro Ser
   500 505 510
   Pro Val Val Val Gly Thr Thr Asp Arg Ser Gly Ala Pro Thr Phe Asn
   515 520 525
   Trp Gly Asp Asn Asp Thr Asp Val Leu Val Leu Asn Asn Thr Arg Pro
   530 535 540
   Pro Leu Gly Asn Trp Phe Gly Cys Thr Trp Met Asn Ser Ser Gly Tyr
   545 550 555 560
   Thr Lys Val Cys Gly Ala Pro Pro Cys Ile Ile Gly Gly Val Gly Asn
   565 570 575
   Asn Thr Leu His Cys Pro Thr Asp Cys Phe Arg Lys His Pro Glu Ala
   580 585 590
   Thr Tyr Ser Arg Cys Gly Ser Gly Pro Trp Ile Thr Pro Arg Cys Leu
   595 600 605
   Val His Tyr Pro Tyr Arg Leu Trp His Tyr Pro Cys Thr Val Asn Tyr
   610 615 620
   Thr Leu Phe Lys Val Arg Met Tyr Val Gly Gly Val Glu His Arg Leu
   625 630 635 640
   Glu Ala Ala Cys Asn Trp Thr Arg Gly Glu Arg Cys Asn Leu Asp Asp
   645 650 655
   Arg Asp Arg Ser Glu Leu Ser Pro Leu Leu Leu Ser Thr Thr Gln Trp
   660 665 670
   Gln Val Leu Pro Cys Ser Phe Thr Thr Leu Pro Ala Leu Thr Thr Gly
   675 680 685
   Leu Ile His Leu His Gln Asn Ile Val Asp Val Gln Tyr Leu Tyr Gly
   690 695 700
   Val Gly Ser Ser Ile Val Ser Trp Ala Ile Lys Trp Glu Tyr Val Ile
   705 710 715 720
   Leu Leu Phe Leu Leu Leu Ala Asp Ala Arg Val Cys Ser Cys Leu Trp
   725 730 735
   Met Met Leu Leu Ile Ser Gln Val Glu Ala
               740                 745
<210> 9
   <211> 746
   <212> PRT
   <213> Hepatitis C virus
<400> 9
   Met Ser Thr Asn Pro Lys Pro Gln Arg Lys Thr Lys Arg Asn Thr Asn
   1               5                   10                  15
   Arg Arg Pro Gln Asp Val Lys Phe Pro Gly Gly Gly Gln Ile Val Gly
               20                  25                  30
   Gly Val Tyr Leu Leu Pro Arg Arg Gly Pro Arg Leu Gly Val Arg Ala
           35                  40                  45
   Thr Arg Lys Thr Ser Glu Arg Ser Gln Pro Arg Gly Arg Arg Gln Pro
       50                  55                  60
   Ile Pro Lys Ala Arg Arg Pro Glu Gly Arg Thr Trp Ala Gln Pro Gly
   65                  70                  75                  80
   Tyr Pro Trp Pro Leu Tyr Gly Asn Glu Gly Cys Gly Trp Ala Gly Trp
                   85                  90                  95
   Leu Leu Ser Pro Arg Gly Ser Arg Pro Ser Trp Gly Pro Thr Asp Pro
               100                 105                 110
   Arg Arg Arg Ser Arg Asn Leu Gly Lys Val Ile Asp Thr Leu Thr Cys
           115                 120                 125
   Gly Phe Ala Asp Leu Met Gly Tyr Ile Pro Leu Val Gly Ala Pro Leu
       130                 135                 140
   Gly Gly Ala Ala Arg Ala Leu Ala His Gly Val Arg Val Leu Glu Asp
   145                 150                 155                 160
   Gly Val Asn Tyr Ala Thr Gly Asn Leu Pro Gly Cys Ser Phe Ser Ile
                   165                 170                 175
   Phe Leu Leu Ala Leu Leu Ser Cys Leu Thr Val Pro Ala Ser Ala Tyr
               180                 185                 190
   Gln Val Arg Asn Ser Ser Gly Leu Tyr His Val Thr Asn Asp Cys Pro
           195                 200                 205
   Asn Ser Ser Ile Val Tyr Glu Thr Ala Asp Thr Ile Leu His Ser Pro
   210 215 220
   Gly Cys Val Pro Cys Val Arg Glu Gly Asn Ala Ser Lys Cys Trp Val
   225 230 235 240
   Ala Val Thr Pro Thr Val Ala Thr Arg Asp Gly Arg Leu Pro Ala Thr
   245 250 255
   Gln Leu Arg Arg His Ile Asp Leu Leu Val Gly Ser Ala Thr Leu Cys
   260 265 270
   Ser Ala Leu Tyr Val Gly Asp Leu Cys Gly Ser Ile Phe Leu Val Gly
   275 280 285
   Gln Leu Phe Thr Phe Ser Pro Arg Arg His Trp Thr Thr Gln Asp Cys
   290 295 300
   Asn Cys Ser Ile Tyr Pro Gly His Ile Thr Gly His Arg Met Ala Trp
   305 310 315 320
   Asp Met Met Met Asn Trp Ser Pro Thr Thr Ala Leu Val Val Ala Gln
   325 330 335
   Leu Leu Arg Val Pro Gln Ala Ile Leu Asp Met Ile Ala Gly Ala His
   340 345 350
   Trp Gly Val Leu Ala Gly Ile Ala Tyr Phe Ser Met Val Gly Asn Trp
   355 360 365
   Ala Lys Val Leu Val Val Leu Leu Leu Phe Ala Gly Val Asp Ala Gly
   370 375 380
   Thr His Val Thr Gly Gly Ser Ala Ala Lys Asp Thr Ser Gly Phe Thr
   385 390 395 400
   Ser Leu Phe Arg Ile Gly Ala Arg Gln Asn Ile Gln Leu Ile Asn Ser
   405 410 415
   Asn Gly Ser Trp His Ile Asn Arg Thr Ala Leu Asn Cys Asn Glu Ser
   420 425 430
   Leu Asp Thr Gly Trp Val Ala Gly Leu Leu Tyr Tyr His Lys Phe Asn
   435 440 445
   Ser Ser Gly Cys Pro Glu Arg Met Ala Ser Cys Arg Ser Leu Ala Asp
   450 455 460
   Phe Asp Gln Gly Trp Gly Pro Ile Ser Tyr Ala Asn Gly Ser Gly Pro
   465 470 475 480
   Glu His Arg Pro Tyr Cys Trp His Tyr Pro Pro Lys Pro Cys Gly Ile
   485 490 495
   Val Pro Ala Gln Ser Val Cys Gly Pro Val Tyr Cys Phe Thr Pro Ser
   500 505 510
   Pro Val Val Val Gly Thr Thr Asp Lys Ser Gly Ala Pro Thr Tyr Asn
   515 520 525
   Trp Gly Cys Asn Glu Thr Asp Val Phe Val Leu Asn Asn Thr Arg Pro
   530 535 540
   Pro Leu Gly Asn Trp Phe Gly Cys Thr Trp Met Asn Ser Ser Gly Phe
   545 550 555 560
   Thr Lys Val Cys Gly Ala Pro Pro Cys Val Ile Gly Gly Val Gly Asn
   565 570 575
   Asn Thr Leu His Cys Pro Thr Asp Cys Phe Arg Lys His Pro Glu Ala
   580 585 590
   Thr Tyr Ser Arg Cys Gly Ser Gly Pro Trp Ile Thr Pro Arg Cys Leu
   595 600 605
   Val His Tyr Ala Tyr Arg Leu Trp His Tyr Pro Cys Thr Val Asn Tyr
   610 615 620
   Thr Leu Phe Lys Val Arg Met Tyr Val Gly Gly Val Glu His Arg Leu
   625 630 635 640
   Asp Val Ala Cys Asn Trp Thr Arg Gly Glu Arg Cys Asn Leu Asp Asp
   645 650 655
   Arg Asp Arg Ser Glu Leu Ser Pro Leu Leu Leu Ser Thr Thr Gln Trp
   660 665 670
   Gln Val Leu Pro Cys Ser Phe Thr Thr Leu Pro Ala Leu Ser Thr Gly
   675 680 685
   Leu Ile His Leu His Gln Asn Ile Val Asp Val Gln Tyr Leu Tyr Gly
   690 695 700
   Val Gly Ser Ser Ile Val Ser Trp Ala Ile Lys Trp Glu Tyr Val Ile
   705 710 715 720
   Leu Leu Phe Leu Leu Leu Ala Asp Ala Arg Ile Cys Ser Cys Leu Trp
   725 730 735
   Met Met Leu Leu Ile Ser Gln Ala Glu Ala
   740 745
<210> 10
   <211> 746
   <212> PRT
   <213> Hepatitis C virus
<400> 10
   Met Ser Thr Asn Pro Lys Pro Gln Arg Lys Thr Lys Arg Asn Thr Asn
   1               5                   10                  15
   Arg Arg Pro Gln Asp Val Lys Phe Pro Gly Gly Gly Gln Ile Val Gly
               20                  25                  30
   Gly Val Tyr Leu Leu Pro Arg Arg Gly Pro Arg Leu Gly Val Arg Ala
           35                  40                  45
   Thr Arg Lys Thr Ser Glu Arg Ser Gln Pro Arg Gly Arg Arg Gln Pro
       50                  55                  60
   Ile Pro Lys Ala Arg Arg Pro Glu Gly Arg Thr Trp Ala Gln Pro Gly
   65                  70                  75                  80
   Tyr Pro Trp Pro Leu Tyr Gly Asn Glu Gly Cys Gly Trp Ala Gly Trp
                   85                  90                  95
   Leu Leu Ser Pro Arg Gly Ser Arg Pro Ser Trp Gly Pro Thr Asp Pro
               100                 105                 110
   Arg Arg Arg Ser Arg Asn Leu Gly Lys Val Ile Asp Thr Leu Thr Cys
           115                 120                 125
   Gly Phe Ala Asp Leu Met Gly Tyr Ile Pro Leu Val Gly Ala Pro Leu
       130                 135                 140
   Gly Gly Ala Ala Arg Ala Leu Ala His Gly Val Arg Val Leu Glu Asp
   145                 150                 155                 160
   Gly Val Asn Tyr Ala Thr Gly Asn Leu Pro Gly Cys Ser Phe Ser Ile
                   165                 170                 175
   Phe Leu Leu Ala Leu Leu Ser Cys Leu Thr Val Pro Ala Ser Ala Tyr
   180 185 190
   Gln Val Arg Asn Ser Ser Gly Leu Tyr His Val Thr Asn Asp Cys Pro
   195 200 205
   Asn Ser Ser Ile Val Tyr Glu Thr Ala Asp Thr Ile Leu His Ser Pro
   210 215 220
   Gly Cys Val Pro Cys Val Arg Glu Gly Asn Ala Ser Lys Cys Trp Val
   225 230 235 240
   Ala Val Thr Pro Thr Val Ala Thr Arg Asp Gly Arg Leu Pro Ala Thr
   245 250 255
   Gln Leu Arg Arg His Ile Asp Leu Leu Val Gly Ser Ala Thr Leu Cys
   260 265 270
   Ser Ala Leu Tyr Val Gly Asp Leu Cys Gly Ser Ile Phe Leu Val Gly
   275 280 285
   Gln Leu Phe Thr Phe Ser Pro Arg Arg His Trp Thr Thr Gln Asp Cys
   290 295 300
   Asn Cys Ser Ile Tyr Pro Gly His Ile Thr Gly His Arg Met Ala Trp
   305 310 315 320
   Asp Met Met Met Asn Trp Ser Pro Thr Thr Ala Leu Val Val Ala Gln
   325 330 335
   Leu Leu Arg Val Pro Gln Ala Ile Leu Asp Met Ile Ala Gly Ala His
   340 345 350
   Trp Gly Val Leu Ala Gly Ile Ala Tyr Phe Ser Met Val Gly Asn Trp
   355 360 365
   Ala Lys Val Leu Val Val Leu Leu Leu Phe Ala Gly Val Asp Ala Gly
   370 375 380
   Thr His Val Thr Gly Gly Ser Ala Ala Lys Asp Thr Ser Gly Phe Thr
   385 390 395 400
   Ser Leu Phe Arg Ile Gly Ala Arg Gln Asn Ile Gln Leu Ile Asn Ser
   405 410 415
   Asn Gly Ser Trp His Ile Asn Arg Thr Ala Leu Asn Cys Asn Glu Ser
   420 425 430
   Leu Asp Thr Gly Trp Val Ala Gly Leu Leu Tyr Tyr His Lys Phe Asn
           435                 440                 445
   Ser Ser Gly Cys Pro Glu Arg Met Ala Ser Cys Arg Ser Leu Ala Asp
       450                 455                 460
   Phe Asp Gln Gly Trp Gly Pro Ile Ser Tyr Ala Asn Gly Ser Gly Pro
   465                 470                 475                 480
   Glu His Arg Pro Tyr Cys Trp His Tyr Pro Pro Lys Pro Cys Gly Ile
                   485                 490                 495
   Val Pro Ala Gln Ser Val Cys Gly Pro Val Tyr Cys Phe Thr Pro Ser
               500                 505                 510
   Pro Val Val Val Gly Thr Thr Asp Lys Ser Gly Ala Pro Thr Tyr Asn
           515                 520                 525
   Trp Gly Cys Asn Glu Thr Asp Val Phe Val Leu Asn Asn Thr Arg Pro
       530                 535                 540
   Pro Leu Gly Asn Trp Phe Gly Cys Thr Trp Met Asn Ser Ser Gly Phe
   545                 550                 555                 560
   Thr Lys Val Cys Gly Ala Pro Pro Cys Val Ile Gly Gly Val Gly Asn
                   565                 570                 575
   Asn Thr Leu His Cys Pro Thr Asp Cys Phe Arg Lys His Pro Glu Ala
               580                 585                 590
   Thr Tyr Ser Arg Cys Gly Ser Gly Pro Trp Ile Thr Pro Arg Cys Leu
           595                 600                 605
   Val His Tyr Ala Tyr Arg Leu Trp His Tyr Pro Cys Thr Val Asn Tyr
       610                 615                 620
   Thr Leu Phe Lys Val Arg Met Tyr Val Gly Gly Val Glu His Arg Leu
   625                 630                 635                 640
   Asp Val Ala Cys Asn Trp Thr Arg Gly Glu Arg Cys Asn Leu Asp Asp
                   645                 650                 655
   Arg Asp Arg Ser Glu Leu Ser Pro Leu Leu Leu Ser Thr Thr Gln Trp
               660                 665                 670
   Gln Val Leu Pro Cys Ser Phe Thr Thr Leu Pro Ala Leu Ser Thr Gly
           675                 680                 685
   Leu Ile His Leu His Gln Asn Ile Val Asp Val Gln Tyr Leu Tyr Gly
   690 695 700
   Val Gly Ser Ser Ile Val Ser Trp Ala Ile Lys Trp Glu Tyr Val Ile
   705 710 715 720
   Leu Leu Phe Leu Leu Leu Ala Asp Ala Arg Ile Cys Ser Cys Leu Trp
   725 730 735
   Met Met Leu Leu Ile Ser Gln Ala Glu Ala
   740 745
<210> 11
   <211> 746
   <212> PRT
   <213> Hepatitis C virus
<400> 11
   Met Ser Thr Asn Pro Lys Pro Gln Arg Lys Thr Lys Arg Asn Thr Asn
   1               5                   10                  15
   Arg Arg Pro Gln Asp Val Lys Phe Pro Gly Gly Gly Gln Ile Val Gly
               20                  25                  30
   Gly Val Tyr Leu Leu Pro Arg Arg Gly Pro Arg Leu Gly Val Arg Ala
           35                  40                  45
   Thr Arg Lys Thr Ser Glu Arg Ser Gln Pro Arg Gly Arg Arg Gln Pro
       50                  55                  60
   Ile Pro Lys Ala Arg Arg Pro Glu Gly Arg Thr Trp Ala Gln Pro Gly
   65                  70                  75                  80
   Tyr Pro Trp Pro Leu Tyr Gly Asn Glu Gly Cys Gly Trp Ala Gly Trp
                   85                  90                  95
   Leu Leu Ser Pro Arg Gly Ser Arg Pro Ser Trp Gly Pro Thr Asp Pro
               100                 105                 110
   Arg Arg Arg Ser Arg Asn Leu Gly Lys Val Ile Asp Thr Leu Thr Cys
           115                 120                 125
   Gly Phe Ala Asp Leu Met Gly Tyr Ile Pro Leu Val Gly Ala Pro Leu
       130                 135                 140
   Gly Gly Ala Ala Arg Ala Leu Ala His Gly Val Arg Val Leu Glu Asp
   145                 150                 155                 160
   Gly Val Asn Tyr Ala Thr Gly Asn Leu Pro Gly Cys Ser Phe Ser Ile
                   165                 170                 175
   Phe Leu Leu Ala Leu Leu Ser Cys Leu Thr Val Pro Ala Ser Ala Tyr
               180                 185                 190
   Gln Val Arg Asn Ser Ser Gly Leu Tyr His Val Thr Asn Asp Cys Pro
           195                 200                 205
   Asn Ser Ser Ile Val Tyr Glu Thr Ala Asp Thr Ile Leu His Ser Pro
       210                 215                 220
   Gly Cys Val Pro Cys Val Arg Glu Gly Asn Ala Ser Lys Cys Trp Val
   225                 230                 235                 240
   Ala Val Thr Pro Thr Val Ala Thr Arg Asp Gly Arg Leu Pro Ala Thr
                   245                 250                 255
   Gln Leu Arg Arg His Ile Asp Leu Leu Val Gly Ser Ala Thr Leu Cys
               260                 265                 270
   Ser Ala Leu Tyr Val Gly Asp Leu Cys Gly Ser Ile Phe Leu Val Gly
           275                 280                 285
   Gln Leu Phe Thr Phe Ser Pro Arg Arg His Trp Thr Thr Gln Asp Cys
       290                 295                 300
   Asn Cys Ser Ile Tyr Pro Gly His Ile Thr Gly His Arg Met Ala Trp
   305                 310                 315                 320
   Asp Met Met Met Asn Trp Ser Pro Thr Thr Ala Leu Val Val Ala Gln
                   325                 330                 335
   Leu Leu Arg Val Pro Gln Ala Ile Leu Asp Met Ile Ala Gly Ala His
               340                 345                 350
   Trp Gly Val Leu Ala Gly Ile Ala Tyr Phe Ser Met Val Gly Asn Trp
           355                 360                 365
   Ala Lys Val Leu Val Val Leu Leu Leu Phe Ala Gly Val Asp Ala Gly
       370                 375                 380
   Thr His Val Thr Gly Gly Ser Ala Ala Lys Asp Thr Ser Gly Phe Thr
   385                 390                 395                 400
   Ser Leu Phe Arg Ile Gly Ala Arg Gln Asn Ile Gln Leu Ile Asn Ser
                   405                 410                 415
   Asn Gly Ser Trp His Ile Asn Arg Thr Ala Leu Asn Cys Asn Glu Ser
               420                 425                 430
   Leu Asp Thr Gly Trp Val Ala Gly Leu Leu Tyr Tyr His Lys Phe Asn
           435                 440                 445
   Ser Ser Gly Cys Pro Glu Arg Met Ala Ser Cys Arg Ser Leu Ala Asp
       450                 455                 460
   Phe Asp Gln Gly Trp Gly Pro Ile Ser Tyr Ala Asn Gly Ser Gly Pro
   465                 470                 475                 480
   Glu His Arg Pro Tyr Cys Trp His Tyr Pro Pro Lys Pro Cys Gly Ile
                   485                 490                 495
   Val Pro Ala Gln Ser Val Cys Gly Pro Val Tyr Cys Phe Thr Pro Ser
               500                 505                 510
   Pro Val Val Val Gly Thr Thr Asp Lys Ser Gly Ala Pro Thr Tyr Asn
           515                 520                 525
   Trp Gly Cys Asn Glu Thr Asp Val Phe Val Leu Asn Asn Thr Arg Pro
       530                 535                 540
   Pro Leu Gly Asn Trp Phe Gly Cys Thr Trp Met Asn Ser Ser Gly Phe
   545                 550                 555                 560
   Thr Lys Val Cys Gly Ala Pro Pro Cys Val Ile Gly Gly Val Gly Asn
                   565                 570                 575
   Asn Thr Leu His Cys Pro Thr Asp Cys Phe Arg Lys His Pro Glu Ala
               580                 585                 590
   Thr Tyr Ser Arg Cys Gly Ser Gly Pro Trp Ile Thr Pro Arg Cys Leu
           595                 600                 605
   Val His Tyr Ala Tyr Arg Leu Trp His Tyr Pro Cys Thr Val Asn Tyr
       610                 615                 620
   Thr Leu Phe Lys Val Arg Met Tyr Val Gly Gly Val Glu His Arg Leu
   625                 630                 635                 640
   Asp Val Ala Cys Asn Trp Thr Arg Gly Glu Arg Cys Asn Leu Asp Asp
                   645                 650                 655
   Arg Asp Arg Ser Glu Leu Ser Pro Leu Leu Leu Ser Thr Thr Gln Trp
   660 665 670
   Gln Val Leu Pro Cys Ser Phe Thr Thr Leu Pro Ala Leu Ser Thr Gly
   675 680 685
   Leu Ile His Leu His Gln Asn Ile Val Asp Val Gln Tyr Leu Tyr Gly
   690 695 700
   Val Gly Ser Ser Ile Val Ser Trp Ala Ile Lys Trp Glu Tyr Val Ile
   705 710 715 720
   Leu Leu Phe Leu Leu Leu Ala Asp Ala Arg Ile Cys Ser Cys Leu Trp
   725 730 735
   Met Met Leu Leu Ile Ser Gln Ala Glu Ala
   740 745
<210> 12
   <211> 746
   <212> PRT
   <213> Hepatitis C virus
<400> 12
   Met Ser Thr Asn Pro Lys Pro Gln Arg Lys Thr Lys Arg Asn Thr Asn
   1               5                   10                  15
   Arg Arg Pro Gln Asp Val Lys Phe Pro Gly Gly Gly Gln Ile Val Gly
               20                  25                  30
   Gly Val Tyr Leu Leu Pro Arg Arg Gly Pro Arg Leu Gly Val Arg Ala
           35                  40                  45
   Thr Arg Lys Thr Ser Glu Arg Ser Gln Pro Arg Gly Arg Arg Gln Pro
       50                  55                  60
   Ile Pro Lys Ala Arg Arg Pro Glu Gly Arg Thr Trp Ala Gln Pro Gly
   65                  70                  75                  80
   Tyr Pro Trp Pro Leu Tyr Gly Asn Glu Gly Cys Gly Trp Ala Gly Trp
                   85                  90                  95
   Leu Leu Ser Pro Arg Gly Ser Arg Pro Ser Trp Gly Pro Thr Asp Pro
               100                 105                 110
   Arg Arg Arg Ser Arg Asn Leu Gly Lys Val Ile Asp Thr Leu Thr Cys
           115                 120                 125
   Gly Phe Ala Asp Leu Met Gly Tyr Ile Pro Leu Val Gly Ala Pro Leu
   130 135 140
   Gly Gly Ala Ala Arg Ala Leu Ala His Gly Val Arg Val Leu Glu Asp
   145 150 155 160
   Gly Val Asn Tyr Ala Thr Gly Asn Leu Pro Gly Cys Ser Phe Ser Ile
   165 170 175
   Phe Leu Leu Ala Leu Leu Ser Cys Leu Thr Val Pro Ala Ser Ala Tyr
   180 185 190
   Gln Val Arg Asn Ser Ser Gly Leu Tyr His Val Thr Asn Asp Cys Pro
   195 200 205
   Asn Ser Ser Ile Val Tyr Glu Thr Ala Asp Ser Ile Leu His Ser Pro
   210 215 220
   Gly Cys Val Pro Cys Val Arg Glu Gly Asn Ala Ser Lys Cys Trp Val
   225 230 235 240
   Ala Val Ala Pro Thr Val Ala Thr Arg Asp Gly Lys Leu Pro Ala Thr
   245 250 255
   Gln Leu Arg Arg His Ile Asp Leu Leu Val Gly Ser Ala Thr Leu Cys
   260 265 270
   Ser Ala Leu Tyr Val Gly Asp Leu Cys Gly Ser Val Phe Leu Val Ser
   275 280 285
   Gln Leu Phe Thr Phe Ser Pro Arg Arg His Trp Thr Thr Gln Asp Cys
   290 295 300
   Asn Cys Ser Ile Tyr Pro Gly His Ile Thr Gly His Arg Met Ala Trp
   305 310 315 320
   Asp Met Met Met Asn Trp Ser Pro Thr Thr Ala Leu Val Val Ser Gln
   325 330 335
   Leu Leu Arg Val Pro Gln Ala Ile Leu Asp Met Ile Ala Gly Ala His
   340 345 350
   Trp Gly Val Leu Ala Gly Ile Ala Tyr Phe Ser Met Val Gly Asn Trp
   355 360 365
   Ala Lys Val Leu Val Val Leu Leu Leu Phe Ala Gly Val Asp Ala Glu
   370 375 380
   Thr His Thr Thr Gly Gly Ser Ala Ala Tyr Ala Thr Ser Gly Phe Val
   385 390 395 400
   Gly Leu Phe Arg Gln Gly Ala Lys Gln Asn Ile Gln Leu Ile Asn Thr
   405 410 415
   Asn Gly Ser Trp His Val Asn Arg Thr Ala Leu Asn Cys Asn Ala Ser
   420 425 430
   Leu Asp Thr Gly Trp Val Ala Gly Leu Phe Tyr Tyr His Lys Phe Asn
   435 440 445
   Ser Ser Gly Cys Pro Glu Arg Leu Ala Ser Cys Lys Pro Leu Ala Asn
   450 455 460
   Phe Asp Gln Gly Trp Gly Ser Ile Ser Tyr Thr Asn Gly Ser Gly Pro
   465 470 475 480
   Glu His Arg Pro Tyr Cys Trp His Tyr Pro Pro Lys Pro Cys Gly Ile
   485 490 495
   Val Pro Ala Gln Asn Val Cys Gly Pro Val Tyr Cys Phe Thr Pro Ser
   500 505 510
   Pro Val Val Val Gly Thr Thr Asp Arg Leu Gly Ala Pro Thr Phe Asn
   515 520 525
   Trp Gly Glu Asn Glu Ser Asp Val Phe Val Leu Asn Asn Thr Arg Pro
   530 535 540
   Pro Ser Gly Asn Trp Phe Gly Cys Thr Trp Met Asn Ser Ser Gly Phe
   545 550 555 560
   Thr Lys Val Cys Gly Ala Pro Pro Cys Asn Ile Gly Gly Val Gly Asn
   565 570 575
   Asn Thr Leu His Cys Pro Thr Asp Cys Phe Arg Lys His Pro Glu Ala
   580 585 590
   Thr Tyr Ser Arg Cys Gly Ser Gly Pro Trp Val Thr Pro Arg Cys Leu
   595 600 605
   Val His Tyr Pro Tyr Arg Leu Trp His Tyr Pro Cys Thr Leu Asn Tyr
   610 615 620
   Ser Leu Phe Lys Val Arg Met Tyr Val Gly Gly Ile Glu His Arg Leu
   625 630 635 640
   Glu Val Ala Cys Asn Trp Thr Arg Gly Glu Arg Cys Asn Leu Asp Asp
                   645                 650                 655
   Arg Asp Arg Ser Glu Leu Ser Pro Leu Leu Leu Thr Thr Thr Gln Trp
               660                 665                 670
   Gln Val Leu Pro Cys Ser Phe Thr Thr Leu Pro Ala Leu Thr Thr Gly
           675                 680                 685
   Leu Ile His Leu His Gln Asn Val Val Asp Val Gln Tyr Leu Tyr Gly
       690                 695                 700
   Val Gly Ser Ser Ile Val Ser Trp Ala Ile Lys Trp Glu Tyr Val Ile
   705                 710                 715                 720
   Leu Leu Phe Leu Leu Leu Ala Asp Ala Arg Ile Cys Ser Cys Leu Trp
                   725                 730                 735
   Met Met Leu Leu Ile Ser Gln Ala Glu Ala
               740                 745
<210> 13
   <211> 746
   <212> PRT
   <213> Hepatitis C virus
<400> 13
   Met Ser Thr Asn Pro Lys Pro Gln Arg Lys Thr Lys Arg Asn Thr Asn
   1               5                   10                  15
   Arg Arg Pro Gln Asp Val Lys Phe Pro Gly Gly Gly Gln Ile Val Gly
               20                  25                  30
   Gly Val Tyr Leu Leu Pro Arg Arg Gly Pro Arg Leu Gly Val Arg Ala
           35                  40                  45
   Thr Arg Lys Thr Ser Glu Arg Ser Gln Pro Arg Gly Arg Arg Gln Pro
       50                  55                  60
   Ile Pro Lys Ala Arg Arg Pro Glu Gly Arg Thr Trp Ala Gln Pro Gly
   65                  70                  75                  80
   Tyr Pro Trp Pro Leu Tyr Gly Asn Glu Gly Cys Gly Trp Ala Gly Trp
                   85                  90                  95
   Leu Leu Ser Pro Arg Gly Ser Arg Pro Ser Trp Gly Pro Thr Asp Pro
               100                 105                 110
   Arg Arg Arg Ser Arg Asn Leu Gly Lys Val Ile Asp Thr Leu Thr Cys
           115                 120                 125
   Gly Phe Ala Asp Leu Met Gly Tyr Ile Pro Leu Val Gly Ala Pro Leu
       130                 135                 140
   Gly Gly Ala Ala Arg Ala Leu Ala His Gly Val Arg Val Leu Glu Asp
   145                 150                 155                 160
   Gly Val Asn Tyr Ala Thr Gly Asn Leu Pro Gly Cys Ser Phe Ser Ile
                   165                 170                 175
   Phe Leu Leu Ala Leu Leu Ser Cys Leu Thr Val Pro Ala Ser Ala Tyr
               180                 185                 190
   Gln Val Arg Asn Ser Ser Gly Leu Tyr His Val Thr Asn Asp Cys Pro
           195                 200                 205
   Asn Ser Ser Ile Val Tyr Glu Thr Ala Asp Thr Ile Leu His Ser Pro
       210                 215                 220
   Gly Cys Val Pro Cys Val Arg Glu Gly Asn Ala Ser Lys Cys Trp Val
   225                 230                 235                 240
   Ala Val Ala Pro Thr Val Ala Thr Arg Asp Gly Arg Leu Pro Thr Thr
                   245                 250                 255
   Gln Leu Arg Arg His Ile Asp Leu Leu Val Gly Ser Ala Thr Leu Cys
               260                 265                 270
   Ser Ala Leu Tyr Val Gly Asp Leu Cys Gly Ser Val Phe Leu Val Gly
           275                 280                 285
   Gln Leu Phe Thr Phe Ser Pro Arg His His Trp Thr Thr Gln Asp Cys
       290                 295                 300
   Asn Cys Ser Ile Tyr Pro Gly His Ile Thr Gly His Arg Met Ala Trp
   305                 310                 315                 320
   Asp Met Met Met Asn Trp Ser Pro Thr Thr Ala Leu Val Val Ala Gln
                   325                 330                 335
   Leu Leu Arg Val Pro Gln Ala Ile Leu Asp Met Ile Ala Gly Ala His
               340                 345                 350
   Trp Gly Val Leu Ala Gly Ile Ala Tyr Phe Ser Met Val Gly Asn Trp
           355                 360                 365
   Ala Lys Val Leu Val Val Leu Leu Leu Phe Ala Gly Val Asp Ala Thr
   370 375 380
   Thr His Thr Thr Gly Gly Ala Val Ala His Asn Thr Arg Met Phe Thr
   385 390 395 400
   Ser Ile Phe Ser Leu Gly Pro Arg Gln Glu Ile Gln Leu Val Asn Thr
   405 410 415
   Asn Gly Ser Trp His Ile Asn Arg Thr Ala Leu Asn Cys Asn Ala Ser
   420 425 430
   Leu Glu Thr Gly Trp Ile Ala Gly Leu Leu Tyr Ala Asn Arg Phe Asn
   435 440 445
   Ser Ser Gly Cys Pro Glu Arg Met Ala Ser Cys Lys Pro Leu Ala Asp
   450 455 460
   Phe Asp Gln Gly Trp Gly Pro Ile Ser Tyr Ala Asn Gly Ser Gly Pro
   465 470 475 480
   Glu His Arg Pro Tyr Cys Trp His Tyr Pro Pro Lys Pro Cys Gly Ile
   485 490 495
   Val Pro Ala Gln Asn Val Cys Gly Pro Val Tyr Cys Phe Thr Pro Ser
   500 505 510
   Pro Val Val Val Gly Thr Thr Asp Arg Leu Gly Thr Pro Thr Tyr Asp
   515 520 525
   Trp Gly Ser Asn Asp Thr Asp Val Phe Val Leu Asn Asn Thr Arg Pro
   530 535 540
   Pro Ala Gly Asn Trp Phe Gly Cys Thr Trp Met Asn Ser Ser Gly Tyr
   545 550 555 560
   Thr Lys Val Cys Gly Ala Pro Pro Cys Val Ile Gly Gly Val Ser Asn
   565 570 575
   Asn Thr Leu His Cys Pro Thr Asp Cys Phe Arg Lys His Pro Glu Ala
   580 585 590
   Thr Tyr Ser Arg Cys Gly Ser Gly Pro Trp Ile Thr Pro Arg Cys Leu
   595 600 605
   Val His Tyr Ala Tyr Arg Leu Trp His Tyr Pro Cys Thr Val Asn Tyr
   610 615 620
   Thr Leu Phe Lys Val Arg Met Tyr Val Gly Gly Val Glu His Arg Leu
   625 630 635 640
   Glu Val Ala Cys Asn Trp Thr Arg Gly Glu Arg Cys Asn Leu Asp Asp
   645 650 655
   Arg Asp Arg Ser Glu Leu Ser Pro Leu Leu Leu Ser Thr Thr Gln Trp
   660 665 670
   Gln Val Leu Pro Cys Ser Phe Thr Thr Leu Pro Ala Leu Thr Thr Gly
   675 680 685
   Leu Ile His Leu His Gln Asn Ile Val Asp Val Gln Tyr Leu Tyr Gly
   690 695 700
   Val Gly Ser Ser Ile Val Ser Trp Ala Val Lys Trp Glu Tyr Val Ile
   705 710 715 720
   Leu Leu Phe Leu Leu Leu Ala Asp Ala Arg Ile Cys Ser Cys Leu Trp
   725 730 735
   Met Met Leu Leu Ile Ser Gln Ala Glu Ala
   740 745
<210> 14
   <211> 746
   <212> PRT
   <213> Hepatitis C virus
<400> 14
   Met Ser Thr Asn Pro Lys Pro Gln Arg Lys Thr Lys Arg Asn Thr Asn
   1               5                   10                  15
   Arg Arg Pro Gln Asp Val Lys Phe Pro Gly Gly Gly Gln Ile Val Gly
               20                  25                  30
   Gly Val Tyr Leu Leu Pro Arg Arg Gly Pro Arg Leu Gly Val Arg Ala
           35                  40                  45
   Thr Arg Lys Thr Ser Glu Arg Ser Gln Pro Arg Gly Arg Arg Gln Pro
       50                  55                  60
   Ile Pro Lys Ala Arg Gln Pro Glu Gly Arg Ala Trp Ala Gln Pro Gly
   65                  70                  75                  80
   Tyr Pro Trp Pro Leu Tyr Gly Asn Glu Gly Leu Gly Trp Ala Gly Trp
   85 90 95
   Leu Leu Ser Pro Arg Gly Ser Arg Pro Ser Trp Gly Pro Thr Asp Pro
   100 105 110
   Arg Arg Arg Ser Arg Asn Leu Gly Lys Val Ile Asp Thr Leu Thr Cys
   115 120 125
   Gly Phe Ala Asp Leu Met Gly Tyr Ile Pro Leu Val Gly Ala Pro Leu
   130 135 140
   Gly Gly Ala Ala Arg Ala Leu Ala His Gly Val Arg Val Leu Glu Asp
   145 150 155 160
   Gly Val Asn Tyr Ala Thr Gly Asn Leu Pro Gly Cys Ser Phe Ser Ile
   165 170 175
   Phe Leu Leu Ala Leu Leu Ser Cys Leu Thr Ile Pro Ala Ser Ala Tyr
   180 185 190
   Glu Val Arg Asn Val Ser Gly Val Tyr His Val Thr Asn Asp Cys Ser
   195 200 205
   Asn Ala Ser Ile Val Tyr Glu Ala Ala Asp Met Ile Met His Thr Pro
   210 215 220
   Gly Cys Val Pro Cys Val Arg Glu Asn Asn Ser Ser Arg Cys Trp Val
   225 230 235 240
   Ala Leu Thr Pro Thr Leu Ala Ala Arg Asn Ala Ser Val Pro Thr Thr
   245 250 255
   Thr Ile Arg Arg His Val Asp Leu Leu Val Gly Ala Ala Ala Leu Cys
   260 265 270
   Ser Ala Met Tyr Val Gly Asp Leu Cys Gly Ser Val Phe Leu Val Ala
   275 280 285
   Gln Leu Phe Thr Phe Ser Pro Arg Arg His Glu Thr Val Gln Asp Cys
   290 295 300
   Asn Cys Ser Ile Tyr Pro Gly His Val Thr Gly His Arg Met Ala Trp
   305 310 315 320
   Asp Met Met Met Asn Trp Ser Pro Thr Ala Ala Leu Val Val Ser Gln
   325 330 335
   Leu Leu Arg Ile Pro Gln Ala Val Val Asp Met Val Ala Gly Ala His
               340                 345                 350
   Trp Gly Val Leu Ala Gly Leu Ala Tyr Tyr Ser Met Val Gly Asn Trp
           355                 360                 365
   Ala Lys Val Leu Ile Val Met Leu Leu Phe Ala Gly Val Asp Gly Gly
       370                 375                 380
   Thr Tyr Val Thr Gly Gly Thr Met Ala Lys Asn Thr Leu Gly Ile Thr
   385                 390                 395                 400
   Ser Leu Phe Ser Pro Gly Ser Ser Gln Lys Ile Gln Leu Val Asn Thr
                   405                 410                 415
   Asn Gly Ser Trp His Ile Asn Arg Thr Ala Leu Asn Cys Asn Asp Ser
               420                 425                 430
   Leu Asn Thr Gly Phe Leu Ala Ala Leu Phe Tyr Val His Lys Phe Asn
           435                 440                 445
   Ser Ser Gly Cys Pro Glu Arg Met Ala Ser Cys Ser Pro Ile Asp Ala
       450                 455                 460
   Phe Ala Gln Gly Trp Gly Pro Ile Thr Tyr Asn Glu Ser His Ser Ser
   465                 470                 475                 480
   Asp Gln Arg Pro Tyr Cys Trp His Tyr Ala Pro Arg Pro Cys Gly Ile
                   485                 490                 495
   Val Pro Ala Ala Gln Val Cys Gly Pro Val Tyr Cys Phe Thr Pro Ser
               500                 505                 510
   Pro Val Val Val Gly Thr Thr Asp Arg Phe Gly Val Pro Thr Tyr Ser
           515                 520                 525
   Trp Gly Glu Asn Glu Thr Asp Val Leu Leu Leu Asn Asn Thr Arg Pro
       530                 535                 540
   Pro Gln Gly Asn Trp Phe Gly Cys Thr Trp Met Asn Ser Thr Gly Phe
   545                 550                 555                 560
   Thr Lys Thr Cys Gly Gly Pro Pro Cys Asn Ile Gly Gly Ile Gly Asn
                   565                 570                 575
   Lys Thr Leu Thr Cys Pro Thr Asp Cys Phe Arg Lys His Pro Glu Ala
               580                 585                 590
   Thr Tyr Thr Lys Cys Gly Ser Gly Pro Trp Leu Thr Pro Arg Cys Leu
           595                 600                 605
   Val His Tyr Pro Tyr Arg Leu Trp His Tyr Pro Cys Thr Val Asn Phe
       610                 615                 620
   Thr Ile Phe Lys Val Arg Met Tyr Val Gly Gly Val Glu His Arg Leu
   625                 630                 635                     640
   Glu Ala Ala Cys Asn Trp Thr Arg Gly Glu Arg Cys Asn Leu Glu Asp
                   645                 650                 655
   Arg Asp Arg Ser Glu Leu Ser Pro Leu Leu Leu Ser Thr Thr Glu Trp
               660                 665                 670
   Gln Val Leu Pro Cys Ser Phe Thr Thr Leu Pro Ala Leu Ser Thr Gly
           675                 680                 685
   Leu Ile His Leu His Gln Asn Val Val Asp Val Gln Tyr Leu Tyr Gly
       690                 695                 700
   Ile Gly Ser Ala Val Val Ser Phe Ala Ile Lys Trp Glu Tyr Val Leu
   705                 710                 715                     720
   Leu Leu Phe Leu Leu Leu Ala Asp Ala Arg Val Cys Ala Cys Leu Trp
                   725                 730                 735
   Met Met Leu Leu Ile Ala Gln Ala Glu Ala
               740                 745
<210> 15
   <211> 746
   <212> PRT
   <213> Hepatitis C virus
<400> 15
   Met Ser Thr Asn Pro Lys Pro Gln Arg Lys Thr Lys Arg Asn Thr Asn
   1               5                   10                  15
   Arg Arg Pro Gln Asp Val Lys Phe Pro Gly Gly Gly Gln Ile Val Gly
               20                  25                  30
   Gly Val Tyr Leu Leu Pro Arg Arg Gly Pro Arg Leu Gly Val Arg Ala
           35                  40                  45
   Thr Arg Lys Thr Ser Glu Arg Ser Gln Pro Arg Gly Arg Arg Gln Pro
       50                  55                  60
   Ile Pro Lys Ala Arg Gln Pro Glu Gly Arg Ala Trp Ala Gln Pro Gly
   65                  70                  75                  80
   Tyr Pro Trp Pro Leu Tyr Gly Asn Glu Gly Met Gly Trp Ala Gly Trp
                   85                  90                  95
   Leu Leu Ser Pro Arg Gly Ser Arg Pro Ser Trp Gly Pro Thr Asp Pro
               100                 105                 110
   Arg Arg Arg Ser Arg Asn Leu Gly Lys Val Ile Asp Thr Leu Thr Cys
           115                 120                 125
   Gly Phe Ala Asp Leu Met Gly Tyr Ile Pro Leu Val Gly Ala Pro Leu
       130                 135                 140
   Gly Gly Val Ala Arg Ala Leu Ala His Gly Val Arg Val Val Glu Asp
   145                 150                 155                 160
   Gly Val Asn Tyr Ala Thr Gly Asn Leu Pro Gly Cys Ser Phe Ser Ile
                   165                 170                 175
   Phe Leu Leu Ala Leu Leu Ser Cys Leu Thr Ile Pro Ala Ser Ala Tyr
               180                 185                 190
   Glu Val Arg Asn Val Ser Gly Ile Tyr His Val Thr Asn Asp Cys Ser
           195                 200                 205
   Asn Ser Ser Ile Val Tyr Glu Ala Ala Asp Met Ile Met His Thr Pro
       210                 215                 220
   Gly Cys Val Pro Cys Val Arg Glu Gly Asn Ser Ser Arg Cys Trp Val
   225                 230                 235                 240
   Ala Leu Thr Pro Thr Leu Ala Ala Arg Asn Ala Ser Val Pro Thr Thr
                   245                 250                 255
   Ala Ile Arg Arg His Val Asp Leu Leu Val Gly Ala Ala Ala Phe Cys
               260                 265                 270
   Ser Ala Met Tyr Val Gly Asp Leu Cys Gly Ser Val Phe Leu Val Ser
           275                 280                 285
   Gln Leu Phe Thr Phe Ser Pro Arg Arg His Glu Thr Ile Gln Asp Cys
       290                 295                 300
   Asn Cys Ser Ile Tyr Pro Gly His Val Ser Gly His Arg Met Ala Trp
   305                 310                 315                 320
   Asp Met Met Met Asn Trp Ser Pro Thr Thr Ala Leu Val Val Ser Gln
   325 330 335
   Leu Leu Arg Ile Pro Gln Ala Ile Val Asp Met Val Ala Gly Ala His
   340 345 350
   Trp Gly Val Leu Ala Gly Leu Ala Tyr Tyr Ser Met Val Gly Asn Trp
   355 360 365
   Ala Lys Val Leu Ile Val Met Leu Leu Phe Ala Gly Val Asp Gly Glu
   370 375 380
   Thr Arg Val Thr Gly Gly Gln Ile Ala Arg Asn Ala Tyr Ser Leu Thr
   385 390 395 400
   Thr Leu Phe Ser Ser Gly Ser Ala Gln Asn Ile Gln Leu Ile Asn Thr
   405 410 415
   Asn Gly Ser Trp His Ile Asn Arg Thr Ala Leu Asn Cys Asn Asp Ser
   420 425 430
   Leu Asn Thr Gly Phe Leu Ala Ala Leu Phe Tyr Thr His Lys Phe Asn
   435 440 445
   Ala Ser Gly Cys Pro Glu Arg Leu Ala Ser Cys Arg Pro Ile Asp Lys
   450 455 460
   Phe Asp Gln Gly Trp Gly Pro Ile Thr Tyr Ala Glu Gln Gly Gly Gln
   465 470 475 480
   Asp Gln Arg Pro Tyr Cys Trp His Tyr Ala Pro Lys Pro Cys Gly Ile
   485 490 495
   Val Ser Ala Ser Lys Val Cys Gly Pro Val Tyr Cys Phe Thr Pro Ser
   500 505 510
   Pro Val Val Val Gly Thr Thr Asp Arg Phe Gly Val Pro Thr Tyr Ser
   515 520 525
   Trp Gly Glu Asn Glu Thr Asp Val Leu Leu Leu Asn Asn Thr Arg Pro
   530 535 540
   Pro Gln Gly Asn Trp Phe Gly Cys Thr Trp Met Asn Gly Thr Gly Phe
   545 550 555 560
   Thr Lys Thr Cys Gly Gly Pro Pro Cys Asn Ile Gly Gly Gly Gly Asn
   565 570 575
   Asn Thr Leu Thr Cys Pro Thr Asp Cys Phe Arg Lys His Pro Ala Ala
   580 585 590
   Thr Tyr Thr Lys Cys Gly Ser Gly Pro Trp Leu Thr Pro Arg Cys Leu
   595 600 605
   Val Asp Tyr Pro Tyr Arg Leu Trp His Tyr Pro Cys Thr Ala Asn Phe
   610 615 620
   Thr Ile Phe Lys Val Arg Met Tyr Val Gly Gly Val Glu His Arg Leu
   625 630 635 640
   Asp Ala Ala Cys Asn Trp Thr Arg Gly Glu Arg Cys Asn Leu Glu Asp
   645 650 655
   Arg Asp Arg Leu Glu Leu Ser Pro Leu Leu Leu Ser Thr Thr Glu Trp
   660 665 670
   Gln Val Leu Pro Cys Ser Phe Thr Thr Leu Pro Ala Leu Ser Thr Gly
   675 680 685
   Leu Ile His Leu His Gln Asn Ile Val Asp Val Gln Tyr Leu Tyr Gly
   690 695 700
   Ile Gly Ser Ala Val Val Ser Phe Ala Ile Lys Trp Asp Tyr Ile Val
   705 710 715 720
   Ile Leu Phe Leu Leu Leu Ala Asp Ala Arg Val Cys Ala Cys Leu Trp
   725 730 735
   Met Met Leu Leu Ile Ala Gln Ala Glu Ala
   740 745
<210> 16
   <211> 747
   <212> PRT
   <213> Hepatitis C virus
<400> 16
   Met Ser Thr Asn Pro Lys Pro Gln Arg Lys Thr Lys Arg Asn Thr Asn
   1               5                   10                  15
   Arg Arg Pro Gln Asp Val Lys Phe Pro Gly Gly Gly Gln Ile Val Gly
               20                  25                  30
   Gly Val Tyr Leu Leu Pro Arg Arg Gly Pro Arg Leu Gly Val Arg Ala
           35                  40                  45
   Ile Arg Lys Thr Ser Glu Arg Ser Gln Pro Arg Gly Arg Arg Gln Pro
       50                  55                  60
   Ile Pro Lys Ala Arg Arg Pro Glu Gly Arg Ala Trp Ala Gln Pro Gly
   65                  70                  75                  80
   Tyr Pro Trp Pro Leu Tyr Gly Asn Glu Gly Met Gly Trp Ala Gly Trp
                   85                  90                  95
   Leu Leu Ser Pro Arg Gly Ser Arg Pro Ser Trp Gly Pro Thr Asp Pro
               100                 105                 110
   Arg Arg Arg Ser Arg Asn Leu Gly Lys Val Ile Asp Thr Leu Thr Cys
           115                 120                 125
   Gly Leu Ala Asp Leu Met Gly Tyr Ile Pro Leu Val Gly Gly Pro Leu
       130                 135                 140
   Gly Gly Ala Ala Arg Ala Leu Ala His Gly Val Arg Val Leu Glu Asp
   145                 150                 155                 160
   Gly Val Asn Tyr Ala Thr Gly Asn Leu Pro Gly Cys Ser Phe Ser Ile
                   165                 170                 175
   Phe Leu Leu Ala Leu Leu Ser Cys Leu Thr Val Pro Ala Ser Ala His
               180                 185                 190
   Glu Val Arg Asn Ala Ser Gly Val Tyr His Val Thr Asn Asp Cys Ser
           195                 200                 205
   Asn Ser Ser Ile Val Phe Glu Ala Ala Asp Leu Ile Met His Thr Pro
       210                 215                 220
   Gly Cys Val Pro Cys Val Arg Glu Gly Asn Ser Ser Arg Cys Trp Val
   225                 230                 235                 240
   Ala Leu Thr Pro Thr Leu Ala Ala Arg Asn Ala Thr Ile Pro Thr Thr
                   245                 250                 255
   Thr Ile Arg His His Val Asp Leu Leu Val Gly Ala Ala Ala Leu Cys
               260                 265                 270
   Ser Ala Met Tyr Val Gly Asp Leu Cys Gly Ser Val Phe Leu Val Ser
           275                 280                 285
   Gln Leu Phe Thr Phe Ser Pro Arg Arg His Ala Thr Leu Gln Asp Cys
       290                 295                 300
   Asn Cys Ser Ile Tyr Pro Gly His Ala Ser Gly His Arg Met Ala Trp
   305                 310                 315                 320
   Asp Met Met Met Asn Trp Ser Pro Thr Thr Ala Leu Val Val Ser Gln
                   325                 330                 335
   Leu Leu Arg Ile Pro Gln Ala Val Ile Asp Met Val Ala Gly Ala His
               340                 345                 350
   Trp Gly Val Leu Ala Gly Leu Ala Tyr Tyr Ser Met Ala Gly Asn Trp
           355                 360                 365
   Ala Lys Val Leu Ile Val Met Leu Leu Phe Ala Gly Val Asp Gly His
       370                 375                 380
   Thr Leu Thr Thr Gly Gly His Ala Ala Arg Leu Thr Ser Gly Phe Ala
   385                 390                 395                 400
   Gly Leu Phe Thr Pro Gly Pro Ser Gln Arg Ile Gln Leu Ile Asn Thr
                   405                 410                 415
   Asn Gly Ser Trp His Ile Asn Arg Thr Ala Leu Asn Cys Asn Asp Ser
               420                 425                 430
   Leu Gln Thr Gly Phe Leu Ala Ala Leu Phe Tyr Ala His Arg Phe Asn
           435                 440                 445
   Ser Ser Gly Cys Pro Glu Arg Met Ala Ser Cys Arg Ser Ile Asp Lys
       450                 455                 460
   Phe Asp Gln Gly Trp Gly Pro Ile Thr Tyr Ala Glu Pro Thr Lys Asp
   465                 470                 475                 480
   Pro Asp Gln Arg Pro Tyr Cys Trp His Tyr Pro Pro Gln Gln Cys Gly
                   485                 490                 495
   Ile Val Pro Ala Ser Gln Val Cys Gly Pro Val Tyr Cys Phe Thr Pro
               500                 505                 510
   Ser Pro Val Val Val Gly Thr Thr Asp Arg Leu Gly Asn Pro Thr Tyr
           515                 520                 525
   Ser Trp Gly Glu Asn Asp Thr Asp Val Leu Leu Leu Asn Asn Thr Arg
       530                 535                 540
   Pro Pro Gln Gly Asn Trp Phe Gly Cys Thr Trp Met Asn Ser Thr Gly
   545 550 555 560
   Phe Thr Lys Thr Cys Gly Ala Pro Pro Cys Asn Ile Gly Gly Val Gly
   565 570 575
   Asn Asn Thr Leu Thr Cys Pro Thr Asp Cys Phe Arg Lys His Pro Glu
   580 585 590
   Ala Thr Tyr Ser Lys Cys Gly Ser Gly Pro Trp Leu Thr Pro Arg Cys
   595 600 605
   Met Val Asp Tyr Pro Tyr Arg Leu Trp His Tyr Pro Cys Thr Val Asn
   610 615 620
   Phe Ser Ile Phe Lys Val Arg Met Tyr Val Gly Gly Val Glu His Arg
   625 630 635 640
   Leu Asn Ala Ala Cys Asn Trp Thr Arg Gly Glu Arg Cys Asn Leu Asp
   645 650 655
   Asp Arg Asp Arg Ser Glu Leu Ser Pro Leu Leu Leu Ser Thr Thr Glu
   660 665 670
   Trp Gln Val Leu Pro Cys Ser Phe Thr Thr Leu Pro Ala Leu Ser Thr
   675 680 685
   Gly Leu Ile His Leu His Gln Asn Ile Val Asp Val Gln Tyr Leu Tyr
   690 695 700
   Gly Ile Gly Ser Ala Val Val Ser Phe Ala Ile Lys Trp Glu Tyr Val
   705 710 715 720
   Val Leu Leu Phe Leu Leu Leu Ala Asp Ala Arg Val Cys Ala Cys Leu
   725 730 735
   Trp Met Met Leu Leu Ile Ala Gln Ala Glu Ala
   740 745
<210> 17
   <211> 746
   <212> PRT
   <213> Hepatitis C virus
<400> 17
   Met Ser Thr Asn Pro Lys Pro Gln Arg Lys Thr Lys Arg Asn Thr Asn
   1 5 10 15
   Arg Arg Pro Gln Asp Val Lys Phe Pro Gly Gly Gly Gln Ile Val Gly
               20                  25                  30
   Gly Val Tyr Leu Leu Pro Arg Arg Gly Pro Arg Leu Gly Val Arg Ala
           35                  40                  45
   Thr Arg Lys Thr Ser Glu Arg Ser Gln Pro Arg Gly Arg Arg Gln Pro
       50                  55                  60
   Ile Pro Lys Ala Arg His Pro Glu Gly Arg Thr Trp Ala Gln Pro Gly
   65                  70                  75                  80
   Tyr Pro Trp Pro Leu Tyr Gly Asn Glu Gly Leu Gly Trp Ala Gly Trp
                   85                  90                  95
   Leu Leu Ser Pro Arg Gly Ser Arg Pro Ser Trp Gly Pro Thr Asp Pro
               100                 105                 110
   Arg Arg Arg Ser Arg Asn Leu Gly Lys Val Ile Asp Thr Leu Thr Cys
           115                 120                 125
   Gly Phe Ala Asp Leu Met Gly Tyr Ile Pro Leu Val Gly Ala Pro Leu
       130                 135                 140
   Gly Gly Val Ala Arg Ala Leu Ala His Gly Val Arg Val Leu Glu Asp
   145                 150                 155                 160
   Gly Val Asn Tyr Ala Thr Gly Asn Leu Pro Gly Cys Ser Phe Ser Ile
                   165                 170                 175
   Phe Leu Leu Ala Leu Leu Ser Cys Leu Thr Ile Pro Ala Ser Ala Tyr
               180                 185                 190
   Glu Val Arg Asn Val Ser Gly Val Tyr His Val Thr Asn Asp Cys Ser
           195                 200                 205
   Asn Ser Ser Ile Val Tyr Glu Ala Ala Asp Val Ile Met His Thr Pro
       210                 215                 220
   Gly Cys Val Pro Cys Val Gln Asp Gly Asn Thr Ser Arg Cys Trp Val
   225                 230                 235                 240
   Ala Leu Thr Pro Thr Leu Ala Ala Arg Asn Ala Ser Val Pro Val Thr
                   245                 250                 255
   Ala Ile Arg Arg His Val Asp Leu Leu Val Gly Thr Ala Ala Phe Cys
               260                 265                 270
   Ser Ala Met Tyr Val Gly Asp Leu Cys Gly Ser Val Phe Pro Val Ser
   275 280 285
   Gln Leu Phe Thr Phe Ser Pro Arg Arg His Gln Thr Val Gln Asp Cys
   290 295 300
   Asn Cys Ser Ile Tyr Pro Gly His Ile Ser Gly His Arg Met Ala Trp
   305 310 315 320
   Asp Met Met Met Asn Trp Ser Pro Thr Ala Ala Leu Val Val Ser Gln
   325 330 335
   Leu Leu Arg Ile Pro Gln Ala Ile Val Asp Met Val Ala Gly Ala His
   340 345 350
   Trp Gly Val Leu Ala Gly Leu Ala Tyr Tyr Ser Met Val Gly Asn Trp
   355 360 365
   Ala Lys Val Met Ile Val Leu Leu Leu Phe Ala Gly Val Asp Gly Thr
   370 375 380
   Thr His Thr Thr Gly Gly Ala Ala Ala Arg Ala Thr Gln Gly Phe Thr
   385 390 395 400
   Ser Phe Phe Ser Leu Gly Pro Ser Gln Lys Ile Gln Leu Ile Asn Thr
   405 410 415
   Asn Gly Ser Trp His Ile Asn Arg Thr Ala Leu Asn Cys Asn Asp Ser
   420 425 430
   Leu Gln Thr Gly Phe Leu Ala Ala Leu Phe Tyr Thr Tyr Arg Phe Asn
   435 440 445
   Ala Ser Gly Cys Pro Glu Arg Met Ala Ser Cys Arg Pro Ile Asp Lys
   450 455 460
   Phe Asp Gln Gly Trp Gly Pro Ile Thr Tyr Ala Glu Pro Asp Ser Ser
   465 470 475 480
   Asp Gln Arg Pro Tyr Cys Trp His Tyr Ala Pro Arg Pro Cys Gly Ile
   485 490 495
   Val Pro Ala Ser Gln Val Cys Gly Pro Val Tyr Cys Phe Thr Pro Ser
   500 505 510
   Pro Val Val Val Gly Thr Thr Asp Arg Phe Gly Val Pro Thr Tyr Thr
   515 520 525
   Trp Gly Glu Asn Glu Thr Asp Val Leu Leu Leu Asn Asn Thr Arg Pro
   530 535 540
   Pro Leu Gly Asn Trp Phe Gly Cys Thr Trp Met Asn Ser Thr Gly Phe
   545 550 555 560
   Thr Lys Thr Cys Gly Gly Pro Pro Cys Asn Ile Gly Gly Ala Gly Asn
   565 570 575
   Thr Thr Leu Thr Cys Pro Thr Asp Cys Phe Arg Lys His Pro Glu Ala
   580 585 590
   Thr Tyr Thr Lys Cys Gly Ser Gly Pro Trp Leu Thr Pro Arg Cys Leu
   595 600 605
   Val Asp Tyr Pro Tyr Arg Leu Trp His Tyr Pro Cys Ala Val Asn Phe
   610 615 620
   Thr Ile Phe Lys Val Arg Met Tyr Val Gly Gly Val Glu His Arg Leu
   625 630 635 640
   Asn Ala Ala Cys Asn Trp Thr Arg Gly Glu Arg Cys Asp Leu Glu Asp
   645 650 655
   Arg Asp Arg Ser Glu Leu Ser Pro Leu Leu Leu Ser Thr Thr Glu Trp
   660 665 670
   Gln Val Leu Pro Cys Ser Phe Thr Thr Leu Pro Ala Leu Ser Thr Gly
   675 680 685
   Leu Ile His Leu His Gln Asn Ile Val Asp Val Gln Tyr Leu Tyr Gly
   690 695 700
   Ile Gly Ser Ala Val Ile Pro Phe Ala Ile Lys Trp Glu Tyr Val Leu
   705 710 715 720
   Leu Leu Phe Leu Leu Leu Ala Asp Ala Arg Val Cys Ala Cys Leu Trp
   725 730 735
   Met Met Leu Leu Ile Ala Gln Ala Glu Ala
   740 745
<210> 18
   <211> 746
   <212> PRT
   <213> Hepatitis C virus
<400> 18
   Met Ser Thr Asn Pro Lys Pro Gln Arg Lys Thr Lys Arg Asn Thr Asn
   1               5                   10                  15
   Arg Arg Pro Gln Asp Val Lys Phe Pro Gly Gly Gly Gln Ile Val Gly
               20                  25                  30
   Gly Val Tyr Leu Leu Pro Arg Arg Gly Pro Arg Leu Gly Val Arg Ala
           35                  40                  45
   Thr Arg Lys Thr Ser Glu Arg Ser Gln Pro Arg Gly Arg Arg Gln Pro
       50                  55                  60
   Ile Pro Lys Ala Arg Arg Pro Glu Gly Arg Thr Trp Ala Gln Pro Gly
   65                  70                  75                  80
   Tyr Pro Trp Pro Leu Tyr Gly Asn Glu Gly Met Gly Trp Ala Gly Trp
                   85                  90                  95
   Leu Leu Ser Pro Arg Gly Ser Arg Pro Asn Trp Gly Pro Thr Asp Pro
               100                 105                 110
   Arg Arg Arg Ser Arg Asn Leu Gly Lys Val Ile Asp Thr Leu Thr Cys
           115                 120                 125
   Gly Phe Ala Asp Leu Met Gly Tyr Ile Pro Leu Val Gly Ala Pro Leu
       130                 135                 140
   Gly Gly Val Ala Arg Ala Leu Ala His Gly Val Arg Val Leu Glu Asp
   145                 150                 155                 160
   Gly Val Asn Tyr Ala Thr Gly Asn Leu Pro Gly Cys Ser Phe Ser Ile
                   165                 170                 175
   Phe Leu Leu Ala Leu Leu Ser Cys Leu Thr Thr Pro Ala Ser Ala Tyr
               180                 185                 190
   Glu Val Arg Asn Val Ser Gly Ile Tyr His Val Thr Asn Asp Cys Ser
           195                 200                 205
   Asn Ser Ser Ile Val Tyr Glu Ala Ala Asp Ile Ile Met His Thr Pro
       210                 215                 220
   Gly Cys Val Pro Cys Val Arg Glu Lys Asn Ile Ser Arg Cys Trp Val
   225                 230                 235                 240
   Ala Leu Thr Pro Thr Leu Ala Ala Arg Asn Ile Ser Val Pro Thr Ala
                   245                 250                 255
   Thr Ile Arg Arg His Val Asp Leu Leu Val Gly Thr Ala Ala Phe Cys
               260                 265                 270
   Ser Ala Met Tyr Val Gly Asp Leu Cys Gly Ser Val Phe Leu Val Ser
           275                 280                 285
   Gln Leu Phe Thr Phe Ser Pro Arg Trp His Glu Thr Val Gln Asp Cys
       290                 295                 300
   Asn Cys Ser Leu Tyr Pro Gly His Val Ser Gly His Arg Met Ala Trp
   305                 310                 315                 320
   Asp Met Met Met Asn Trp Ser Pro Thr Ala Ala Leu Val Val Ser Gln
                   325                 330                 335
   Leu Leu Arg Ile Pro Gln Ala Val Val Asp Met Val Ala Gly Ala His
               340                 345                 350
   Trp Gly Val Leu Ala Gly Leu Ala Tyr Tyr Ser Met Ile Gly Asn Trp
           355                 360                 365
   Ala Lys Val Leu Ile Val Met Leu Leu Phe Ala Gly Ala Asp Gly Thr
       370                 375                 380
   Thr His Val Thr Gly Gly Val Gln Ala His Gly Ala Tyr Gly Leu Ala
   385                 390                 395                 400
   Ser Leu Phe Asn Val Gly Pro His Gln Lys Ile Gln Leu Val Asn Thr
                   405                 410                 415
   Asn Gly Ser Trp His Ile Asn Arg Thr Ala Leu Asn Cys Asn Asp Thr
               420                 425                 430
   Leu Gln Thr Gly Phe Leu Ala Ala Leu Phe Tyr Lys His Arg Phe Asn
           435                 440                 445
   Ala Ser Gly Cys Pro Glu Arg Met Ala Ser Cys Arg Pro Ile Asp Lys
       450                 455                 460
   Phe Ala Gln Gly Trp Gly Pro Ile Thr Tyr Ala Glu Pro Asp Arg Leu
   465                 470                 475                 480
   Asp Gln Arg Pro Tyr Cys Trp His Tyr Pro Pro Arg Pro Cys Gly Ile
                   485                 490                 495
   Val Pro Ala Leu Glu Val Cys Gly Pro Val Tyr Cys Phe Thr Pro Ser
               500                 505                 510
   Pro Val Val Val Gly Thr Thr Asp Arg Phe Gly Val Pro Thr Tyr Ser
           515                 520                 525
   Trp Gly Glu Asn Glu Thr Asp Val Leu Leu Leu Asn Asn Thr Arg Pro
       530                 535                 540
   Pro Gln Gly Asn Trp Phe Gly Cys Thr Trp Met Asn Ser Thr Gly Tyr
   545                 550                 555                 560
   Thr Lys Thr Cys Gly Gly Pro Pro Cys Asn Ile Gly Gly Val Gly Asn
                   565                 570                 575
   Asn Thr Leu Thr Cys Pro Thr Asp Cys Phe Arg Lys His Pro Glu Ala
               580                 585                 590
   Thr Tyr Thr Lys Cys Gly Ser Gly Pro Trp Leu Thr Pro Arg Cys Leu
           595                 600                 605
   Val His Tyr Pro Tyr Arg Leu Trp His Tyr Pro Cys Thr Val Asn Phe
       610                 615                 620
   Thr Ile Phe Lys Val Arg Met Tyr Val Gly Gly Ile Glu His Arg Leu
   625                 630                 635                 640
   Asp Ala Ala Cys Asn Trp Thr Arg Gly Glu Arg Cys Asp Leu Glu Asp
                   645                 650                 655
   Arg Asp Arg Ser Glu Leu Ser Pro Leu Leu Leu Ser Thr Thr Glu Trp
               660                 665                 670
   Gln Ile Leu Pro Cys Ser Phe Thr Thr Leu Pro Ala Leu Ser Thr Gly
           675                 680                 685
   Leu Ile His Leu His Arg Asn Ile Val Asp Val Gln Tyr Leu Tyr Gly
       690                 695                 700
   Ile Gly Ser Ala Val Val Ser Phe Ala Ile Lys Trp Glu Tyr Val Leu
   705                 710                 715                 720
   Leu Leu Phe Leu Leu Leu Ala Asp Ala Arg Val Cys Ala Cys Leu Trp
                   725                 730                 735
   Met Met Leu Leu Ile Ala Gln Ala Glu Ala
               740                 745
<210> 19
   <211> 746
   <212> PRT
   <213> Hepatitis C virus
<400> 19
   Met Ser Thr Asn Pro Lys Pro Gln Arg Lys Thr Lys Arg Asn Thr Asn
   1               5                   10                  15
   Arg Arg Pro Gln Asp Val Lys Phe Pro Gly Gly Gly Gln Ile Val Gly
               20                  25                  30
   Gly Val Tyr Leu Leu Pro Arg Arg Gly Pro Arg Leu Gly Val Arg Ala
           35                  40                  45
   Thr Arg Lys Thr Ser Glu Arg Ser Gln Pro Arg Gly Arg Arg Gln Pro
       50                  55                  60
   Ile Pro Lys Ala Arg Arg Pro Glu Gly Arg Thr Trp Ala Gln Pro Gly
   65                  70                  75                  80
   Tyr Pro Trp Pro Leu Tyr Gly Asn Glu Gly Leu Gly Trp Ala Gly Trp
                   85                  90                  95
   Leu Leu Ser Pro Arg Gly Ser Arg Pro Ser Trp Gly Pro Asn Asp Pro
               100                 105                 110
   Arg Arg Arg Ser Arg Asn Leu Gly Lys Val Ile Asp Thr Leu Thr Cys
           115                 120                 125
   Gly Phe Ala Asp Leu Met Gly Tyr Ile Pro Leu Val Gly Ala Pro Leu
       130                 135                 140
   Gly Gly Ala Ala Arg Ala Leu Ala His Gly Val Arg Val Leu Glu Asp
   145                 150                 155                 160
   Gly Val Asn Tyr Ala Thr Gly Asn Leu Pro Gly Cys Ser Phe Ser Ile
                   165                 170                 175
   Phe Leu Leu Ala Leu Leu Ser Cys Leu Thr Ile Pro Ala Ser Ala Tyr
               180                 185                 190
   Glu Val Arg Asn Val Ser Gly Ile Tyr His Val Thr Asn Asp Cys Ser
           195                 200                 205
   Asn Ser Ser Ile Val Tyr Glu Ala Ala Asp Val Ile Met His Ala Pro
       210                 215                 220
   Gly Cys Val Pro Cys Val Arg Glu Asn Asn Ser Ser Arg Cys Trp Val
   225 230 235 240
   Ala Leu Thr Pro Thr Leu Ala Ala Arg Asn Ala Ser Val Pro Thr Thr
   245 250 255
   Thr Leu Arg Arg His Val Asp Leu Leu Val Gly Thr Ala Ala Phe Cys
   260 265 270
   Ser Ala Met Tyr Val Gly Asp Leu Cys Gly Ser Val Phe Leu Ile Ser
   275 280 285
   Gln Leu Phe Thr Phe Ser Pro Arg Arg His Glu Thr Val Gln Asp Cys
   290 295 300
   Asn Cys Ser Ile Tyr Pro Gly His Val Ser Gly His Arg Met Ala Trp
   305 310 315 320
   Asp Met Met Met Asn Trp Ser Pro Thr Ala Ala Leu Val Val Ser Gln
   325 330 335
   Leu Leu Arg Ile Pro Gln Ala Val Met Asp Met Val Ala Gly Ala His
   340 345 350
   Trp Gly Val Leu Ala Gly Leu Ala Tyr Tyr Ser Met Val Gly Asn Trp
   355 360 365
   Ala Lys Val Leu Ile Val Met Leu Leu Phe Ala Gly Val Asp Gly His
   370 375 380
   Thr Arg Val Thr Gly Gly Val Gln Gly His Val Thr Ser Thr Leu Thr
   385 390 395 400
   Ser Leu Phe Arg Pro Gly Ala Ser Gln Lys Ile Gln Leu Val Asn Thr
   405 410 415
   Asn Gly Ser Trp His Ile Asn Arg Thr Ala Leu Asn Cys Asn Asp Ser
   420 425 430
   Leu Lys Thr Gly Phe Leu Ala Ala Leu Phe Tyr Thr His Lys Phe Asn
   435 440 445
   Ala Ser Gly Cys Pro Glu Arg Met Ala Ser Cys Arg Ser Ile Asp Lys
   450 455 460
   Phe Asp Gln Gly Trp Gly Pro Ile Thr Tyr Ala Gln Pro Asp Asn Ser
   465                 470                 475                 480
   Asp Gln Arg Pro Tyr Cys Trp His Tyr Ala Pro Arg Gln Cys Gly Ile
                   485                 490                 495
   Val Pro Ala Ser Gln Val Cys Gly Pro Val Tyr Cys Phe Thr Pro Ser
               500                 505                 510
   Pro Val Val Val Gly Thr Thr Asp Arg Phe Gly Ala Pro Thr Tyr Asn
           515                 520                 525
   Trp Gly Asp Asn Glu Thr Asp Val Leu Leu Leu Asn Asn Thr Arg Pro
       530                 535                 540
   Pro His Gly Asn Trp Phe Gly Cys Thr Trp Met Asn Ser Thr Gly Phe
   545                 550                 555                 560
   Thr Lys Thr Cys Gly Gly Pro Pro Cys Asn Ile Arg Gly Val Gly Asn
                   565                 570                 575
   Asn Thr Leu Thr Cys Pro Thr Asp Cys Phe Arg Lys His Pro Asp Ala
               580                 585                 590
   Thr Tyr Thr Lys Cys Gly Ser Gly Pro Trp Leu Thr Pro Arg Cys Leu
           595                 600                 605
   Val Asp Tyr Pro Tyr Arg Leu Trp His Tyr Pro Cys Thr Val Asn Phe
       610                 615                 620
   Thr Ile Phe Lys Val Arg Met Tyr Val Gly Gly Val Glu His Arg Leu
   625                 630                 635                 640
   Asp Ala Ala Cys Asn Trp Thr Arg Gly Glu Arg Cys Asp Leu Glu Asp
                   645                 650                 655
   Arg Asp Arg Ala Glu Leu Ser Pro Leu Leu Leu Ser Thr Thr Glu Trp
               660                 665                 670
   Gln Ile Leu Pro Cys Ser Tyr Thr Thr Leu Pro Ala Leu Ser Thr Gly
           675                 680                 685
   Leu Ile His Leu His Gln Asn Ile Val Asp Ile Gln Tyr Leu Tyr Gly
       690                 695                 700
   Ile Gly Ser Ala Val Val Ser Ile Ala Ile Lys Trp Glu Tyr Val Val
   705                 710                 715                 720
   Leu Leu Phe Leu Leu Leu Ala Asp Ala Arg Val Cys Ala Cys Leu Trp
   725 730 735
   Met Met Leu Leu Ile Ala Gln Ala Glu Ala
   740 745
<210> 20
   <211> 746
   <212> PRT
   <213> Hepatitis C virus
<400> 20
   Met Ser Thr Asn Pro Lys Pro Gln Arg Lys Thr Lys Arg Asn Thr Asn
   1 5 10 15
   Arg Arg Pro Gln Asp Val Lys Phe Pro Gly Gly Gly Gln Ile Val Gly
   20 25 30
   Gly Val Tyr Leu Leu Pro Arg Arg Gly Pro Arg Leu Gly Val Arg Ala
   35 40 45
   Thr Arg Lys Thr Ser Glu Arg Ser Gln Pro Arg Gly Arg Arg Gln Pro
   50 55 60
   Ile Pro Lys Ala Arg Arg Pro Glu Gly Arg Thr Trp Ala Gln Pro Gly
   65 70 75 80
   Tyr Pro Trp Pro Leu Tyr Gly Asp Glu Gly Met Gly Trp Ala Gly Trp
   85 90 95
   Leu Leu Ser Pro Arg Gly Ser Arg Pro Asn Trp Gly Pro Thr Asp Pro
   100 105 110
   Arg Arg Arg Ser Arg Asn Leu Gly Lys Val Ile Asp Thr Leu Thr Cys
   115 120 125
   Gly Phe Ala Asp Leu Met Gly Tyr Ile Pro Leu Val Gly Ala Pro Leu
   130 135 140
   Gly Gly Val Ala Arg Ala Leu Ala His Gly Val Arg Val Leu Glu Asp
   145 150 155 160
   Gly Val Asn Tyr Ala Thr Gly Asn Leu Pro Gly Cys Ser Phe Ser Ile
   165 170 175
   Phe Leu Leu Ala Leu Leu Ser Cys Leu Thr Thr Pro Ala Ser Ala Tyr
   180 185 190
   Glu Val Arg Asn Val Ser Gly Ile Tyr His Val Thr Asn Asp Cys Ser
   195 200 205
   Asn Ser Ser Ile Val Tyr Glu Ala Ala Asp Ile Ile Met His Thr Pro
   210 215 220
   Gly Cys Val Pro Cys Val Arg Glu Lys Asn Thr Ser Arg Cys Trp Val
   225 230 235 240
   Ala Leu Thr Pro Thr Leu Ala Ala Arg Asn Ile Ser Val Pro Thr Thr
   245 250 255
   Thr Ile Arg Arg His Val Asp Leu Leu Val Gly Thr Ala Ala Phe Cys
   260 265 270
   Ser Ala Met Tyr Val Gly Asp Leu Cys Gly Ser Val Phe Leu Val Ser
   275 280 285
   Gln Leu Phe Thr Phe Ser Pro Arg Arg His Glu Thr Val Gln Asp Cys
   290 295 300
   Asn Cys Ser Leu Tyr Pro Gly His Val Ser Gly His Arg Met Ala Trp
   305 310 315 320
   Asp Met Met Met Asn Trp Ser Pro Thr Ala Ala Leu Val Val Ser Gln
   325 330 335
   Leu Leu Arg Ile Pro Gln Ala Ile Val Asp Met Val Ala Gly Ala His
   340 345 350
   Trp Gly Val Leu Ala Gly Leu Ala Tyr Tyr Ser Met Val Gly Asn Trp
   355 360 365
   Ala Lys Val Leu Ile Val Met Leu Leu Phe Ala Gly Ala Asp Gly Thr
   370 375 380
   Thr His Val Thr Gly Gly Val Gln Ala His Gly Ala Tyr Gly Leu Ala
   385 390 395 400
   Ser Leu Phe Asn Val Gly Pro His Gln Lys Ile Gln Leu Val Asn Thr
   405 410 415
   Asn Gly Ser Trp His Ile Asn Arg Thr Ala Leu Asn Cys Asn Asp Thr
   420 425 430
   Leu Gln Thr Gly Phe Leu Ala Ala Leu Phe Tyr Lys His Arg Phe Asn
   435 440 445
   Ala Ser Gly Cys Pro Glu Arg Met Ala Ser Cys Arg Pro Ile Asp Lys
       450                 455                 460
   Phe Ala Gln Gly Trp Gly Pro Ile Thr Tyr Ala Glu Pro Asp Arg Leu
   465                 470                 475                 480
   Asp Gln Arg Pro Tyr Cys Trp His Tyr Pro Pro Arg Pro Cys Gly Ile
                   485                 490                 495
   Val Pro Ala Leu Glu Val Cys Gly Pro Val Tyr Cys Phe Thr Pro Ser
               500                 505                 510
   Pro Val Val Val Gly Thr Thr Asp Arg Phe Gly Val Pro Thr Tyr Ser
           515                 520                 525
   Trp Gly Glu Asn Glu Thr Asp Val Leu Leu Leu Asn Asn Thr Arg Pro
       530                 535                 540
   Pro Gln Gly Asn Trp Phe Gly Cys Thr Trp Met Asn Asn Thr Gly Phe
   545                 550                 555                 560
   Thr Lys Thr Cys Gly Gly Pro Pro Cys Asn Ile Gly Gly Val Gly Asn
                   565                 570                 575
   Asn Thr Leu Thr Cys Pro Thr Asp Cys Phe Arg Lys His Pro Glu Ala
               580                 585                 590
   Thr Tyr Thr Lys Cys Gly Ser Gly Pro Trp Leu Thr Pro Arg Cys Leu
           595                 600                 605
   Val Asp Tyr Pro Tyr Arg Leu Trp His Tyr Pro Cys Thr Val Asn Phe
       610                 615                 620
   Thr Val Phe Lys Val Arg Met Tyr Val Gly Gly Ile Glu His Arg Leu
   625                 630                 635                 640
   Asp Ala Ala Cys Asn Trp Thr Arg Gly Glu Arg Cys Asp Leu Glu Asp
                   645                 650                 655
   Arg Asp Arg Ser Glu Leu Ser Pro Leu Leu Leu Ser Thr Thr Glu Trp
               660                 665                 670
   Gln Ile Leu Pro Cys Ser Phe Thr Thr Leu Pro Ala Leu Ser Thr Gly
           675                 680                 685
   Leu Ile His Leu His Arg Asn Ile Val Asp Val Gln Tyr Leu Tyr Gly
       690                 695                 700
   Ile Gly Ser Ala Val Val Ser Phe Ala Ile Lys Trp Glu Tyr Ile Leu
   705 710 715 720
   Leu Leu Phe Leu Leu Leu Ala Asp Ala Arg Val Cys Ala Cys Leu Trp
   725 730 735
   Met Met Leu Leu Ile Ala Gln Ala Glu Ala
   740 745
<210> 21
   <211> 746
   <212> PRT
   <213> Hepatitis C virus
<400> 21
   Met Ser Thr Asn Pro Lys Pro Gln Arg Lys Thr Lys Arg Asn Thr Asn
   1 5 10 15
   Arg Arg Pro Gln Asp Val Lys Phe Pro Gly Gly Gly Gln Ile Val Gly
   20 25 30
   Gly Val Tyr Leu Leu Pro Arg Arg Gly Pro Arg Leu Gly Val Arg Ala
   35 40 45
   Thr Arg Lys Thr Ser Glu Arg Ser Gln Pro Arg Gly Arg Arg Gln Pro
   50 55 60
   Ile Pro Lys Ala Arg Gln Pro Glu Gly Arg Ala Trp Ala Gln Pro Gly
   65 70 75 80
   Tyr Pro Trp Pro Leu Tyr Gly Asn Glu Gly Leu Gly Trp Ala Gly Trp
   85 90 95
   Leu Leu Ser Pro Arg Gly Ser Arg Pro Ser Trp Gly Pro Thr Asp Pro
   100 105 110
   Arg Arg Arg Ser Arg Asn Leu Gly Lys Val Ile Asp Thr Leu Thr Cys
   115 120 125
   Gly Phe Ala Asp Leu Met Gly Tyr Ile Pro Leu Val Gly Ala Pro Leu
   130 135 140
   Gly Gly Ala Ala Arg Ala Leu Ala His Gly Val Arg Val Leu Glu Asp
   145 150 155 160
   Gly Val Asn Tyr Ala Thr Gly Asn Leu Pro Gly Cys Ser Phe Ser Ile
   165 170 175
   Phe Leu Leu Ala Leu Leu Ser Cys Leu Thr Ile Pro Ala Ser Ala Tyr
               180                 185                 190
   Glu Val Arg Asn Val Ser Gly Val Tyr His Val Thr Asn Asp Cys Ser
           195                 200                 205
   Asn Ser Ser Ile Val Tyr Glu Ala Ala Asp Met Ile Met His Thr Pro
       210                 215                 220
   Gly Cys Val Pro Cys Val Arg Glu Asp Asn Ser Ser Arg Cys Trp Val
   225                 230                 235                 240
   Ala Leu Thr Pro Thr Leu Ala Ala Arg Asn Ser Ser Val Pro Thr Thr
                   245                 250                 255
   Thr Ile Arg Arg His Val Asp Leu Leu Val Gly Ala Ala Ala Phe Cys
               260                 265                 270
   Ser Ala Met Tyr Val Gly Asp Leu Cys Gly Ser Val Phe Leu Ile Ser
           275                 280                 285
   Gln Leu Phe Thr Phe Ser Pro Arg Arg Tyr Glu Thr Val Gln Asp Cys
       290                 295                 300
   Asn Cys Ser Leu Tyr Pro Gly His Val Ser Gly His Arg Met Ala Trp
   305                 310                 315                 320
   Asp Met Met Met Asn Trp Ser Pro Thr Thr Ala Leu Val Val Ser Gln
                   325                 330                 335
   Leu Leu Arg Ile Pro Gln Ala Val Val Asp Met Val Ala Gly Ala His
               340                 345                 350
   Trp Gly Val Leu Ala Gly Leu Ala Tyr Tyr Ser Met Val Gly Asn Trp
           355                 360                 365
   Ala Lys Val Leu Ile Val Met Leu Leu Phe Ala Gly Val Asp Gly Asn
       370                 375                 380
   Thr Arg Val Ser Gly Gly Glu Ala Ala Lys Asn Thr Met Gly Phe Ala
   385                 390                 395                 400
   Ser Leu Phe Val Ser Gly Pro Ser Gln Lys Ile Gln Leu Ile Asn Thr
                   405                 410                 415
   Asn Gly Ser Trp His Ile Asn Arg Thr Ala Leu Asn Cys Asp Asp Ser
               420                 425                 430
   Leu His Thr Gly Phe Leu Ala Ala Leu Phe Tyr Ala His Lys Phe Asn
           435                 440                 445
   Ser Ser Gly Cys Ser Gly Arg Met Ala Ser Cys Arg Pro Ile Asp Glu
       450                 455                 460
   Phe Ala Gln Gly Trp Gly Pro Ile Thr His Gly Val Pro Asp Asn Leu
   465                 470                 475                 480
   Asp Gln Arg Pro Tyr Cys Trp His Tyr Ala Pro Arg Pro Cys Gly Ile
                   485                 490                 495
   Val Pro Ala Ser Gln Val Cys Gly Pro Val Tyr Cys Phe Thr Pro Ser
               500                 505                 510
   Pro Val Val Val Gly Thr Thr Asp Arg Phe Gly Ala Pro Thr Tyr Ser
           515                 520                 525
   Trp Gly Glu Asn Glu Thr Asp Val Leu Leu Leu Asn Asn Thr Arg Pro
       530                 535                 540
   Pro Gln Gly Asn Trp Phe Gly Cys Thr Trp Met Asn Ser Thr Gly Phe
   545                 550                 555                 560
   Thr Lys Thr Cys Gly Gly Pro Pro Cys Asn Ile Gly Gly Val Gly Asn
                   565                 570                 575
   Asn Thr Leu Thr Cys Pro Thr Asp Cys Phe Arg Lys His Pro Glu Ala
               580                 585                 590
   Thr Tyr Thr Lys Cys Gly Ser Gly Pro Trp Leu Thr Pro Arg Cys Met
           595                 600                 605
   Val Asp Tyr Pro Tyr Arg Leu Trp His Tyr Pro Cys Thr Val Asn Phe
       610                 615                 620
   Thr Ile Phe Lys Val Arg Met Tyr Val Gly Gly Val Glu His Arg Leu
   625                 630                 635                 640
   Asp Ala Ala Cys Asn Trp Thr Arg Gly Glu Arg Cys Asn Val Glu Asp
                   645                 650                 655
   Arg Asp Arg Ser Glu Leu Ser Pro Leu Leu Leu Ser Thr Thr Glu Trp
               660                 665                 670
   Gln Ile Leu Pro Cys Ser Phe Thr Thr Leu Pro Ala Leu Ser Thr Gly
   675 680 685
   Leu Ile His Leu His Gln Asn Ile Val Asp Val Gln Tyr Leu Tyr Gly
   690 695 700
   Val Gly Ser Val Val Val Ser Val Val Ile Arg Trp Glu Tyr Val Val
   705 710 715 720
   Leu Leu Phe Leu Leu Leu Ala Asp Ala Arg Val Cys Ala Cys Leu Trp
   725 730 735
   Met Met Leu Leu Ile Ala Gln Ala Glu Ala
   740 745
<210> 22
   <211> 746
   <212> PRT
   <213> Hepatitis C virus
<400> 22
   Met Ser Thr Asn Pro Lys Pro Gln Arg Lys Thr Lys Arg Asn Thr Asn
   1 5 10 15
   Arg Arg Pro Gln Asp Val Lys Phe Pro Gly Gly Gly Gln Ile Val Gly
   20 25 30
   Gly Val Tyr Leu Leu Pro Arg Arg Gly Pro Arg Leu Gly Val Arg Ala
   35 40 45
   Thr Arg Lys Thr Ser Glu Arg Ser Gln Pro Arg Gly Arg Arg Gln Pro
   50 55 60
   Ile Pro Lys Ala Arg Gln Pro Glu Gly Arg Ala Trp Ala Gln Pro Gly
   65 70 75 80
   Tyr Pro Trp Pro Leu Tyr Gly Asn Glu Gly Met Gly Trp Ala Gly Trp
   85 90 95
   Leu Leu Ser Pro Arg Gly Ser Arg Pro Ser Trp Gly Pro Thr Asp Pro
   100 105 110
   Arg Arg Arg Ser Arg Asn Leu Gly Lys Val Ile Asp Thr Leu Thr Cys
   115 120 125
   Gly Phe Ala Asp Leu Met Gly Tyr Ile Pro Leu Val Gly Ala Pro Leu
   130 135 140
   Gly Gly Ala Ala Arg Ala Leu Ala His Gly Val Arg Val Leu Glu Asp
   145                 150                 155                 160
   Gly Val Asn Tyr Ala Thr Gly Asn Leu Pro Gly Cys Ser Phe Ser Ile
                   165                 170                 175
   Phe Leu Leu Ala Leu Leu Ser Cys Leu Thr Ile Pro Ala Ser Ala Tyr
               180                 185                 190
   Glu Val Arg Asn Val Ser Gly Met Tyr His Val Thr Asn Asp Cys Ser
           195                 200                 205
   Asn Ser Ser Ile Val Tyr Glu Ala Ala Asp Met Ile Leu His Ala Pro
       210                 215                 220
   Gly Cys Val Pro Cys Val Arg Glu Asn Asn Ser Ser Arg Cys Trp Val
   225                 230                 235                 240
   Ala Leu Thr Pro Thr Leu Ala Ala Arg Asn Ala Ser Val Pro Thr Thr
                   245                 250                 255
   Ala Ile Arg Arg His Val Asp Leu Leu Val Gly Ala Ala Ala Phe Cys
               260                 265                 270
   Ser Ala Met Tyr Val Gly Asp Leu Cys Gly Ser Val Leu Leu Val Ser
           275                 280                 285
   Gln Ile Phe Thr Phe Ser Pro Arg Arg His Glu Thr Met Gln Asp Cys
       290                 295                 300
   Asn Cys Ser Ile Tyr Pro Gly His Ile Thr Gly His Arg Met Ala Trp
   305                 310                 315                 320
   Asp Met Met Met Asn Trp Ser Pro Thr Thr Ala Leu Val Val Ser Gln
                   325                 330                 335
   Leu Leu Arg Ile Pro Gln Ala Ile Val Asp Met Val Ala Gly Ala His
               340                 345                 350
   Trp Gly Val Leu Ala Gly Leu Ala Tyr Tyr Ser Met Val Gly Asn Trp
           355                 360                 365
   Ala Lys Val Leu Ile Val Met Leu Leu Phe Ala Gly Val Asp Gly Gly
       370                 375                 380
   Thr Tyr Val Thr Gly Gly Glu Ala Gly Arg Arg Thr Ser Gly Phe Ala
   385                 390                 395                 400
   Ser Ile Phe Thr Pro Gly Ala Ser Gln Asn Ile Gln Leu Ile Asn Thr
                   405                 410                 415
   Asn Gly Ser Trp His Ile Asn Arg Thr Ala Leu Asn Cys Asn Asp Ser
               420                 425                 430
   Leu His Thr Gly Phe Ile Ala Ala Leu Phe Tyr His His Lys Phe Asn
           435                 440                 445
   Ala Ser Gly Cys Pro Glu Arg Met Ala Ser Cys Arg Pro Ile Gly Glu
       450                 455                 460
   Phe Ala Gln Gly Trp Gly Pro Ile Ser Tyr Thr Glu Pro Pro Ser Ser
   465                 470                 475                 480
   Asp Gln Arg Pro Tyr Cys Trp His Tyr Pro Pro Arg Pro Cys Gly Ile
                   485                 490                 495
   Val Pro Ala Ser Gln Val Cys Gly Pro Val Tyr Cys Phe Thr Pro Ser
               500                 505                 510
   Pro Val Val Val Gly Thr Thr Asp Arg Leu Gly Ala Pro Thr Tyr Asn
           515                 520                 525
   Trp Gly Asp Asn Asp Thr Asp Val Leu Leu Leu Asn Asn Thr Arg Pro
       530                 535                 540
   Pro Gln Gly Asn Trp Phe Gly Cys Thr Trp Met Asn Gly Thr Gly Phe
   545                 550                 555                 560
   Thr Lys Thr Cys Gly Gly Pro Pro Cys Asn Ile Gly Gly Val Gly Asn
                   565                 570                 575
   Asn Thr Leu Thr Cys Pro Thr Asp Cys Phe Arg Lys His Pro Glu Ala
               580                 585                 590
   Thr Tyr Thr Lys Cys Gly Ser Gly Pro Trp Leu Thr Pro Arg Cys Ile
           595                 600                 605
   Val Asp Tyr Pro Tyr Arg Leu Trp His Tyr Pro Cys Thr Val Asn Phe
       610                 615                 620
   Thr Ile Thr Lys Ile Arg Met Tyr Val Gly Gly Val Glu His Arg Leu
   625                 630                 635                 640
   Thr Ala Ala Cys Asn Trp Thr Arg Gly Glu Arg Cys Asp Leu Glu Asp
                   645                 650                 655
   Arg Asp Arg Ser Glu Leu Ser Pro Leu Leu Leu Ser Thr Thr Glu Trp
   660 665 670
   Gln Ile Met Pro Cys Ser Phe Thr Thr Leu Pro Ala Leu Ser Thr Gly
   675 680 685
   Leu Ile His Leu His Gln Asn Ile Val Asp Ile Gln Tyr Leu Tyr Gly
   690 695 700
   Ile Gly Ser Ala Ala Val Ser Phe Ala Ile Arg Trp Glu Tyr Val Leu
   705 710 715 720
   Leu Leu Phe Leu Leu Leu Ala Asp Ala Arg Val Cys Ala Cys Leu Trp
   725 730 735
   Met Met Leu Leu Ile Ala Gln Ala Glu Ala
   740 745
<210> 23
   <211> 746
   <212> PRT
   <213> Hepatitis C virus
<400> 23
   Met Ser Thr Asn Pro Lys Pro Gln Arg Lys Thr Lys Arg Asn Thr Asn
   1 5 10 15
   Arg Arg Pro Gln Asp Val Lys Phe Pro Gly Gly Gly Gln Ile Val Gly
   20 25 30
   Gly Val Tyr Leu Leu Pro Arg Arg Gly Pro Arg Leu Gly Val Arg Ala
   35 40 45
   Thr Arg Lys Thr Ser Glu Arg Ser Gln Pro Arg Gly Arg Arg Gln Pro
   50 55 60
   Ile Pro Lys Ala Arg Gln Pro Glu Gly Arg Ala Trp Ala Gln Pro Gly
   65 70 75 80
   Tyr Pro Trp Pro Leu Tyr Gly Asn Glu Gly Met Gly Trp Ala Gly Trp
   85 90 95
   Leu Leu Ser Pro Arg Gly Ser Arg Pro Ser Trp Gly Pro Thr Asp Pro
   100 105 110
   Arg Arg Arg Ser Arg Asn Leu Gly Lys Val Ile Asp Thr Leu Thr Cys
   115 120 125
   Gly Phe Ala Asp Leu Met Gly Tyr Ile Pro Leu Val Gly Ala Pro Leu
       130                 135                 140
   Gly Gly Ala Ala Arg Ala Leu Ala His Gly Val Arg Val Leu Glu Asp
   145                 150                 155                 160
   Gly Val Asn Tyr Ala Thr Gly Asn Leu Pro Gly Cys Ser Phe Ser Ile
                   165                 170                 175
   Phe Leu Leu Ala Leu Leu Ser Cys Leu Thr Ile Pro Ala Ser Ala Tyr
               180                 185                 190
   Glu Val Arg Asn Val Ser Gly Met Tyr His Val Thr Asn Asp Cys Ser
           195                 200                 205
   Asn Ser Ser Ile Val Tyr Glu Ala Ala Asp Met Ile Leu His Ala Pro
       210                 215                 220
   Gly Cys Val Pro Cys Val Arg Glu Asn Asn Ser Ser Arg Cys Trp Val
   225                 230                 235                 240
   Ala Leu Thr Pro Thr Leu Ala Ala Arg Asn Ala Ser Val Pro Thr Thr
                   245                 250                 255
   Ala Ile Arg Arg His Val Asp Leu Leu Val Gly Ala Ala Ala Phe Cys
               260                 265                 270
   Ser Ala Met Tyr Val Gly Asp Leu Cys Gly Ser Val Leu Leu Val Ser
           275                 280                 285
   Gln Ile Phe Thr Phe Ser Pro Arg Arg His Glu Thr Met Gln Asp Cys
       290                 295                 300
   Asn Cys Ser Ile Tyr Pro Gly His Ile Thr Gly His Arg Met Ala Trp
   305                 310                 315                 320
   Asp Met Met Met Asn Trp Ser Pro Thr Thr Ala Leu Val Val Ser Gln
                   325                 330                 335
   Leu Leu Arg Ile Pro Gln Ala Ile Val Asp Met Val Ala Gly Ala His
               340                 345                 350
   Trp Gly Val Leu Ala Gly Leu Ala Tyr Tyr Ser Met Val Gly Asn Trp
           355                 360                 365
   Ala Lys Val Leu Ile Val Met Leu Leu Phe Ala Gly Val Asp Gly Gly
   370 375 380
   Thr Tyr Val Thr Gly Gly Glu Ala Gly Arg Arg Thr Ser Gly Phe Ala
   385 390 395 400
   Ser Ile Phe Thr Pro Gly Ala Ser Gln Asn Ile Gln Leu Ile Asn Thr
   405 410 415
   Asn Gly Ser Trp His Ile Asn Arg Thr Ala Leu Asn Cys Asn Asp Ser
   420 425 430
   Leu His Thr Gly Phe Ile Ala Ala Leu Phe Tyr His His Lys Phe Asn
   435 440 445
   Ala Ser Gly Cys Pro Glu Arg Met Ala Ser Cys Arg Pro Ile Gly Glu
   450 455 460
   Phe Ala Gln Gly Trp Gly Pro Ile Ser Tyr Thr Glu Pro Pro Ser Ser
   465 470 475 480
   Asp Gln Arg Pro Tyr Cys Trp His Tyr Pro Pro Arg Pro Cys Gly Ile
   485 490 495
   Val Pro Ala Ser Gln Val Cys Gly Pro Val Tyr Cys Phe Thr Pro Ser
   500 505 510
   Pro Val Val Val Gly Thr Thr Asp Arg Leu Gly Ala Pro Thr Tyr Asn
   515 520 525
   Trp Gly Asp Asn Asp Thr Asp Val Leu Leu Leu Asn Asn Thr Arg Pro
   530 535 540
   Pro Gln Gly Asn Trp Phe Gly Cys Thr Trp Met Asn Gly Thr Gly Phe
   545 550 555 560
   Thr Lys Thr Cys Gly Gly Pro Pro Cys Asn Ile Gly Gly Val Gly Asn
   565 570 575
   Asn Thr Leu Thr Cys Pro Thr Asp Cys Phe Arg Lys His Pro Glu Ala
   580 585 590
   Thr Tyr Thr Lys Cys Gly Ser Gly Pro Trp Leu Thr Pro Arg Cys Ile
   595 600 605
   Val Asp Tyr Pro Tyr Arg Leu Trp His Tyr Pro Cys Thr Val Asn Phe
   610 615 620
   Thr Ile Thr Lys Ile Arg Met Tyr Val Gly Gly Val Glu His Arg Leu
   625                 630                 635                 640
   Thr Ala Ala Cys Asn Trp Thr Arg Gly Glu Arg Cys Asp Leu Glu Asp
                   645                 650                 655
   Arg Asp Arg Ser Glu Leu Ser Pro Leu Leu Leu Ser Thr Thr Glu Trp
               660                 665                 670
   Gln Ile Met Pro Cys Ser Phe Thr Thr Leu Pro Ala Leu Ser Thr Gly
           675                 680                 685
   Leu Ile His Leu His Gln Asn Ile Val Asp Ile Gln Tyr Leu Tyr Gly
       690                 695                 700
   Ile Gly Ser Ala Ala Val Ser Phe Ala Ile Arg Trp Glu Tyr Val Leu
   705                 710                 715                 720
   Leu Leu Phe Leu Leu Leu Ala Asp Ala Arg Val Cys Ala Cys Leu Trp
                   725                 730                 735
   Met Met Leu Leu Ile Ala Gln Ala Glu Ala
               740                 745
<210> 24
   <211> 746
   <212> PRT
   <213> Hepatitis C virus
<400> 24
   Met Ser Thr Asn Pro Lys Pro Gln Arg Lys Thr Lys Arg Asn Thr Asn
   1               5                   10                  15
   Arg Arg Pro Gln Asp Val Lys Phe Pro Gly Gly Gly Gln Ile Val Gly
               20                  25                  30
   Gly Val Tyr Leu Leu Pro Arg Arg Gly Pro Arg Leu Gly Val Arg Ala
           35                  40                  45
   Thr Arg Lys Thr Ser Glu Arg Ser Gln Pro Arg Gly Arg Arg Gln Pro
       50                  55                  60
   Ile Pro Lys Ala Arg Arg Pro Glu Gly Arg Ala Trp Ala Gln Pro Gly
   65                  70                  75                  80
   Tyr Pro Trp Pro Leu Tyr Gly Asn Glu Gly Leu Gly Trp Ala Gly Trp
                   85                  90                  95
   Leu Leu Ser Pro Arg Gly Ser Arg Pro Ser Trp Gly Pro Thr Asp Pro
               100                 105                 110
   Arg Arg Arg Ser Arg Asn Leu Gly Lys Val Ile Asp Thr Leu Thr Cys
           115                 120                 125
   Gly Phe Ala Asp Leu Met Gly Tyr Ile Pro Leu Val Gly Ala Pro Leu
       130                 135                 140
   Gly Gly Thr Ala Arg Ala Leu Ala His Gly Val Arg Val Leu Glu Asp
   145                 150                 155                 160
   Gly Val Asn Tyr Ala Thr Gly Asn Leu Pro Gly Cys Ser Phe Ser Ile
                   165                 170                 175
   Phe Leu Leu Ala Leu Leu Ser Cys Leu Thr Ile Pro Ala Ser Ala Tyr
               180                 185                 190
   Glu Val Arg Asn Val Ser Gly Val Tyr His Val Thr Asn Asp Cys Ser
           195                 200                 205
   Asn Ser Ser Ile Val Tyr Glu Thr Ala Asp Met Ile Met His Thr Pro
       210                 215                 220
   Gly Cys Val Pro Cys Val Arg Glu Asp Asn Phe Thr Arg Cys Trp Val
   225                 230                 235                 240
   Ala Leu Thr Pro Thr Leu Ala Ala Arg Asn Gly Ser Val Pro Thr Thr
                   245                 250                 255
   Ala Ile Arg Arg His Val Asp Leu Leu Val Gly Ala Ala Ala Phe Cys
               260                 265                 270
   Ser Ala Met Tyr Val Gly Asp Leu Cys Gly Ser Val Phe Leu Val Ser
           275                 280                 285
   Gln Leu Phe Thr Phe Ser Pro Arg Trp His Glu Thr Val Gln Glu Cys
       290                 295                 300
   Asn Cys Ser Ile Tyr Pro Gly His Val Thr Gly His Arg Met Ala Trp
   305                 310                 315                 320
   Asp Met Met Met Asn Trp Ser Pro Thr Thr Ala Leu Val Val Ser Gln
                   325                 330                 335
   Leu Leu Arg Ile Pro Gln Ala Val Val Asp Met Val Ala Gly Ala His
               340                 345                 350
   Trp Gly Val Leu Ala Gly Leu Ala Tyr Tyr Ser Met Val Gly Asn Trp
           355                 360                 365
   Ala Lys Val Leu Ile Val Thr Leu Leu Phe Ala Gly Val Asp Gly Asn
       370                 375                 380
   Thr His Thr Ile Gly Gly Lys Gln Ala Gln Ala Thr Gly Gly Phe Val
   385                 390                 395                 400
   Ala Trp Leu Ala Arg Gly Pro Ser Gln Glu Ile Gln Leu Ile Asn Thr
                   405                 410                 415
   Asn Gly Ser Trp His Ile Asn Arg Thr Ala Leu Asn Cys Asn Asp Ser
               420                 425                 430
   Leu Lys Thr Gly Phe Ile Ala Ala Leu Phe Tyr Ala His Arg Phe Asn
           435                 440                 445
   Ser Ser Gly Cys Pro Glu Arg Met Ala Ser Cys Arg Pro Ile Asp Lys
       450                 455                 460
   Phe Ala Gln Gly Trp Gly Pro Ile Thr Tyr Ala Lys Pro Asp Ser Leu
   465                 470                 475                 480
   Asp Gln Arg Pro Tyr Cys Trp His Tyr Ala Pro Gln Pro Cys Gly Ile
                   485                 490                 495
   Val Pro Ala Ser Glu Val Cys Gly Pro Val Tyr Cys Phe Thr Pro Ser
               500                 505                 510
   Pro Val Val Val Gly Thr Thr Asp Arg Ser Gly Val Pro Thr Tyr Arg
           515                 520                 525
   Trp Gly Glu Asn Glu Thr Asp Val Leu Leu Leu Asn Asn Thr Arg Pro
       530                 535                 540
   Pro Gln Gly Asn Trp Phe Gly Cys Thr Trp Met Asn Ala Thr Gly Phe
   545                 550                 555                 560
   Thr Lys Thr Cys Gly Gly Pro Pro Cys Lys Ile Gly Gly Leu Gly Asn
                   565                 570                 575
   Asn Thr Leu Thr Cys Pro Thr Asp Cys Phe Arg Lys His Pro Glu Ala
               580                 585                 590
   Thr Tyr Thr Lys Cys Gly Ser Gly Pro Trp Leu Thr Pro Arg Cys Ile
           595                 600                 605
   Val Asp Tyr Pro Tyr Arg Leu Trp His Tyr Pro Cys Thr Val Asn Phe
   610 615 620
   Thr Ile Phe Lys Val Arg Met Tyr Val Gly Gly Ile Glu His Arg Leu
   625 630 635 640
   Ser Ala Ala Cys Asn Trp Thr Arg Gly Glu Arg Cys Asp Leu Glu Asp
   645 650 655
   Arg Asp Arg Ser Glu Leu Ser Pro Leu Leu Leu Ser Thr Thr Glu Trp
   660 665 670
   Gln Ile Leu Pro Cys Ser Phe Thr Thr Leu Pro Ala Leu Ser Thr Gly
   675 680 685
   Leu Ile His Leu His Gln Asn Thr Val Asp Val Gln Tyr Leu Tyr Gly
   690 695 700
   Val Gly Ser Val Leu Val Ser Phe Ala Ile Lys Trp Glu Tyr Ile Leu
   705 710 715 720
   Leu Phe Phe Leu Leu Leu Ala Asp Ala Arg Val Cys Ala Cys Leu Trp
   725 730 735
   Met Met Leu Leu Ile Ala Gln Ala Glu Ala
   740 745
<210> 25
   <211> 746
   <212> PRT
   <213> Hepatitis C virus
<400> 25
   Met Ser Thr Asn Pro Lys Pro Gln Arg Lys Thr Lys Arg Asn Thr Asn
   1 5 10 15
   Arg Arg Pro Gln Asn Val Lys Phe Pro Gly Gly Gly Gln Ile Val Gly
   20 25 30
   Gly Val Cys Leu Leu Pro Arg Arg Gly Pro Arg Val Gly Val Arg Ala
   35 40 45
   Thr Arg Lys Thr Ser Glu Arg Ser Gln Pro Arg Gly Arg Arg Gln Pro
   50 55 60
   Ile Pro Lys Ala Arg Arg Pro Glu Gly Arg Ser Trp Ala Gln Pro Gly
   65 70 75 80
   Tyr Pro Trp Pro Leu Tyr Gly Asn Glu Gly Cys Gly Trp Ala Gly Trp
                   85                  90                  95
   Leu Leu Ser Pro Arg Gly Ser Arg Pro Ser Arg Gly Pro Ser Asp Pro
               100                 105                 110
   Arg Arg Arg Ser Arg Asn Leu Gly Lys Val Ile Asp Thr Leu Thr Cys
           115                 120                 125
   Gly Phe Ala Asp Leu Met Gly Tyr Ile Pro Leu Val Gly Ala Pro Leu
       130                 135                 140
   Gly Gly Ala Ala Arg Ala Leu Ala His Gly Val Arg Val Leu Glu Asp
   145                 150                 155                 160
   Gly Val Asn Tyr Ala Thr Gly Asn Leu Pro Gly Cys Ser Phe Ser Ile
                   165                 170                 175
   Phe Leu Leu Ala Leu Leu Ser Cys Leu Thr Val Pro Ala Ser Ala Val
               180                 185                 190
   Glu Val Arg Asn Ser Ser Gly Ile Tyr His Val Thr Asn Asp Cys Pro
           195                 200                 205
   Asn Ala Ser Val Val Tyr Glu Thr Asp Ser Leu Ile Ile His Leu Pro
       210                 215                 220
   Gly Cys Val Pro Cys Val Arg Glu Gly Asn Ala Ser Arg Cys Trp Val
   225                 230                 235                 240
   Ser Leu Ser Pro Thr Val Ala Ala Lys Asp Pro Gly Val Pro Val Asn
                   245                 250                 255
   Glu Ile Arg Arg His Val Asp Leu Ile Val Gly Ala Ala Ala Phe Cys
               260                 265                 270
   Ser Ala Met Tyr Val Gly Asp Leu Cys Gly Ser Ile Phe Leu Val Gly
           275                 280                 285
   Gln Leu Phe Thr Leu Ser Pro Arg Arg His Trp Thr Thr Gln Asp Cys
       290                 295                 300
   Asn Cys Ser Ile Tyr Pro Gly His Val Thr Gly His Arg Met Ala Trp
   305                 310                 315                 320
   Asp Met Met Met Asn Trp Ser Pro Thr Gly Ala Leu Val Val Ala Gln
   325 330 335
   Leu Leu Arg Ile Pro Gln Ala Val Leu Asp Met Ile Ala Gly Ala His
   340 345 350
   Trp Gly Val Leu Ala Gly Pro Ala Tyr Tyr Ser Met Val Gly Asn Trp
   355 360 365
   Ala Lys Val Leu Val Val Leu Leu Leu Phe Ala Gly Val Asp Ala Thr
   370 375 380
   Thr Gln Val Thr Gly Gly Thr Ala Gly Arg Asn Ala Tyr Arg Leu Ala
   385 390 395 400
   Ser Leu Phe Ser Thr Gly Pro Ser Gln Asn Ile Gln Leu Ile Asn Ser
   405 410 415
   Asn Gly Ser Trp His Ile Asn Arg Thr Ala Leu Asn Cys Asn Asp Ser
   420 425 430
   Leu His Thr Gly Trp Val Ala Ala Leu Phe Tyr Ser His Lys Phe Asn
   435 440 445
   Ser Ser Gly Arg Pro Glu Arg Met Ala Ser Cys Arg Pro Leu Thr Ala
   450 455 460
   Phe Asp Gln Gly Trp Gly Pro Ile Thr Tyr Gly Gly Lys Ala Ser Asn
   465 470 475 480
   Asp Gln Arg Pro Tyr Cys Trp His Tyr Ala Pro Arg Pro Cys Gly Ile
   485 490 495
   Val Pro Ala Lys Glu Val Cys Gly Pro Val Tyr Cys Phe Thr Pro Ser
   500 505 510
   Pro Val Val Val Gly Thr Thr Asp Lys Tyr Gly Val Pro Thr Tyr Thr
   515 520 525
   Trp Gly Glu Asn Glu Thr Asp Val Leu Leu Leu Asn Asn Ser Arg Pro
   530 535 540
   Pro Ile Gly Asn Trp Phe Gly Cys Thr Trp Met Asn Ser Thr Gly Phe
   545 550 555 560
   Thr Lys Thr Cys Gly Ala Pro Ala Cys Asn Val Gly Gly Ser Glu Thr
   565 570 575
   Asn Thr Leu Ser Cys Pro Thr Asp Cys Phe Arg Arg His Pro Asp Ala
               580                 585                 590
   Thr Tyr Ala Lys Cys Gly Ser Gly Pro Trp Leu Asn Pro Arg Cys Met
           595                 600                 605
   Val Asp Tyr Pro Tyr Arg Leu Trp His Tyr Pro Cys Thr Val Asn Tyr
       610                 615                 620
   Thr Ile Phe Lys Ile Arg Met Phe Val Gly Gly Ile Glu His Arg Leu
   625                 630                 635                 640
   Thr Ala Ala Cys Asn Trp Thr Arg Gly Glu Arg Cys Asp Leu Asp Asp
                   645                 650                 655
   Arg Asp Arg Ala Glu Leu Ser Pro Leu Leu Leu Ser Thr Thr Gln Trp
               660                 665                 670
   Gln Val Leu Pro Cys Ser Phe Thr Thr Leu Pro Ala Leu Ser Thr Gly
           675                 680                 685
   Leu Ile His Leu His Gln Asn Ile Val Asp Val Gln Tyr Leu Tyr Gly
       690                 695                 700
   Leu Ser Ser Val Val Thr Ser Trp Ala Ile Arg Trp Glu Tyr Val Val
   705                 710                 715                 720
   Leu Leu Phe Leu Leu Leu Ala Asp Ala Arg Ile Cys Ala Cys Leu Trp
                   725                 730                 735
   Met Met Leu Leu Ile Ser Gln Val Glu Ala
               740                 745
<210> 26
   <211> 746
   <212> PRT
   <213> Hepatitis C virus
<400> 26
   Met Ser Thr Asn Pro Lys Pro Gln Arg Lys Thr Lys Arg Asn Thr Asn
   1               5                   10                  15
   Arg Arg Pro Gln Asp Val Lys Phe Pro Gly Gly Gly Gln Ile Val Gly
               20                  25                  30
   Gly Val Tyr Leu Leu Pro Arg Arg Gly Pro Arg Leu Gly Val Arg Ala
           35                  40                  45
   Thr Arg Lys Thr Ser Glu Arg Ser Gln Pro Arg Gly Arg Arg Gln Pro
       50                  55                  60
   Ile Pro Lys Ala Arg Arg Pro Glu Gly Arg Thr Trp Ala Gln Pro Gly
   65                  70                  75                  80
   Tyr Pro Trp Pro Leu Tyr Gly Asn Glu Gly Cys Gly Trp Ala Gly Trp
                   85                  90                  95
   Leu Leu Ser Pro Arg Gly Ser Arg Pro Ser Trp Gly Pro Thr Asp Pro
               100                 105                 110
   Arg Arg Arg Ser Arg Asn Leu Gly Lys Val Ile Asp Thr Leu Thr Tyr
           115                 120                 125
   Gly Phe Ala Asp Leu Met Gly Tyr Ile Pro Leu Val Gly Ala Pro Leu
       130                 135                 140
   Gly Gly Ala Ala Arg Ala Leu Ala His Gly Val Arg Val Leu Glu Asp
   145                 150                 155                 160
   Gly Val Asn Tyr Ala Thr Gly Asn Leu Pro Gly Cys Ser Phe Ser Ile
                   165                 170                 175
   Phe Leu Leu Ala Leu Leu Ser Cys Leu Thr Val Pro Ala Ser Ala Val
               180                 185                 190
   Gly Val Arg Asn Ser Ser Gly Val Tyr His Val Thr Asn Asp Cys Pro
           195                 200                 205
   Asn Ala Ser Val Val Tyr Glu Thr Asp Ser Leu Ile Ile His Leu Pro
       210                 215                 220
   Gly Cys Val Pro Cys Val Arg Glu Gly Asn Gly Ser Arg Cys Trp Val
   225                 230                 235                 240
   Ser Leu Ser Pro Thr Val Ala Ala Lys Asp Pro Gly Val Pro Val Asn
                   245                 250                 255
   Glu Ile Arg Arg His Val Asp Leu Ile Ala Gly Ala Ala Ala Phe Cys
               260                 265                 270
   Ser Ala Met Tyr Val Gly His Leu Cys Gly Ser Ile Phe Leu Val Gly
           275                 280                 285
   Gln Leu Phe Thr Leu Ser Pro Arg Arg His Trp Thr Thr Gln Asp Cys
       290                 295                 300
   Asn Cys Ser Ile Tyr Pro Gly His Val Thr Gly His Arg Met Ala Trp
   305 310 315 320
   Asp Met Met Met Asn Trp Ser Pro Thr Thr Ala Leu Val Val Ala Gln
   325 330 335
   Leu Leu Arg Ile Pro Gln Ala Ile Leu Asp Met Ile Ala Gly Ala His
   340 345 350
   Trp Gly Val Leu Ala Gly Ile Ala Tyr Phe Ser Met Val Gly Asn Trp
   355 360 365
   Thr Lys Val Leu Val Val Leu Leu Leu Phe Ala Gly Val Asp Ala Thr
   370 375 380
   Thr Ile Val Ser Gly Gly Ser Ala Gly Arg Ser Thr Ala Gly Leu Val
   385 390 395 400
   Gly Leu Phe Ser Pro Gly Ala Arg Gln Asn Ile Gln Leu Ile Asn Thr
   405 410 415
   Asn Gly Ser Trp His Ile Asn Arg Thr Ala Leu Asn Cys Asn Asp Thr
   420 425 430
   Leu Gln Thr Gly Trp Val Ala Gly Leu Phe Tyr Thr Asn Lys Phe Asn
   435 440 445
   Ser Ser Gly Cys Pro Glu Arg Leu Ala Ser Cys Arg Pro Leu Ala Asp
   450 455 460
   Phe Asp Gln Gly Trp Gly Pro Ile Ser Tyr Thr Asn Gly Ser Gly Pro
   465 470 475 480
   Asp Gln Arg Pro Tyr Cys Trp His Tyr Pro Pro Lys Pro Cys Gly Ile
   485 490 495
   Val Pro Ala Glu Ser Val Cys Gly Pro Val Tyr Cys Phe Thr Pro Ser
   500 505 510
   Pro Val Val Val Gly Thr Thr Asp Arg Ser Gly Ala Pro Thr Tyr Asn
   515 520 525
   Trp Gly Glu Asn Glu Thr Asp Val Phe Val Leu Asn Asn Thr Arg Pro
   530 535 540
   Arg Leu Gly Asn Trp Phe Gly Gly Thr Trp Met Asn Ser Thr Gly Phe
   545 550 555 560
   Thr Lys Val Cys Gly Ala Pro Pro Cys Ala Ile Gly Gly Val Gly Asn
   565 570 575
   Asn Thr Leu Tyr Cys Pro Thr Asp Cys Phe Arg Lys His Pro Glu Ala
   580 585 590
   Thr Tyr Ser Arg Cys Gly Ser Gly Pro Trp Ile Thr Pro Arg Cys Leu
   595 600 605
   Ile His Tyr Pro Tyr Arg Leu Trp His Tyr Pro Cys Thr Ile Asn Tyr
   610 615 620
   Thr Ile Phe Lys Ile Arg Met Phe Val Gly Gly Val Glu His Arg Leu
   625 630 635 640
   Asp Ala Ala Cys Asn Trp Thr Arg Gly Glu Arg Cys Asp Leu Asp Asp
   645 650 655
   Arg Asp Arg Ala Glu Leu Ser Pro Leu Leu Leu Ser Thr Thr Gln Trp
   660 665 670
   Gln Val Leu Pro Cys Ser Phe Thr Thr Leu Pro Ala Leu Ser Thr Gly
   675 680 685
   Leu Ile His Leu His Gln Asn Ile Val Asp Val Gln Tyr Leu Tyr Gly
   690 695 700
   Leu Ser Ser Ala Val Thr Ser Trp Val Ile Lys Trp Glu Tyr Val Val
   705 710 715 720
   Leu Leu Phe Leu Leu Leu Ala Asp Ala Arg Ile Cys Ala Cys Leu Trp
   725 730 735
   Met Met Leu Leu Ile Ser Gln Val Glu Ala
   740 745
<210> 27
   <211> 746
   <212> PRT
   <213> Hepatitis C virus
<400> 27
   Met Ser Thr Asn Pro Lys Pro Gln Arg Lys Thr Lys Arg Asn Thr Asn
   1 5 10 15
   Arg Arg Pro Gln Asp Val Lys Phe Pro Gly Gly Gly Gln Ile Val Gly
   20 25 30
   Gly Val Tyr Leu Leu Pro Arg Arg Gly Pro Arg Val Gly Val Arg Ala
           35                  40                  45
   Thr Arg Lys Thr Ser Glu Arg Ser Gln Pro Arg Gly Arg Arg Gln Pro
       50                  55                  60
   Ile Pro Lys Ala Arg Arg Pro Glu Gly Arg Ser Trp Ala Gln Pro Gly
   65                  70                  75                  80
   Tyr Pro Trp Pro Leu Tyr Gly Asn Glu Gly Cys Gly Trp Ala Gly Trp
                   85                  90                  95
   Leu Leu Ser Pro Arg Gly Ser Arg Pro Ser Trp Gly Pro Ser Asp Pro
               100                 105                 110
   Arg Arg Arg Ser Arg Asn Leu Gly Lys Val Ile Asp Thr Leu Thr Cys
           115                 120                 125
   Gly Phe Ala Asp Leu Met Gly Tyr Ile Pro Leu Val Gly Ala Pro Leu
       130                 135                 140
   Gly Gly Ala Ala Arg Ala Leu Ala His Gly Val Arg Val Leu Glu Asp
   145                 150                 155                 160
   Gly Val Asn Tyr Ala Thr Gly Asn Leu Pro Gly Cys Ser Phe Ser Ile
                   165                 170                 175
   Phe Leu Leu Ala Leu Leu Ser Cys Leu Thr Val Pro Ala Ser Ala Val
               180                 185                 190
   Gly Val Arg Asn Ser Ser Gly Val Tyr His Val Thr Asn Asp Cys Pro
           195                 200                 205
   Asn Ala Ser Val Val Tyr Glu Thr Glu Asn Leu Ile Met His Leu Pro
       210                 215                 220
   Gly Cys Val Pro Tyr Val Arg Glu Gly Asn Ala Ser Arg Cys Trp Val
   225                 230                 235                 240
   Ser Leu Ser Pro Thr Val Ala Ala Arg Asp Ser Arg Val Pro Val Ser
                   245                 250                 255
   Glu Val Arg Arg Arg Val Asp Ser Ile Val Gly Ala Ala Ala Phe Cys
               260                 265                 270
   Ser Ala Met Tyr Val Gly Asp Leu Cys Gly Ser Ile Phe Leu Val Gly
           275                 280                 285
   Gln Ile Phe Thr Phe Ser Pro Arg His His Trp Thr Thr Gln Asp Cys
       290                 295                 300
   Asn Cys Ser Ile Tyr Pro Gly His Val Thr Gly His Arg Met Ala Trp
   305                 310                 315                 320
   Asp Met Met Met Asn Trp Ser Pro Thr Gly Ala Leu Val Val Ala Gln
                   325                 330                 335
   Leu Leu Arg Ile Pro Gln Ala Ile Val Asp Met Ile Ala Gly Ala His
               340                 345                 350
   Trp Gly Val Leu Ala Gly Leu Ala Tyr Tyr Ser Met Val Gly Asn Trp
           355                 360                 365
   Ala Lys Val Val Val Val Leu Leu Leu Phe Ala Gly Val Asp Ala Glu
       370                 375                 380
   Thr Arg Val Thr Gly Gly Ala Ala Gly His Thr Ala Phe Gly Phe Ala
   385                 390                 395                 400
   Ser Phe Leu Ala Pro Gly Ala Lys Gln Lys Ile Gln Leu Ile Asn Thr
                   405                 410                 415
   Asn Gly Ser Trp His Ile Asn Arg Thr Ala Leu Asn Cys Asn Glu Ser
               420                 425                 430
   Leu Asp Thr Gly Trp Leu Ala Gly Leu Leu Tyr Tyr His Lys Phe Asn
           435                 440                 445
   Ser Ser Gly Cys Pro Glu Arg Met Ala Ser Cys Gln Pro Leu Thr Ala
       450                 455                 460
   Phe Asp Gln Gly Trp Gly Pro Ile Thr His Glu Gly Asn Ala Ser Asp
   465                 470                 475                 480
   Asp Gln Arg Pro Tyr Cys Trp His Tyr Ala Leu Arg Pro Cys Gly Ile
                   485                 490                 495
   Val Pro Ala Lys Lys Val Cys Gly Pro Val Tyr Cys Phe Thr Pro Ser
               500                 505                 510
   Pro Val Val Val Gly Thr Thr Asp Arg Ala Gly Val Pro Thr Tyr Arg
           515                 520                 525
   Trp Gly Ala Asn Glu Thr Asp Val Leu Leu Leu Asn Asn Ser Arg Pro
       530                 535                 540
   Pro Met Gly Asn Trp Phe Gly Cys Thr Trp Met Asn Ser Ser Gly Phe
   545                 550                 555                 560
   Thr Lys Thr Cys Gly Ala Pro Ala Cys Asn Ile Gly Gly Ser Gly Asn
                   565                 570                 575
   Asn Thr Leu Leu Cys Pro Thr Asp Cys Phe Arg Lys His Pro Asp Ala
               580                 585                 590
   Thr Tyr Ser Arg Cys Gly Ser Gly Pro Trp Leu Thr Pro Arg Cys Leu
           595                 600                 605
   Val Asp Tyr Pro Tyr Arg Leu Trp His Tyr Pro Cys Thr Val Asn Tyr
       610                 615                 620
   Thr Ile Phe Lys Ile Arg Met Phe Val Gly Gly Val Glu His Arg Leu
   625                 630                 635                 640
   Asp Ala Ala Cys Asn Trp Thr Arg Gly Glu Arg Cys Asp Leu Asp Asp
                   645                 650                 655
   Arg Asp Arg Ala Glu Leu Ser Pro Leu Leu Leu Ser Thr Thr Gln Trp
               660                 665                 670
   Gln Val Leu Pro Cys Ser Phe Thr Thr Leu Pro Ala Leu Ser Thr Gly
           675                 680                 685
   Leu Ile His Leu His Gln Asn Ile Val Asp Val Gln Tyr Leu Tyr Gly
       690                 695                 700
   Leu Ser Ser Ala Val Thr Ser Trp Val Ile Lys Trp Glu Tyr Val Val
   705                 710                 715                 720
   Leu Leu Phe Leu Leu Leu Ala Asp Ala Arg Ile Cys Ala Cys Leu Trp
                   725                 730                 735
   Met Met Leu Leu Ile Ser Gln Val Glu Ala
               740                 745
<210> 28
   <211> 752
   <212> PRT
   <213> Hepatitis C virus
<400> 28
   Met Ser Thr Leu Pro Lys Pro Gln Arg Lys Thr Lys Arg Asn Thr Ile
   1               5                   10                  15
   Arg Arg Pro Gln Asp Val Lys Phe Pro Gly Gly Gly Gln Ile Val Gly
               20                  25                  30
   Gly Val Tyr Val Leu Pro Arg Arg Gly Pro Arg Leu Gly Val Arg Ala
           35                  40                  45
   Thr Arg Lys Thr Ser Glu Arg Ser Gln Pro Arg Gly Arg Arg Gln Pro
       50                  55                  60
   Ile Pro Lys Ala Arg Arg Ser Glu Gly Arg Ser Trp Ala Gln Pro Gly
   65                  70                  75                  80
   Tyr Pro Trp Pro Leu Tyr Gly Asn Glu Gly Cys Gly Trp Ala Gly Trp
                   85                  90                  95
   Leu Leu Ser Pro Arg Gly Ser Arg Pro Ser Trp Gly Pro Asn Asp Pro
               100                 105                 110
   Arg Arg Arg Ser Arg Asn Leu Gly Lys Val Ile Asp Thr Leu Thr Cys
           115                 120                 125
   Gly Phe Ala Asp Leu Met Gly Tyr Ile Pro Leu Val Gly Ala Pro Val
       130                 135                 140
   Gly Gly Val Ala Arg Ala Leu Ala His Gly Val Arg Ala Leu Glu Asp
   145                 150                 155                 160
   Gly Ile Asn Phe Ala Thr Gly Asn Leu Pro Gly Cys Ser Phe Ser Ile
                   165                 170                 175
   Phe Leu Leu Ala Leu Phe Ser Cys Leu Ile His Pro Ala Ala Ser Leu
               180                 185                 190
   Glu Trp Arg Asn Thr Ser Gly Leu Tyr Val Leu Thr Asn Asp Cys Ser
           195                 200                 205
   Asn Ser Ser Ile Val Tyr Glu Ala Asp Asp Val Ile Leu His Thr Pro
       210                 215                 220
   Gly Cys Val Pro Cys Val Gln Asp Gly Asn Thr Ser Thr Cys Trp Thr
   225                 230                 235                 240
   Pro Val Thr Pro Thr Val Ala Val Arg Tyr Val Gly Ala Thr Thr Ala
                   245                 250                 255
   Ser Ile Arg Ser His Val Asp Leu Leu Val Gly Ala Ala Thr Met Cys
               260                 265                 270
   Ser Ala Leu Tyr Val Gly Asp Met Cys Gly Ala Val Phe Leu Val Gly
           275                 280                 285
   Gln Ala Phe Thr Phe Arg Pro Arg Arg His Gln Thr Val Gln Thr Cys
       290                 295                 300
   Asn Cys Ser Leu Tyr Pro Gly His Leu Ser Gly His Arg Met Ala Trp
   305                 310                 315                 320
   Asp Met Met Met Asn Trp Ser Pro Ala Val Gly Met Val Val Ala His
                   325                 330                 335
   Val Leu Arg Leu Pro Gln Thr Leu Phe Asp Ile Met Ala Gly Ala His
               340                 345                 350
   Trp Gly Ile Leu Ala Gly Leu Ala Tyr Tyr Ser Met Gln Gly Asn Trp
           355                 360                 365
   Ala Lys Val Ala Ile Ile Met Val Met Phe Ser Gly Val Asp Ala His
       370                 375                 380
   Thr Tyr Thr Thr Gly Gly Thr Ala Ser Arg His Thr Gln Ala Phe Ala
   385                 390                 395                 400
   Gly Leu Phe Asp Ile Gly Pro Gln Gln Lys Leu Gln Leu Val Asn Thr
                   405                 410                 415
   Asn Gly Ser Trp His Ile Asn Ser Thr Ala Leu Asn Cys Asn Glu Ser
               420                 425                 430
   Ile Asn Thr Gly Phe Ile Ala Gly Leu Phe Tyr Tyr His Lys Phe Asn
           435                 440                 445
   Ser Thr Gly Cys Pro Gln Arg Leu Ser Ser Cys Lys Pro Ile Thr Phe
       450                 455                 460
   Phe Arg Gln Gly Trp Gly Pro Leu Thr Asp Ala Asn Ile Thr Gly Pro
   465                 470                 475                 480
   Ser Asp Asp Arg Pro Tyr Cys Trp His Tyr Ala Pro Arg Pro Cys Asp
                   485                 490                 495
   Ile Val Pro Ala Ser Ser Val Cys Gly Pro Val Tyr Cys Phe Thr Pro
               500                 505                 510
   Ser Pro Val Val Val Gly Thr Thr Asp Ala Arg Gly Val Pro Thr Tyr
   515 520 525
   Thr Trp Gly Glu Asn Glu Lys Asp Val Phe Leu Leu Lys Ser Gln Arg
   530 535 540
   Pro Pro Ser Gly Arg Trp Phe Gly Cys Ser Trp Met Asn Ser Thr Gly
   545 550 555 560
   Phe Leu Lys Thr Cys Gly Ala Pro Pro Cys Asn Ile Tyr Gly Gly Glu
   565 570 575
   Gly Asn Pro His Asn Glu Ser Asp Leu Phe Cys Pro Thr Asp Cys Phe
   580 585 590
   Arg Lys His Pro Glu Thr Thr Tyr Ser Arg Cys Gly Ala Gly Pro Trp
   595 600 605
   Leu Thr Pro Arg Cys Met Val Asp Tyr Pro Tyr Arg Leu Trp His Tyr
   610 615 620
   Pro Cys Thr Val Asp Phe Arg Leu Phe Lys Val Arg Met Phe Val Gly
   625 630 635 640
   Gly Phe Glu His Arg Phe Thr Ala Ala Cys Asn Trp Thr Arg Gly Glu
   645 650 655
   Arg Cys Asp Ile Glu Asp Arg Asp Arg Ser Glu Gln His Pro Leu Leu
   660 665 670
   His Ser Thr Thr Glu Leu Ala Ile Leu Pro Cys Ser Phe Thr Pro Met
   675 680 685
   Pro Ala Leu Ser Thr Gly Leu Ile His Leu His Gln Asn Ile Val Asp
   690 695 700
   Val Gln Tyr Leu Tyr Gly Val Gly Ser Gly Met Val Gly Trp Ala Leu
   705 710 715 720
   Lys Trp Glu Phe Val Ile Leu Val Phe Leu Leu Leu Ala Asp Ala Arg
   725 730 735
   Val Cys Val Ala Leu Trp Leu Met Leu Met Ile Ser Gln Thr Glu Ala
   740 745 750
<210> 29
   <211> 752
   <212> PRT
   <213> Hepatitis C virus
<400> 29
   Met Ser Thr Leu Pro Lys Pro Gln Arg Lys Thr Lys Arg Asn Thr Ile
   1               5                   10                  15
   Arg Arg Pro Gln Asp Val Lys Phe Pro Gly Gly Gly Gln Ile Val Gly
               20                  25                  30
   Gly Val Tyr Val Leu Pro Arg Arg Gly Pro Arg Leu Gly Val Arg Ala
           35                  40                  45
   Thr Arg Lys Thr Ser Glu Arg Ser Gln Pro Arg Gly Arg Arg Gln Pro
       50                  55                  60
   Ile Pro Lys Ala Arg Arg Ser Glu Gly Arg Ser Trp Ala Gln Pro Gly
   65                  70                  75                  80
   Tyr Pro Trp Pro Leu Tyr Gly Asn Glu Gly Cys Gly Trp Ala Gly Trp
                   85                  90                  95
   Leu Leu Ser Pro Arg Gly Ser Arg Pro Ser Trp Gly Gln Asn Asp Pro
               100                 105                 110
   Arg Arg Arg Ser Arg Asn Leu Gly Lys Val Ile Asp Thr Leu Thr Cys
           115                 120                 125
   Gly Phe Ala Asp Leu Met Gly Tyr Ile Pro Leu Val Gly Ala Pro Val
       130                 135                 140
   Gly Gly Val Ala Arg Ala Leu Ala His Gly Val Arg Ala Leu Glu Asp
   145                 150                 155                 160
   Gly Ile Asn Phe Ala Thr Gly Asn Leu Pro Gly Cys Ser Phe Ser Ile
                   165                 170                 175
   Phe Leu Leu Ala Leu Phe Ser Cys Leu Ile His Pro Ala Ala Ser Leu
               180                 185                 190
   Glu Trp Arg Asn Thr Ser Gly Leu Tyr Val Leu Thr Asn Asp Cys Ser
           195                 200                 205
   Asn Ser Ser Ile Val Tyr Glu Ala Asp Asp Val Ile Leu His Thr Pro
       210                 215                 220
   Gly Cys Val Pro Cys Val Gln Asp Gly Asn Thr Ser Thr Cys Trp Thr
   225                 230                 235                 240
   Pro Val Thr Pro Thr Val Ala Val Arg Tyr Val Gly Ala Thr Thr Ala
                   245                 250                 255
   Ser Ile Arg Ser His Val Asp Leu Leu Val Gly Ala Ala Thr Met Cys
               260                 265                 270
   Ser Ala Leu Tyr Val Gly Asp Met Cys Gly Ala Val Phe Leu Val Gly
           275                 280                 285
   Gln Ala Phe Thr Phe Arg Pro Arg Arg His Gln Thr Val Gln Thr Cys
       290                 295                 300
   Asn Cys Ser Leu Tyr Pro Gly His Leu Ser Gly His Arg Met Ala Trp
   305                 310                 315                 320
   Asp Met Met Met Asn Trp Ser Pro Ala Val Gly Met Val Val Ser His
                   325                 330                 335
   Val Leu Arg Leu Pro Gln Thr Leu Phe Asp Ile Ile Ala Gly Ala His
               340                 345                 350
   Trp Gly Ile Leu Ala Gly Leu Ala Tyr Tyr Ser Met Gln Gly Asn Trp
           355                 360                 365
   Ala Lys Val Ala Val Ile Met Val Met Phe Ser Gly Val Asp Ala Glu
       370                 375                 380
   Thr Tyr Ile Thr Gly Gly Ser Ala Ala His Gly Val Ser Thr Leu Thr
   385                 390                 395                 400
   Ser Leu Phe Ser Ser Gly Pro Gln Gln Lys Leu Gln Leu Val Lys Thr
                   405                 410                 415
   Asn Gly Ser Trp His Ile Asn Ser Thr Ala Leu Asn Cys Asn Glu Ser
               420                 425                 430
   Ile Asn Thr Gly Phe Ile Ala Gly Leu Phe Tyr Tyr His Lys Phe Asn
           435                 440                 445
   Ser Thr Gly Cys Pro Gln Arg Leu Ser Ser Cys Lys Pro Ile Thr Phe
       450                 455                 460
   Phe Arg Gln Gly Trp Gly Ser Leu Thr Asp Ala Asn Val Thr Gly Ala
   465                 470                 475                 480
   Ser Ala Asp Lys Pro Tyr Cys Trp His Tyr Ala Pro Arg Pro Cys Asp
                   485                 490                 495
   Val Val Pro Ala Leu Asn Val Cys Gly Pro Val Tyr Cys Phe Thr Pro
               500                 505                 510
   Ser Pro Val Val Val Gly Thr Thr Asp Arg Lys Gly Val Pro Thr Tyr
           515                 520                 525
   Asn Trp Gly Glu Asn Glu Ser Asp Val Phe Leu Leu Glu Ser Leu Arg
       530                 535                 540
   Pro Pro Ser Gly Arg Trp Phe Gly Cys Ala Trp Met Asn Ser Thr Gly
   545                 550                 555                 560
   Phe Leu Lys Thr Cys Gly Ala Pro Pro Cys Asn Ile Tyr Gly Gly Gly
                   565                 570                 575
   Gly Asn Pro Asn Asn Glu Ser His Leu Phe Cys Pro Thr Asp Cys Phe
               580                 585                 590
   Arg Lys His Pro Asp Ala Thr Tyr Ser Arg Cys Gly Ala Gly Pro Trp
           595                 600                 605
   Leu Thr Pro Arg Cys Met Val Asp Tyr Pro Tyr Arg Leu Trp His Tyr
       610                 615                 620
   Pro Cys Thr Val Asn Phe Thr Leu Phe Lys Val Arg Met Phe Val Gly
   625                 630                 635                 640
   Gly Phe Glu His Arg Phe Thr Ala Ala Cys Asn Trp Thr Arg Gly Glu
                   645                 650                 655
   Arg Cys Asn Ile Glu Asp Arg Asp Arg Ser Glu Gln His Pro Leu Leu
               660                 665                 670
   His Ser Thr Thr Glu Leu Ala Ile Leu Pro Cys Ser Phe Thr Pro Met
           675                 680                 685
   Pro Ala Leu Ser Thr Gly Leu Ile His Leu His Gln Asn Ile Val Asp
       690                 695                 700
   Val Gln Tyr Leu Tyr Gly Val Gly Ser Gly Met Val Gly Trp Ala Leu
   705                 710                 715                 720
   Lys Trp Glu Phe Val Ile Leu Ile Phe Leu Leu Leu Ala Asp Ala Arg
                   725                 730                 735
   Val Cys Val Ala Leu Trp Leu Met Leu Met Ile Ser Gln Ala Glu Ala
   740 745 750
<210> 30
   <211> 752
   <212> PRT
   <213> Hepatitis C virus
<400> 30
   Met Ser Thr Leu Pro Lys Pro Gln Arg Lys Thr Lys Arg Asn Thr Ile
   1               5                   10                  15
   Arg Arg Pro Gln Asp Val Lys Phe Pro Gly Gly Gly Gln Ile Val Gly
               20                  25                  30
   Gly Val Tyr Val Leu Pro Arg Arg Gly Pro Arg Leu Gly Val Cys Ala
           35                  40                  45
   Thr Arg Lys Thr Ser Glu Arg Ser Gln Pro Arg Arg Arg Arg Gln Pro
       50                  55                  60
   Ile Pro Lys Ala Arg Gln Ser Gly Gly Arg Ser Trp Ala Gln Pro Gly
   65                  70                  75                  80
   Tyr Pro Trp Pro Leu Tyr Gly Asn Glu Gly Cys Gly Trp Ala Gly Trp
                   85                  90                  95
   Leu Leu Ser Pro Arg Gly Ser Arg Pro Ser Trp Gly Pro Asn Asp Pro
               100                 105                 110
   Arg Arg Arg Ser Arg Asn Leu Gly Lys Val Ile Asp Thr Leu Thr Cys
           115                 120                 125
   Gly Phe Ala Asp Leu Met Gly Tyr Ile Pro Leu Val Gly Ala Pro Val
       130                 135                 140
   Gly Gly Val Ala Arg Ala Leu Ala His Gly Val Arg Ala Leu Glu Asp
   145                 150                 155                 160
   Gly Ile Asn Phe Ala Thr Gly Asn Leu Pro Gly Cys Ser Phe Ser Ile
                   165                 170                 175
   Phe Leu Leu Ala Leu Phe Ser Cys Leu Ile His Pro Ala Ala Ser Leu
               180                 185                 190
   Glu Trp Arg Asn Thr Ser Gly Leu Tyr Val Leu Thr Asn Asp Cys Ser
           195                 200                 205
   Asn Ser Ser Ile Val Tyr Glu Ala Asp Asp Val Ile Leu His Thr Pro
       210                 215                 220
   Gly Cys Val Pro Cys Val Gln Asn Asp Asn Ile Ser Thr Cys Trp Thr
   225                 230                 235                 240
   Pro Val Thr Pro Thr Val Ala Val Arg Tyr Val Gly Ala Thr Thr Ala
                   245                 250                 255
   Ser Ile Arg Ser His Val Asp Leu Leu Val Gly Ala Ala Thr Met Cys
               260                 265                 270
   Ser Ala Leu Tyr Val Gly Asp Met Cys Gly Ala Val Phe Leu Val Gly
           275                 280                 285
   Gln Ala Phe Thr Phe Arg Pro Arg Arg His Gln Thr Val Gln Thr Cys
       290                 295                 300
   Asn Cys Ser Leu Tyr Pro Gly His Leu Ser Gly His Arg Met Ala Trp
   305                 310                 315                 320
   Asp Met Met Met Asn Trp Phe Pro Ala Leu Gly Met Ala Val Ala His
                   325                 330                 335
   Val Leu Arg Val Pro Gln Thr Leu Phe Asp Ile Ile Ala Gly Ala His
               340                 345                 350
   Trp Gly Ile Leu Ala Gly Leu Ala Tyr Tyr Ser Met Gln Gly Asn Trp
           355                 360                 365
   Ala Lys Val Ala Ile Ile Met Val Met Phe Ser Gly Val Asp Ala Val
       370                 375                 380
   Thr Tyr Thr Thr Gly Gly Ser Ala Ala His Ala Thr Arg Gly Leu Thr
   385                 390                 395                 400
   Ser Leu Phe Ser Val Gly Ala Gln Gln Lys Leu Gln Leu Val Asn Thr
                   405                 410                 415
   Asn Gly Ser Trp His Ile Asn Ser Thr Ala Leu Asn Cys Asn Glu Ser
               420                 425                 430
   Ile Asn Thr Gly Phe Ile Ala Gly Leu Phe Tyr Tyr His Arg Phe Asn
           435                 440                 445
   Ser Thr Gly Cys Pro Gln Arg Leu Ser Ser Cys Lys Pro Ile Thr Phe
       450                 455                 460
   Phe Lys Gln Gly Trp Gly Pro Leu Thr Asp Ala Asn Ile Ser Gly Pro
   465                 470                 475                 480
   Ser Asp Asp Lys Pro Tyr Cys Trp His Tyr Ala Pro Arg Pro Cys Lys
                   485                 490                 495
   Val Val Pro Ala Ser Gly Val Cys Gly Pro Val Tyr Cys Phe Thr Pro
               500                 505                 510
   Ser Pro Val Val Val Gly Thr Thr Asp Ala Lys Gly Val Pro Thr Tyr
           515                 520                 525
   Thr Trp Gly Ala Asn Asp Thr Asp Val Phe Leu Leu Glu Ser Leu Arg
       530                 535                 540
   Pro Pro Gly Gly Arg Trp Phe Gly Cys Thr Trp Met Asn Ser Thr Gly
   545                 550                 555                 560
   Phe Val Lys Thr Cys Gly Ala Ser Pro Cys Asp Ile Tyr Gly Gly Gly
                   565                 570                 575
   Gly Asn Ser Gly Asn Glu Ser Asp Leu Phe Cys Pro Thr Asp Cys Phe
               580                 585                 590
   Arg Lys His Pro Glu Ala Thr Tyr Ser Arg Cys Gly Ala Gly Pro Trp
           595                 600                 605
   Leu Thr Pro Arg Cys Met Val Asp Tyr Pro Tyr Arg Leu Trp His Tyr
       610                 615                 620
   Pro Cys Thr Val Asn Phe Thr Leu Phe Lys Val Arg Met Phe Val Gly
   625                 630                 635                 640
   Gly Phe Glu His Arg Phe Thr Ala Ala Cys Asn Trp Thr Arg Gly Glu
                   645                 650                 655
   Arg Cys Asp Ile Glu Asp Arg Asp Arg Ser Glu Gln His Pro Leu Leu
               660                 665                 670
   His Ser Thr Thr Glu Leu Ala Ile Leu Pro Cys Ser Phe Thr Pro Met
           675                 680                 685
   Pro Ala Leu Ser Thr Gly Leu Ile His Leu His Gln Asn Ile Val Asp
       690                 695                 700
   Val Gln Tyr Leu Tyr Gly Val Gly Ser Gly Met Val Gly Trp Ala Leu
   705 710 715 720
   Lys Trp Glu Phe Val Ile Leu Ile Phe Leu Leu Leu Ala Asp Arg Arg
   725 730 735
   Val Cys Val Ala Leu Trp Leu Met Leu Met Ile Thr Gln Ala Glu Ala
   740 745 750
<210> 31
   <211> 752
   <212> PRT
   <213> Hepatitis C virus
<400> 31
   Met Ser Thr Leu Pro Lys Pro Gln Arg Lys Thr Lys Arg Asn Thr Val
   1               5                   10                  15
   Cys Arg Pro Gln Asp Val Lys Phe Pro Gly Gly Gly Gln Ile Val Gly
               20                  25                  30
   Gly Val Tyr Val Leu Pro Arg Arg Gly Pro Arg Leu Gly Val Arg Ala
           35                  40                  45
   Thr Arg Lys Thr Ser Glu Arg Ser Gln Pro Arg Gly Arg Arg Gln Pro
       50                  55                  60
   Ile Pro Lys Ala Arg Arg Ser Glu Gly Arg Ser Trp Ala Gln Pro Gly
   65                  70                  75                  80
   Tyr Pro Trp Pro Leu Tyr Gly Asn Glu Gly Cys Gly Trp Ala Gly Trp
                   85                  90                  95
   Leu Leu Ser Pro Arg Gly Ser Arg Pro Ser Trp Gly Pro Asn Asp Pro
               100                 105                 110
   Arg Arg Arg Ser Arg Asn Leu Gly Lys Val Ile Asp Thr Leu Thr Cys
           115                 120                 125
   Gly Phe Ala Asp Leu Met Gly Tyr Ile Pro Leu Val Gly Ala Pro Val
       130                 135                 140
   Gly Gly Val Ala Arg Ala Leu Ala His Gly Val Arg Ala Leu Glu Asp
   145                 150                 155                 160
   Gly Ile Asn Phe Ala Thr Gly Asn Leu Pro Gly Cys Ser Phe Ser Ile
                   165                 170                 175
   Phe Leu Leu Ala Leu Phe Ser Cys Leu Ile His Pro Ala Ala Ser Leu
   180 185 190
   Glu Trp Arg Asn Val Ser Gly Leu Tyr Ile Leu Thr Asn Asp Cys Pro
   195 200 205
   Asn Ser Ser Ile Val Tyr Glu Ala Asp Asp Val Ile Leu His Thr Pro
   210 215 220
   Gly Cys Ile Pro Cys Val Gln Asp Gly Asn Thr Ser Thr Cys Trp Thr
   225 230 235 240
   Ala Leu Thr Pro Thr Val Ala Val Arg Tyr Val Gly Ala Thr Thr Ala
   245 250 255
   Ser Ile Arg Ser His Val Asp Leu Leu Val Gly Ala Ala Thr Met Cys
   260 265 270
   Ser Ala Leu Tyr Val Gly Asp Met Cys Gly Ala Val Phe Leu Val Gly
   275 280 285
   Gln Ala Phe Thr Phe Arg Pro Arg Arg His Gln Thr Val Gln Thr Cys
   290 295 300
   Asn Cys Ser Leu Tyr Pro Gly His Leu Thr Gly His Arg Met Ala Trp
   305 310 315 320
   Asp Met Met Met Asn Trp Ser Pro Ala Val Gly Met Val Val Ala His
   325 330 335
   Val Leu Arg Leu Pro Gln Thr Leu Phe Asp Ile Ile Ala Gly Ala His
   340 345 350
   Trp Gly Ile Leu Ala Gly Leu Ala Tyr Tyr Ser Met Gln Gly Asn Trp
   355 360 365
   Ala Lys Val Ile Ile Ile Met Val Met Phe Ser Gly Val Asp Ala Thr
   370 375 380
   Thr His Val Thr Gly Gly Thr Ala Gly Leu Thr Ala Phe Arg Leu Thr
   385 390 395 400
   Gly Leu Phe Thr Val Gly Pro Gln Gln Lys Leu Gln Leu Val Asn Thr
   405 410 415
   Asn Gly Ser Trp His Ile Asn Arg Thr Ala Leu Asn Cys Asn Asp Ser
   420 425 430
   Leu Asn Thr Gly Phe Ile Ala Gly Leu Phe Arg Phe His Lys Phe Asn
           435                 440                 445
   Ser Thr Gly Cys Pro Glu Met Leu Ser Ser Cys Lys Pro Ile Thr Ser
       450                 455                 460
   Phe Lys Gln Gly Trp Gly Pro Leu Thr Asp Ala Asn Ile Thr Ile Pro
   465                 470                 475                 480
   Ser Asp Asp Arg Pro Tyr Cys Trp His Tyr Pro Pro Arg Ser Cys Glu
                   485                 490                 495
   Val Val Pro Ala Leu Ser Val Cys Gly Pro Val Tyr Cys Phe Thr Pro
               500                 505                 510
   Ser Pro Val Val Val Gly Thr Thr Asp Ala Lys Gly Val Pro Thr Tyr
           515                 520                 525
   Thr Trp Gly Glu Asn Glu Thr Asp Val Phe Leu Leu Lys Ser Leu Arg
       530                 535                 540
   Pro Pro Gly Gly Arg Trp Phe Gly Cys Thr Trp Met Asn Ser Thr Gly
   545                 550                 555                 560
   Phe Val Gln Thr Cys Gly Ala Pro Pro Cys Asn Ile Tyr Gly Gly Gly
                   565                 570                 575
   Gly Asp Leu Lys Asn Glu Ser Asp Leu Phe Cys Pro Thr Asp Cys Phe
               580                 585                 590
   Arg Lys His Pro Glu Ala Thr Tyr Ser Arg Cys Gly Ala Gly Pro Trp
           595                 600                 605
   Leu Thr Pro Arg Cys Met Val Asp Tyr Pro Tyr Arg Leu Trp His Tyr
       610                 615                 620
   Pro Cys Thr Val Asn Phe Thr Leu Phe Lys Val Arg Met Phe Val Gly
   625                 630                 635                 640
   Gly Phe Glu His Arg Phe Thr Ala Ala Cys Asn Trp Thr Arg Gly Glu
                   645                 650                 655
   Arg Cys Asp Ile Glu Asp Arg Asp Arg Ser Glu Leu His Pro Leu Leu
               660                 665                 670
   His Ser Thr Thr Glu Leu Ala Ile Leu Pro Cys Ser Phe Thr Pro Met
           675                 680                 685
   Pro Ala Leu Ser Thr Gly Leu Ile His Leu His Gln Asn Ile Val Asp
       690                 695                 700
   Val Gln Tyr Leu Tyr Gly Val Gly Ser Gly Met Val Gly Trp Ala Val
   705                 710                 715                 720
   Lys Trp Glu Phe Val Ile Leu Val Phe Leu Leu Leu Ala Asp Ala Arg
                   725                 730                 735
   Val Cys Val Ala Leu Trp Leu Met Leu Met Ile Ser Gln Ala Glu Ala
               740                 745                 750
<210> 32
   <211> 752
   <212> PRT
   <213> Hepatitis C virus
<400> 32
   Met Ser Thr Leu Pro Lys Pro Gln Arg Lys Thr Lys Arg Asn Thr Ile
   1               5                   10                  15
   Arg Arg Pro Gln Asp Val Lys Phe Pro Gly Gly Gly Gln Ile Val Gly
               20                  25                  30
   Gly Val Tyr Val Leu Pro Arg Arg Gly Pro Arg Leu Gly Val Arg Ala
           35                  40                  45
   Thr Arg Lys Thr Ser Glu Arg Ser Gln Pro Arg Gly Arg Arg Gln Pro
       50                  55                  60
   Ile Pro Lys Ala Arg Arg Ser Glu Gly Arg Ser Trp Ala Gln Pro Gly
   65                  70                  75                  80
   Tyr Pro Trp Pro Leu Tyr Gly Asn Glu Gly Cys Gly Trp Ala Gly Trp
                   85                  90                  95
   Leu Leu Ser Pro Arg Gly Ser Arg Pro Ser Trp Gly Pro Asn Asp Pro
               100                 105                 110
   Arg Arg Arg Ser Arg Asn Leu Gly Lys Val Ile Asp Thr Leu Thr Cys
           115                 120                 125
   Gly Phe Ala Asp Leu Met Gly Tyr Ile Pro Leu Val Gly Ala Pro Val
       130                 135                 140
   Gly Gly Val Ala Arg Ala Leu Ala His Gly Val Arg Ala Leu Glu Asp
   145                 150                 155                 160
   Gly Ile Asn Phe Ala Thr Gly Asn Leu Pro Gly Cys Ser Phe Ser Ile
                   165                 170                 175
   Phe Leu Leu Ala Leu Phe Ser Cys Leu Ile His Pro Ala Ala Ser Leu
               180                 185                 190
   Gln Trp Arg Asn Thr Ser Gly Leu Tyr Val Leu Thr Asn Asp Cys Ser
           195                 200                 205
   Asn Ser Ser Ile Val Tyr Glu Ala Asp Asp Val Ile Leu His Thr Pro
       210                 215                 220
   Gly Cys Val Pro Cys Val Gln Asp Gly Asn Thr Ser Thr Cys Trp Thr
   225                 230                 235                 240
   Pro Val Thr Pro Thr Val Ala Val Arg Tyr Val Gly Ala Thr Thr Ala
                   245                 250                 255
   Ser Ile Arg Ser His Val Asp Leu Leu Val Gly Ala Ala Thr Met Cys
               260                 265                 270
   Ser Ala Leu Tyr Val Gly Asp Met Cys Gly Ala Val Phe Leu Val Gly
           275                 280                 285
   Gln Ala Phe Thr Phe Arg Pro Arg Arg His Gln Thr Val Gln Thr Cys
       290                 295                 300
   Asn Cys Ser Leu Tyr Pro Gly His Leu Ser Gly His Arg Met Ala Trp
   305                 310                 315                 320
   Asp Met Met Met Asn Trp Ser Pro Ala Ala Gly Met Val Val Ala His
                   325                 330                 335
   Ile Leu Arg Leu Pro Gln Thr Leu Phe Asp Ile Ile Ala Gly Ala His
               340                 345                 350
   Trp Gly Ile Leu Ala Gly Leu Ala Tyr Tyr Ser Met Gln Gly Asn Trp
           355                 360                 365
   Ala Lys Val Ala Ile Ile Met Val Met Phe Ser Gly Val Asp Ala Thr
       370                 375                 380
   Thr Tyr Thr Ser Gly Gly Ser Val Ala Gln Gln Ala Arg Gly Leu Ala
   385                 390                 395                 400
   Asp Leu Phe Ser Val Gly Ala Lys Gln Asn Leu Gln Leu Val Asn Thr
                   405                 410                 415
   Asn Gly Ser Trp His Ile Asn Ser Thr Ala Leu Asn Cys Asp Asp Ser
   420 425 430
   Ile Asn Thr Gly Phe Ile Ala Gly Leu Phe Tyr Tyr His Lys Phe Asn
   435 440 445
   Ser Thr Gly Cys Pro Gln Arg Leu Ser Asp Cys Lys Pro Ile Thr Phe
   450 455 460
   Phe Lys Gln Gly Trp Gly Pro Leu Thr Asp Ala Asn Ile Thr Gly Pro
   465 470 475 480
   Ser Asp Asp Lys Pro Tyr Cys Trp His Tyr Ala Pro Arg Arg Cys Gly
   485 490 495
   Val Val Pro Ala Ser Ser Val Cys Gly Pro Val Tyr Cys Phe Thr Pro
   500 505 510
   Ser Pro Val Val Val Gly Thr Thr Asp Ala Lys Gly Val Pro Thr Tyr
   515 520 525
   Thr Trp Gly Ala Asn Glu Thr Asp Val Phe Leu Leu Glu Ser Leu Arg
   530 535 540
   Pro Pro Ser Gly Arg Trp Phe Gly Cys Ala Trp Met Asn Ser Thr Gly
   545 550 555 560
   Phe Leu Lys Thr Cys Gly Ala Pro Pro Cys Asn Ile Tyr Gly Gly Gly
   565 570 575
   Gly Asn Pro His Asn Glu Ser Asp Leu Phe Cys Pro Thr Asp Cys Phe
   580 585 590
   Arg Lys His Pro Glu Ala Thr Tyr Ser Arg Cys Gly Ala Gly Pro Trp
   595 600 605
   Leu Thr Pro Arg Cys Met Val Asp Tyr Pro Tyr Arg Leu Trp His Tyr
   610 615 620
   Pro Cys Thr Val Asn Phe Thr Leu Phe Lys Met Arg Thr Phe Val Gly
   625 630 635 640
   Gly Phe Glu His Arg Phe Thr Ala Ala Cys Asn Trp Thr Arg Gly Glu
   645 650 655
   Arg Cys Asp Ile Glu Asp Arg Asp Arg Ser Glu Gln His Pro Leu Leu
   660 665 670
   His Ser Thr Thr Glu Leu Ala Ile Leu Pro Cys Ser Phe Thr Pro Met
   675 680 685
   Pro Ala Leu Ser Thr Gly Leu Ile His Leu His Gln Asn Ile Val Asp
   690 695 700
   Val Gln Tyr Leu Tyr Gly Val Gly Ser Gly Met Val Gly Trp Ala Leu
   705 710 715 720
   Lys Trp Glu Phe Val Ile Leu Val Phe Leu Leu Leu Ala Asp Ala Arg
   725 730 735
   Val Cys Val Ala Leu Trp Leu Met Leu Met Ile Ser Gln Ala Glu Ala
   740 745 750
<210> 33
   <211> 752
   <212> PRT
   <213> Hepatitis C virus
<400> 33
   Met Ser Thr Leu Pro Lys Pro Gln Arg Lys Thr Lys Arg Asn Thr Ile
   1               5                   10                  15
   Arg Arg Pro Gln Asp Val Lys Phe Pro Gly Gly Gly Gln Ile Val Gly
               20                  25                  30
   Gly Val Tyr Val Leu Pro Arg Arg Gly Pro Arg Leu Gly Val Arg Ala
           35                  40                  45
   Thr Arg Lys Thr Ser Glu Arg Ser Gln Pro Arg Gly Arg Arg Gln Pro
       50                  55                  60
   Ile Pro Lys Ala Arg Arg Ser Glu Gly Arg Ser Trp Ala Gln Pro Gly
   65                  70                  75                  80
   Tyr Pro Trp Pro Leu Tyr Gly Asn Glu Gly Cys Gly Trp Ala Gly Trp
                   85                  90                  95
   Leu Leu Ser Pro Arg Gly Ser Arg Pro Ser Trp Gly Pro Asn Asp Pro
               100                 105                 110
   Arg Arg Arg Ser Arg Asn Leu Gly Lys Val Ile Asp Thr Leu Thr Cys
           115                 120                 125
   Gly Phe Ala Asp Leu Met Gly Tyr Ile Pro Leu Val Gly Ala Pro Val
   130 135 140
   Gly Gly Val Ala Arg Ala Leu Ala His Gly Val Arg Ala Leu Glu Asp
   145 150 155 160
   Gly Ile Asn Phe Ala Thr Gly Asn Leu Pro Gly Cys Ser Phe Ser Ile
   165 170 175
   Phe Leu Leu Ala Leu Phe Ser Cys Leu Ile His Pro Ala Ala Ser Leu
   180 185 190
   Gln Trp Arg Asn Thr Ser Gly Leu Tyr Val Leu Thr Asn Asp Cys Ser
   195 200 205
   Asn Ser Ser Ile Val Tyr Glu Ala Asp Asp Val Ile Leu His Thr Pro
   210 215 220
   Gly Cys Val Pro Cys Val Gln Asp Gly Asn Thr Ser Thr Cys Trp Thr
   225 230 235 240
   Pro Val Thr Pro Thr Val Ala Val Arg Tyr Val Gly Ala Thr Thr Ala
   245 250 255
   Ser Ile Arg Ser His Val Asp Leu Leu Val Gly Ala Ala Thr Met Cys
   260 265 270
   Ser Ala Leu Tyr Val Gly Asp Met Cys Gly Ala Val Phe Leu Val Gly
   275 280 285
   Gln Ala Phe Thr Phe Arg Pro Arg Arg His Gln Thr Val Gln Thr Cys
   290 295 300
   Asn Cys Ser Leu Tyr Pro Gly His Leu Ser Gly His Arg Met Ala Trp
   305 310 315 320
   Asp Met Met Met Asn Trp Ser Pro Ala Ala Gly Met Val Val Ala His
   325 330 335
   Ile Leu Arg Leu Pro Gln Thr Leu Phe Asp Ile Ile Ala Gly Ala His
   340 345 350
   Trp Gly Ile Leu Ala Gly Leu Ala Tyr Tyr Ser Met Gln Gly Asn Trp
   355 360 365
   Ala Lys Val Ala Ile Ile Met Val Met Phe Ser Gly Val Asp Ala Thr
   370 375 380
   Thr Tyr Thr Ser Gly Gly Ser Val Ala Gln Gln Ala Arg Gly Leu Ala
   385 390 395 400
   Asp Leu Phe Ser Val Gly Ala Lys Gln Asn Leu Gln Leu Val Asn Thr
   405 410 415
   Asn Gly Ser Trp His Ile Asn Ser Thr Ala Leu Asn Cys Asp Asp Ser
   420 425 430
   Ile Asn Thr Gly Phe Ile Ala Gly Leu Phe Tyr Tyr His Lys Phe Asn
   435 440 445
   Ser Thr Gly Cys Pro Gln Arg Leu Ser Asp Cys Lys Pro Ile Thr Phe
   450 455 460
   Phe Lys Gln Gly Trp Gly Pro Leu Thr Asp Ala Asn Ile Thr Gly Pro
   465 470 475 480
   Ser Asp Asp Lys Pro Tyr Cys Trp His Tyr Ala Pro Arg Arg Cys Gly
   485 490 495
   Val Val Pro Ala Ser Ser Val Cys Gly Pro Val Tyr Cys Phe Thr Pro
   500 505 510
   Ser Pro Val Val Val Gly Thr Thr Asp Ala Lys Gly Val Pro Thr Tyr
   515 520 525
   Thr Trp Gly Ala Asn Glu Thr Asp Val Phe Leu Leu Glu Ser Leu Arg
   530 535 540
   Pro Pro Ser Gly Arg Trp Phe Gly Cys Ala Trp Met Asn Ser Thr Gly
   545 550 555 560
   Phe Leu Lys Thr Cys Gly Ala Pro Pro Cys Asn Ile Tyr Gly Gly Gly
   565 570 575
   Gly Asn Pro His Asn Glu Ser Asp Leu Phe Cys Pro Thr Asp Cys Phe
   580 585 590
   Arg Lys His Pro Glu Ala Thr Tyr Ser Arg Cys Gly Ala Gly Pro Trp
   595 600 605
   Leu Thr Pro Arg Cys Met Val Asp Tyr Pro Tyr Arg Leu Trp His Tyr
   610 615 620
   Pro Cys Thr Val Asn Phe Thr Leu Phe Lys Met Arg Thr Phe Val Gly
   625 630 635 640
   Gly Phe Glu His Arg Phe Thr Ala Ala Cys Asn Trp Thr Arg Gly Glu
   645 650 655
   Arg Cys Asp Ile Glu Asp Arg Asp Arg Ser Glu Gln His Pro Leu Leu
   660 665 670
   His Ser Thr Thr Glu Leu Ala Ile Leu Pro Cys Ser Phe Thr Pro Met
   675 680 685
   Pro Ala Leu Ser Thr Gly Leu Ile His Leu His Gln Asn Ile Val Asp
   690 695 700
   Val Gln Tyr Leu Tyr Gly Val Gly Ser Gly Met Val Gly Trp Ala Leu
   705 710 715 720
   Lys Trp Glu Phe Val Ile Leu Val Phe Leu Leu Leu Ala Asp Ala Arg
   725 730 735
   Val Cys Val Ala Leu Trp Leu Met Leu Met Ile Ser Gln Ala Glu Ala
   740 745 750
<210> 34
   <211> 752
   <212> PRT
   <213> Hepatitis C virus
<400> 34
   Met Ser Thr Leu Pro Lys Pro Gln Arg Lys Thr Lys Arg Asn Thr Ile
   1               5                   10                  15
   Arg Arg Pro Gln Asp Val Lys Phe Pro Gly Gly Gly Gln Ile Val Gly
               20                  25                  30
   Gly Val Tyr Val Leu Pro Arg Arg Gly Pro Arg Leu Gly Val Cys Ala
           35                  40                  45
   Thr Arg Lys Thr Ser Glu Arg Ser Gln Pro Arg Gly Arg Arg Gln Pro
       50                  55                  60
   Ile Pro Lys Ala Arg Arg Ser Glu Gly Arg Ser Trp Ala Gln Pro Gly
   65                  70                  75                  80
   Tyr Pro Trp Pro Leu Tyr Gly Asn Glu Gly Cys Gly Trp Ala Gly Trp
                   85                  90                  95
   Leu Leu Ser Pro Arg Gly Ser Arg Pro Ser Trp Gly Pro Asn Asp Pro
               100                 105                 110
   Arg Arg Arg Ser Arg Asn Leu Gly Lys Val Ile Asp Thr Leu Thr Cys
           115                 120                 125
   Gly Phe Ala Asp Leu Met Gly Tyr Ile Pro Leu Val Gly Ala Pro Val
       130                 135                 140
   Gly Gly Val Ala Arg Ala Leu Ala His Gly Val Arg Ala Leu Glu Asp
   145                 150                 155                 160
   Gly Ile Asn Phe Ala Thr Gly Asn Leu Pro Gly Cys Ser Phe Ser Ile
                   165                 170                 175
   Phe Leu Leu Ala Leu Phe Ser Cys Leu Val His Pro Ala Ala Ser Leu
               180                 185                 190
   Glu Trp Arg Asn Thr Ser Gly Leu Tyr Val Leu Thr Asn Asp Cys Pro
           195                 200                 205
   Asn Ser Ser Ile Val Tyr Glu Ala Asp Asp Val Ile Leu His Thr Pro
       210                 215                 220
   Gly Cys Ile Pro Cys Val Gln Asp Gly Asn Lys Ser Thr Cys Trp Thr
   225                 230                 235                 240
   Ser Val Thr Pro Thr Val Ala Val Lys Tyr Val Gly Ala Thr Thr Ala
                   245                 250                 255
   Ser Ile Arg Ser His Val Asp Leu Leu Val Gly Ala Ala Thr Met Cys
               260                 265                 270
   Ser Ala Leu Tyr Val Gly Asp Met Cys Gly Ala Val Phe Leu Val Gly
           275                 280                 285
   Gln Ala Phe Thr Phe Arg Pro Arg Arg His Gln Thr Val Gln Thr Cys
       290                 295                 300
   Asn Cys Ser Leu Tyr Pro Gly His Leu Ser Gly His Arg Met Ala Trp
   305                 310                 315                 320
   Asp Met Met Met Asn Trp Ser Pro Ala Val Gly Met Val Val Ala His
                   325                 330                 335
   Val Leu Arg Leu Pro Gln Thr Leu Phe Asp Ile Ile Ala Gly Ala His
               340                 345                 350
   Trp Gly Ile Leu Ala Gly Leu Ala Tyr Tyr Ser Met Gln Gly Asn Trp
           355                 360                 365
   Ala Lys Val Ala Ile Ile Met Val Met Phe Ser Gly Val Asp Ala Thr
   370 375 380
   Thr Tyr Thr Thr Gly Gly Asn Ala Ala Arg Gly Ala Ser Gly Ile Val
   385 390 395 400
   Ser Leu Phe Thr Pro Gly Ala Lys Gln Asn Leu Gln Leu Val Asn Thr
   405 410 415
   Asn Gly Ser Trp His Ile Asn Arg Thr Ala Leu Asn Cys Asn Asp Ser
   420 425 430
   Ile Asn Thr Gly Phe Ile Ala Gly Leu Ile Tyr Tyr His Lys Phe Asn
   435 440 445
   Ser Thr Gly Cys Pro Gln Arg Leu Ser Ser Cys Lys Pro Ile Thr Phe
   450 455 460
   Phe Arg Gln Gly Trp Gly Ser Leu Thr Asp Ala Asn Ile Thr Gly Pro
   465 470 475 480
   Ser Asp Asp Lys Pro Tyr Cys Trp His Tyr Pro Pro Arg Pro Cys Asp
   485 490 495
   Thr Ile Arg Ala Ser Ser Val Cys Gly Pro Val Tyr Cys Phe Thr Pro
   500 505 510
   Ser Pro Val Val Val Gly Thr Thr Asp Ala Lys Gly Ala Pro Thr Tyr
   515 520 525
   Asn Trp Gly Ala Asn Glu Thr Asp Met Phe Leu Leu Gln Ser Leu Arg
   530 535 540
   Pro Pro Ser Gly Arg Trp Phe Gly Cys Thr Trp Met Asn Ser Thr Gly
   545 550 555 560
   Phe Thr Lys Thr Cys Gly Ala Pro Pro Cys Asn Ile Tyr Gly Gly Gly
   565 570 575
   Gly Asn Leu Asn Asn Glu Ser Asp Leu Phe Cys Pro Thr Asp Cys Phe
   580 585 590
   Arg Lys His Pro Glu Ala Thr Tyr Ser Arg Cys Gly Ala Gly Pro Trp
   595 600 605
   Leu Thr Pro Arg Cys Leu Val Asp Tyr Pro Tyr Arg Leu Trp His Tyr
   610 615 620
   Pro Cys Thr Val Asn Phe Thr Leu Phe Arg Met Arg Thr Phe Val Gly
   625 630 635 640
   Gly Phe Glu His Arg Phe Thr Ala Ala Cys Asn Trp Thr Arg Gly Glu
   645 650 655
   Arg Cys Asn Ile Glu Asp Arg Asp Arg Ser Glu Gln His Pro Leu Leu
   660 665 670
   His Ser Thr Thr Glu Leu Ala Ile Leu Pro Cys Ser Phe Thr Pro Met
   675 680 685
   Pro Ala Leu Ser Thr Gly Leu Ile His Leu His Gln Asn Ile Val Asp
   690 695 700
   Val Gln Tyr Leu Tyr Gly Ile Gly Ser Gly Val Val Gly Trp Ala Leu
   705 710 715 720
   Lys Trp Glu Phe Val Ile Leu Val Phe Leu Leu Leu Ala Asp Ala Arg
   725 730 735
   Val Cys Val Ala Leu Trp Leu Met Leu Met Ile Ser Gln Ala Glu Ala
   740 745 750
<210> 35
   <211> 752
   <212> PRT
   <213> Hepatitis C virus
<400> 35
   Met Ser Thr Leu Pro Lys Pro Gln Arg Lys Thr Lys Arg Asn Thr Ile
   1               5                   10                  15
   Arg Arg Pro Gln Asp Val Lys Phe Pro Gly Gly Gly Val Ile Tyr Val
               20                  25                  30
   Gly Val Tyr Val Leu Pro Arg Arg Gly Pro Arg Leu Gly Val Arg Ala
           35                  40                  45
   Thr Arg Lys Thr Ser Glu Arg Ser Gln Pro Arg Gly Arg Arg Lys Pro
       50                  55                  60
   Ile Pro Lys Ala Arg Arg Ser Glu Gly Arg Ser Trp Ala Gln Pro Gly
   65                  70                  75                  80
   Tyr Pro Trp Pro Leu Tyr Gly Asn Glu Gly Cys Gly Trp Ala Gly Trp
   85 90 95
   Leu Leu Ser Pro Arg Gly Ser Arg Pro Asn Trp Ala Pro Asn Asp Pro
   100 105 110
   Arg Arg Arg Ser Arg Asn Leu Gly Lys Val Ile Asp Thr Leu Thr Cys
   115 120 125
   Gly Phe Ala Asp Leu Met Gly Tyr Ile Pro Leu Val Gly Ala Pro Leu
   130 135 140
   Gly Gly Ala Ala Arg Ala Leu Ala His Gly Val Arg Ala Leu Glu Asp
   145 150 155 160
   Gly Ile Asn Phe Ala Thr Gly Asn Leu Pro Gly Cys Ser Phe Ser Ile
   165 170 175
   Phe Leu Leu Ala Leu Phe Ser Cys Leu Ile His Pro Ala Ala Ser Leu
   180 185 190
   Glu Trp Arg Asn Thr Ser Gly Leu Tyr Val Leu Thr Asn Asp Cys Ser
   195 200 205
   Asn Ser Ser Ile Val Tyr Glu Ala Asp Asp Val Ile Leu His Thr Pro
   210 215 220
   Gly Cys Ile Pro Cys Val Gln Asp Gly Asn Thr Ser Thr Cys Trp Thr
   225 230 235 240
   Pro Val Thr Pro Thr Val Ala Val Arg Tyr Val Gly Ala Thr Thr Ala
   245 250 255
   Ser Ile Arg Ser His Val Asp Leu Leu Val Gly Ala Gly Thr Met Cys
   260 265 270
   Ser Ala Leu Tyr Val Gly Asp Met Cys Gly Pro Val Phe Leu Val Gly
   275 280 285
   Gln Ala Phe Thr Phe Arg Pro Arg Arg His Arg Thr Val Gln Thr Cys
   290 295 300
   Asn Cys Ser Leu Tyr Pro Gly His Leu Ser Gly Gln Arg Met Ala Trp
   305 310 315 320
   Asp Met Met Met Asn Trp Ser Pro Ala Val Gly Met Val Val Ala His
   325 330 335
   Ile Leu Arg Leu Pro Gln Thr Leu Phe Asp Val Val Ala Gly Ala His
               340                 345                 350
   Trp Gly Ile Ile Ala Gly Leu Ala Tyr Tyr Ser Met Gln Gly Asn Trp
           355                 360                 365
   Ala Lys Val Ala Ile Ile Met Val Met Phe Ser Gly Val Asp Ala Ser
       370                 375                 380
   Thr His Val Thr Ala Gly Gln Ala Ala Arg Asn Ala Tyr Gly Ile Thr
   385                 390                 395                 400
   Ser Leu Phe Ser Val Gly Ala Lys Gln Asn Leu Gln Leu Ile Asn Thr
                   405                 410                 415
   Asn Gly Ser Trp His Ile Asn Arg Thr Ala Leu Asn Cys Asn Glu Ser
               420                 425                 430
   Ile Asn Thr Gly Phe Ile Ala Gly Leu Phe Tyr Tyr His Lys Phe Asn
           435                 440                 445
   Ser Thr Gly Cys Pro Gln Arg Leu Ser Ser Cys Lys Pro Ile Thr Phe
       450                 455                 460
   Phe Lys Gln Gly Trp Gly Pro Leu Thr Asp Ala Asn Ile Thr Gly Pro
   465                 470                 475                 480
   Ser Asp Asp Lys Pro Tyr Cys Trp His Tyr Ala Pro Arg Pro Cys Gly
                   485                 490                 495
   Ile Val Pro Ala Leu Asn Val Cys Gly Pro Val Tyr Cys Phe Thr Pro
               500                 505                 510
   Ser Pro Val Val Val Gly Thr Thr Asp Ala Lys Gly Ala Pro Thr Tyr
           515                 520                 525
   Thr Trp Gly Ala Asn Lys Thr Asp Val Phe Leu Leu Glu Ser Leu Arg
       530                 535                 540
   Pro Pro Ser Gly Arg Trp Phe Gly Cys Thr Trp Met Asn Ser Thr Gly
   545                 550                 555                 560
   Phe Val Lys Thr Cys Gly Ala Pro Pro Cys Asn Ile Tyr Gly Asp Gly
                   565                 570                 575
   Arg Asp Ala Gln Asn Glu Ser Asp Leu Phe Cys Pro Thr Asp Cys Phe
               580                 585                 590
   Arg Lys His Pro Glu Ala Thr Tyr Ser Arg Cys Gly Ala Gly Pro Trp
   595 600 605
   Leu Thr Pro Arg Cys Leu Val Asp Tyr Pro Tyr Arg Leu Trp His Tyr
   610 615 620
   Pro Cys Thr Val Asn Phe Thr Leu Phe Lys Val Arg Met Phe Val Gly
   625 630 635 640
   Gly Phe Glu His Arg Phe Thr Ala Ala Cys Asn Trp Thr Arg Gly Glu
   645 650 655
   Arg Cys Asp Ile Glu Asp Arg Asp Arg Ser Glu Gln His Pro Leu Leu
   660 665 670
   His Ser Thr Thr Glu Leu Ala Ile Leu Pro Cys Ser Phe Thr Pro Met
   675 680 685
   Pro Ala Leu Ser Thr Gly Leu Ile His Leu His Gln Asn Ile Val Asp
   690 695 700
   Val Gln Tyr Leu Tyr Gly Ile Gly Ser Gly Met Val Gly Trp Ala Leu
   705 710 715 720
   Lys Trp Glu Phe Val Ile Leu Ile Phe Leu Leu Leu Ala Asp Ala Arg
   725 730 735
   Val Cys Val Ala Leu Trp Leu Ile Leu Thr Ile Ser Gln Ala Glu Ala
   740 745 750
<210> 36
   <211> 754
   <212> PRT
   <213> Hepatitis C virus
<400> 36
   Met Ser Thr Leu Pro Lys Pro Lys Arg Gln Thr Lys Arg Asn Thr Leu
   1               5                   10                  15
   Arg Arg Pro Lys Asn Val Lys Phe Pro Ala Gly Gly Gln Ile Val Gly
               20                  25                  30
   Glu Val Tyr Val Leu Pro Arg Arg Gly Pro Gln Leu Gly Val Arg Glu
           35                  40                  45
   Val Arg Lys Thr Ser Glu Arg Ser Gln Pro Arg Gly Arg Arg Gln Pro
       50                  55                  60
   Thr Pro Lys Ala Arg Pro Arg Glu Gly Arg Ser Trp Ala Gln Pro Gly
   65 70 75 80
   Tyr Pro Trp Pro Leu Tyr Gly Asn Glu Gly Cys Gly Trp Ala Gly Trp
   85 90 95
   Leu Leu Pro Pro Arg Gly Ser Arg Pro Ser Trp Gly Gln Asn Asp Pro
   100 105 110
   Arg Arg Arg Ser Arg Asn Leu Gly Lys Val Ile Asp Thr Leu Thr Cys
   115 120 125
   Gly Phe Ala Asp Leu Met Gly Tyr Ile Pro Leu Ile Gly Ala Pro Val
   130 135 140
   Gly Gly Val Ala Arg Ala Leu Ala His Gly Val Arg Ala Leu Glu Asp
   145 150 155 160
   Gly Val Asn Tyr Ala Thr Gly Asn Leu Pro Gly Cys Ser Phe Ser Ile
   165 170 175
   Phe Leu Leu Ala Leu Phe Ser Cys Leu Thr Cys Pro Ala Ser Ser Leu
   180 185 190
   Glu Tyr Arg Asn Ala Ser Gly Leu Tyr Leu Leu Thr Asn Asp Cys Ser
   195 200 205
   Asn Arg Ser Ile Val Tyr Glu Ala Asp Asp Val Ile Leu His Leu Pro
   210 215 220
   Gly Cys Val Pro Cys Val Glu Thr Asp Asn Asn Asn Thr Ser Cys Trp
   225 230 235 240
   Thr Pro Ile Ser Pro Thr Val Ala Val Lys His Pro Gly Val Thr Thr
   245 250 255
   Ala Ser Ile Arg Asn His Val Asn Met Leu Val Ala Pro Pro Thr Leu
   260 265 270
   Cys Ser Ala Leu Tyr Val Glu Asp Ala Phe Gly Ala Val Ser Leu Val
   275 280 285
   Gly Gln Ala Phe Thr Phe Arg Pro Arg Gln His Lys Thr Val Gln Thr
   290 295 300
   Cys Asn Cys Ser Ile Tyr Pro Gly His Val Ser Gly His Arg Met Ala
   305 310 315 320
   Trp Asp Met Met Met Asn Trp Ser Pro Ala Ile Gly Leu Val Ile Ser
                   325                 330                 335
   His Leu Met Arg Leu Pro Gln Thr Phe Phe Asp Leu Val Val Gly Ala
               340                 345                 350
   His Trp Gly Val Met Ala Gly Leu Ala Tyr Phe Ser Met Gln Gly Asn
           355                 360                 365
   Trp Ala Lys Val Val Ile Val Leu Ile Met Phe Ser Gly Val Asp Ala
       370                 375                 380
   Thr Thr His Thr Thr Gly Gly Ser Ala Ala Gln Ala Thr Ala Gly Phe
   385                 390                 395                 400
   Thr Ser Phe Phe Thr Arg Gly Pro Ser Gln Asn Leu Gln Leu Val Asn
                   405                 410                 415
   Ser Asn Gly Ser Trp His Ile Asn Ser Thr Ala Leu Asn Cys Asn Asp
               420                 425                 430
   Ser Leu Asn Thr Gly Phe Ile Ala Gly Leu Phe Tyr Tyr His Lys Phe
           435                 440                 445
   Asn Ser Ser Gly Cys Pro Glu Arg Met Ser Ser Cys Lys Pro Ile Thr
       450                 455                 460
   Tyr Phe Asn Gln Gly Trp Gly Pro Leu Thr Asp Ala Asn Ile Asn Gly
   465                 470                 475                 480
   Pro Ser Glu Asp Arg Pro Tyr Cys Trp His Tyr Pro Pro Arg Pro Cys
                   485                 490                 495
   Asn Ile Thr Lys Pro Leu Asn Val Cys Gly Pro Val Tyr Cys Phe Thr
               500                 505                 510
   Pro Ser Pro Val Val Val Gly Thr Thr Asp Ile Lys Gly Leu Pro Thr
           515                 520                 525
   Tyr Arg Phe Gly Val Asn Glu Ser Asp Val Phe Leu Leu Thr Ser Leu
       530                 535                 540
   Arg Pro Pro Gln Gly Arg Trp Phe Gly Cys Val Trp Met Asn Ser Thr
   545                 550                 555                 560
   Gly Phe Val Lys Thr Cys Gly Ala Pro Pro Cys Asn Ile Tyr Gly Gly
   565 570 575
   Met Lys Asp Ile Glu Ala Asn Gln Thr His Leu Lys Cys Pro Thr Asp
   580 585 590
   Cys Phe Arg Lys His His Asp Ala Thr Phe Thr Arg Cys Gly Ser Gly
   595 600 605
   Pro Trp Leu Thr Pro Arg Cys Leu Val Asp Tyr Pro Tyr Arg Leu Trp
   610 615 620
   His Tyr Pro Cys Thr Val Asn Phe Ser Ile Phe Lys Val Arg Met Phe
   625 630 635 640
   Val Gly Gly His Glu His Arg Phe Ser Ala Ala Cys Asn Trp Thr Arg
   645 650 655
   Gly Glu Arg Cys Asp Leu Glu Asp Arg Asp Arg Ser Glu Gln Gln Pro
   660 665 670
   Leu Leu His Ser Thr Thr Asp Ser Leu Ile Leu Pro Cys Ser Phe Thr
   675 680 685
   Pro Met Arg Arg Leu Ser Thr Gly Leu Ile His Leu His Gln Asn Ile
   690 695 700
   Val Asp Val Gln Tyr Leu Tyr Gly Val Gly Ser Ala Val Val Gly Trp
   705 710 715 720
   Ala Leu Lys Trp Glu Phe Val Val Leu Val Phe Leu Leu Leu Ala Asp
   725 730 735
   Ala Arg Val Cys Val Ala Leu Trp Met Met Leu Leu Ile Ser Gln Ala
   740 745 750
   Glu Ala
<210> 37
   <211> 751
   <212> PRT
   <213> Hepatitis C virus
<400> 37
   Met Ser Thr Leu Pro Lys Pro Gln Arg Ile Thr Lys Arg Asn Ile Asn
   1               5                   10                  15
   Arg Arg Pro Gln Asp Val Lys Phe Pro Gly Gly Gly Gln Ile Val Gly
   20 25 30
   Gly Val Tyr Val Leu Pro Arg Arg Gly Pro Lys Leu Gly Val Arg Ala
   35 40 45
   Val Arg Lys Thr Ser Glu Arg Ser Gln Pro Arg Ser Arg Arg Gln Pro
   50 55 60
   Ile Pro Arg Ala Arg Arg Thr Glu Gly Arg Ser Trp Ala Gln Pro Gly
   65 70 75 80
   Tyr Pro Trp Pro Leu Tyr Gly Asn Glu Gly Cys Gly Trp Ala Gly Trp
   85 90 95
   Leu Leu Ser Pro Arg Gly Ser Arg Pro Ser Trp Gly Pro Asn Asp Pro
   100 105 110
   Arg Arg Arg Ser Arg Asn Leu Gly Lys Val Ile Asp Thr Leu Thr Cys
   115 120 125
   Gly Phe Ala Asp Leu Met Gly Tyr Ile Pro Leu Val Gly Ala Pro Val
   130 135 140
   Gly Gly Val Ala Arg Ala Leu Ala His Gly Val Arg Ala Leu Glu Asp
   145 150 155 160
   Gly Ile Asn Phe Ala Thr Gly Asn Leu Pro Gly Cys Ser Phe Ser Ile
   165 170 175
   Phe Leu Leu Ala Leu Leu Ser Cys Leu Leu Thr Pro Thr Ala Gly Leu
   180 185 190
   Glu Tyr Arg Asn Ala Ser Gly Leu Tyr Thr Val Thr Asn Asp Cys Ser
   195 200 205
   Asn Gly Ser Ile Val Tyr Glu Ala Gly Asp Val Ile Leu His Leu Pro
   210 215 220
   Gly Cys Ile Pro Cys Val Arg Leu Asn Asn Ala Ser Lys Cys Trp Thr
   225 230 235 240
   Pro Val Ser Pro Thr Val Ala Val Ser Arg Pro Gly Ala Ala Thr Ala
   245 250 255
   Ser Leu Arg Thr His Val Asp Met Met Val Gly Ala Ala Thr Leu Cys
   260 265 270
   Ser Ala Leu Tyr Val Gly Asp Leu Cys Gly Ala Leu Phe Leu Val Gly
           275                 280                 285
   Gln Gly Phe Ser Trp Arg His Arg Gln His Trp Thr Val Gln Asp Cys
       290                 295                 300
   Asn Cys Ser Ile Tyr Pro Gly His Leu Thr Gly His Arg Met Ala Trp
   305                 310                 315                 320
   Asp Met Met Met Asn Trp Ser Pro Ala Met Thr Leu Ile Val Ser Gln
                   325                 330                 335
   Val Leu Arg Leu Pro Gln Thr Met Phe Asp Leu Val Ile Gly Ala His
               340                 345                 350
   Trp Gly Val Met Ala Gly Val Ala Tyr Tyr Ser Met Gln Gly Asn Trp
           355                 360                 365
   Ala Lys Val Phe Leu Val Leu Cys Leu Phe Ser Gly Val Asp Ala Ser
       370                 375                 380
   Thr Thr Ile Thr Gly Gly Val Ala Ala Ser Gly Ala Phe Thr Ile Thr
   385                 390                 395                 400
   Ser Leu Phe Ser Thr Gly Ala Lys Gln Pro Leu His Leu Val Asn Thr
                   405                 410                 415
   Asn Gly Ser Trp His Ile Asn Arg Thr Ala Leu Asn Cys Asn Asp Ser
               420                 425                 430
   Leu Asn Thr Gly Phe Ile Ala Gly Leu Leu Tyr Tyr His Lys Phe Asn
           435                 440                 445
   Ser Ser Gly Cys Val Glu Arg Met Ser Ala Cys Ser Pro Leu Asp Arg
       450                 455                 460
   Phe Ala Gln Gly Trp Gly Pro Leu Gly Pro Ala Asn Ile Ser Gly Pro
   465                 470                 475                 480
   Ser Ser Glu Lys Pro Tyr Cys Trp His Tyr Ala Pro Arg Pro Cys Asp
                   485                 490                 495
   Thr Val Pro Ala Gln Ser Val Cys Gly Pro Val Tyr Cys Phe Thr Pro
               500                 505                 510
   Ser Pro Val Val Val Gly Ala Thr Asp Lys Arg Gly Ala Pro Thr Tyr
           515                 520                 525
   Thr Trp Gly Glu Asn Glu Ser Asp Val Phe Leu Leu Glu Ser Ala Arg
   530 535 540
   Pro Pro Thr Glu Pro Trp Phe Gly Cys Thr Trp Met Asn Gly Ser Gly
   545 550 555 560
   Tyr Val Lys Thr Cys Gly Ala Pro Pro Cys His Ile Tyr Gly Gly Arg
   565 570 575
   Glu Gly Lys Ser Asn Asn Ser Leu Val Cys Pro Thr Asp Cys Phe Arg
   580 585 590
   Lys His Pro Asp Ala Thr Tyr Asn Arg Cys Gly Ala Gly Pro Trp Leu
   595 600 605
   Thr Pro Arg Cys Leu Val Asp Tyr Pro Tyr Arg Leu Trp His Tyr Pro
   610 615 620
   Cys Thr Val Asn Tyr Thr Ile Phe Lys Val Arg Met Phe Val Gly Gly
   625 630 635 640
   Leu Glu His Arg Phe Asn Ala Ala Cys Asn Trp Thr Arg Gly Glu Arg
   645 650 655
   Cys Asn Leu Glu Asp Arg Asp Arg Ser Glu Met Tyr Pro Leu Leu His
   660 665 670
   Ser Thr Thr Glu Gln Ala Ile Leu Pro Cys Ser Phe Val Pro Ile Pro
   675 680 685
   Ala Leu Ser Thr Gly Leu Ile His Leu His Gln Asn Ile Val Asp Val
   690 695 700
   Gln Tyr Leu Tyr Gly Ile Ser Ser Gly Leu Val Gly Trp Ala Ile Lys
   705 710 715 720
   Trp Glu Phe Val Ile Leu Ile Phe Leu Leu Leu Ala Asp Ala Arg Val
   725 730 735
   Cys Val Val Leu Trp Met Met Met Leu Ile Ser Gln Ala Glu Ala
   740 745 750
<210> 38
   <211> 745
   <212> PRT
   <213> Hepatitis C virus
<220>
   <221> misc_feature
   <222> (198)..(198)
   <223> Xaa can be any naturally occurring amino acid
<220>
   <221> misc_feature
   <222> (217)..(217)
   <223> Xaa can be any naturally occurring amino acid
<220>
   <221> misc_feature
   <222> (223)..(223)
   <223> Xaa can be any naturally occurring amino acid
<220>
   <221> misc_feature
   <222> (235)..(235)
   <223> Xaa can be any naturally occurring amino acid
<220>
   <221> misc_feature
   <222> (242)..(242)
   <223> Xaa can be any naturally occurring amino acid
<220>
   <221> misc_feature
   <222> (268)..(268)
   <223> Xaa can be any naturally occurring amino acid
<220>
   <221> misc_feature
   <222> (297)..(297)
   <223> Xaa can be any naturally occurring amino acid
<220>
   <221> misc_feature
   <222> (313)..(313)
   <223> Xaa can be any naturally occurring amino acid
<220>
   <221> misc_feature
   <222> (329)..(329)
   <223> Xaa can be any naturally occurring amino acid
<220>
   <221> misc_feature
   <222> (344)..(344)
   <223> Xaa can be any naturally occurring amino acid
<220>
   <221> misc_feature
   <222> (379)..(379)
   <223> Xaa can be any naturally occurring amino acid
<220>
   <221> misc_feature
   <222> (390)..(390)
   <223> Xaa can be any naturally occurring amino acid
<220>
   <221> misc_feature
   <222> (392)..(392)
   <223> Xaa can be any naturally occurring amino acid
<220>
   <221> misc_feature
   <222> (394)..(394)
   <223> Xaa can be any naturally occurring amino acid
<220>
   <221> misc_feature
   <222> (396)..(396)
   <223> Xaa can be any naturally occurring amino acid
<220>
   <221> misc_feature
   <222> (402)..(404)
   <223> Xaa can be any naturally occurring amino acid
<220>
   <221> misc_feature
   <222> (406)..(406)
   <223> Xaa can be any naturally occurring amino acid
<220>
   <221> misc_feature
   <222> (411)..(411)
   <223> Xaa can be any naturally occurring amino acid
<220>
   <221> misc_feature
   <222> (423)..(423)
   <223> Xaa can be any naturally occurring amino acid
<220>
   <221> misc_feature
   <222> (430)..(430)
   <223> Xaa can be any naturally occurring amino acid
<220>
   <221> misc_feature
   <222> (441)..(441)
   <223> Xaa can be any naturally occurring amino acid
<220>
   <221> misc_feature
   <222> (443)..(445)
   <223> Xaa can be any naturally occurring amino acid
<220>
   <221> misc_feature
   <222> (473)..(474)
   <223> Xaa can be any naturally occurring amino acid
<220>
   <221> misc_feature
   <222> (479)..(479)
   <223> Xaa can be any naturally occurring amino acid
<220>
   <221> misc_feature
   <222> (527)..(527)
   <223> Xaa can be any naturally occurring amino acid
<220>
   <221> misc_feature
   <222> (530)..(530)
   <223> Xaa can be any naturally occurring amino acid
<220>
   <221> misc_feature
   <222> (569)..(569)
   <223> Xaa can be any naturally occurring amino acid
<220>
   <221> misc_feature
   <222> (579)..(579)
   <223> Xaa can be any naturally occurring amino acid
<220>
   <221> misc_feature
   <222> (590)..(590)
   <223> Xaa can be any naturally occurring amino acid
<220>
   <221> misc_feature
   <222> (607)..(607)
   <223> Xaa can be any naturally occurring amino acid
<220>
   <221> misc_feature
   <222> (625)..(625)
   <223> Xaa can be any naturally occurring amino acid
<220>
   <221> misc_feature
   <222> (709)..(709)
   <223> Xaa can be any naturally occurring amino acid
<400> 38
   Met Ser Thr Asn Pro Lys Pro Gln Arg Lys Thr Lys Arg Asn Thr Asn
   1               5                   10                  15
   Arg Arg Pro Gln Asp Val Lys Phe Pro Gly Gly Gly Gln Ile Val Gly
               20                  25                  30
   Gly Val Tyr Leu Leu Pro Arg Arg Gly Pro Arg Leu Gly Val Arg Ala
           35                  40                  45
   Thr Arg Lys Thr Ser Glu Arg Ser Gln Pro Arg Gly Arg Arg Gln Pro
       50                  55                  60
   Ile Pro Lys Ala Arg Arg Pro Glu Gly Arg Thr Trp Ala Gln Pro Gly
   65                  70                  75                  80
   Tyr Pro Trp Pro Leu Tyr Gly Asn Glu Gly Cys Gly Trp Ala Gly Trp
                   85                  90                  95
   Leu Leu Ser Pro Arg Gly Ser Arg Pro Ser Trp Gly Pro Thr Asp Pro
               100                 105                 110
   Arg Arg Arg Ser Arg Asn Leu Gly Lys Val Ile Asp Thr Leu Thr Cys
           115                 120                 125
   Gly Phe Ala Asp Leu Met Gly Tyr Ile Pro Leu Val Gly Ala Pro Leu
       130                 135                 140
   Gly Gly Ala Ala Arg Ala Leu Ala His Gly Val Arg Val Leu Glu Asp
   145                 150                 155                 160
   Gly Val Asn Tyr Ala Thr Gly Asn Leu Pro Gly Cys Ser Phe Ser Ile
                   165                 170                 175
   Phe Leu Leu Ala Leu Leu Ser Cys Leu Thr Val Pro Ala Ser Ala Tyr
               180                 185                 190
   Gln Val Arg Asn Ser Xaa Gly Leu Tyr His Val Thr Asn Asp Cys Pro
           195                 200                 205
   Asn Ser Ser Ile Val Tyr Glu Ala Xaa Asp Ala Ile Leu His Xaa Pro
       210                 215                 220
   Gly Cys Val Pro Cys Val Arg Glu Gly Asn Xaa Ser Arg Cys Trp Val
   225                 230                 235                 240
   Ala Xaa Thr Pro Thr Val Ala Thr Arg Asp Gly Lys Leu Pro Thr Thr
                   245                 250                 255
   Gln Leu Arg Arg His Ile Asp Leu Leu Val Gly Xaa Ala Thr Leu Cys
               260                 265                 270
   Ser Ala Leu Tyr Val Gly Asp Leu Cys Gly Ser Val Phe Leu Val Gly
           275                 280                 285
   Gln Leu Phe Thr Phe Ser Pro Arg Xaa His Trp Thr Thr Gln Asp Cys
       290                 295                 300
   Asn Cys Ser Tyr Pro Gly His Ile Xaa Gly His Arg Met Ala Trp Asp
   305                 310                 315                 320
   Met Met Met Asn Trp Ser Pro Thr Xaa Ala Leu Val Val Ala Gln Leu
                   325                 330                 335
   Leu Arg Ile Pro Gln Ala Ile Xaa Asp Met Ile Ala Gly Ala His Trp
               340                 345                 350
   Gly Val Leu Ala Gly Ile Ala Tyr Phe Ser Met Val Gly Asn Trp Ala
           355                 360                 365
   Lys Val Leu Val Val Leu Leu Leu Phe Ala Xaa Val Asp Ala Glu Thr
   370 375 380
   His Val Thr Gly Gly Xaa Ala Xaa Arg Xaa Thr Xaa Gly Leu Val Gly
   385 390 395 400
   Leu Xaa Xaa Xaa Gly Xaa Lys Gln Asx Ile Xaa Leu Ile Asn Thr Asn
   405 410 415
   Gly Ser Trp His Ile Asn Xaa Thr Ala Leu Asn Cys Asn Xaa Ser Leu
   420 425 430
   Asn Thr Gly Trp Leu Ala Gly Leu Xaa Tyr Xaa Xaa Xaa Phe Asn Ser
   435 440 445
   Ser Gly Cys Pro Glu Arg Leu Ala Ser Cys Arg Arg Leu Thr Asp Phe
   450 455 460
   Ala Gln Gly Trp Gly Pro Ile Ser Xaa Xaa Asn Gly Ser Gly Xaa Asp
   465 470 475 480
   Glu Arg Pro Tyr Cys Trp His Tyr Pro Pro Arg Pro Cys Gly Ile Val
   485 490 495
   Pro Ala Lys Ser Val Cys Gly Pro Val Tyr Cys Phe Thr Pro Ser Pro
   500 505 510
   Val Val Val Gly Thr Thr Asp Arg Ser Gly Ala Pro Thr Tyr Xaa Trp
   515 520 525
   Gly Xaa Asn Asp Thr Asp Val Phe Val Leu Asn Asn Thr Arg Pro Pro
   530 535 540
   Leu Gly Asn Trp Phe Gly Cys Thr Trp Met Asn Ser Thr Gly Phe Thr
   545 550 555 560
   Lys Val Cys Gly Ala Pro Pro Cys Xaa Ile Gly Gly Val Gly Asn Asn
   565 570 575
   Thr Leu Xaa Cys Pro Thr Asp Cys Phe Arg Lys His Pro Xaa Ala Thr
   580 585 590
   Tyr Ser Arg Cys Gly Ser Gly Pro Trp Ile Thr Pro Arg Cys Xaa Val
   595 600 605
   Asp Tyr Pro Tyr Arg Leu Trp His Tyr Pro Cys Thr Ile Asn Tyr Thr
   610 615 620
   Xaa Phe Lys Val Arg Met Tyr Val Gly Gly Val Glu His Arg Leu Glu
   625 630 635 640
   Ala Ala Cys Asn Trp Thr Arg Gly Glu Arg Cys Asx Leu Glu Asp Arg
   645 650 655
   Asp Arg Ser Glu Leu Ser Pro Leu Leu Leu Ser Thr Thr Gln Trp Gln
   660 665 670
   Val Leu Pro Cys Ser Phe Thr Thr Leu Pro Ala Leu Ser Thr Gly Leu
   675 680 685
   Ile His Leu His Gln Asn Ile Val Asp Val Gln Tyr Leu Tyr Gly Val
   690 695 700
   Gly Ser Ser Ile Xaa Ser Trp Ala Ile Lys Trp Glu Tyr Val Val Leu
   705 710 715 720
   Leu Phe Leu Leu Leu Ala Asp Ala Arg Val Cys Ser Cys Leu Trp Met
   725 730 735
   Met Leu Leu Ile Ser Gln Ala Glu Ala
   740 745
<210> 39
   <211> 746
   <212> PRT
   <213> Hepatitis C virus
<400> 39
   Met Ser Thr Asn Pro Lys Pro Gln Arg Lys Thr Lys Arg Asn Thr Asn
   1               5                   10                  15
   Arg Arg Pro Gln Asp Val Lys Phe Pro Gly Gly Gly Gln Ile Val Gly
               20                  25                  30
   Gly Val Tyr Leu Leu Pro Arg Arg Gly Pro Arg Leu Gly Val Arg Ala
           35                  40                  45
   Thr Arg Lys Thr Ser Glu Arg Ser Gln Pro Arg Gly Arg Arg Gln Pro
       50                  55                  60
   Ile Pro Lys Ala Arg Arg Pro Glu Gly Arg Thr Trp Ala Gln Pro Gly
   65                  70                  75                  80
   Tyr Pro Trp Pro Leu Tyr Gly Asn Glu Gly Cys Gly Trp Ala Gly Trp
   85 90 95
   Leu Leu Ser Pro Arg Gly Ser Arg Pro Ser Trp Gly Pro Thr Asp Pro
   100 105 110
   Arg Arg Arg Ser Arg Asn Leu Gly Lys Val Ile Asp Thr Leu Thr Cys
   115 120 125
   Gly Phe Ala Asp Leu Met Gly Tyr Ile Pro Leu Val Gly Ala Pro Leu
   130 135 140
   Gly Gly Ala Ala Arg Ala Leu Ala His Gly Val Arg Val Leu Glu Asp
   145 150 155 160
   Gly Val Asn Tyr Ala Thr Gly Asn Leu Pro Gly Cys Ser Phe Ser Ile
   165 170 175
   Phe Leu Leu Ala Leu Leu Ser Cys Leu Thr Val Pro Ala Ser Ala Tyr
   180 185 190
   Gln Val Arg Asn Ser Thr Gly Leu Tyr His Val Thr Asn Asp Cys Pro
   195 200 205
   Asn Ser Ser Ile Val Tyr Glu Ala Asp Asp Ala Ile Leu His Ala Pro
   210 215 220
   Gly Cys Val Pro Cys Val Arg Glu Gly Asn Ile Ser Arg Cys Trp Val
   225 230 235 240
   Ala Met Thr Pro Thr Val Ala Thr Arg Asp Gly Lys Leu Pro Thr Thr
   245 250 255
   Gln Leu Arg Arg His Ile Asp Leu Leu Val Gly Gly Ala Thr Leu Cys
   260 265 270
   Ser Ala Leu Tyr Val Gly Asp Leu Cys Gly Ser Val Phe Leu Val Gly
   275 280 285
   Gln Leu Phe Thr Phe Ser Pro Arg Leu His Trp Thr Thr Gln Asp Cys
   290 295 300
   Asn Cys Ser Leu Tyr Pro Gly His Ile Ser Gly His Arg Met Ala Trp
   305 310 315 320
   Asp Met Met Met Asn Trp Ser Pro Thr Thr Ala Leu Val Val Ala Gln
   325 330 335
   Leu Leu Arg Ile Pro Gln Ala Ile Leu Asp Met Ile Ala Gly Ala His
   340 345 350
   Trp Gly Val Leu Ala Gly Ile Ala Tyr Phe Ser Met Val Gly Asn Trp
   355 360 365
   Ala Lys Val Leu Val Val Leu Leu Leu Phe Ala Ser Val Asp Ala Glu
   370 375 380
   Thr His Val Thr Gly Gly Asn Ala Ala Arg Ser Thr Ser Gly Leu Val
   385 390 395 400
   Gly Leu Phe Ser Ala Gly Pro Lys Gln Asp Ile Arg Leu Ile Asn Thr
   405 410 415
   Asn Gly Ser Trp His Ile Asn Arg Thr Ala Leu Asn Cys Asn Asp Ser
   420 425 430
   Leu Asn Thr Gly Trp Leu Ala Gly Leu Leu Tyr Arg Tyr Asn Phe Asn
   435 440 445
   Ser Ser Gly Cys Pro Glu Arg Leu Ala Ser Cys Arg Arg Leu Thr Asp
   450 455 460
   Phe Ala Gln Gly Trp Gly Pro Ile Ser His Val Asn Gly Ser Gly Pro
   465 470 475 480
   Asp Glu Arg Pro Tyr Cys Trp His Tyr Pro Pro Arg Pro Cys Gly Ile
   485 490 495
   Val Pro Ala Lys Ser Val Cys Gly Pro Val Tyr Cys Phe Thr Pro Ser
   500 505 510
   Pro Val Val Val Gly Thr Thr Asp Arg Ser Gly Ala Pro Thr Tyr Asn
   515 520 525
   Trp Gly Glu Asn Asp Thr Asp Val Phe Val Leu Asn Asn Thr Arg Pro
   530 535 540
   Pro Leu Gly Asn Trp Phe Gly Cys Thr Trp Met Asn Ser Thr Gly Phe
   545 550 555 560
   Thr Lys Val Cys Gly Ala Pro Pro Cys Ala Ile Gly Gly Val Gly Asn
   565 570 575
   Asn Thr Leu Arg Cys Pro Thr Asp Cys Phe Arg Lys His Pro Asp Ala
   580 585 590
   Thr Tyr Ser Arg Cys Gly Ser Gly Pro Trp Ile Thr Pro Arg Cys Leu
   595 600 605
   Val Asp Tyr Pro Tyr Arg Leu Trp His Tyr Pro Cys Thr Ile Asn Tyr
   610 615 620
   Thr Val Phe Lys Val Arg Met Tyr Val Gly Gly Val Glu His Arg Leu
   625 630 635 640
   Glu Ala Ala Cys Asn Trp Thr Arg Gly Glu Arg Cys Asn Leu Glu Asp
   645 650 655
   Arg Asp Arg Ser Glu Leu Ser Pro Leu Leu Leu Ser Thr Thr Gln Trp
   660 665 670
   Gln Val Leu Pro Cys Ser Phe Thr Thr Leu Pro Ala Leu Ser Thr Gly
   675 680 685
   Leu Ile His Leu His Gln Asn Ile Val Asp Val Gln Tyr Leu Tyr Gly
   690 695 700
   Val Gly Ser Ser Ile Val Ser Trp Ala Ile Lys Trp Glu Tyr Val Val
   705 710 715 720
   Leu Leu Phe Leu Leu Leu Ala Asp Ala Arg Val Cys Ser Cys Leu Trp
   725 730 735
   Met Met Leu Leu Ile Ser Gln Ala Glu Ala
   740 745
<210> 40
   <211> 750
   <212> PRT
   <213> Hepatitis C virus
<400> 40
   Met Ser Thr Asn Pro Lys Pro Gln Arg Leu Thr Lys Arg Asn Thr Val
   1               5                   10                  15
   Arg Arg Pro Gln Asn Val Lys Phe Pro Gly Gly Gly Gln Ile Val Gly
               20                  25                  30
   Gly Val Tyr Leu Leu Pro Arg Arg Gly Pro Arg Leu Gly Val Arg Gly
           35                  40                  45
   Thr Arg Lys Ser Ser Glu Arg Ser Gln Pro Arg Gly Arg Arg Gln Arg
       50                  55                  60
   Ile Pro Lys Ala Ala Ser Ser Gln Gly Lys Ala Trp Gly Lys Pro Gly
   65                  70                  75                  80
   Tyr Pro Trp Pro Leu Tyr Gly Asn Glu Gly Cys Gly Trp Ala Gly Trp
                   85                  90                  95
   Leu Leu Ser Pro Arg Gly Ser Arg Pro Thr Trp Gly Pro Thr Asp Pro
               100                 105                 110
   Arg His Arg Ser Arg Asn Leu Gly Lys Val Ile Asp Thr Met Thr Cys
           115                 120                 125
   Gly Phe Ala Asp Leu Met Gly Tyr Ile Pro Val Leu Gly Ala Pro Leu
       130                 135                 140
   Gly Gly Val Ala Arg Ala Leu Ala His Gly Val Arg Val Leu Glu Asp
   145                 150                 155                 160
   Gly Val Asn Tyr Ala Thr Gly Asn Leu Pro Gly Cys Ser Phe Ser Ile
                   165                 170                 175
   Phe Leu Leu Ala Leu Leu Ser Cys Leu Thr Val Pro Ala Ser Ala Tyr
               180                 185                 190
   Glu Val Arg Asn Ser Ser Gly Val Tyr His Leu Thr Asn Asp Cys Pro
           195                 200                 205
   Asn Ala Ser Ile Val Tyr Glu Thr Asp Asn Ala Ile Leu His Glu Pro
       210                 215                 220
   Gly Cys Val Pro Cys Val Arg Glu Gly Asn Thr Ser Arg Cys Trp Glu
   225                 230                 235                 240
   Pro Val Ala Pro Thr Leu Ala Val Arg Tyr Arg Gly Ala Leu Thr Asp
                   245                 250                 255
   Asp Leu Arg Thr His Ile Asp Leu Val Val Ala Ser Ala Thr Leu Cys
               260                 265                 270
   Ser Ala Leu Tyr Val Gly Asp Ile Cys Gly Ala Ile Phe Ile Ala Ser
           275                 280                 285
   Gln Ala Val Leu Trp Lys Pro Gly Gly Gly Arg Ile Val Gln Asp Cys
       290                 295                 300
   Asn Cys Ser Ile Tyr Pro Gly His Val Thr Gly His Arg Met Ala Trp
   305                 310                 315                 320
   Asp Met Met Gln Asn Trp Ala Pro Ala Leu Ser Met Val Ala Ala Tyr
                   325                 330                 335
   Ala Val Arg Val Pro Gly Val Ile Ile Thr Thr Val Ala Gly Gly His
               340                 345                 350
   Trp Gly Val Leu Phe Gly Leu Ala Tyr Phe Gly Met Ala Gly Asn Trp
           355                 360                 365
   Ala Lys Val Ile Leu Ile Met Leu Leu Met Ser Gly Val Asp Ala Glu
       370                 375                 380
   Thr Met Ala Val Gly Ala Arg Ala Ala His Thr Thr Gly Ala Leu Val
   385                 390                 395                 400
   Ser Leu Leu Asn Pro Gly Pro Ser Gln Arg Leu Gln Leu Ile Asn Thr
                   405                 410                 415
   Asn Gly Ser Trp His Ile Asn Arg Thr Ala Leu Asn Cys Asn Asp Ser
               420                 425                 430
   Leu Gln Thr Gly Phe Ile Ala Ala Leu Phe Tyr Thr His Arg Phe Asn
           435                 440                 445
   Ser Ser Gly Cys Pro Glu Arg Met Ala Ser Cys Lys Pro Leu Ser Asp
       450                 455                 460
   Phe Asp Gln Gly Trp Gly Pro Leu Trp Tyr Asn Ser Thr Glu Arg Pro
   465                 470                 475                 480
   Ser Asp Gln Arg Pro Tyr Cys Trp His Tyr Ala Pro Ser Pro Cys Gly
                   485                 490                 495
   Ile Val Pro Ala Lys Asp Val Cys Gly Pro Val Tyr Cys Phe Thr Pro
               500                 505                 510
   Ser Pro Val Val Val Gly Thr Thr Asp Arg Arg Gly Val Pro Thr Tyr
           515                 520                 525
   Thr Trp Gly Glu Asn Glu Ser Asp Val Phe Leu Leu Asn Ser Thr Arg
       530                 535                 540
   Pro Pro Gln Gly Ser Trp Phe Gly Cys Ser Trp Met Asn Thr Thr Gly
   545                 550                 555                 560
   Phe Thr Lys Thr Cys Gly Gly Pro Pro Cys Lys Ile Arg Pro Gln Gly
   565 570 575
   Ala Gln Ser Asn Thr Ser Leu Thr Cys Pro Thr Asp Cys Phe Arg Lys
   580 585 590
   His Pro Arg Ala Thr Tyr Ser Ala Cys Gly Ser Gly Pro Trp Leu Thr
   595 600 605
   Pro Arg Cys Met Val His Tyr Pro Tyr Arg Leu Trp His Tyr Pro Cys
   610 615 620
   Thr Val Asn Phe Thr Ile His Lys Val Arg Leu Tyr Ile Gly Gly Val
   625 630 635 640
   Glu His Arg Leu Asp Ala Ala Cys Asn Trp Thr Arg Gly Glu Arg Cys
   645 650 655
   Asp Leu Glu Asp Arg Asp Arg Val Asp Met Ser Pro Leu Leu His Ser
   660 665 670
   Thr Thr Glu Leu Ala Ile Leu Pro Cys Ser Phe Val Pro Leu Pro Ala
   675 680 685
   Leu Ser Thr Gly Leu Ile His Leu His Gln Asn Ile Val Asp Ala Gln
   690 695 700
   Tyr Leu Tyr Gly Leu Ser Pro Ala Ile Ile Ser Trp Ala Ile Arg Trp
   705 710 715 720
   Glu Trp Val Val Leu Val Phe Leu Leu Leu Ala Asp Ala Arg Ile Cys
   725 730 735
   Ala Cys Leu Trp Met Met Met Leu Met Ala Gln Ala Glu Ala
   740 745 750
<210> 41
   <211> 750
   <212> PRT
   <213> Hepatitis C virus
<400> 41
   Met Ser Thr Asn Pro Lys Pro Gln Arg Lys Thr Lys Arg Asn Thr Asn
   1               5                   10                  15
   Arg Arg Pro Glu Asp Val Lys Phe Pro Gly Gly Gly Gln Ile Val Gly
               20                  25                  30
   Gly Val Tyr Leu Leu Pro Arg Arg Gly Pro Arg Leu Gly Val Arg Thr
   35 40 45
   Thr Arg Lys Thr Ser Glu Arg Ser Gln Pro Arg Gly Arg Arg Gln Pro
   50 55 60
   Ile Pro Lys Asp Arg Arg Ser Thr Gly Lys Ala Trp Gly Lys Pro Gly
   65 70 75 80
   Arg Pro Trp Pro Leu Tyr Gly Asn Glu Gly Leu Gly Trp Ala Gly Trp
   85 90 95
   Leu Leu Ser Pro Arg Gly Ser Arg Pro Ser Trp Gly Pro Thr Asp Pro
   100 105 110
   Arg His Arg Ser Arg Asn Val Gly Lys Val Ile Asp Thr Leu Thr Cys
   115 120 125
   Gly Phe Ala Asp Leu Met Gly Tyr Ile Pro Val Val Gly Ala Pro Leu
   130 135 140
   Ser Gly Ala Ala Arg Ala Val Ala His Gly Val Arg Val Leu Glu Asp
   145 150 155 160
   Gly Val Asn Tyr Ala Thr Gly Asn Leu Pro Gly Phe Pro Phe Ser Ile
   165 170 175
   Phe Leu Leu Ala Leu Leu Ser Cys Ile Thr Val Pro Val Ser Ala Ala
   180 185 190
   Gln Val Lys Asn Thr Ser Ser Ser Tyr Met Val Thr Asn Asp Cys Ser
   195 200 205
   Asn Asp Ser Ile Thr Trp Gln Leu Glu Ala Ala Val Leu His Val Pro
   210 215 220
   Gly Cys Val Pro Cys Glu Arg Val Gly Asn Thr Ser Arg Cys Trp Val
   225 230 235 240
   Pro Val Ser Pro Asn Met Ala Val Arg Gln Pro Gly Ala Leu Thr Gln
   245 250 255
   Gly Leu Arg Thr His Ile Asp Met Val Val Met Ser Ala Thr Phe Cys
   260 265 270
   Ser Ala Leu Tyr Val Gly Asp Leu Cys Gly Gly Val Met Leu Ala Ala
   275 280 285
   Gln Val Phe Ile Val Ser Pro Gln Tyr His Trp Phe Val Gln Glu Cys
   290 295 300
   Asn Cys Ser Ile Tyr Pro Gly Thr Ile Thr Gly His Arg Met Ala Trp
   305 310 315 320
   Asp Met Met Met Asn Trp Ser Pro Thr Ala Thr Met Ile Leu Ala Tyr
   325 330 335
   Val Met Arg Val Pro Glu Val Ile Ile Asp Ile Val Ser Gly Ala His
   340 345 350
   Trp Gly Val Met Phe Gly Leu Ala Tyr Phe Ser Met Gln Gly Ala Trp
   355 360 365
   Ala Lys Val Ile Val Ile Leu Leu Leu Ala Ala Gly Val Asp Ala Gly
   370 375 380
   Thr Thr Thr Val Gly Gly Ala Val Ala Arg Ser Thr Asn Val Ile Ala
   385 390 395 400
   Gly Val Phe Ser His Gly Pro Gln Gln Asn Ile Gln Leu Ile Asn Thr
   405 410 415
   Asn Gly Ser Trp His Ile Asn Arg Thr Ala Leu Asn Cys Asn Asp Ser
   420 425 430
   Leu Asn Thr Gly Phe Leu Ala Ala Leu Phe Tyr Thr Asn Arg Phe Asn
   435 440 445
   Ser Ser Gly Cys Pro Gly Arg Leu Ser Ala Cys Arg Asn Ile Glu Ala
   450 455 460
   Phe Arg Ile Gly Trp Gly Thr Leu Gln Tyr Glu Asp Asn Val Thr Asn
   465 470 475 480
   Pro Glu Asp Met Arg Pro Tyr Cys Trp His Tyr Pro Pro Lys Pro Cys
   485 490 495
   Gly Val Val Pro Ala Arg Ser Val Cys Gly Pro Val Tyr Cys Phe Thr
   500 505 510
   Pro Ser Pro Val Val Val Gly Thr Thr Asp Arg Arg Gly Val Pro Thr
   515 520 525
   Tyr Thr Trp Gly Glu Asn Glu Thr Asp Val Phe Leu Leu Asn Ser Thr
   530 535 540
   Arg Pro Pro Gln Gly Ser Trp Phe Gly Cys Thr Trp Met Asn Ser Thr
   545                 550                 555                 560
   Gly Phe Thr Lys Thr Cys Gly Ala Pro Pro Cys Arg Thr Arg Ala Asp
                   565                 570                 575
   Phe Asn Ala Ser Thr Asp Leu Leu Cys Pro Thr Asp Cys Phe Arg Lys
               580                 585                 590
   His Pro Asp Ala Thr Tyr Ile Lys Cys Gly Ser Gly Pro Trp Leu Thr
           595                 600                 605
   Pro Lys Cys Leu Val His Tyr Pro Tyr Arg Leu Trp His Tyr Pro Cys
       610                 615                 620
   Thr Val Asn Phe Thr Ile Phe Lys Ile Arg Met Tyr Val Gly Gly Val
   625                 630                 635                 640
   Glu His Arg Leu Thr Ala Ala Cys Asn Phe Thr Arg Gly Asp Arg Cys
                   645                 650                 655
   Asp Leu Glu Asp Arg Asp Arg Ser Gln Leu Ser Pro Leu Leu His Ser
               660                 665                 670
   Thr Thr Glu Trp Ala Ile Leu Pro Cys Thr Tyr Ser Asp Leu Pro Ala
           675                 680                 685
   Leu Ser Thr Gly Leu Leu His Leu His Gln Asn Ile Val Asp Val Gln
       690                 695                 700
   Tyr Met Tyr Gly Leu Ser Pro Ala Ile Thr Lys Tyr Val Val Arg Trp
   705                 710                 715                 720
   Glu Trp Val Val Leu Leu Phe Leu Leu Leu Ala Asp Ala Arg Val Cys
                   725                 730                 735
   Ala Cys Leu Trp Met Leu Ile Leu Leu Gly Gln Ala Glu Ala
               740                 745                 750
<210> 42
   <211> 749
   <212> PRT
   <213> Hepatitis C virus
<400> 42
   Met Ser Thr Asn Pro Lys Pro Gln Arg Lys Thr Lys Arg Asn Thr Asn
   1               5                   10                  15
   Arg Arg Pro Gln Asp Val Lys Phe Pro Gly Gly Gly Gln Ile Val Gly
               20                  25                  30
   Gly Val Tyr Leu Leu Pro Arg Arg Gly Pro Arg Leu Gly Val Arg Ala
           35                  40                  45
   Thr Arg Lys Thr Ser Glu Arg Ser Gln Pro Arg Gly Arg Arg Gln Pro
       50                  55                  60
   Ile Pro Lys Asp Arg Arg Ser Thr Gly Lys Ser Trp Gly Lys Pro Gly
   65                  70                  75                  80
   Tyr Pro Trp Pro Leu Tyr Gly Asn Glu Gly Leu Gly Trp Ala Gly Trp
                   85                  90                  95
   Leu Leu Ser Pro Arg Gly Ser Arg Pro Ser Trp Gly Pro Thr Asp Pro
               100                 105                 110
   Arg His Arg Ser Arg Asn Val Gly Lys Val Ile Asp Thr Leu Thr Cys
           115                 120                 125
   Gly Phe Ala Asp Leu Met Gly Tyr Ile Pro Val Val Gly Ala Pro Leu
       130                 135                 140
   Gly Gly Val Ala Arg Ala Leu Ala His Gly Val Arg Val Leu Glu Asp
   145                 150                 155                 160
   Gly Val Asn Tyr Ala Thr Gly Asn Leu Pro Gly Cys Ser Phe Ser Ile
                   165                 170                 175
   Phe Leu Leu Ala Leu Leu Ser Cys Ile Thr Thr Pro Val Ser Ala Ala
               180                 185                 190
   Gln Val Lys Asn Thr Ser Asp Ile Tyr Met Val Thr Asn Asp Cys Ser
           195                 200                 205
   Asn Asp Ser Ile Thr Trp Gln Leu Gln Ala Ala Ala Leu His Val Pro
       210                 215                 220
   Gly Cys Val Pro Cys Glu Arg Val Gly Asn Thr Ser Arg Cys Trp Ile
   225                 230                 235                 240
   Pro Val Ser Pro Asn Val Ala Val Arg Gln Pro Gly Ala Leu Thr Gln
                   245                 250                 255
   Gly Leu Arg Thr His Ile Asp Met Val Val Met Ser Ala Thr Leu Cys
               260                 265                 270
   Ser Ala Leu Tyr Val Gly Asp Leu Cys Gly Gly Val Met Leu Ala Ala
               275                 280                 285
   Gln Met Phe Ile Ile Ser Pro Gln His His Trp Phe Val Gln Glu Cys
       290                 295                 300
   Asn Cys Ser Ile Tyr Pro Gly Thr Ile Thr Gly His Arg Met Ala Trp
   305                 310                 315                 320
   Asp Met Met Met Asn Trp Ser Pro Thr Thr Thr Met Ile Leu Ala Tyr
                   325                 330                 335
   Val Met Arg Val Pro Glu Val Ile Ile Asp Ile Ile Gly Gly Ala His
               340                 345                 350
   Trp Gly Val Met Phe Gly Leu Ala Tyr Phe Ser Met Gln Gly Ala Trp
           355                 360                 365
   Ala Lys Val Val Val Ile Leu Leu Leu Ala Ala Gly Val Asp Ala His
       370                 375                 380
   Thr Arg Thr Gly Ser Ser Val Gly Tyr Ala Thr Ser Gly Ile Val Gly
   385                 390                 395                 400
   Leu Phe Thr Ser Gly Pro Lys Gln Asn Ile Gln Leu Ile Asn Thr Asn
                   405                 410                 415
   Gly Ser Trp His Ile Asn Arg Thr Ala Leu Asn Cys Asn Asp Ser Leu
               420                 425                 430
   Asn Thr Gly Phe Ile Val Ser Leu Phe Tyr Ala Arg Asn Phe Asn Ser
           435                 440                 445
   Thr Gly Cys Pro Glu Arg Leu Ser Ala Cys Arg Gly Ile Glu Gly Phe
       450                 455                 460
   Arg Ile Gly Trp Gly Thr Leu Gln Tyr Glu Asp Asn Val Thr Asn Pro
   465                 470                 475                 480
   Glu Asp Met Arg Pro Tyr Cys Trp His Tyr Pro Pro Lys Gln Cys Gly
                   485                 490                 495
   Ile Val Pro Ala Gly Ser Val Cys Gly Pro Val Tyr Cys Phe Thr Pro
               500                 505                 510
   Ser Pro Val Val Val Gly Thr Thr Asp Arg Leu Gly Val Pro Thr Tyr
   515 520 525
   Thr Trp Gly Glu Asn Glu Thr Asp Val Phe Leu Leu Asn Ser Thr Arg
   530 535 540
   Pro Pro Val Gly Ser Trp Phe Gly Cys Thr Trp Met Asn Ser Thr Gly
   545 550 555 560
   Phe Thr Lys Thr Cys Gly Ala Pro Pro Cys Arg Ile Arg Ala Asp Phe
   565 570 575
   Asn Ala Ser Thr Asp Leu Leu Cys Pro Thr Asp Cys Phe Arg Lys His
   580 585 590
   Pro Glu Ala Thr Tyr Ile Lys Cys Gly Ser Gly Pro Trp Leu Thr Pro
   595 600 605
   Arg Cys Leu Val Asp Tyr Pro Tyr Arg Leu Trp His Tyr Pro Cys Thr
   610 615 620
   Val Asn Tyr Ser Ile Phe Lys Ile Arg Met Tyr Val Gly Gly Val Glu
   625 630 635 640
   His Arg Leu Thr Ala Ala Cys Asn Phe Thr Arg Gly Asp Arg Cys Asn
   645 650 655
   Leu Glu Asp Arg Asp Arg Ser Gln Leu Thr Pro Leu Leu His Ser Thr
   660 665 670
   Thr Glu Trp Ala Ile Leu Pro Cys Thr Tyr Ser Asp Leu Pro Ala Leu
   675 680 685
   Ser Thr Gly Leu Leu His Leu His Gln Asn Ile Val Asp Val Gln Tyr
   690 695 700
   Met Tyr Gly Leu Ser Pro Ala Leu Thr Lys Tyr Val Val Arg Trp Glu
   705 710 715 720
   Trp Val Val Leu Leu Phe Leu Leu Leu Ala Asp Ala Arg Val Cys Ala
   725 730 735
   Cys Val Trp Met Leu Ile Leu Leu Gly Gln Ala Glu Ala
   740 745
<210> 43
   <211> 749
   <212> PRT
   <213> Hepatitis C virus
<220>
   <221> misc_feature
   <222> (386)..(386)
   <223> Xaa can be any naturally occurring amino acid
<220>
   <221> misc_feature
   <222> (392)..(394)
   <223> Xaa can be any naturally occurring amino acid
<220>
   <221> misc_feature
   <222> (398)..(399)
   <223> Xaa can be any naturally occurring amino acid
<220>
   <221> misc_feature
   <222> (444)..(444)
   <223> Xaa can be any naturally occurring amino acid
<220>
   <221> misc_feature
   <222> (496)..(496)
   <223> Xaa can be any naturally occurring amino acid
<220>
   <221> misc_feature
   <222> (525)..(525)
   <223> Xaa can be any naturally occurring amino acid
<220>
   <221> misc_feature
   <222> (538)..(538)
   <223> Xaa can be any naturally occurring amino acid
<220>
   <221> misc_feature
   <222> (712)..(712)
   <223> Xaa can be any naturally occurring amino acid
<220>
   <221> misc_feature
   <222> (722)..(722)
   <223> Xaa can be any naturally occurring amino acid
<400> 43
   Met Ser Thr Asn Pro Lys Pro Gln Arg Lys Thr Lys Arg Asn Thr Asn
   1               5                   10                  15
   Arg Arg Pro Gln Asp Val Lys Phe Pro Gly Gly Gly Gln Ile Val Gly
               20                  25                  30
   Gly Val Tyr Leu Leu Pro Arg Arg Gly Pro Arg Leu Gly Val Arg Ala
           35                  40                  45
   Thr Arg Lys Thr Ser Glu Arg Ser Gln Pro Arg Gly Arg Arg Gln Pro
       50                  55                  60
   Ile Pro Lys His Arg Arg Ser Thr Gly Lys Ser Trp Gly Thr Pro Gly
   65                  70                  75                  80
   Tyr Pro Trp Pro Leu Tyr Gly Asn Glu Gly Leu Gly Trp Ala Gly Trp
                   85                  90                  95
   Leu Leu Ser Pro Arg Gly Ser Arg Pro Thr Trp Gly Pro Thr Asp Pro
               100                 105                 110
   Arg His Arg Ser Arg Asn Val Gly Lys Val Ile Asp Thr Leu Thr Cys
           115                 120                 125
   Gly Phe Ala Asp Leu Met Gly Tyr Ile Pro Val Val Gly Ala Pro Leu
       130                 135                 140
   Gly Gly Val Ala Arg Ala Leu Ala His Gly Val Arg Val Leu Glu Asp
   145                 150                 155                 160
   Gly Val Asn Tyr Ala Thr Gly Asn Leu Pro Gly Cys Ser Phe Ser Ile
                   165                 170                 175
   Phe Leu Leu Ala Leu Leu Ser Cys Ile Thr Ile Pro Val Ser Ala Ala
               180                 185                 190
   Gln Val Lys Asn Thr Ser Ser Gly Tyr Met Val Thr Asn Asp Cys Ser
           195                 200                 205
   Asn Asp Ser Ile Thr Trp Gln Leu Glu Ala Ala Val Leu His Val Pro
       210                 215                 220
   Gly Cys Val Pro Cys Glu Lys Val Gly Asn Thr Ser Arg Cys Trp Ile
   225                 230                 235                 240
   Pro Val Ser Pro Asn Val Ala Val Gln Arg Pro Gly Ala Leu Thr Gln
                   245                 250                 255
   Gly Leu Arg Thr His Ile Asp Met Val Val Met Ser Ala Thr Leu Cys
               260                 265                 270
   Ser Ala Leu Tyr Val Gly Asp Phe Cys Gly Gly Val Met Leu Ala Ala
           275                 280                 285
   Gln Met Phe Ile Val Ser Pro Gln His His Trp Phe Val Gln Glu Cys
       290                 295                 300
   Asn Cys Ser Ile Tyr Pro Gly Thr Ile Ser Gly His Arg Met Ala Trp
   305                 310                 315                 320
   Asp Met Met Met Asn Trp Ser Pro Thr Ala Thr Met Ile Met Ala Tyr
                   325                 330                 335
   Val Met Arg Val Pro Glu Val Ile Ile Asp Ile Ile Ala Gly Ala His
               340                 345                 350
   Trp Gly Val Met Phe Gly Leu Ala Tyr Phe Ser Met Gln Gly Ala Trp
           355                 360                 365
   Ala Lys Val Val Val Ile Leu Leu Leu Thr Ala Gly Val Asp Ala His
       370                 375                 380
   Thr Xaa Ile Gly Ser Ala Val Xaa Xaa Xaa Thr Ser Gly Xaa Xaa Gly
   385                 390                 395                 400
   Leu Phe Tyr Arg Gly Ala Gln Gln Asn Ile Gln Leu Ile Asn Thr Asn
                   405                 410                 415
   Gly Ser Trp His Ile Asn Arg Thr Ala Leu Asn Cys Asn Asp Ser Leu
               420                 425                 430
   Asn Thr Gly Phe Leu Ala Ser Leu Phe Tyr Ala Xaa Arg Phe Asn Ser
           435                 440                 445
   Ser Gly Cys Pro Glu Arg Leu Ser Val Cys Arg Asn Ile Glu Ala Phe
       450                 455                 460
   Arg Ile Gly Trp Gly Thr Leu Gln Tyr Glu Asp Asn Val Thr Asn Pro
   465                 470                 475                 480
   Glu Asp Met Arg Pro Tyr Cys Trp His Tyr Pro Pro Lys Pro Cys Xaa
                   485                 490                 495
   Ile Val Pro Ala Gly Ser Val Cys Gly Pro Val Tyr Cys Phe Thr Pro
               500                 505                 510
   Ser Pro Val Val Val Gly Thr Thr Asp Arg Leu Gly Xaa Pro Thr Tyr
           515                 520                 525
   Thr Trp Gly Glu Asn Glu Thr Asp Val Xaa Leu Leu Asn Ser Thr Arg
       530                 535                 540
   Pro Pro Gln Gly Ser Trp Phe Gly Cys Thr Trp Met Asn Ser Thr Gly
   545                 550                 555                 560
   Phe Thr Lys Thr Cys Gly Ala Pro Pro Cys Arg Thr Arg Ala Asp Phe
                   565                 570                 575
   Asn Ala Ser Thr Asp Leu Leu Cys Pro Thr Asp Cys Phe Arg Lys His
               580                 585                 590
   Pro Glu Ala Thr Tyr Ile Lys Cys Gly Ser Gly Pro Trp Leu Thr Pro
           595                 600                 605
   Arg Cys Met Val Asp Tyr Pro Tyr Arg Leu Trp His Tyr Pro Cys Thr
       610                 615                 620
   Val Asn Tyr Ser Ile Phe Lys Ile Arg Met Tyr Val Gly Gly Val Glu
   625                 630                 635                 640
   His Arg Leu Met Ala Ala Cys Asn Phe Thr Arg Gly Asp Arg Cys Asp
                   645                 650                 655
   Leu Glu Asp Arg Asp Arg Ser Gln Leu Ser Pro Leu Leu His Ser Thr
               660                 665                 670
   Thr Glu Trp Ala Ile Leu Pro Cys Ser Tyr Ser Asp Leu Pro Ala Leu
           675                 680                 685
   Ser Thr Gly Leu Leu His Leu His Gln Asn Ile Val Asp Val Gln Tyr
       690                 695                 700
   Met Tyr Gly Leu Ser Pro Ala Xaa Thr Lys Tyr Val Val Arg Trp Glu
   705                 710                 715                 720
   Trp Xaa Val Leu Leu Phe Leu Leu Leu Ala Asp Ala Arg Val Cys Ala
                   725                 730                 735
   Cys Leu Trp Met Leu Ile Leu Leu Gly Gln Ala Glu Ala
               740                 745
<210> 44
   <211> 750
   <212> PRT
   <213> Hepatitis C virus
<400> 44
   Met Ser Thr Asn Pro Lys Pro Gln Arg Lys Thr Lys Arg Asn Thr Ser
   1               5                   10                  15
   Arg Arg Pro Gln Asp Val Lys Phe Pro Gly Gly Gly Gln Ile Val Gly
               20                  25                  30
   Gly Val Tyr Leu Leu Pro Arg Arg Gly Pro Arg Leu Gly Val Arg Ala
   35 40 45
   Thr Arg Lys Thr Ser Glu Arg Ser Gln Pro Arg Gly Arg Arg Gln Pro
   50 55 60
   Ile Pro Lys Asp Arg Arg Ser Thr Gly Lys Ser Trp Gly Lys Pro Gly
   65 70 75 80
   Tyr Pro Trp Pro Leu Tyr Gly Asn Glu Gly Leu Gly Trp Ala Gly Trp
   85 90 95
   Leu Leu Ser Pro Arg Gly Ser Arg Pro Ser Trp Gly Pro Thr Asp Pro
   100 105 110
   Arg His Arg Ser Arg Asn Val Gly Lys Val Ile Asp Thr Leu Thr Cys
   115 120 125
   Gly Phe Ala Asp Leu Met Gly Tyr Ile Pro Val Val Gly Ala Pro Leu
   130 135 140
   Gly Gly Val Ala Arg Ala Leu Ala His Gly Val Arg Val Leu Glu Asp
   145 150 155 160
   Gly Val Asn Tyr Ala Thr Gly Asn Leu Pro Gly Cys Ser Phe Ser Ile
   165 170 175
   Phe Leu Leu Ala Leu Leu Ser Cys Ile Thr Val Pro Val Ser Ala Val
   180 185 190
   Gln Val Lys Asn Ile Ser Asp Ser Tyr Met Val Thr Asn Asp Cys Ser
   195 200 205
   Asn Asp Ser Ile Thr Trp Gln Leu Gln Ala Ala Val Leu His Val Pro
   210 215 220
   Gly Cys Val Pro Cys Glu Lys Met Gly Asn Ile Ser Arg Cys Trp Ile
   225 230 235 240
   Pro Val Ser Pro Asn Val Ala Val Gln Arg Pro Gly Ala Leu Thr Gln
   245 250 255
   Gly Leu Arg Ala His Ile Asp Met Val Val Met Ser Ala Thr Leu Cys
   260 265 270
   Ser Ala Leu Tyr Val Gly Asp Leu Cys Gly Gly Val Met Leu Ala Ala
   275 280 285
   Gln Met Phe Ile Val Ser Pro Gln His His His Phe Val Gln Glu Cys
       290                 295                 300
   Asn Cys Ser Ile Tyr Pro Gly Ala Ile Thr Gly His Arg Met Ala Trp
   305                 310                 315                 320
   Asp Met Met Met Asn Trp Ser Pro Thr Ala Thr Met Ile Leu Ala Tyr
                   325                 330                 335
   Ala Met Arg Val Pro Glu Val Ile Ile Asp Ile Ile Ser Gly Ala His
               340                 345                 350
   Trp Gly Val Met Phe Gly Leu Ala Tyr Phe Ser Met Gln Gly Ala Trp
           355                 360                 365
   Ala Lys Val Val Val Ile Leu Leu Leu Thr Ala Gly Val Asp Ala His
       370                 375                 380
   Thr Arg Ser Ile Ala Gly Ser Val Ala His Ala Thr Ser Gly Leu Ala
   385                 390                 395                 400
   Gly Leu Phe Thr Ser Gly Ala Lys Gln Asn Ile Gln Leu Ile Asn Thr
                   405                 410                 415
   Asn Gly Ser Trp His Ile Asn Arg Thr Ala Leu Asn Cys Asn Asp Ser
               420                 425                 430
   Leu Asn Thr Gly Phe Ile Ala Ser Leu Phe Tyr Thr Tyr Arg Phe Asn
           435                 440                 445
   Ser Ser Gly Cys Pro Glu Arg Leu Ser Ala Cys Arg Gly Ile Gln Ala
       450                 455                 460
   Phe Arg Ile Gly Trp Gly Thr Leu Arg Tyr Glu Asp Asn Val Thr Asn
   465                 470                 475                 480
   Pro Glu Asp Met Arg Pro Tyr Cys Trp His Tyr Pro Pro Lys Gln Cys
                   485                 490                 495
   Gly Ile Val Ser Ala Arg Ser Val Cys Gly Pro Val Tyr Cys Phe Thr
               500                 505                 510
   Pro Ser Pro Val Val Val Gly Thr Thr Asp Arg Leu Gly Val Pro Thr
           515                 520                 525
   Tyr Thr Trp Gly Glu Asn Glu Thr Asp Val Phe Ile Leu Asn Ser Thr
       530                 535                 540
   Arg Pro Pro Gly Gly Ser Trp Phe Gly Cys Thr Trp Met Asn Ser Thr
   545 550 555 560
   Gly Phe Thr Lys Thr Cys Gly Ala Pro Pro Cys Arg Ile Arg Ala Asp
   565 570 575
   Phe Asn Ala Ser Met Asp Leu Leu Cys Pro Thr Asp Cys Phe Arg Lys
   580 585 590
   His Pro Asp Ala Thr Tyr Ile Lys Cys Gly Ser Gly Pro Trp Leu Thr
   595 600 605
   Pro Arg Cys Leu Val Asp Tyr Pro Tyr Arg Leu Trp His Tyr Pro Cys
   610 615 620
   Thr Ile Asn Tyr Thr Ile Phe Lys Ile Arg Met Tyr Val Gly Gly Val
   625 630 635 640
   Glu His Arg Leu Thr Ala Ala Cys Asn Phe Thr Arg Gly Asp Pro Cys
   645 650 655
   Asn Leu Glu Asp Arg Asp Arg Ser Gln Leu Ser Pro Leu Leu His Ser
   660 665 670
   Thr Thr Glu Trp Ala Ile Leu Pro Cys Ser Tyr Ser Asp Leu Pro Ala
   675 680 685
   Leu Ser Thr Gly Leu Leu His Leu His Gln Asn Ile Val Asp Val Gln
   690 695 700
   Tyr Met Tyr Gly Leu Ser Pro Ala Leu Thr Lys Tyr Ile Val Arg Trp
   705 710 715 720
   Glu Trp Val Val Leu Leu Phe Leu Leu Leu Ala Asp Ala Arg Val Cys
   725 730 735
   Ala Cys Leu Trp Met Leu Ile Leu Leu Gly Gln Ala Glu Ala
   740 745 750
<210> 45
   <211> 750
   <212> PRT
   <213> Hepatitis C virus
<220>
   <221> misc_feature
   <222> (458)..(458)
   <223> Xaa can be any naturally occurring amino acid
<400> 45
   Met Ser Thr Asn Pro Lys Pro Gln Arg Lys Thr Lys Arg Asn Thr Asn
   1               5                   10                  15
   Arg Arg Pro Gln Asp Val Lys Phe Pro Gly Gly Gly Gln Ile Val Gly
               20                  25                  30
   Gly Val Tyr Leu Leu Pro Arg Arg Gly Pro Arg Leu Gly Val Arg Ala
           35                  40                  45
   Thr Arg Lys Thr Ser Glu Arg Ser Gln Pro Arg Gly Arg Arg Gln Pro
       50                  55                  60
   Ile Pro Lys Asp Arg Arg Ser Thr Gly Lys Ser Trp Gly Lys Pro Gly
   65                  70                  75                  80
   Tyr Pro Trp Pro Leu Tyr Gly Asn Glu Gly Leu Gly Trp Ala Gly Trp
                   85                  90                  95
   Leu Leu Ser Pro Arg Gly Ser Arg Pro Ser Trp Gly Pro Thr Asp Pro
               100                 105                 110
   Arg His Arg Ser Arg Asn Val Gly Lys Val Ile Asp Thr Leu Thr Cys
           115                 120                 125
   Gly Phe Ala Asp Leu Met Gly Tyr Ile Pro Val Val Gly Ala Pro Leu
       130                 135                 140
   Gly Gly Val Ala Arg Ala Leu Ala His Gly Val Arg Val Leu Glu Asp
   145                 150                 155                 160
   Gly Val Asn Tyr Ala Thr Gly Asn Leu Pro Gly Cys Ser Phe Ser Ile
                   165                 170                 175
   Phe Leu Leu Ala Leu Leu Ser Cys Val Thr Val Pro Val Ser Ala Val
               180                 185                 190
   Gln Val Lys Asn Thr Ser Glu Thr Tyr Met Val Thr Asn Asp Cys Ser
           195                 200                 205
   Asn Asp Ser Ile Thr Trp Gln Leu Gln Ala Ala Val Leu His Val Pro
       210                 215                 220
   Gly Cys Val Pro Cys Glu Arg Thr Gly Asn Thr Ser Arg Cys Trp Ile
   225                 230                 235                 240
   Pro Val Ser Pro Asn Val Ala Val Gln Gln Pro Gly Ala Leu Thr Gln
                   245                 250                 255
   Gly Leu Arg Thr His Ile Asp Met Val Val Met Ser Ala Thr Leu Cys
               260                 265                 270
   Ser Ala Leu Tyr Val Gly Asp Leu Cys Gly Gly Val Val Leu Ala Ala
           275                 280                 285
   Gln Leu Phe Ile Val Ser Pro Arg Arg His Trp Phe Val Gln Glu Cys
       290                 295                 300
   Asn Cys Ser Ile Tyr Pro Gly Ala Ile Thr Gly His Arg Met Ala Trp
   305                 310                 315                 320
   Asp Met Met Met Asn Trp Ser Pro Thr Thr Thr Met Val Leu Ala Tyr
                   325                 330                 335
   Ala Met Arg Val Pro Glu Val Ile Ile Asp Ile Ile Ser Gly Ala His
               340                 345                 350
   Trp Gly Val Met Phe Gly Leu Ala Tyr Phe Ser Met Gln Gly Ala Trp
           355                 360                 365
   Ala Lys Val Ala Val Ile Leu Leu Leu Thr Ala Gly Val Glu Ala Arg
       370                 375                 380
   Thr His Thr Thr Gly Ser Val Ala Gly Arg Thr Thr Ser Gly Phe Ala
   385                 390                 395                 400
   Gly Ile Phe Thr Ser Gly Pro Lys Gln Asn Ile Gln Leu Ile Asn Thr
                   405                 410                 415
   Asn Gly Ser Trp His Ile Asn Arg Thr Ala Leu Asn Cys Asn Asp Ser
               420                 425                 430
   Leu Asn Thr Gly Phe Met Ala Ala Leu Phe Tyr Thr Lys Asn Phe Asn
           435                 440                 445
   Ser Ser Gly Cys Pro Glu Arg Leu Ser Xaa Cys Arg Asn Ile Glu Ala
       450                 455                 460
   Phe Arg Ile Gly Trp Gly Thr Leu Gln Tyr Glu Asp Asp Val Thr Asn
   465                 470                 475                 480
   Pro Glu Asp Met Arg Pro Tyr Cys Trp His Tyr Pro Pro Lys Gln Cys
                   485                 490                 495
   Gly Ile Phe Pro Ala Gly Ser Val Cys Gly Pro Val Tyr Cys Phe Thr
               500                 505                 510
   Pro Ser Pro Val Val Val Gly Thr Thr Asn Lys Leu Gly Val Pro Thr
           515                 520                 525
   Tyr Thr Trp Gly Glu Asn Glu Thr Asp Val Phe Ile Leu Asn Ser Thr
       530                 535                 540
   Arg Pro Pro Arg Gly Ser Trp Phe Gly Cys Thr Trp Met Asn Ser Thr
   545                 550                 555                 560
   Gly Phe Thr Lys Thr Cys Gly Ala Pro Pro Cys Arg Ile Arg Ala Asp
                   565                 570                 575
   Phe Asn Ala Ser Thr Asp Leu Leu Cys Pro Thr Asp Cys Phe Arg Lys
               580                 585                 590
   His Pro Glu Ala Thr Tyr Ile Lys Cys Gly Ser Gly Pro Trp Leu Thr
           595                 600                 605
   Pro Arg Cys Leu Val Asp Tyr Pro Tyr Arg Leu Trp His Tyr Pro Cys
       610                 615                 620
   Thr Val Asn Tyr Ser Ile Phe Lys Ile Arg Met Tyr Val Gly Gly Val
   625                 630                 635                 640
   Glu His Arg Leu Thr Ala Ala Cys Asn Phe Ser Arg Gly Asp Arg Cys
                   645                 650                 655
   Asn Leu Glu Asp Arg Asp Arg Ser Gln Leu Thr Pro Leu Leu His Ser
               660                 665                 670
   Thr Thr Glu Trp Ala Ile Leu Pro Cys Ser Tyr Ser Asp Leu Pro Ala
           675                 680                 685
   Leu Ser Thr Gly Leu Leu His Leu His Gln Asn Ile Val Asp Val Gln
       690                 695                 700
   Tyr Met Tyr Gly Leu Thr Pro Ala Leu Thr Lys Tyr Val Val Arg Trp
   705                 710                 715                 720
   Glu Trp Val Val Leu Leu Phe Leu Leu Leu Ala Asp Ala Arg Val Cys
                   725                 730                 735
   Ala Cys Leu Trp Met Leu Ile Leu Leu Gly Gln Ala Glu Ala
   740 745 750
<210> 46
   <211> 751
   <212> PRT
   <213> Hepatitis C virus
<400> 46
   Met Ser Thr Asn Pro Lys Pro Gln Arg Lys Thr Gln Arg Asn Thr Asn
   1               5                   10                  15
   Arg Arg Pro Gln Asp Val Lys Phe Pro Gly Gly Gly Gln Ile Val Gly
               20                  25                  30
   Gly Val Tyr Leu Leu Pro Arg Arg Gly Pro Arg Leu Gly Val Arg Thr
           35                  40                  45
   Ala Arg Lys Thr Ser Glu Arg Ser Gln Pro Arg Gly Arg Arg Gln Pro
       50                  55                  60
   Ile Pro Lys Asp Arg Arg Ser Thr Gly Lys Ser Trp Gly Arg Pro Gly
   65                  70                  75                  80
   Tyr Pro Trp Pro Leu Tyr Gly Asn Glu Gly Leu Gly Trp Ala Gly Trp
                   85                  90                  95
   Leu Leu Ser Pro Arg Gly Ser Arg Pro Ser Trp Gly Pro Thr Asp Pro
               100                 105                 110
   Arg His Arg Ser Arg Asn Val Gly Lys Val Ile Asp Thr Leu Thr Cys
           115                 120                 125
   Gly Phe Ala Asp Leu Met Gly Tyr Ile Pro Val Val Gly Ala Pro Leu
       130                 135                 140
   Gly Gly Val Ala Arg Ala Leu Ala His Gly Val Arg Val Leu Glu Asp
   145                 150                 155                 160
   Gly Val Asn Tyr Ala Thr Gly Asn Leu Pro Gly Cys Ser Phe Ser Ile
                   165                 170                 175
   Phe Leu Leu Ala Leu Leu Ser Cys Ile Thr Ile Pro Ala Ser Ala Val
               180                 185                 190
   Glu Val Lys Asn Thr Ser Thr Gly Tyr Met Val Thr Asn Asp Cys Ala
           195                 200                 205
   Asn Ser Ser Ile Thr Trp Gln Leu His Ala Ala Val Leu His Val Pro
   210 215 220
   Gly Cys Val Pro Cys Glu Arg Val Asp Asn Asn Thr Ser Arg Cys Trp
   225 230 235 240
   Ile Pro Val Ser Pro Asn Ile Ala Val Gln Arg Pro Gly Ala Leu Thr
   245 250 255
   Gln Gly Leu Arg Ser His Ile Asp Ile Val Val Met Ser Ala Thr Leu
   260 265 270
   Cys Ser Ala Leu Tyr Val Gly Asp Leu Cys Gly Gly Val Met Leu Ala
   275 280 285
   Ala Gln Met Phe Val Val Ser Pro Glu His His Trp Phe Val Gln Glu
   290 295 300
   Cys Asn Cys Ser Ile Tyr Pro Gly Thr Ile Thr Gly His Arg Met Ala
   305 310 315 320
   Trp Asp Met Met Met Asn Trp Ser Pro Thr Ala Thr Met Ile Leu Ala
   325 330 335
   Tyr Ala Met Arg Val Pro Glu Val Ile Ile Asp Ile Ile Gly Gly Ala
   340 345 350
   His Trp Gly Val Met Phe Gly Leu Ala Tyr Phe Ser Met Gln Gly Ala
   355 360 365
   Trp Ala Lys Val Val Val Ile Leu Leu Leu Ala Ala Gly Val Asp Ala
   370 375 380
   Tyr Thr His Thr Val Gly Gly Ala Ala Ala Ser Thr Ala Asn Ser Ile
   385 390 395 400
   Ala Gly Leu Leu Ser Arg Gly Pro Arg Gln Asn Leu Gln Leu Ile Asn
   405 410 415
   Ser Asn Gly Ser Trp His Ile Asn Arg Thr Ala Leu Asn Cys His Asp
   420 425 430
   Ser Leu Gln Thr Gly Phe Ile Thr Ala Leu Phe Tyr Ala Arg His Phe
   435 440 445
   Asn Ser Ser Gly Cys Pro Glu Arg Leu Ala Ala Cys Arg Asn Ile Glu
   450 455 460
   Ala Phe Arg Val Gly Trp Gly Ala Leu Gln Tyr Glu Asp Asn Val Thr
   465 470 475 480
   Asn Pro Glu Asp Met Arg Pro Tyr Cys Trp His Tyr Pro Pro Lys Gln
   485 490 495
   Cys Gly Ile Val Pro Ala Arg Ser Val Cys Gly Pro Val Tyr Cys Phe
   500 505 510
   Thr Pro Ser Pro Val Val Val Gly Thr Thr Asp Lys Leu Gly Val Pro
   515 520 525
   Thr Tyr Thr Trp Gly Glu Asn Glu Thr Asp Val Phe Leu Leu Asn Ser
   530 535 540
   Thr Arg Pro Pro Gln Gly Pro Trp Phe Gly Cys Thr Trp Met Asn Ser
   545 550 555 560
   Thr Gly Phe Thr Lys Thr Cys Gly Ala Pro Pro Cys Arg Thr Arg Ala
   565 570 575
   Asp Phe Asn Ala Ser Thr Asp Leu Leu Cys Pro Thr Asp Cys Phe Arg
   580 585 590
   Lys His Pro Asp Ala Thr Tyr Asn Lys Cys Gly Ser Gly Pro Trp Leu
   595 600 605
   Thr Pro Arg Cys Leu Ile Asp Tyr Pro Tyr Arg Leu Trp His Tyr Pro
   610 615 620
   Cys Thr Val Asn Tyr Thr Thr Phe Lys Ile Arg Met Tyr Val Gly Gly
   625 630 635 640
   Val Glu His Arg Leu Met Ala Ala Cys Asn Phe Thr Arg Gly Asp Ser
   645 650 655
   Cys Asp Leu Ser Gln Arg Asp Arg Gly Gln Leu Ser Pro Leu Leu His
   660 665 670
   Ser Thr Thr Glu Trp Ala Ile Leu Pro Cys Ser Phe Ser Asp Leu Pro
   675 680 685
   Ala Leu Ser Thr Gly Leu Leu His Leu His Gln Asn Ile Val Asp Val
   690 695 700
   Gln Tyr Met Tyr Gly Leu Ser Pro Ala Leu Thr Lys Tyr Ile Val Arg
   705 710 715 720
   Trp Glu Trp Val Val Leu Leu Phe Leu Leu Leu Ala Asp Ala Arg Val
                   725                 730                 735
   Cys Ala Cys Ile Trp Met Leu Ile Leu Leu Gly Gln Ala Glu Ala
               740                 745                 750
<210> 47
   <211> 750
   <212> PRT
   <213> Hepatitis C virus
<400> 47
   Met Ser Thr Asn Pro Lys Pro Gln Arg Lys Thr Lys Arg Asn Thr Asn
   1 5 10 15
   Arg Arg Pro Gln Asp Val Lys Phe Pro Gly Gly Gly Gln Ile Val Gly
   20 25 30
   Gly Val Tyr Leu Leu Pro Arg Arg Gly Pro Arg Leu Gly Val Arg Ala
   35 40 45
   Thr Arg Lys Thr Ser Glu Arg Ser Gln Pro Arg Gly Arg Arg Gln Pro
   50 55 60
   Ile Pro Lys Asp Arg Arg Ser Thr Gly Lys Ser Trp Gly Lys Pro Gly
   65 70 75 80
   Tyr Pro Trp Pro Leu Tyr Gly Asn Glu Gly Leu Gly Trp Ala Gly Trp
   85 90 95
   Leu Leu Ser Pro Arg Gly Ser Arg Pro Ser Trp Gly Pro Asn Asp Pro
   100 105 110
   Arg His Arg Ser Arg Asn Val Gly Lys Val Ile Asp Thr Leu Thr Cys
   115 120 125
   Gly Phe Ala Asp Leu Met Gly Tyr Ile Pro Val Val Gly Ala Pro Leu
   130 135 140
   Gly Gly Val Ala Arg Ala Leu Ala His Gly Val Arg Val Leu Glu Asp
   145 150 155 160
   Gly Val Asn Phe Ala Thr Gly Asn Leu Pro Gly Cys Ser Phe Ser Ile
   165 170 175
   Phe Leu Leu Ala Leu Leu Ser Cys Ile Thr Thr Pro Val Ser Ala Ala
   180 185 190
   Glu Val Lys Asn Ile Ser Thr Gly Tyr Met Val Thr Asn Asp Cys Thr
           195                 200                 205
   Asn Asp Ser Ile Thr Trp Gln Leu Gln Ala Ala Val Leu His Val Pro
       210                 215                 220
   Gly Cys Val Pro Cys Glu Lys Val Gly Asn Thr Ser Arg Cys Trp Ile
   225                 230                 235                 240
   Pro Val Ser Pro Asn Val Ala Val Gln Gln Pro Gly Ala Leu Thr Gln
                   245                 250                 255
   Gly Leu Arg Thr His Ile Asp Met Val Val Met Ser Ala Thr Leu Cys
               260                 265                 270
   Ser Ala Leu Tyr Val Gly Asp Leu Cys Gly Gly Val Met Leu Ala Ala
           275                 280                 285
   Gln Met Phe Ile Val Ser Pro Gln His His Trp Phe Val Gln Asp Cys
       290                 295                 300
   Asn Cys Ser Ile Tyr Pro Gly Thr Ile Thr Gly His Arg Met Ala Trp
   305                 310                 315                 320
   Asp Met Met Met Asn Trp Ser Pro Thr Ala Thr Met Ile Leu Ala Tyr
                   325                 330                 335
   Ala Met Arg Val Pro Glu Val Ile Ile Asp Ile Ile Gly Gly Ala His
               340                 345                 350
   Trp Gly Val Met Phe Gly Leu Ala Tyr Phe Ser Met Gln Gly Ala Trp
           355                 360                 365
   Ala Lys Val Val Val Ile Leu Leu Leu Ala Ala Gly Val Asp Ala Gln
       370                 375                 380
   Thr His Thr Val Gly Gly Ser Thr Ala His Asn Ala Arg Thr Leu Thr
   385                 390                 395                 400
   Gly Met Phe Ser Leu Gly Ala Arg Gln Lys Ile Gln Leu Ile Asn Thr
                   405                 410                 415
   Asn Gly Ser Trp His Ile Asn Arg Thr Ala Leu Asn Cys Asn Asp Ser
               420                 425                 430
   Leu His Thr Gly Phe Leu Ala Ser Leu Phe Tyr Thr His Ser Phe Asn
           435                 440                 445
   Ser Ser Gly Cys Pro Glu Arg Met Ser Ala Cys Arg Ser Ile Glu Ala
   450 455 460
   Phe Arg Val Gly Trp Gly Ala Leu Gln Tyr Glu Asp Asn Val Thr Asn
   465 470 475 480
   Pro Glu Asp Met Arg Pro Tyr Cys Trp His Tyr Pro Pro Arg Gln Cys
   485 490 495
   Gly Val Val Ser Ala Ser Ser Val Cys Gly Pro Val Tyr Cys Phe Thr
   500 505 510
   Pro Ser Pro Val Val Val Gly Thr Thr Asp Arg Leu Gly Ala Pro Thr
   515 520 525
   Tyr Thr Trp Gly Glu Asn Glu Thr Asp Val Phe Leu Leu Asn Ser Thr
   530 535 540
   Arg Pro Pro Gln Gly Ser Trp Phe Gly Cys Thr Trp Met Asn Ser Thr
   545 550 555 560
   Gly Tyr Thr Lys Thr Cys Gly Ala Pro Pro Cys Arg Ile Arg Ala Asp
   565 570 575
   Phe Asn Ala Ser Met Asp Leu Leu Cys Pro Thr Asp Cys Phe Arg Lys
   580 585 590
   His Pro Asp Thr Thr Tyr Ile Lys Cys Gly Ser Gly Pro Trp Leu Thr
   595 600 605
   Pro Arg Cys Leu Ile Asp Tyr Pro Tyr Arg Leu Trp His Tyr Pro Cys
   610 615 620
   Thr Val Asn Tyr Thr Ile Phe Lys Ile Arg Met Tyr Val Gly Gly Val
   625 630 635 640
   Glu His Arg Leu Thr Ala Ala Cys Asn Phe Thr Arg Gly Asp Arg Cys
   645 650 655
   Asn Leu Glu Asp Arg Asp Arg Ser Gln Leu Ser Pro Leu Leu His Ser
   660 665 670
   Thr Thr Glu Trp Ala Ile Leu Pro Cys Thr Tyr Ser Asp Leu Pro Ala
   675 680 685
   Leu Ser Thr Gly Leu Leu His Leu His Gln Asn Ile Val Asp Val Gln
   690 695 700
   Phe Met Tyr Gly Leu Ser Pro Ala Leu Thr Lys Tyr Ile Val Arg Trp
   705 710 715 720
   Glu Trp Val Val Leu Leu Phe Leu Leu Leu Ala Asp Ala Arg Val Cys
   725 730 735
   Ala Cys Leu Trp Met Leu Ile Leu Leu Gly Gln Ala Glu Ala
   740 745 750
<210> 48
   <211> 750
   <212> PRT
   <213> Hepatitis C virus
<400> 48
   Met Ser Thr Asn Pro Lys Pro Gln Arg Lys Thr Lys Arg Asn Thr Asn
   1               5                   10                  15
   Arg Arg Pro Gln Asp Val Lys Phe Pro Gly Gly Gly Gln Ile Val Gly
               20                  25                  30
   Gly Val Tyr Leu Leu Pro Arg Arg Gly Pro Arg Leu Gly Val Arg Ala
           35                  40                  45
   Thr Arg Lys Thr Ser Glu Arg Ser Gln Pro Arg Gly Arg Arg Gln Pro
       50                  55                  60
   Ile Pro Lys Asp Arg Arg Ser Thr Gly Lys Ser Trp Gly Lys Pro Gly
   65                  70                  75                  80
   Tyr Pro Trp Pro Leu Tyr Gly Asn Glu Gly Leu Gly Trp Ala Gly Trp
                   85                  90                  95
   Leu Leu Ser Pro Arg Gly Ser Arg Pro Ser Trp Gly Pro Asn Asp Pro
               100                 105                 110
   Arg His Arg Ser Arg Asn Val Gly Lys Val Ile Asp Thr Leu Thr Cys
           115                 120                 125
   Gly Phe Ala Asp Leu Met Gly Tyr Ile Pro Val Val Gly Ala Pro Leu
       130                 135                 140
   Gly Gly Val Ala Arg Ala Leu Ala His Gly Val Arg Val Leu Glu Asp
   145                 150                 155                 160
   Gly Val Asn Phe Ala Thr Gly Asn Leu Pro Gly Cys Ser Phe Ser Ile
   165 170 175
   Phe Leu Leu Ala Leu Leu Ser Cys Ile Thr Thr Pro Val Ser Ala Ala
   180 185 190
   Glu Val Lys Asn Ile Ser Thr Gly Tyr Met Val Thr Asn Asp Cys Thr
   195 200 205
   Asn Asp Ser Ile Thr Trp Gln Leu Gln Ala Ala Val Leu His Val Pro
   210 215 220
   Gly Cys Val Pro Cys Glu Lys Val Gly Asn Ala Ser Gln Cys Trp Ile
   225 230 235 240
   Pro Val Ser Pro Asn Val Ala Val Gln Arg Pro Gly Ala Leu Thr Gln
   245 250 255
   Gly Leu Arg Thr His Ile Asp Met Val Val Met Ser Ala Thr Leu Cys
   260 265 270
   Ser Ala Leu Tyr Val Gly Asp Leu Cys Gly Gly Val Met Leu Ala Ala
   275 280 285
   Gln Met Phe Ile Val Ser Pro Gln His His Trp Phe Val Gln Asp Cys
   290 295 300
   Asn Cys Ser Ile Tyr Pro Gly Thr Ile Thr Gly His Arg Met Ala Trp
   305 310 315 320
   Asp Met Met Met Asn Trp Ser Pro Thr Ala Thr Met Ile Leu Ala Tyr
   325 330 335
   Ala Met Arg Val Pro Glu Val Ile Ile Asp Ile Ile Ser Gly Ala His
   340 345 350
   Trp Gly Val Met Phe Gly Leu Ala Tyr Phe Ser Met Gln Gly Ala Trp
   355 360 365
   Ala Lys Val Val Val Ile Leu Leu Leu Ala Ala Gly Val Asp Ala Arg
   370 375 380
   Thr His Thr Val Gly Gly Ser Ala Ala Gln Thr Thr Gly Arg Leu Thr
   385 390 395 400
   Ser Leu Phe Asp Met Gly Pro Arg Gln Lys Ile Gln Leu Val Asn Thr
   405 410 415
   Asn Gly Ser Trp His Ile Asn Arg Thr Ala Leu Asn Cys Asn Asp Ser
   420 425 430
   Leu His Thr Gly Phe Ile Ala Ser Leu Phe Tyr Thr His Ser Phe Asn
   435 440 445
   Ser Ser Gly Cys Pro Glu Arg Met Ser Ala Cys Arg Ser Ile Glu Ala
   450 455 460
   Phe Arg Val Gly Trp Gly Ala Leu Gln Tyr Glu Asp Asn Val Thr Asn
   465 470 475 480
   Pro Glu Asp Met Arg Pro Tyr Cys Trp His Tyr Pro Pro Arg Gln Cys
   485 490 495
   Gly Val Val Ser Ala Lys Thr Val Cys Gly Pro Val Tyr Cys Phe Thr
   500 505 510
   Pro Ser Pro Val Val Val Gly Thr Thr Asp Arg Leu Gly Ala Pro Thr
   515 520 525
   Tyr Thr Trp Gly Glu Asn Glu Thr Asp Val Phe Leu Leu Asn Ser Thr
   530 535 540
   Arg Pro Pro Leu Gly Ser Trp Phe Gly Cys Thr Trp Met Asn Ser Ser
   545 550 555 560
   Gly Tyr Thr Lys Thr Cys Gly Ala Pro Pro Cys Arg Thr Arg Ala Asp
   565 570 575
   Phe Asn Ala Ser Thr Asp Leu Leu Cys Pro Thr Asp Cys Phe Arg Lys
   580 585 590
   His Pro Asp Thr Thr Tyr Leu Lys Cys Gly Ser Gly Pro Trp Leu Thr
   595 600 605
   Pro Arg Cys Leu Ile Asp Tyr Pro Tyr Arg Leu Trp His Tyr Pro Cys
   610 615 620
   Thr Val Asn Tyr Thr Ile Phe Lys Ile Arg Met Tyr Val Gly Gly Val
   625 630 635 640
   Glu His Arg Leu Thr Ala Ala Cys Asn Phe Thr Arg Gly Asp Arg Cys
   645 650 655
   Asn Leu Glu Asp Arg Asp Arg Ser Gln Leu Ser Pro Leu Leu His Ser
   660 665 670
   Thr Thr Glu Trp Ala Ile Leu Pro Cys Ser Tyr Ser Asp Leu Pro Ala
           675                 680                 685
   Leu Ser Thr Gly Leu Leu His Leu His Gln Asn Ile Val Asp Val Gln
       690                 695                 700
   Phe Met Tyr Gly Leu Ser Pro Ala Leu Thr Lys Tyr Ile Val Arg Trp
   705                 710                 715                 720
   Glu Trp Val Ile Leu Leu Phe Leu Leu Leu Ala Asp Ala Arg Val Cys
                   725                 730                 735
   Ala Cys Leu Trp Met Leu Ile Leu Leu Gly Gln Ala Glu Ala
               740                 745                 750
<210> 49
   <211> 750
   <212> PRT
   <213> Hepatitis C virus
<400> 49
   Met Ser Thr Asn Pro Lys Pro Gln Arg Lys Thr Lys Arg Asn Thr Asn
   1               5                   10                  15
   Arg Arg Pro Gln Asp Val Lys Phe Pro Gly Gly Gly Gln Ile Val Gly
               20                  25                  30
   Gly Val Tyr Leu Leu Pro Arg Arg Gly Pro Arg Leu Gly Val Arg Ala
           35                  40                  45
   Thr Arg Lys Thr Ser Glu Arg Ser Gln Pro Arg Gly Arg Arg Gln Pro
       50                  55                  60
   Ile Pro Lys Asp Arg Arg Ser Thr Gly Lys Ser Trp Gly Lys Pro Gly
   65                  70                  75                  80
   Tyr Pro Trp Pro Leu Tyr Gly Asn Glu Gly Cys Gly Trp Ala Gly Trp
                   85                  90                  95
   Leu Leu Ser Pro Arg Gly Ser Arg Pro Thr Trp Gly Pro Thr Asp Pro
               100                 105                 110
   Arg His Arg Ser Arg Asn Leu Gly Arg Val Ile Asp Thr Ile Thr Cys
           115                 120                 125
   Gly Phe Ala Asp Leu Met Gly Tyr Ile Pro Val Val Gly Ala Pro Val
       130                 135                 140
   Gly Gly Val Ala Arg Ala Leu Ala His Gly Val Arg Val Leu Glu Asp
   145                 150                 155                 160
   Gly Ile Asn Tyr Ala Thr Gly Asn Leu Pro Gly Cys Ser Phe Ser Ile
                   165                 170                 175
   Phe Leu Leu Ala Leu Leu Ser Cys Val Thr Val Pro Val Ser Ala Val
               180                 185                 190
   Glu Val Arg Asn Ile Ser Ser Ser Tyr Tyr Ala Thr Asn Asp Cys Ser
           195                 200                 205
   Asn Asn Ser Ile Thr Trp Gln Leu Thr Asp Ala Val Leu His Leu Pro
       210                 215                 220
   Gly Cys Val Pro Cys Glu Asn Asp Asn Gly Thr Leu His Cys Trp Ile
   225                 230                 235                 240
   Gln Val Thr Pro Asn Val Ala Val Lys His Arg Gly Ala Leu Thr Arg
                   245                 250                 255
   Ser Leu Arg Thr His Val Asp Met Ile Val Met Ala Ala Thr Ala Cys
               260                 265                 270
   Ser Ala Leu Tyr Val Gly Asp Val Cys Gly Ala Val Met Ile Leu Ser
           275                 280                 285
   Gln Ala Phe Met Val Ser Pro Gln Arg His Asn Phe Thr Gln Glu Cys
       290                 295                 300
   Asn Cys Ser Ile Tyr Gln Gly His Ile Thr Gly His Arg Met Ala Trp
   305                 310                 315                 320
   Asp Met Met Leu Ser Trp Ser Pro Thr Leu Thr Met Ile Leu Ala Tyr
                   325                 330                 335
   Ala Ala Arg Val Pro Glu Leu Val Leu Glu Ile Ile Phe Gly Gly His
               340                 345                 350
   Trp Gly Val Val Phe Gly Leu Ala Tyr Phe Ser Met Gln Gly Ala Trp
           355                 360                 365
   Ala Lys Val Ile Ala Ile Leu Leu Leu Val Ala Gly Val Asp Ala Thr
       370                 375                 380
   Thr Tyr Ser Ser Gly Gln Glu Ala Gly Arg Thr Val Ala Gly Phe Ala
   385                 390                 395                 400
   Gly Leu Phe Thr Thr Gly Ala Lys Gln Asn Leu Tyr Leu Ile Asn Thr
                   405                 410                 415
   Asn Gly Ser Trp His Ile Asn Arg Thr Ala Leu Asn Cys Asn Asp Ser
               420                 425                 430
   Leu Gln Thr Gly Phe Leu Ala Ser Leu Phe Tyr Thr His Lys Phe Asn
           435                 440                 445
   Ser Ser Gly Cys Pro Glu Arg Leu Ser Ser Cys Arg Gly Leu Asp Asp
       450                 455                 460
   Phe Arg Ile Gly Trp Gly Thr Leu Glu Tyr Glu Thr Asn Val Thr Asn
   465                 470                 475                 480
   Asp Gly Asp Met Arg Pro Tyr Cys Trp His Tyr Pro Pro Arg Pro Cys
                   485                 490                 495
   Gly Ile Val Pro Ala Arg Thr Val Cys Gly Pro Val Tyr Cys Phe Thr
               500                 505                 510
   Pro Ser Pro Val Val Val Gly Thr Thr Asp Lys Gln Gly Val Pro Thr
           515                 520                 525
   Tyr Thr Trp Gly Glu Asn Glu Thr Asp Val Phe Leu Leu Asn Ser Thr
       530                 535                 540
   Arg Pro Pro Arg Gly Ala Trp Phe Gly Cys Thr Trp Met Asn Gly Thr
   545                 550                 555                 560
   Gly Phe Thr Lys Thr Cys Gly Ala Pro Pro Cys Arg Ile Arg Lys Asp
                   565                 570                 575
   Tyr Asn Ser Thr Ile Asp Leu Leu Cys Pro Thr Asp Cys Phe Arg Lys
               580                 585                 590
   His Pro Asp Ala Thr Tyr Leu Lys Cys Gly Ala Gly Pro Trp Leu Thr
           595                 600                 605
   Pro Arg Cys Leu Val Asp Tyr Pro Tyr Arg Leu Trp His Tyr Pro Cys
       610                 615                 620
   Thr Val Asn Phe Thr Ile Phe Lys Ala Arg Met Tyr Val Gly Gly Val
   625                 630                 635                 640
   Glu His Arg Phe Ser Ala Ala Cys Asn Phe Thr Arg Gly Asp Arg Cys
   645 650 655
   Arg Leu Glu Asp Arg Asp Arg Gly Gln Gln Ser Pro Leu Leu His Ser
   660 665 670
   Thr Thr Glu Trp Ala Val Leu Pro Cys Ser Phe Ser Asp Leu Pro Ala
   675 680 685
   Leu Ser Thr Gly Leu Leu His Leu His Gln Asn Ile Val Asp Val Gln
   690 695 700
   Tyr Leu Tyr Gly Leu Ser Pro Ala Leu Thr Arg Tyr Ile Val Lys Trp
   705 710 715 720
   Glu Trp Val Ile Leu Leu Phe Leu Leu Leu Ala Asp Ala Arg Ile Cys
   725 730 735
   Ala Cys Leu Trp Met Leu Ile Ile Leu Gly Gln Ala Glu Ala
   740 745 750
<210> 50
   <211> 750
   <212> PRT
   <213> Hepatitis C virus
<400> 50
   Met Ser Thr Asn Pro Lys Pro Gln Arg Lys Thr Lys Arg Asn Thr Asn
   1               5                   10                  15
   Arg Arg Pro Gln Asp Val Lys Phe Pro Gly Gly Gly Gln Ile Val Gly
               20                  25                  30
   Gly Val Tyr Leu Leu Pro Arg Arg Gly Pro Arg Leu Gly Val Arg Ala
           35                  40                  45
   Thr Arg Lys Thr Ser Glu Arg Ser Gln Pro Arg Gly Arg Arg Gln Pro
       50                  55                  60
   Ile Pro Lys Asp Arg Arg Ser Thr Gly Lys Ser Trp Gly Lys Pro Gly
   65                  70                  75                  80
   Tyr Pro Trp Pro Leu Tyr Gly Asn Glu Gly Cys Gly Trp Ala Gly Trp
                   85                  90                  95
   Leu Leu Ser Pro Arg Gly Ser Arg Pro Thr Trp Gly Pro Ser Asp Pro
               100                 105                 110
   Arg His Arg Ser Arg Asn Leu Gly Arg Val Ile Asp Thr Ile Thr Cys
   115 120 125
   Gly Phe Ala Asp Leu Met Gly Tyr Ile Pro Val Val Gly Ala Pro Val
   130 135 140
   Gly Gly Val Ala Arg Ala Leu Ala His Gly Val Arg Val Leu Glu Asp
   145 150 155 160
   Gly Ile Asn Tyr Ala Thr Arg Asn Leu Pro Gly Cys Ser Phe Ser Ile
   165 170 175
   Phe Leu Leu Ala Leu Leu Ser Cys Val Thr Val Pro Val Ser Ser Val
   180 185 190
   Glu Ile Arg Asn Ile Ser Thr Ser Tyr Tyr Ala Thr Asn Asp Cys Ser
   195 200 205
   Asn Asn Ser Ile Thr Trp Gln Leu Thr Asn Ala Val Leu His Leu Pro
   210 215 220
   Gly Cys Val Pro Cys Glu Asn Asp Asn Gly Thr Leu Arg Cys Trp Ile
   225 230 235 240
   Gln Val Thr Pro Asn Val Ala Val Lys His Arg Gly Ala Leu Thr His
   245 250 255
   Asn Leu Arg Ala His Val Asp Val Ile Val Met Ala Ala Thr Val Cys
   260 265 270
   Ser Ala Leu Tyr Val Gly Asp Val Cys Gly Ala Val Met Ile Val Ser
   275 280 285
   Gln Ala Leu Ile Val Ser Pro Glu Arg His Asn Phe Thr Gln Glu Cys
   290 295 300
   Asn Cys Ser Ile Tyr Gln Gly His Ile Thr Gly Gln Arg Met Ala Trp
   305 310 315 320
   Asp Met Met Leu Asn Trp Ser Pro Thr Leu Thr Met Ile Leu Ala Tyr
   325 330 335
   Ala Ala Arg Val Pro Glu Leu Val Leu Glu Ile Val Phe Gly Gly His
   340 345 350
   Trp Gly Val Val Phe Gly Leu Ala Tyr Phe Ser Met Gln Gly Ala Trp
   355 360 365
   Ala Lys Val Ile Ala Ile Leu Leu Leu Val Ala Gly Val Asp Ala Thr
   370 375 380
   Thr Tyr Ser Thr Gly Ala Thr Val Gly Arg Thr Val Gly Ser Phe Ala
   385 390 395 400
   Gly Leu Phe Lys Leu Gly Ala Gln Gln Asn Val Gln Leu Ile Asn Thr
   405 410 415
   Asn Gly Ser Trp His Ile Asn Arg Thr Ala Leu Asn Cys Asn Asp Ser
   420 425 430
   Leu His Thr Gly Phe Met Ala Ala Leu Phe Tyr Ala Asn Lys Phe Asn
   435 440 445
   Ser Ser Gly Cys Pro Glu Arg Leu Ser Ser Cys Arg Gly Leu Asp Asp
   450 455 460
   Phe Arg Ile Gly Trp Gly Thr Leu Glu Tyr Glu Thr Asn Val Thr Asn
   465 470 475 480
   Val Glu Asp Met Arg Pro Tyr Cys Trp His Tyr Pro Pro Lys Pro Cys
   485 490 495
   Gly Ile Val Pro Ala Gln Ser Val Cys Gly Pro Val Tyr Cys Phe Thr
   500 505 510
   Pro Ser Pro Val Val Val Gly Thr Thr Asp Arg Gln Gly Val Pro Thr
   515 520 525
   Tyr Asn Trp Gly Asp Asn Glu Thr Asp Val Phe Leu Leu Asn Ser Thr
   530 535 540
   Arg Pro Pro Arg Gly Ala Trp Phe Gly Cys Thr Trp Met Asn Gly Thr
   545 550 555 560
   Gly Phe Thr Lys Thr Cys Gly Ala Pro Pro Cys Arg Ile Arg Lys Asp
   565 570 575
   Phe Asn Ser Thr Leu Asp Leu Leu Cys Pro Thr Asp Cys Phe Arg Lys
   580 585 590
   His Pro Asp Ala Thr Tyr Val Lys Cys Gly Ala Gly Pro Trp Leu Thr
   595 600 605
   Pro Arg Cys Leu Ile Asp Tyr Pro Tyr Arg Leu Trp His Tyr Pro Cys
   610 615 620
   Thr Val Asn Phe Thr Ile Phe Lys Val Arg Met Tyr Val Gly Gly Val
   625 630 635 640
   Glu His Arg Phe Ser Ala Ala Cys Asn Phe Thr Arg Gly Asp Arg Cys
   645 650 655
   Arg Leu Glu Asp Arg Asp Arg Gly Gln Gln Ser Pro Leu Leu His Ser
   660 665 670
   Thr Thr Glu Trp Ala Val Leu Pro Cys Ser Phe Ser Asp Leu Pro Ala
   675 680 685
   Leu Ser Thr Gly Leu Leu His Leu His Gln Asn Ile Val Asp Val Gln
   690 695 700
   Tyr Leu Tyr Gly Leu Ser Pro Ala Val Thr Lys Tyr Ile Val Lys Trp
   705 710 715 720
   Glu Trp Val Val Leu Leu Phe Leu Leu Leu Ala Asp Ala Arg Ile Cys
   725 730 735
   Ala Cys Leu Trp Met Leu Ile Ile Leu Gly Gln Ala Glu Ala
   740 745 750
<210> 51
   <211> 750
   <212> PRT
   <213> Hepatitis C virus
<400> 51
   Met Ser Thr Asn Pro Lys Pro Gln Arg Lys Thr Lys Arg Asn Thr Asn
   1               5                   10                  15
   Arg Arg Pro Gln Asp Val Lys Phe Pro Gly Gly Gly Gln Ile Val Gly
               20                  25                  30
   Gly Val Tyr Leu Leu Pro Arg Arg Gly Pro Arg Leu Gly Val Arg Ala
           35                  40                  45
   Thr Arg Lys Thr Ser Glu Arg Ser Gln Pro Arg Gly Arg Arg Gln Pro
       50                  55                  60
   Ile Pro Lys Asp Arg Arg Ser Thr Gly Lys Ser Trp Gly Lys Pro Gly
   65                  70                  75                  80
   Tyr Pro Trp Pro Leu Tyr Gly Asn Glu Gly Cys Gly Trp Ala Gly Trp
                   85                  90                  95
   Leu Leu Ser Pro Arg Gly Ser Arg Pro His Trp Gly Pro Thr Asp Pro
               100                 105                 110
   Arg His Arg Ser Arg Asn Leu Gly Lys Val Ile Asp Thr Ile Thr Cys
           115                 120                 125
   Gly Phe Ala Asp Leu Met Gly Tyr Ile Pro Val Ile Gly Ala Pro Val
       130                 135                 140
   Gly Gly Val Ala Arg Ala Leu Ala His Gly Val Arg Val Leu Glu Asp
   145                 150                 155                 160
   Gly Val Asn Tyr Ala Thr Gly Asn Leu Pro Gly Cys Ser Phe Ser Ile
                   165                 170                 175
   Phe Leu Leu Ala Leu Leu Ser Cys Val Thr Val Pro Val Ser Ala Val
               180                 185                 190
   Glu Val Arg Asn Ile Ser Ser Ser Tyr Tyr Ala Thr Asn Asp Cys Ser
           195                 200                 205
   Asn Asn Ser Ile Thr Trp Gln Leu Glu Asn Ala Val Leu His Leu Pro
       210                 215                 220
   Gly Cys Val Pro Cys Glu Asn Asp Asn Gly Thr Leu Arg Cys Trp Thr
   225                 230                 235                 240
   Gln Val Thr Pro Asn Val Ala Val Lys His Arg Gly Ala Leu Thr Gln
                   245                 250                 255
   Asn Leu Arg Thr His Val Asp Val Ile Val Val Ala Ala Thr Val Cys
               260                 265                 270
   Ser Ala Leu Tyr Val Gly Asp Val Cys Gly Ala Val Met Ile Ala Ser
           275                 280                 285
   Gln Ala Leu Ile Val Ser Pro Ala Arg His Asn Phe Thr Gln Glu Cys
       290                 295                 300
   Asn Cys Ser Ile Tyr Gln Gly Arg Ile Thr Gly His His Met Ala Trp
   305                 310                 315                 320
   Asp Met Met Leu Asn Trp Ser Pro Thr Ile Thr Met Ile Leu Ala Tyr
                   325                 330                 335
   Ala Ala Arg Ile Pro Glu Leu Val Leu Glu Val Ile Phe Gly Gly His
               340                 345                 350
   Trp Gly Val Met Phe Gly Leu Ala Tyr Phe Ser Met Gln Gly Ala Trp
           355                 360                 365
   Ala Lys Val Ile Val Ile Leu Leu Leu Val Ala Gly Val Asp Ala Arg
       370                 375                 380
   His His Thr Thr Gly Leu Gln Ala Gly Lys Thr Leu Ala Arg Val Thr
   385                 390                 395                 400
   Ser Leu Phe Ser Ile Gly Ala Lys Gln Asn Ile Gln Leu Ile Asn Thr
                   405                 410                 415
   Asn Gly Ser Trp His Ile Asn Arg Thr Ala Leu Asn Cys Asn Asp Ser
               420                 425                 430
   Leu Gln Thr Gly Phe Ile Ala Ser Leu Phe Tyr Val Asn Asn Ile Asn
           435                 440                 445
   Ser Ser Gly Cys Pro Glu Arg Met Ser Ser Cys Arg Glu Leu Asp Asp
       450                 455                 460
   Phe Arg Ile Gly Trp Gly Thr Leu Glu Tyr Glu Thr Asn Val Thr Asn
   465                 470                 475                 480
   Asp Glu Asp Met Arg Pro Tyr Cys Trp His Tyr Pro Pro Lys Pro Cys
                   485                 490                 495
   Gly Ile Val Pro Ala Arg Thr Val Cys Gly Pro Val Tyr Cys Phe Thr
               500                 505                 510
   Pro Ser Pro Ile Val Val Gly Thr Thr Asp Lys Gln Gly Val Pro Thr
           515                 520                 525
   Tyr Ser Trp Gly Glu Asn Glu Thr Asp Val Phe Leu Leu Asn Ser Thr
       530                 535                 540
   Arg Pro Pro Arg Gly Ser Trp Phe Gly Cys Thr Trp Met Asn Gly Thr
   545                 550                 555                 560
   Gly Phe Thr Lys Thr Cys Gly Ala Pro Pro Cys Arg Ile Arg Arg Asp
                   565                 570                 575
   Tyr Asn Ser Thr Leu Asp Leu Leu Cys Pro Thr Asp Cys Phe Arg Lys
               580                 585                 590
   His Pro Asp Thr Thr Tyr Leu Lys Cys Gly Ser Gly Pro Trp Leu Thr
               595                 600                 605
   Pro Lys Cys Leu Val Glu Tyr Pro Tyr Arg Leu Trp His Tyr Pro Cys
       610                 615                 620
   Thr Val Asn Phe Thr Ile Phe Lys Val Arg Met Tyr Val Gly Ala Val
   625                 630                 635                 640
   Glu His Arg Phe Ser Ala Ala Cys Asn Phe Thr Arg Gly Asp Arg Cys
                   645                 650                 655
   Arg Leu Glu Asp Arg Asp Arg Gly Gln Gln Ser Pro Leu Leu His Ser
               660                 665                 670
   Thr Thr Glu Trp Ala Val Leu Pro Cys Ser Phe Ser Asp Leu Pro Ala
           675                 680                 685
   Leu Ser Thr Gly Leu Leu His Leu His Gln Asn Ile Val Asp Val Gln
       690                 695                 700
   Tyr Leu Tyr Gly Leu Ser Pro Ala Ile Thr Arg Tyr Ile Val Lys Trp
   705                 710                 715                 720
   Glu Trp Val Val Leu Leu Phe Leu Leu Leu Ala Asp Ala Arg Val Cys
                   725                 730                 735
   Ala Cys Leu Trp Met Leu Ile Ile Leu Gly Gln Ala Glu Ala
               740                 745                 750
<210> 52
   <211> 750
   <212> PRT
   <213> Hepatitis C virus
<400> 52
   Met Ser Thr Asn Pro Lys Pro Gln Arg Lys Thr Lys Arg Asn Thr Asn
   1               5                   10                  15
   Arg Arg Pro Gln Asp Val Lys Phe Pro Gly Gly Gly Gln Ile Val Gly
               20                  25                  30
   Gly Val Tyr Leu Leu Pro Arg Arg Gly Pro Arg Leu Gly Val Arg Ala
           35                  40                  45
   Thr Arg Lys Thr Ser Glu Arg Ser Gln Pro Arg Gly Arg Arg Gln Pro
       50                  55                  60
   Ile Pro Lys Asp Arg Arg Ser Thr Gly Lys Ser Trp Gly Lys Pro Gly
   65 70 75 80
   Tyr Pro Trp Pro Leu Tyr Gly Asn Glu Gly Cys Gly Trp Ala Gly Trp
   85 90 95
   Leu Leu Ser Pro Arg Gly Ser Arg Pro Thr Trp Gly Pro Thr Asp Pro
   100 105 110
   Arg His Arg Ser Arg Asn Leu Gly Arg Val Ile Asp Thr Ile Thr Cys
   115 120 125
   Gly Phe Ala Asp Leu Met Gly Tyr Ile Pro Val Val Gly Ala Pro Val
   130 135 140
   Gly Gly Val Ala Arg Ala Leu Ala His Gly Val Arg Val Leu Glu Asp
   145 150 155 160
   Gly Ile Asn Tyr Ala Thr Gly Asn Leu Pro Gly Cys Ser Phe Ser Ile
   165 170 175
   Phe Leu Leu Ala Leu Leu Ser Cys Cys Thr Val Pro Val Ser Ala Val
   180 185 190
   Glu Val Arg Asn Ile Ser Thr Ser Tyr Tyr Ala Thr Asn Asp Cys Ser
   195 200 205
   Asn Thr Ser Ile Thr Trp Gln Leu Thr Asn Ala Val Leu His Leu Pro
   210 215 220
   Gly Cys Val Pro Cys Glu Asn Asp Asn Gly Thr Leu Arg Cys Trp Ile
   225 230 235 240
   Gln Val Thr Pro Asn Val Ala Val Lys His Arg Gly Ala Leu Thr His
   245 250 255
   Asn Leu Arg Thr His Val Asp Val Ile Val Met Ala Ala Thr Val Cys
   260 265 270
   Ser Ala Leu Tyr Val Gly Asp Ile Cys Gly Ala Val Met Ile Val Ser
   275 280 285
   Gln Ala Phe Ile Ile Ser Pro Glu Arg His Asn Phe Thr Gln Glu Cys
   290 295 300
   Asn Cys Ser Met Tyr Gln Gly His Ile Thr Gly His Arg Met Ala Trp
   305 310 315 320
   Asp Met Met Leu Asn Trp Ser Pro Thr Leu Thr Met Ile Leu Ala Tyr
                   325                 330                 335
   Ala Ala Arg Val Pro Glu Leu Val Leu Glu Val Ile Phe Gly Gly His
               340                 345                 350
   Trp Gly Val Val Phe Gly Leu Ala Tyr Phe Ser Met Gln Gly Ala Trp
           355                 360                 365
   Ala Lys Val Ile Ala Ile Leu Leu Leu Val Ala Gly Val Asp Ala Asn
       370                 375                 380
   Thr Tyr Ser Ser Gly Val Thr Val Gly His Thr Thr Ser Thr Phe Ala
   385                 390                 395                 400
   Asn Ile Phe Ser Val Gly Pro Ser Gln Lys Ile Asn Leu Ile Asn Thr
                   405                 410                 415
   Asn Gly Ser Trp His Ile Asn Arg Thr Ala Leu Asn Cys Asn Asp Ser
               420                 425                 430
   Leu Gln Thr Gly Phe Leu Ala Ser Leu Phe Tyr Val Arg Asn Phe Asn
           435                 440                 445
   Ser Ser Gly Cys Arg Glu Arg Leu Ser Ser Cys Arg Arg Leu Asp Asp
       450                 455                 460
   Phe Arg Ile Gly Trp Gly Thr Leu Glu Tyr Glu Thr Asn Val Thr Asn
   465                 470                 475                 480
   Asp Glu Asp Met Arg Pro Tyr Cys Trp His Tyr Pro Pro Lys Pro Cys
                   485                 490                 495
   Gly Ile Val Ser Ala Arg Thr Val Cys Gly Pro Val Tyr Cys Phe Thr
               500                 505                 510
   Pro Ser Pro Val Val Val Gly Thr Thr Asp Arg Gln Gly Val Pro Thr
           515                 520                 525
   Tyr Ser Trp Gly Glu Asn Glu Thr Asp Val Phe Leu Leu Asn Ser Thr
       530                 535                 540
   Arg Pro Pro Arg Gly Ala Trp Phe Gly Cys Thr Trp Met Asn Gly Thr
   545                 550                 555                 560
   Gly Phe Thr Lys Thr Cys Gly Ala Pro Pro Cys Arg Ile Arg Arg Asp
                   565                 570                 575
   Tyr Asn Ser Thr Leu Asp Leu Leu Cys Pro Thr Asp Cys Phe Arg Lys
               580                 585                 590
   His Pro Asp Thr Thr Tyr Leu Lys Cys Gly Ala Gly Pro Trp Leu Thr
           595                 600                 605
   Pro Lys Cys Leu Val Asp Tyr Pro Tyr Arg Leu Trp His Tyr Pro Cys
       610                 615                 620
   Thr Val Asn Phe Thr Ile Phe Lys Val Arg Met Tyr Val Gly Gly Val
   625                 630                 635                 640
   Glu His Arg Leu Ser Ala Ala Cys Asn Phe Thr Arg Gly Asp Arg Cys
                   645                 650                 655
   Gly Leu Glu Asp Arg Asp Arg Gly Gln Gln Ser Pro Leu Leu His Ser
               660                 665                 670
   Thr Thr Glu Trp Ala Val Leu Pro Cys Ser Phe Ser Asp Leu Pro Ala
           675                 680                 685
   Leu Ser Thr Gly Leu Leu His Leu His Gln Asn Ile Val Asp Val Gln
       690                 695                 700
   Tyr Leu Tyr Gly Leu Ser Pro Ala Ile Thr Arg Tyr Ile Val Lys Trp
   705                 710                 715                 720
   Glu Trp Val Val Leu Leu Phe Leu Leu Leu Ala Asp Ala Arg Val Cys
                   725                 730                 735
   Ala Cys Leu Trp Met Leu Ile Ile Leu Gly Gln Ala Glu Ala
               740                 745                 750
<210> 53
   <211> 750
   <212> PRT
   <213> Hepatitis C virus
<220>
   <221> misc_feature
   <222> (203)..(203)
   <223> Xaa can be any naturally occurring amino acid
<220>
   <221> misc_feature
   <222> (295)..(295)
   <223> Xaa can be any naturally occurring amino acid
<220>
   <221> misc_feature
   <222> (343)..(343)
   <223> Xaa can be any naturally occurring amino acid
<220>
   <221> misc_feature
   <222> (401)..(401)
   <223> Xaa can be any naturally occurring amino acid
<220>
   <221> misc_feature
   <222> (426)..(426)
   <223> Xaa can be any naturally occurring amino acid
<220>
   <221> misc_feature
   <222> (626)..(626)
   <223> Xaa can be any naturally occurring amino acid
<400> 53
   Met Ser Thr Asp Pro Lys Pro Gln Arg Lys Thr Lys Arg Asn Thr Asn
   1               5                   10                  15
   Arg Arg Pro Gln Asp Val Lys Phe Pro Gly Gly Gly Gln Ile Val Gly
               20                  25                  30
   Gly Val Tyr Leu Leu Pro Arg Arg Gly Pro Arg Leu Gly Val Arg Ala
           35                  40                  45
   Ala Arg Lys Thr Ser Glu Arg Ser Gln Pro Arg Gly Arg Arg Gln Pro
       50                  55                  60
   Ile Pro Lys Asp Arg Arg Ser Pro Gly Lys Ser Trp Gly Lys Pro Gly
   65                  70                  75                  80
   Tyr Pro Trp Pro Leu Tyr Gly Asn Glu Gly Cys Gly Trp Ala Gly Trp
                   85                  90                  95
   Leu Leu Ser Pro Arg Gly Ser Arg Pro Thr Trp Gly Pro Thr Asp Pro
               100                 105                 110
   Arg His Arg Ser Arg Asn Leu Gly Lys Val Ile Asp Thr Ile Thr Cys
           115                 120                 125
   Gly Phe Ala Asp Leu Met Gly Tyr Ile Pro Val Val Gly Ala Pro Val
       130                 135                 140
   Gly Gly Val Ala Arg Ala Leu Ala His Gly Val Arg Val Leu Glu Asp
   145                 150                 155                 160
   Gly Ile Asn Tyr Ala Thr Gly Asn Leu Pro Gly Cys Ser Phe Ser Ile
                   165                 170                 175
   Phe Leu Leu Ala Leu Leu Ser Cys Leu Thr Val Pro Ala Ser Ala Val
             180                 185                 190
   Glu Val Arg Asn Ile Ser Ser Gly Tyr Tyr Xaa Thr Asn Asp Cys Ser
           195                 200                 205
   Asn Ser Ser Ile Thr Trp Gln Leu Thr Asn Ala Val Leu His Leu Pro
       210                 215                 220
   Gly Cys Val Pro Cys Glu Asn Asp Asn Gly Thr Leu Arg Cys Trp Ile
   225                 230                 235                 240
   Gln Val Thr Pro Asn Val Ala Val Lys Tyr Arg Gly Ala Leu Thr His
                   245                 250                 255
   Asn Leu Arg Thr His Val Asp Met Ile Val Met Ala Ala Thr Val Cys
               260                 265                 270
   Ser Ala Leu Tyr Val Gly Asp Val Cys Gly Ala Val Met Ile Val Ser
           275                 280                 285
   Gln Ala Phe Ile Met Ser Xaa Glu Arg His Asn Phe Thr Gln Glu Cys
       290                 295                 300
   Asn Cys Ser Ile Tyr Gln Gly His Ile Thr Gly His Arg Met Ala Trp
   305                 310                 315                 320
   Asp Met Met Leu Gly Trp Ser Pro Thr Leu Thr Met Ile Leu Ala Tyr
                   325                 330                 335
   Ala Ala Arg Val Pro Glu Xaa Val Leu Glu Val Val Phe Gly Gly His
               340                 345                 350
   Trp Gly Val Val Phe Gly Leu Ala Tyr Phe Ser Met Gln Gly Ala Trp
           355                 360                 365
   Ala Lys Val Ile Ala Ile Leu Leu Leu Val Ala Gly Val Asp Ala Gly
       370                 375                 380
   Thr Tyr Ser Ser Gly Ala Thr Ile Gly Gln Gly Thr Arg Gly Leu Val
   385                 390                 395                 400
   Xaa Leu Phe Ser Ala Gly Pro Ser Gln Lys Ile Ser Leu Ile Asn Thr
                   405                 410                 415
   Asn Gly Ser Trp His Ile Asn Arg Thr Xaa Leu Asn Cys Asn Asp Ser
               420                 425                 430
   Leu Gln Thr Gly Phe Ile Ala Ser Leu Phe Tyr Ala Lys Ser Phe Asn
           435                 440                 445
   Ser Ser Gly Cys Pro Glu Arg Leu Ser Ser Cys Arg Gly Leu Asp Asp
       450                 455                 460
   Phe Arg Ile Gly Trp Gly Thr Leu Glu Tyr Glu Asn Asn Val Thr Asn
   465                 470                 475                 480
   Asp Glu Asp Met Arg Pro Tyr Cys Trp His Tyr Pro Pro Lys Pro Cys
                   485                 490                 495
   Gly Ile Val Pro Ala Arg Thr Val Cys Gly Pro Val Tyr Cys Phe Thr
               500                 505                 510
   Pro Ser Pro Val Val Val Gly Thr Thr Asp Lys Gln Gly Val Pro Thr
           515                 520                 525
   Tyr Ser Trp Gly Glu Asn Glu Thr Asp Val Phe Leu Leu Asn Ser Thr
       530                 535                 540
   Arg Pro Pro Gln Gly Ala Trp Phe Gly Cys Thr Trp Met Asn Gly Thr
   545                 550                 555                 560
   Gly Phe Thr Lys Thr Cys Gly Ala Pro Pro Cys Arg Ile Arg Arg Asp
                   565                 570                 575
   His Thr Ser Thr Leu Asp Leu Leu Cys Pro Thr Asp Cys Phe Arg Lys
               580                 585                 590
   His Pro Asp Thr Thr Tyr Leu Lys Cys Gly Ala Gly Pro Trp Leu Thr
           595                 600                 605
   Pro Lys Cys Leu Val Asp Tyr Pro Tyr Arg Leu Trp His Tyr Pro Cys
       610                 615                 620
   Thr Xaa Asn Phe Thr Ile Phe Lys Val Arg Met Tyr Val Gly Gly Val
   625                 630                 635                 640
   Glu His Arg Phe Ser Ala Ala Cys Asn Phe Thr Arg Gly Asp Arg Cys
                   645                 650                 655
   Arg Leu Glu Asp Arg Asp Arg Gly Gln Gln Ser Pro Leu Leu His Ser
               660                 665                 670
   Thr Thr Glu Trp Ala Val Leu Pro Cys Ser Phe Ser Asp Leu Pro Ala
               675                 680                 685
   Leu Ser Thr Gly Leu Leu His Leu His Gln Asn Ile Val Asp Val Gln
       690                 695                 700
   Tyr Leu Tyr Gly Leu Ser Pro Ala Ile Thr Lys Tyr Ile Val Lys Trp
   705                 710                 715                 720
   Glu Trp Val Ile Leu Leu Phe Leu Leu Leu Ala Asp Ala Arg Val Cys
                   725                 730                 735
   Ala Cys Leu Trp Met Leu Ile Ile Leu Gly Gln Ala Glu Ala
               740                 745                 750